# EUROPEAN PATENT SPECIFICATION

(11) **EP 3 608 425 B1**
(45) Date of publication and mention of the grant of the patent: **13.04.2022**
(21) Application number: 19194614.4
(22) Date of filing: 09.07.2015
(51) Int. Cl.: C12Q 1/6883, C12Q 1/70

(54) **HCBI, MSBI, MSSI AND CMI SEQUENCES AS AN EARLY MARKER FOR THE FUTURE DEVELOPMENT OF CANCER AND DISEASES OF THE CNS AND AS A TARGET FOR THE TREATMENT AND PREVENTION OF THESE DISEASES**
HCBIO-, MSBI-, MSSI- UND CMI-SEQUENZEN ALS FRÜHE MARKER FÜR DIE ZUKÜNFTIGE ENTWICKLUNG VON KREBS UND ERKRANKUNGEN DES ZNS UND ALS ZIEL ZUR BEHANDLUNG UND PRÄVENTION DIESER ERKRANKUNGEN
SÉQUENCES HCBI, MSBI, MSSI ET CMI COMME MARQUEUR PRÉCOCE POUR LE DÉVELOPPEMENT FUTUR DU CANCER ET DE MALADIES DU SNC ET COMME CIBLE POUR LE TRAITEMENT ET LA PRÉVENTION DE CES MALADIES

(30) Priority: 10.07.2014 EP 14176624
(43) Date of publication of application: 12.02.2020
(62) Divisional of application: 15745146.9
(73) Proprietor: Deutsches Krebsforschungszentrum, 69120 Heidelberg (DE)
(72) Inventor: de Villiers-zur Hausen, Ethel-Michele, 69483 Wald-Michelbach (DE); zur Hausen, Harald, 69483 Wald-Michelbach (DE); Gunst, Karin, 69493 Hirschberg (DE); Whitley, Corinna, 68519 Vierheim (DE); Pérez, Iranzu Lamberto, 31006 Pamplona (ES)
(74) Representative: Schüssler, Andrea

(56) References cited:
- LAURA MANUELIDIS: "Nuclease resistant circular DNAs copurify with infectivity in scrapie and CJD", JOURNAL OF NEUROVIROLOGY, vol. 17, no. 2, 7 December 2010 (2010-12-07), pages 131-145, XP055158494, ISSN: 1355-0284, DOI: 10.1007/s13365-010-0007-0 & DATABASE EMBL [Online] 10 December 2010 (2010-12-10), "TSE-associated circular DNA isolate Sphinx 1.76, complete sequence.", retrieved from EBI accession no. EM_STD:HQ444404 Database accession no. HQ444404
- EMMANUEL DIAS NETO ET AL: "SHOTGUN SEQUENCING OF THE HUMAN TRANSCRIPTOME WITH ORF EXPRESSED SEQUENCE TAGS", PROCEEDINGS OF THE NATIONAL ACADEMY OF SCIENCES, NATIONAL ACADEMY OF SCIENCES, US, vol. 97, no. 7, 28 March 2000 (2000-03-28) , pages 3491-3496, XP000996193, ISSN: 0027-8424, DOI: 10.1073/PNAS.97.7.3491 & DATABASE EMBL [Online] 17 December 2004 (2004-12-17), "QV0-CT0181-041199-048-g08 CT0181 Homo sapiens cDNA, mRNA sequence.", retrieved from EBI accession no. EM_EST:CK327030 Database accession no. CK327030 & DATABASE EMBL [Online] 20 June 2000 (2000-06-20), "CM2-BT0368-171299-056-b03 BT0368 Homo sapiens cDNA, mRNA sequence.", retrieved from EBI accession no. EM_EST:BE068140 Database accession no. BE068140
- B. PALENIK ET AL: "Coastal Synechococcus metagenome reveals major roles for horizontal gene transfer and plasmids in population diversity", ENVIRONMENTAL MICROBIOLOGY, vol. 11, no. 2, 1 February 2009 (2009-02-01), pages 349-359, XP055158637, ISSN: 1462-2912, DOI: 10.1111/j.1462-2920.2008.01772.x & DATABASE EMBL [Online] 28 September 2011 (2011-09-28), "Uncultured bacterium plasmid clone S6GIO-17 genomic sequence.", retrieved from EBI accession no. EM_STD:JN541288 Database accession no. JN541288

### SEQUENCE LISTING

<110> Deutsches Krebsforschungszentrum
<120> HCBI, MSBI, MSSI and CMI sequences as an early marker for the future development of cancer and diseases of the CNS and as a target for the treatment and prevention of these diseases
<130> K 3530EP
<150> EPEP14176624.6
   <151> 2014-07-10
<160> 248
<170> BiSSAP 1.0
<210> 1
   <211> 2544
   <212> DNA
   <213> Artificial Sequence
<220>
   <221> source
   <222> 1..2544
   <223> /mol_type="DNA" /note="HCBI6.252" /organism="Artificial Sequence"
<400> 1
   ttttgttttt caaaggaagt aattagcgag tgtctacgag cgacacaatg aaagttcgat      60
   tattccctcc tctaaaaaac cgcttttaaa aatattggtt gctagatggt tttaactatc     120
   gtgagctttt gcttttaaaa atacacgagc aaagcgagtt catagtcgct tttgatctgc     180
   tcttactaac agctcatttt taatgggccg ttagtaagag atgcttttgg ggtttcgggg     240
   cttgccctga acaaggtcac aaagtgggaa tttatcacgg tagtgaaaaa gtaccctttg     300
   tgtatccttg ctttatgctt tttaaacctt tgaacttttc ccttaatttg cagaaattgc     360
   ccctcgacta agcttgctta gtcaaaaaag tttgagcaaa gcgaaaacat agggcaattt     420
   tcatgatgaa aatgggcttt taaagttttt aaatgctttt aaatgctttt agacatgttg     480
   aaatgcaagc ctagcaaggc atacagagga cataccccta cgtttaccga tcaatacccc    540
   tacgtttacc gatcaatacc cctacgttta ccgatcaata cccctacgtt taccttgcat     600
   atcactacaa acaatattaa tgtagtaata acttcaaaat aataaatgta ggttatgagc     660
   gatttaatag taaaagataa cgccctaatg aacgctagtt acaacttagc tttggttgaa     720
   cagcgtttaa tccttttagc tatattggaa gcgagagaaa caggcaaagg gattaatgcc     780
   aatgatccct taacagttca tgcaagtagc tatatcaatc aatttaacgt ggaaagacat     840
   acggcatatc aagcattaaa agatgcttgc aaagacttgt ttgcacgtca atttagttac     900
   caagaaaagc gagaacgtgg acgaattaat attacaagtc gatgggtttc acaaattggt     960
   tatatggacg atacagcaac cgttgaaatt atttttgccc ctgcggttgt tcctttgatt    1020
   acacgtttag aggaacaatt cactcaatac gatattgaac aaattagcga gttatcgagt 1080
   gcttatgctg tccgcctata tgagttacta atttgttggc gtagcacagg aaaaacccca 1140
   attattgatc taacagagtt tagaaagcgt ctaggtgttt tagataccga atacactaga 1200
   acagataact tgaagatgcg agtgattgag ctaggactta aacaaatcaa cgagcatacg 1260
   gacattacag caagctacga acagcacaaa aaagggcgga caatcacagg attctcattc 1320
   aagtttaagc agaagaagaa aacaggggcg gaaatgccaa aaaatagcga ttctagccca 1380
   catatcgaaa aacctagtca aatccctgcc aacatcgcaa aacagcctga aaatgccaaa 1440
   aaagatgatt tagggcacag agcaagcaag attacagggt taattatgtc tgaatggttt 1500
   agcagatcgg tttaaacgtg gtgatgagtc agtaatagac atgatgaaac gcatcaaaga 1560
   ggaaattaca actgatacaa cagcagacca atgggaaaac aagctagaag aatttggcgt 1620
   gatttttcag tcatgatcaa tttcccgaag gacatataaa aaataatgta gttaaatata 1680
   gtagttataa tattgactac aatatttaac tactatattg tagtcttaaa tcaacttaca 1740
   tagaatttaa attatgtctc ttcgccaaat tttagaacaa tctctctttg atcaaactga 1800
   tgaaagctct actgtgccac tatcaattag acttccaaac aacttgaata atgagcttga 1860
   agaactgact ataacactag ataaatccaa gagttttcta ctcctagagt tcattaaggc 1920
   tggaattagt gagactagca atatgttaga agacaaagcg aagaacccta tacttgaaag 1980
   tccaagagat tctagtgact ttacaactca taagaagttt atgttgaaca ctaatttcaa 2040
   taacaataaa gcatcacaca tgactatgtt ggagaatcaa gaagctgctg catttcgtaa 2100
   aggttggaaa gaatatatct gtaatttaga agaaggcgat aaggtttacc tttatcaaag 2160
   tggcgttggt tttgtagcat ctggtattgt cactggtgac ttaatcaaaa gtgagtatcg 2220
   tggacaacca gaagaaaaat attctaaaaa ataaaggatt ttaaaatagg atttaaagca 2280
   atttctgcaa aagagtttaa gaccttaact ggcggtggtg ctagctttcg tacaacaatg 2340
   agcgagctaa agcctgaaca atgtagaaaa ttagatatgg aaatagaaaa acggatgaag 2400
   tcagtaagtt aaaaatatga gttgttgttt tagtgtgtca cgtagcaatc acttagaagt 2460
   tccctttgta ttacttgtct agcaatggtt ttaaaaagcg tcactactga tagtgacgct 2520
   ttatacccct acgtttaccg atca 2544
<210> 2
   <211> 1331
   <212> DNA
   <213> Artificial Sequence
<220>
   <221> source
   <222> 1..1331
   <223> /mol_type="DNA"
   /note="Sphinx 1.76"
   /organism="Artificial Sequence"
<400> 2
   ttttaaacct ttgaactttt cccttaattt gcagaaattg cccctcgact aagcttgctt      60
   agtcaaaaaa gtttgagcaa agcgaaaaca tagggcaatt ttcatgatga aaatgggctt     120
   ttaaagtttt taaatgcttt taaatgcttt tagacatgtt gaaatgcaag cctagcaagg     180
   catacagagg acatacccct acgtttaccg atcaataccc ctacgtttac cgatcaatac     240
   ccctacgttt accgatcaat acccctacgt ttaccttgca tatcactaca aacaatatta     300
   atgtagtaat aacttcaaaa taataaatgt aggttatgag cgatttaata gtaaaagata     360
   acgccctaat gaacgctagt tacaacttag ctttggttga acagcgttta atccttttag     420
   ctatattgga agcgagagaa acaggcaaag ggattaatgc caatgatccc ttaacagttc     480
   atgcaagtag ctatatcaat caatttaacg tggaaagaca tacggcatat caagcattaa     540
   aagatgcttg caaagacttg tttgcacgtc aatttagtta ccaagaaaag cgagaacgtg     600
   gacgaattaa tattacaagt cgatgggttt cacaaattgg ttatatggac gatacagcaa     660
   ccgttgaaat tatttttgcc cctgcggttg ttcctttgat tacacgttta gaggaacaat     720
   tcactcaata cgatattgaa caaattagcg agttatcgag tgcttatgct gtccgcctat     780
   atgagttact aatttgttgg cgtagcacag gaaaaacccc aattattgat ctaacagagt     840
   ttagaaagcg tctaggtgtt ttagataccg aatacactag aacagataac ttgaagatgc     900
   gagtgattga gctaggactt aaacaaatca acgagcatac ggacattaca gcaagctacg     960
   aacagcacaa aaaagggcgg acaatcacag gattctcatt caagtttaag cagaagaaga    1020
   aaacaggggc ggaaatgcca aaaaatagcg attctagccc acatatcgaa aaacctagtc    1080
   aaatccctgc caacatcgca aaacagcctg aaaatgccaa aaaagatgat ttagggcaca    1140
   gagcaagcaa gattacaggg ttaattatgt cgaatggttt agcagatcgg tttaaacgtg    1200
   gtgatgagtc agtaatagac atgatgaaac gcatcaaaga ggaaattaca actgatacaa    1260
   cagcagacca atgggaaaac aagctagaag aatttggcgt gatttttcag tcatgatcaa    1320
   tttcccgaag g                                                         1331
<210> 3
   <211> 1324
   <212> DNA
   <213> Artificial Sequence
<220>
   <221> source
   <222> 1..1324
   <223> /mol_type="DNA"
   /note="subject"
   /organism="Artificial Sequence"
<400> 3
   ttctaaacct ttgacttctt ccccattgtt tgcagaaatt gcccctcgaa gcttgcttag 60
   tcaaaaaagt ttgagcaaag cgaaaacata gggcaatttt catgaagaaa ttgggctttt 120
   aaagttttta aatgttttaa atgcttttag acatgctaag aagcccacac agcaaggcat 180
   acagaggaca ttctcctacg tttaccgatc aatacccccg tttaccgatc aataccccta 240
   cgtttaccga tcaatacccc tacgtttacc ttgcgtataa ctacaaagaa tactagtgta 300
   gtataacttc aaaagaataa ttgtaggtta tgagcgattt aatagtaaaa gataacgccc 360
   taatgaatgc tagttataac ttagctttgg ttgaacagag gttaattcta ttagcaatca 420
   tagaagcgag agaaacaggc aaagggatta atgccaatga tcctcttacg gttcatgcag 480
   gtagctatat caatcaattt aacgtacaaa ggcatacggc atatcaagcc ctcaaagatg 540
   cttgtaaaga cttgtttgcc cgtcaattca gttaccaaga aaagcgagaa cgaggacgaa 600
   ttaatattac aagtcgatgg gtttcgcaaa ttggctatat ggacgataca gcaaccgttg 660
   agattatttt tgcccctgcg gttgttcctc tgattacacg gctagaggaa cagttcaccc 720
   agtacgatat tgagcaaatt agcggtttat cgagtgcata tgctgttcgt atgtacgaac 780
   tgctgatttg ttggcgtagc acaggcaaaa caccaattat tgagctagac gagtttagaa 840
   agcgaatagg tgttttagat actgaataca ctagaacaga taatttaaag atgcaagtta 900
   ttgaattagc cctaaaacaa atcaacgaac atactgacat cacagcaagc tatgaacaac 960
   acaaaaaagg gcgagtgatt accggattct cattcatgtt taagcacaag aaacaaaaca 1020
   gcgataaaac gcctgatact aacgcttcta gcccacgtat cgtaaaacat agtcaaatcc 1080
   ctaccaacat tgtaaaacag cctgaaaacg ccaaaatgag cgatttagaa catagagcga 1140
   gccgtgttac aggggaaata atgcgaaatc gtctgtcaga tcggtttaaa caaggcgatg 1200
   aatcagcaat cgacatgatg aaacgtattc aaagtgaaat aataaccgat gcaatagcag 1260
   accagtggga aagcaaactg gaggagtttg gcgtggtttt ttagtcatga cgatttcccg 1320
   aagg 1324
<210> 4
   <211> 153
   <212> DNA
   <213> Artificial Sequence
<220>
   <221> source
   <222> 1..153
   <223> /mol_type="DNA"
   /note="query"
   /organism="Artificial Sequence"
<400> 4
   attagcgagt gtctacgagc gacacaatga aagttcgatt attccctcct ctaaaaaacc 60
   gcttttaaaa atattggttg ctagatggtt ttaactatcg tgagcttttg cttttaaaaa 120
   tacacgagca aagcgagttc atagtcgctt ttg 153
<210> 5
   <211> 152
   <212> DNA
   <213> Artificial Sequence
<220>
   <221> source
   <222> 1..152
   <223> /mol_type="DNA"
   /note="subject"
   /organism="Artificial Sequence"
<400> 5
   attagcgagt gtctacgagc gactcaatga aagttcgatt attccccctc tggaaaaccg 60
   cttttaaaaa tattggctgc tagatggttt ttactatagt gaggttttgc ttttaaaaaa 120
   acacgagcaa agcgagttca tagttgcttt tg 152
<210> 6
   <211> 324
   <212> PRT
   <213> Artificial Sequence
<220>
   <221> SOURCE
   <222> 1..324
   <223> /mol_type="protein"
   /note="query"
   /organism="Artificial Sequence"
<400> 6
   Met Ser Asp Leu Ile Val Lys Asp Asn Ala Leu Met Asn Ala Ser Tyr
   1               5                   10                  15
   Asn Leu Ala Leu Val Glu Gln Arg Leu Ile Leu Leu Ala Ile Leu Glu
               20                  25                  30
   Ala Arg Glu Thr Gly Lys Gly Ile Asn Ala Asn Asp Pro Leu Thr Val
           35                  40                  45
   His Ala Ser Ser Tyr Ile Asn Gln Phe Asn Val Glu Arg His Thr Ala
       50                  55                  60
   Tyr Gln Ala Leu Lys Asp Ala Cys Lys Asp Leu Phe Ala Arg Gln Phe
   65                  70                  75                  80
   Ser Tyr Gln Glu Lys Arg Glu Arg Gly Arg Ile Asn Ile Thr Ser Arg
                   85                  90                  95
   Trp Val Ser Gln Ile Gly Tyr Met Asp Asp Thr Ala Thr Val Glu Ile
               100                 105                 110
   Ile Phe Ala Pro Ala Val Val Pro Leu Ile Thr Arg Leu Glu Glu Gln
           115                 120                 125
   Phe Thr Gln Tyr Asp Ile Glu Gln Ile Ser Glu Leu Ser Ser Ala Tyr
       130                 135                 140
   Ala Val Arg Leu Tyr Glu Leu Leu Ile Cys Trp Arg Ser Thr Gly Lys
   145                 150                 155                 160
   Thr Pro Ile Ile Asp Leu Thr Glu Phe Arg Lys Arg Leu Gly Val Leu
                   165                 170                 175
   Asp Thr Glu Tyr Thr Arg Thr Asp Asn Leu Lys Met Arg Val Ile Glu
               180                 185                 190
   Leu Gly Leu Lys Gln Ile Asn Glu His Thr Asp Ile Thr Ala Ser Tyr
           195                 200                 205
   Glu Gln His Lys Lys Gly Arg Thr Ile Thr Gly Phe Ser Phe Lys Phe
       210                 215                 220
   Lys Gln Lys Lys Lys Thr Gly Ala Glu Met Pro Lys Asn Ser Asp Ser
   225                 230                 235                 240
   Ser Pro His Ile Glu Lys Pro Ser Gln Ile Pro Ala Asn Ile Ala Lys
                   245                 250                 255
   Gln Pro Glu Asn Ala Lys Lys Asp Asp Leu Gly His Arg Ala Ser Lys
               260                 265                 270
   Ile Thr Gly Leu Ile Met Ser Asn Gly Leu Ala Asp Arg Phe Lys Arg
           275                 280                 285
   Gly Asp Glu Ser Val Ile Asp Met Met Lys Arg Ile Lys Glu Glu Ile
       290                 295                 300
   Thr Thr Asp Thr Thr Ala Asp Gln Trp Glu Asn Lys Leu Glu Glu Phe
   305                 310                 315                 320
   Gly Val Ile Phe
<210> 7
   <211> 324
   <212> PRT
   <213> Artificial Sequence
<220>
   <221> SOURCE
   <222> 1..324
   <223> /mol_type="protein"
   /note="subject"
   /organism="Artificial Sequence"
<400> 7
   Met Ser Asp Leu Ile Val Lys Asp Asn Ala Leu Met Asn Ala Ser Tyr
   1               5                   10                  15
   Asn Leu Ala Leu Val Glu Gln Arg Leu Ile Leu Leu Ala Ile Ile Glu
               20                  25                  30
   Ala Arg Glu Thr Gly Lys Gly Ile Asn Ala Asn Asp Pro Leu Thr Val
           35                  40                  45
   His Ala Gly Ser Tyr Ile Asn Gln Phe Asn Val Gln Arg His Thr Ala
       50                  55                  60
   Tyr Gln Ala Leu Lys Asp Ala Cys Lys Asp Leu Phe Ala Arg Gln Phe
   65                  70                  75                  80
   Ser Tyr Gln Glu Lys Arg Glu Arg Gly Arg Ile Asn Ile Thr Ser Arg
                   85                  90                  95
   Trp Val Ser Gln Ile Gly Tyr Met Asp Asp Thr Ala Thr Val Glu Ile
               100                 105                 110
   Ile Phe Ala Pro Ala Val Val Pro Leu Ile Thr Arg Leu Glu Glu Gln
           115                 120                 125
   Phe Thr Gln Tyr Asp Ile Glu Gln Ile Ser Gly Leu Ser Ser Ala Tyr
       130                 135                 140
   Ala Val Arg Met Tyr Glu Leu Leu Ile Cys Trp Arg Ser Thr Gly Lys
   145                 150                 155                 160
   Thr Pro Ile Ile Glu Leu Asp Glu Phe Arg Lys Arg Ile Gly Val Leu
                   165                 170                 175
   Asp Thr Glu Tyr Thr Arg Thr Asp Asn Leu Lys Met Gln Val Ile Glu
               180                 185                 190
   Leu Ala Leu Lys Gln Ile Asn Glu His Thr Asp Ile Thr Ala Ser Tyr
           195                 200                 205
   Glu Gln His Lys Lys Gly Arg Val Ile Thr Gly Phe Ser Phe Met Phe
       210                 215                 220
   Lys His Lys Lys Gln Asn Ser Asp Lys Thr Pro Asp Thr Asn Ala Ser
   225                 230                 235                 240
   Ser Pro Arg Ile Val Lys His Ser Gln Ile Pro Thr Asn Ile Val Lys
                   245                 250                 255
   Gln Pro Glu Asn Ala Lys Met Ser Asp Leu Glu His Arg Ala Ser Arg
               260                 265                 270
   Val Thr Gly Glu Ile Met Arg Asn Arg Leu Ser Asp Arg Phe Lys Gln
           275                 280                 285
   Gly Asp Glu Ser Ala Ile Asp Met Met Lys Arg Ile Gln Ser Glu Ile
       290                 295                 300
   Ile Thr Asp Ala Ile Ala Asp Gln Trp Glu Ser Lys Leu Glu Glu Phe
   305                 310                 315                 320
   Gly Val Val Phe
<210> 8
   <211> 324
   <212> PRT
   <213> Artificial Sequence
<220>
   <221> SOURCE
   <222> 1..324
   <223> /mol_type="protein"
   /note="query"
   /organism="Artificial Sequence"
<400> 8
   Met Ser Asp Leu Ile Val Lys Asp Asn Ala Leu Met Asn Ala Ser Tyr
   1               5                   10                  15
   Asn Leu Ala Leu Val Glu Gln Arg Leu Ile Leu Leu Ala Ile Leu Glu
               20                  25                  30
   Ala Arg Glu Thr Gly Lys Gly Ile Asn Ala Asn Asp Pro Leu Thr Val
           35                  40                  45
   His Ala Ser Ser Tyr Ile Asn Gln Phe Asn Val Glu Arg His Thr Ala
       50                  55                  60
   Tyr Gln Ala Leu Lys Asp Ala Cys Lys Asp Leu Phe Ala Arg Gln Phe
   65                  70                  75                  80
   Ser Tyr Gln Glu Lys Arg Glu Arg Gly Arg Ile Asn Ile Thr Ser Arg
                   85                  90                  95
   Trp Val Ser Gln Ile Gly Tyr Met Asp Asp Thr Ala Thr Val Glu Ile
               100                 105                 110
   Ile Phe Ala Pro Ala Val Val Pro Leu Ile Thr Arg Leu Glu Glu Gln
           115                 120                 125
   Phe Thr Gln Tyr Asp Ile Glu Gln Ile Ser Glu Leu Ser Ser Ala Tyr
       130                 135                 140
   Ala Val Arg Leu Tyr Glu Leu Leu Ile Cys Trp Arg Ser Thr Gly Lys
   145                 150                 155                 160
   Thr Pro Ile Ile Asp Leu Thr Glu Phe Arg Lys Arg Leu Gly Val Leu
                   165                 170                 175
   Asp Thr Glu Tyr Thr Arg Thr Asp Asn Leu Lys Met Arg Val Ile Glu
               180                 185                 190
   Leu Gly Leu Lys Gln Ile Asn Glu His Thr Asp Ile Thr Ala Ser Tyr
           195                 200                 205
   Glu Gln His Lys Lys Gly Arg Thr Ile Thr Gly Phe Ser Phe Lys Phe
       210                 215                 220
   Lys Gln Lys Lys Lys Thr Gly Ala Glu Met Pro Lys Asn Ser Asp Ser
   225                 230                 235                 240
   Ser Pro His Ile Glu Lys Pro Ser Gln Ile Pro Ala Asn Ile Ala Lys
                   245                 250                 255
   Gln Pro Glu Asn Ala Lys Lys Asp Asp Leu Gly His Arg Ala Ser Lys
               260                 265                 270
   Ile Thr Gly Leu Ile Met Ser Asn Gly Leu Ala Asp Arg Phe Lys Arg
           275                 280                 285
   Gly Asp Glu Ser Val Ile Asp Met Met Lys Arg Ile Lys Glu Glu Ile
       290                 295                 300
   Thr Thr Asp Thr Thr Ala Asp Gln Trp Glu Asn Lys Leu Glu Glu Phe
   305                 310                 315                 320
   Gly Val Ile Phe
<210> 9
   <211> 324
   <212> PRT
   <213> Artificial Sequence
<220>
   <221> SOURCE
   <222> 1..324
   <223> /mol_type="protein"
   /note="subject"
   /organism="Artificial Sequence"
<400> 9
   Met Ser Glu Leu Ile Val Lys Asp Asn Ala Leu Met Asn Ala Ser Tyr
   1               5                   10                  15
   Asn Leu Asp Leu Val Glu Gln Arg Leu Ile Leu Leu Ala Ile Val Glu
               20                  25                  30
   Ala Arg Glu Ser Gly Lys Gly Ile Asn Ala Asn Asp Pro Leu Thr Val
           35                  40                  45
   His Ala Glu Ser Tyr Ile Asn Gln Phe Gly Val His Arg Thr Thr Ala
       50                  55                  60
   Tyr Gln Ala Leu Lys Asp Ala Cys Lys Asp Leu Phe Ala Arg Gln Phe
   65                  70                  75                  80
   Ser Tyr Gln Glu Lys Arg Glu Arg Gly Arg Ala Asn Ile Thr Ser Arg
                   85                  90                  95
   Trp Val Ser Gln Ile Ala Tyr Ile Asp Glu Thr Ala Thr Val Glu Ile
               100                 105                 110
   Ile Phe Ala Pro Ala Val Val Pro Leu Ile Thr Arg Leu Glu Lys Gln
           115                 120                 125
   Phe Thr Gln Tyr Asp Ile Glu Gln Ile Ser Gly Leu Ser Ser Ala Tyr
       130                 135                 140
   Ala Val Arg Met Tyr Glu Leu Leu Ile Cys Trp Arg Ser Thr Gly Lys
   145                 150                 155                 160
   Thr Pro Val Ile Glu Leu Gly Glu Phe Arg Lys Arg Ile Gly Val Leu
                   165                 170                 175
   Asp Thr Glu Tyr Ile Arg Thr Asp Asn Leu Lys Met Arg Val Ile Glu
               180                 185                 190
   Leu Ala Leu Lys Gln Ile Asn Asp His Thr Asp Ile Thr Ala Thr Tyr
           195                 200                 205
   Glu Gln His Lys Lys Gly Arg Thr Ile Thr Gly Phe Ser Phe Lys Phe
       210                 215                 220
   Lys Gln Lys Arg Lys Thr Glu Leu Glu Thr Pro Lys Asn Ser Asp Ser
   225                 230                 235                 240
   Asp Val Pro Lys Gln Lys Ser Val Glu Ile Pro Thr Asn Leu Val Lys
                   245                 250                 255
   Gln Pro Glu Asn Ala Asn Met Ser Asp Leu Gln His Arg Ala Ser Lys
               260                 265                 270
   Ile Thr Gly Leu Ile Met Ser Asn Arg Leu Ser Asp Arg Phe Lys Gln
           275                 280                 285
   Ser Asp Glu Ser Ile Met Gln Met Met Ala Arg Ile Gln Ser Glu Ile
       290                 295                 300
   Thr Asp Glu Ala Ile Ala Asn Gln Trp Glu Asn Lys Leu Glu Glu Phe
   305                 310                 315                 320
   Gly Val Ile Phe
<210> 10
   <211> 324
   <212> PRT
   <213> Artificial Sequence
<220>
   <221> SOURCE
   <222> 1..324
   <223> /mol_type="protein"
   /note="SPTREMBL:E5LG72 9ZZZZ"
   /organism="Artificial Sequence"
<400> 10
   Met Ser Asp Leu Ile Val Lys Asp Asn Ala Leu Met Asn Ala Ser Tyr
   1               5                   10                  15
   Asn Leu Ala Leu Val Glu Gln Arg Leu Ile Leu Leu Ala Ile Ile Glu
               20                  25                  30
   Ala Arg Glu Thr Gly Lys Gly Ile Asn Ala Asn Asp Pro Leu Thr Val
           35                  40                  45
   His Ala Gly Ser Tyr Ile Asn Gln Phe Asn Val Gln Arg His Thr Ala
       50                  55                  60
   Tyr Gln Ala Leu Lys Asp Ala Cys Lys Asp Leu Phe Ala Arg Gln Phe
   65                  70                  75                  80
   Ser Tyr Gln Glu Lys Arg Glu Arg Gly Arg Ile Asn Ile Thr Ser Arg
                   85                  90                  95
   Trp Val Ser Gln Ile Gly Tyr Met Asp Asp Thr Ala Thr Val Glu Ile
               100                 105                 110
   Ile Phe Ala Pro Ala Val Val Pro Leu Ile Thr Arg Leu Glu Glu Gln
           115                 120                 125
   Phe Thr Gln Tyr Asp Ile Glu Gln Ile Ser Gly Leu Ser Ser Ala Tyr
       130                 135                 140
   Ala Val Arg Met Tyr Glu Leu Leu Ile Cys Trp Arg Ser Thr Gly Lys
   145                 150                 155                 160
   Thr Pro Ile Ile Glu Leu Asp Glu Phe Arg Lys Arg Ile Gly Val Leu
                   165                 170                 175
   Asp Thr Glu Tyr Thr Arg Thr Asp Asn Leu Lys Met Gln Val Ile Glu
               180                 185                 190
   Leu Ala Leu Lys Gln Ile Asn Glu His Thr Asp Ile Thr Ala Ser Tyr
           195                 200                 205
   Glu Gln His Lys Lys Gly Arg Val Ile Thr Gly Phe Ser Phe Met Phe
       210                 215                 220
   Lys His Lys Lys Gln Asn Ser Asp Lys Thr Pro Asp Thr Asn Ala Ser
   225                 230                 235                 240
   Ser Pro Arg Ile Val Lys His Ser Gln Ile Pro Thr Asn Ile Val Lys
                   245                 250                 255
   Gln Pro Glu Asn Ala Lys Met Ser Asp Leu Glu His Arg Ala Ser Arg
               260                 265                 270
   Val Thr Gly Glu Ile Met Arg Asn Arg Leu Ser Asp Arg Phe Lys Gln
           275                 280                 285
   Gly Asp Glu Ser Ala Ile Asp Met Met Lys Arg Ile Gln Ser Glu Ile
       290                 295                 300
   Ile Thr Asp Ala Ile Ala Asp Gln Trp Glu Ser Lys Leu Glu Glu Phe
   305                 310                 315                 320
   Gly Val Val Phe
<210> 11
   <211> 1613
   <212> DNA
   <213> Artificial Sequence
<220>
   <221> source
   <222> 1..1613
   <223> /mol_type="DNA"
   /note="HCBI6.159"
   /organism="Artificial Sequence"
<400> 11
   ttttgttttt caaaggaagt aattggcgag tgtctacgag cgacacaatg aaagttcgat      60
   tattccctcc tctaaaaaac cgcttttaaa aatattggtt gctagatggt tttaactatc     120
   gtgagctttt gcttttaaaa atacacgagc aaagcgagtt catagtcgct tttgatctgc     180
   tcttactaac agctcatttt taatgggccg ttagtaagag atgcttttgg ggtttcgggg     240
   cttgccctga acaaggtcac aaagtgggaa tttatcacgg tagtgaaaaa gtaccctttg     300
   tgtatccttg ctttatgctt tttaaacctt tgaacttttc ccttaatttg cagaaattgc     360
   ccctcgacta agcttgctta gtcaaaaaag tttgagcaaa gcgaaaacat agggcaattt     420
   tcatgatgaa aatgggcttt taaagttttt aaatgctttt aaatgctttt agacatgttg     480
   aaatgcaagc ctagcaaggc acacagagga catacccctg cgtttaccga tcaatacccc     540
   tacgtttacc gatcaatacc cctacgttta ccgatcaata cccctacgtt taccttgcat     600
   atcactacaa acaatattaa tgtagtaata acttcaaaat aataaatgta ggttatgagc     660
   gatttaatag taaaagataa cgccctaatg aacgctagtt acaacttagc tttggttgaa     720
   cagcgtttaa tccttttagc tatattggaa gcgagagaaa caggcaaagg gattaatgcc     780
   aatgatccct taacagttca tgcaagtagc tatatcaatc aatttaacgt ggaaagacat     840
   acggcatatc aagcattaaa agatgcttgc aaagacttgt ttgcacgtca atttagttac     900
   caagaaaagc gagaacgtgg acgaattaat attacaagtc gatgggtttc acaaattggt     960
   tatatggacg atacagcaac cgttgaaatt atttttgccc ctgcggttgt tcctttgatt    1020
   acacgtttag aggaacaatt cactcaatac gatattgaac aaattagcga gttatcgagt    1080
   gcttatgctg tccgcctata tgagttacta atttgttggc gtagcacagg actatgttgg    1140
   agaatcaaga agctgctgca tttcgtaaag gttggaaaga atatatctgt aatttagaag    1200
   aaggcgataa ggtttacctt tatcaaagtg gcgttggttt tgtagcatct ggtattgtca    1260
   ctggtgactt aatcaaaagt gagtatcgtg gacaaccaga agaaaaatat tctaaaaaat    1320
   taaaggattt taaaatagga tttaaagcaa tttctgcaaa agagtttaag accttaactg    1380
   gcggtggtgc tagctttcgt acaacaatga gcgagctaaa gcctgaacaa tgtagaaaat    1440
   tagatatgga aatagaaaaa cggatgaagt cagtaagtta aaaatatgag ttgttgtttt    1500
   agtgtgtcac gtagcaatca cttagaagtt ccctttgtat tacttgtcta gcaatggttt    1560
   taaaaagcgt cactactgat agtgacgctt tataccccta cgtttaccga tca           1613
<210> 12
   <211> 811
   <212> DNA
   <213> Artificial Sequence
<220>
   <221> source
   <222> 1..811
   <223> /mol_type="DNA"
   /note="query"
   /organism="Artificial Sequence"
<400> 12
   ttttaaacct ttgaactttt cccttaattt gcagaaattg cccctcgact aagcttgctt      60
   agtcaaaaaa gtttgagcaa agcgaaaaca tagggcaatt ttcatgatga aaatgggctt     120
   ttaaagtttt taaatgcttt taaatgcttt tagacatgtt gaaatgcaag cctagcaagg     180
   cacacagagg acatacccct gcgtttaccg atcaataccc ctacgtttac cgatcaatac     240
   ccctacgttt accgatcaat acccctacgt ttaccttgca tatcactaca aacaatatta     300
   atgtagtaat aacttcaaaa taataaatgt aggttatgag cgatttaata gtaaaagata     360
   acgccctaat gaacgctagt tacaacttag ctttggttga acagcgttta atccttttag     420
   ctatattgga agcgagagaa acaggcaaag ggattaatgc caatgatccc ttaacagttc     480
   atgcaagtag ctatatcaat caatttaacg tggaaagaca tacggcatat caagcattaa     540
   aagatgcttg caaagacttg tttgcacgtc aatttagtta ccaagaaaag cgagaacgtg     600
   gacgaattaa tattacaagt cgatgggttt cacaaattgg ttatatggac gatacagcaa     660
   ccgttgaaat tatttttgcc cctgcggttg ttcctttgat tacacgttta gaggaacaat     720
   tcactcaata cgatattgaa caaattagcg agttatcgag tgcttatgct gtccgcctat     780
   atgagttact aatttgttgg cgtagcacag g                                    811
<210> 13
   <211> 805
   <212> DNA
   <213> Artificial Sequence
<220>
   <221> source
   <222> 1..805
   <223> /mol_type="DNA"
   /note="subject"
   /organism="Artificial Sequence"
<400> 13
   ttctaaacct ttgacttctt ccccattgtt tgcagaaatt gcccctcgaa gcttgcttag 60
   tcaaaaaagt ttgagcaaag cgaaaacata gggcaatttt catgaagaaa ttgggctttt 120
   aaagttttta aatgttttaa atgcttttag acatgctaag aagcccacac agcaaggcat 180
   acagaggaca ttctcctacg tttaccgatc aatacccccg tttaccgatc aataccccta 240
   cgtttaccga tcaatacccc tacgtttacc ttgcgtataa ctacaaagaa tactagtgta 300
   gtataacttc aaaagaataa ttgtaggtta tgagcgattt aatagtaaaa gataacgccc 360
   taatgaatgc tagttataac ttagctttgg ttgaacagag gttaattcta ttagcaatca 420
   tagaagcgag agaaacaggc aaagggatta atgccaatga tcctcttacg gttcatgcag 480
   gtagctatat caatcaattt aacgtacaaa ggcatacggc atatcaagcc ctcaaagatg 540
   cttgtaaaga cttgtttgcc cgtcaattca gttaccaaga aaagcgagaa cgaggacgaa 600
   ttaatattac aagtcgatgg gtttcgcaaa ttggctatat ggacgataca gcaaccgttg 660
   agattatttt tgcccctgcg gttgttcctc tgattacacg gctagaggaa cagttcaccc 720
   agtacgatat tgagcaaatt agcggtttat cgagtgcata tgctgttcgt atgtacgaac 780
   tgctgatttg ttggcgtagc acagg 805
<210> 14
   <211> 153
   <212> DNA
   <213> Artificial Sequence
<220>
   <221> source
   <222> 1..153
   <223> /mol_type="DNA"
   /note="query"
   /organism="Artificial Sequence"
<400> 14
   attggcgagt gtctacgagc gacacaatga aagttcgatt attccctcct ctaaaaaacc 60
   gcttttaaaa atattggttg ctagatggtt ttaactatcg tgagcttttg cttttaaaaa 120
   tacacgagca aagcgagttc atagtcgctt ttg 153
<210> 15
   <211> 152
   <212> DNA
   <213> Artificial Sequence
<220>
   <221> source
   <222> 1..152
   <223> /mol_type="DNA"
   /note="subject"
   /organism="Artificial Sequence"
<400> 15
   attagcgagt gtctacgagc gactcaatga aagttcgatt attccccctc tggaaaaccg 60
   cttttaaaaa tattggctgc tagatggttt ttactatagt gaggttttgc ttttaaaaaa 120
   acacgagcaa agcgagttca tagttgcttt tg 152
<210> 16
   <211> 59
   <212> DNA
   <213> Artificial Sequence
<220>
   <221> source
   <222> 1..59
   <223> /mol_type="DNA"
   /note="query"
   /organism="Artificial Sequence"
<400> 16
   ctttttaaac ctttgaactt ttcccttaat ttgcagaaat tgcccctcga ctaagcttg 59
<210> 17
   <211> 59
   <212> DNA
   <213> Artificial Sequence
<220>
   <221> source
   <222> 1..59
   <223> /mol_type="DNA"
   /note="subject"
   /organism="Artificial Sequence"
<400> 17
   ctttttaaac ctttcgactt tccctatcct ttgcataaat tgcccctgac gtagcaaag 59
<210> 18
   <211> 159
   <212> PRT
   <213> Artificial Sequence
<220>
   <221> SOURCE
   <222> 1..159
   <223> /mol_type="protein"
   /note="query"
   /organism="Artificial Sequence"
<400> 18
   Met Ser Asp Leu Ile Val Lys Asp Asn Ala Leu Met Asn Ala Ser Tyr
   1               5                   10                  15
   Asn Leu Ala Leu Val Glu Gln Arg Leu Ile Leu Leu Ala Ile Leu Glu
               20                  25                  30
   Ala Arg Glu Thr Gly Lys Gly Ile Asn Ala Asn Asp Pro Leu Thr Val
           35                  40                  45
   His Ala Ser Ser Tyr Ile Asn Gln Phe Asn Val Glu Arg His Thr Ala
       50                  55                  60
   Tyr Gln Ala Leu Lys Asp Ala Cys Lys Asp Leu Phe Ala Arg Gln Phe
   65                  70                  75                  80
   Ser Tyr Gln Glu Lys Arg Glu Arg Gly Arg Ile Asn Ile Thr Ser Arg
                   85                  90                  95
   Trp Val Ser Gln Ile Gly Tyr Met Asp Asp Thr Ala Thr Val Glu Ile
               100                 105                 110
   Ile Phe Ala Pro Ala Val Val Pro Leu Ile Thr Arg Leu Glu Glu Gln
           115                 120                 125
   Phe Thr Gln Tyr Asp Ile Glu Gln Ile Ser Glu Leu Ser Ser Ala Tyr
       130                 135                 140
   Ala Val Arg Leu Tyr Glu Leu Leu Ile Cys Trp Arg Ser Thr Gly
   145                 150                 155
<210> 19
   <211> 159
   <212> PRT
   <213> Artificial Sequence
<220>
   <221> SOURCE
   <222> 1..159
   <223> /mol_type="protein"
   /note="subject"
   /organism="Artificial Sequence"
<400> 19
   Met Ser Asp Leu Ile Val Lys Asp Asn Ala Leu Met Asn Ala Ser Tyr
   1               5                   10                  15
   Asn Leu Ala Leu Val Glu Gln Arg Leu Ile Leu Leu Ala Ile Ile Glu
               20                  25                  30
   Ala Arg Glu Thr Gly Lys Gly Ile Asn Ala Asn Asp Pro Leu Thr Val
           35                  40                  45
   His Ala Gly Ser Tyr Ile Asn Gln Phe Asn Val Gln Arg His Thr Ala
       50                  55                  60
   Tyr Gln Ala Leu Lys Asp Ala Cys Lys Asp Leu Phe Ala Arg Gln Phe
   65                  70                  75                  80
   Ser Tyr Gln Glu Lys Arg Glu Arg Gly Arg Ile Asn Ile Thr Ser Arg
                   85                  90                  95
   Trp Val Ser Gln Ile Gly Tyr Met Asp Asp Thr Ala Thr Val Glu Ile
               100                 105                 110
   Ile Phe Ala Pro Ala Val Val Pro Leu Ile Thr Arg Leu Glu Glu Gln
           115                 120                 125
   Phe Thr Gln Tyr Asp Ile Glu Gln Ile Ser Gly Leu Ser Ser Ala Tyr
       130                 135                 140
   Ala Val Arg Met Tyr Glu Leu Leu Ile Cys Trp Arg Ser Thr Gly
   145                 150                 155
<210> 20
   <211> 159
   <212> PRT
   <213> Artificial Sequence
<220>
   <221> SOURCE
   <222> 1..159
   <223> /mol_type="protein"
   /note="query"
   /organism="Artificial Sequence"
<400> 20
   Met Ser Asp Leu Ile Val Lys Asp Asn Ala Leu Met Asn Ala Ser Tyr
   1               5                   10                  15
   Asn Leu Ala Leu Val Glu Gln Arg Leu Ile Leu Leu Ala Ile Leu Glu
               20                  25                  30
   Ala Arg Glu Thr Gly Lys Gly Ile Asn Ala Asn Asp Pro Leu Thr Val
           35                  40                  45
   His Ala Ser Ser Tyr Ile Asn Gln Phe Asn Val Glu Arg His Thr Ala
       50                  55                  60
   Tyr Gln Ala Leu Lys Asp Ala Cys Lys Asp Leu Phe Ala Arg Gln Phe
   65                  70                  75                  80
   Ser Tyr Gln Glu Lys Arg Glu Arg Gly Arg Ile Asn Ile Thr Ser Arg
                   85                  90                  95
   Trp Val Ser Gln Ile Gly Tyr Met Asp Asp Thr Ala Thr Val Glu Ile
               100                 105                 110
   Ile Phe Ala Pro Ala Val Val Pro Leu Ile Thr Arg Leu Glu Glu Gln
           115                 120                 125
   Phe Thr Gln Tyr Asp Ile Glu Gln Ile Ser Glu Leu Ser Ser Ala Tyr
       130                 135                 140
   Ala Val Arg Leu Tyr Glu Leu Leu Ile Cys Trp Arg Ser Thr Gly
   145                 150                 155
<210> 21
   <211> 159
   <212> PRT
   <213> Artificial Sequence
<220>
   <221> SOURCE
   <222> 1..159
   <223> /mol_type="protein"
   /note="subject"
   /organism="Artificial Sequence"
<400> 21
   Met Ser Glu Leu Ile Val Lys Asp Asn Ala Leu Met Asn Ala Ser Tyr
   1               5                   10                  15
   Asn Leu Asp Leu Val Glu Gln Arg Leu Ile Leu Leu Ala Ile Val Glu
               20                  25                  30
   Ala Arg Glu Ser Gly Lys Gly Ile Asn Ala Asn Asp Pro Leu Thr Val
           35                  40                  45
   His Ala Glu Ser Tyr Ile Asn Gln Phe Gly Val His Arg Thr Thr Ala
       50                  55                  60
   Tyr Gln Ala Leu Lys Asp Ala Cys Lys Asp Leu Phe Ala Arg Gln Phe
   65                  70                  75                  80
   Ser Tyr Gln Glu Lys Arg Glu Arg Gly Arg Ala Asn Ile Thr Ser Arg
                   85                  90                  95
   Trp Val Ser Gln Ile Ala Tyr Ile Asp Glu Thr Ala Thr Val Glu Ile
               100                 105                 110
   Ile Phe Ala Pro Ala Val Val Pro Leu Ile Thr Arg Leu Glu Lys Gln
           115                 120                 125
   Phe Thr Gln Tyr Asp Ile Glu Gln Ile Ser Gly Leu Ser Ser Ala Tyr
       130                 135                 140
   Ala Val Arg Met Tyr Glu Leu Leu Ile Cys Trp Arg Ser Thr Gly
   145                 150                 155
<210> 22
   <211> 159
   <212> PRT
   <213> Artificial Sequence
<220>
   <221> SOURCE
   <222> 1..159
   <223> /mol_type="protein"
   /note="query"
   /organism="Artificial Sequence"
<400> 22
   Met Ser Asp Leu Ile Val Lys Asp Asn Ala Leu Met Asn Ala Ser Tyr
   1               5                   10                  15
   Asn Leu Ala Leu Val Glu Gln Arg Leu Ile Leu Leu Ala Ile Leu Glu
               20                  25                  30
   Ala Arg Glu Thr Gly Lys Gly Ile Asn Ala Asn Asp Pro Leu Thr Val
           35                  40                  45
   His Ala Ser Ser Tyr Ile Asn Gln Phe Asn Val Glu Arg His Thr Ala
       50                  55                  60
   Tyr Gln Ala Leu Lys Asp Ala Cys Lys Asp Leu Phe Ala Arg Gln Phe
   65                  70                  75                  80
   Ser Tyr Gln Glu Lys Arg Glu Arg Gly Arg Ile Asn Ile Thr Ser Arg
                   85                  90                  95
   Trp Val Ser Gln Ile Gly Tyr Met Asp Asp Thr Ala Thr Val Glu Ile
               100                 105                 110
   Ile Phe Ala Pro Ala Val Val Pro Leu Ile Thr Arg Leu Glu Glu Gln
           115                 120                 125
   Phe Thr Gln Tyr Asp Ile Glu Gln Ile Ser Glu Leu Ser Ser Ala Tyr
       130                 135                 140
   Ala Val Arg Leu Tyr Glu Leu Leu Ile Cys Trp Arg Ser Thr Gly
   145                 150                 155
<210> 23
   <211> 160
   <212> PRT
   <213> Artificial Sequence
<220>
   <221> SOURCE
   <222> 1..160
   <223> /mol_type="protein"
   /note="subject"
   /organism="Artificial Sequence"
<400> 23
   Met Ser Glu Leu Ile Val Lys Asp Asn Ala Leu Met Asn Ala Ser Tyr
   1               5                   10                  15
   Asn Leu Ala Leu Val Glu Gln Arg Leu Ile Leu Leu Ala Ile Ile Glu
               20                  25                  30
   Ala Arg Glu Ser Gly Arg Gly Ile Asn Ala Asn Asp Pro Leu Thr Val
           35                  40                  45
   His Ala Glu Ser Tyr Ile Asn Gln Phe Lys Val Glu Lys His Thr Ala
       50                  55                  60
   Tyr Gln Ala Leu Lys Asp Ala Cys Lys Asp Leu Phe Ala Arg Gln Phe
   65                  70                  75                  80
   Ser Tyr Gln Gln Arg Ser Ala Lys Gly Asn Thr Thr Asn Met Thr Ser
                   85                  90                  95
   Arg Trp Ile Ser Gln Ile Gly Tyr Thr Glu Asn Glu Ala Thr Val Gln
               100                 105                 110
   Leu Val Phe Ala Pro Ala Val Val Pro Leu Ile Thr Arg Leu Glu Glu
           115                 120                 125
   Gln Phe Thr Lys Tyr Asp Ile Glu Gln Ile Ser Ser Leu Ser Ser Ala
       130                 135                 140
   Tyr Ala Val Arg Leu Tyr Glu Leu Leu Ile Cys Trp Arg Ser Thr Gly
   145                 150                 155                 160
<210> 24
   <211> 1766
   <212> DNA
   <213> Artificial Sequence
<220>
   <221> source
   <222> 1..1766
   <223> /mol_type="DNA"
   /note="MSBI1.176"
   /organism="Artificial Sequence"
<400> 24
   aagcttgctt agtcaaaaaa gtttgagcaa agcgaaaaca tagggcaatt ttcatgaaga      60
   aattgggctt ttaaagtttt taaatgcttt taaatgcttt tagacatgct aagaagccca     120
   cacagcaagg catacagagg acattctcct acgtttaccg atcaataccc ctacgtttac     180
   cgatcaatac ccctacgttt accgatcaat acccctacgt ttaccttgcg tataactaca     240
   aagaatacta gtgtagtaat aacttcaaaa gaataattgt aggttatgag cgatttaata     300
   gtaaaagata acgccctaat gaatgctagt tataacttag ctttggttga acagaggtta 360
   attctattag caatcataga agcgagagaa acaggcaaag ggattaatgc caatgatcct 420
   ttaacagttc atgcaagtag ctatatcaat caatttaacg tagaaaggca tacggcatat 480
   caagccctca aagatgcttg taaagacttg tttgcccgtc aattcagtta ccaagaaaag 540
   cgagaacgag gacgaattaa tattacaagt cgatgggttt cgcaaattgg ctatatggac 600
   gatacagcaa ccgttgagat tatttttgcc cctgcggttg ttcctctgat tacacggcta 660
   gaggaacagt tcacccagta cgatattgag caaattagcg gtttatcgag tgcatatgct 720
   gttcgtatgt acgaactgct gatttgttgg cgtagcacag gcaaaacacc aattattgag 780
   ctagacgagt ttagaaagcg aataggtgtt ttagatactg aatacactag aacagataat 840
   ttaaagatgc gagttattga attagcccta aaacaaatca acgaacatac agacatcaca 900
   gcaagctatg aacaacacaa aaaagggcga gtgattacag gattctcatt caagtttaag 960
   cacaagaaac aaaacagcga taaaacgcca aaaaatagcg attctagccc acgtatcgta 1020
   aaacatagtc aaatccctac caacattgta aaacagcctg aaaacgccaa aatgagcgat 1080
   ttagaacata gagcgagccg tgttacaggg gaaataatgc gaaatcgtct gtcagatcgg 1140
   tttaaacaag gcgatgaatc agcaatcgac atgatgaaac gtattcaaag tgaaataata 1200
   accgatgcaa tagcagacca gtgggaaagc aaactggagg agtttggcgt ggttttttag 1260
   tcatgacgat ttcccgaagg gcgcacttag ccattgagaa aaatcttcga ttttttcaat 1320
   ggaagtccgt gggggtaaac ccctcaaccc caaaagcaaa aacactgtaa tcagggaaaa 1380
   aacatttttg attttgatcc ttgtttgtca ctcgtagaca ctcgttttgt tttgctcttt 1440
   ctagaattca caaaaaagat attagcgagt gtctacgagc gactcaatga aagttcgatt 1500
   attccccctc tggaaaaccg cttttaaaaa tattggctgc tagatggttt ttactatagt 1560
   gaggttttgc ttttaaaaaa acacgagcaa agcgagttca tagttgcttt tgcttgtttt 1620
   cgggtcttag gggaaatccc ctaacaagtc ctcgaatatc aaaatgtggc tacattttgt 1680
   atatacgggt aggcttgctt atttgatttt ttttcttcta aacctttgac ttcttcccca 1740
   ttgtttgcag aaattgcccc tcgact 1766
<210> 25
   <211> 1439
   <212> DNA
   <213> Artificial Sequence
<220>
   <221> source
   <222> 1..1439
   <223> /mol_type="DNA"
   /note="query"
   /organism="Artificial Sequence"
<400> 25
   aagcttgctt agtcaaaaaa gtttgagcaa agcgaaaaca tagggcaatt ttcatgaaga      60
   aattgggctt ttaaagtttt taaatgcttt taaatgcttt tagacatgct aagaagccca     120
   cacagcaagg catacagagg acattctcct acgtttaccg atcaataccc ctacgtttac     180
   cgatcaatac ccctacgttt accgatcaat acccctacgt ttaccttgcg tataactaca     240
   aagaatacta gtgtagtaat aacttcaaaa gaataattgt aggttatgag cgatttaata     300
   gtaaaagata acgccctaat gaatgctagt tataacttag ctttggttga acagaggtta     360
   attctattag caatcataga agcgagagaa acaggcaaag ggattaatgc caatgatcct     420
   ttaacagttc atgcaagtag ctatatcaat caatttaacg tagaaaggca tacggcatat     480
   caagccctca aagatgcttg taaagacttg tttgcccgtc aattcagtta ccaagaaaag     540
   cgagaacgag gacgaattaa tattacaagt cgatgggttt cgcaaattgg ctatatggac     600
   gatacagcaa ccgttgagat tatttttgcc cctgcggttg ttcctctgat tacacggcta     660
   gaggaacagt tcacccagta cgatattgag caaattagcg gtttatcgag tgcatatgct     720
   gttcgtatgt acgaactgct gatttgttgg cgtagcacag gcaaaacacc aattattgag     780
   ctagacgagt ttagaaagcg aataggtgtt ttagatactg aatacactag aacagataat     840
   ttaaagatgc gagttattga attagcccta aaacaaatca acgaacatac agacatcaca     900
   gcaagctatg aacaacacaa aaaagggcga gtgattacag gattctcatt caagtttaag     960
   cacaagaaac aaaacagcga taaaacgcca aaaaatagcg attctagccc acgtatcgta    1020
   aaacatagtc aaatccctac caacattgta aaacagcctg aaaacgccaa aatgagcgat    1080
   ttagaacata gagcgagccg tgttacaggg gaaataatgc gaaatcgtct gtcagatcgg    1140
   tttaaacaag gcgatgaatc agcaatcgac atgatgaaac gtattcaaag tgaaataata    1200
   accgatgcaa tagcagacca gtgggaaagc aaactggagg agtttggcgt ggttttttag    1260
   tcatgacgat ttcccgaagg gcgcacttag ccattgagaa aaatcttcga ttttttcaat    1320
   ggaagtccgt gggggtaaac ccctcaaccc caaaagcaaa aacactgtaa tcagggaaaa    1380
   aacatttttg attttgatcc ttgtttgtca ctcgtagaca ctcgttttgt tttgctctt     1439
<210> 26
   <211> 1434
   <212> DNA
   <213> Artificial Sequence
<220>
   <221> source
   <222> 1..1434
   <223> /mol_type="DNA"
   /note="subject"
   /organism="Artificial Sequence"
<400> 26
   aagcttgctt agtcaaaaaa gtttgagcaa agcgaaaaca tagggcaatt ttcatgaaga      60
   aattgggctt ttaaagtttt taaatgtttt aaatgctttt agacatgcta agaagcccac     120
   acagcaaggc atacagagga cattctccta cgtttaccga tcaatacccc cgtttaccga     180
   tcaatacccc tacgtttacc gatcaatacc cctacgttta ccttgcgtat aactacaaag     240
   aatactagtg tagtataact tcaaaagaat aattgtaggt tatgagcgat ttaatagtaa     300
   aagataacgc cctaatgaat gctagttata acttagcttt ggttgaacag aggttaattc     360
   tattagcaat catagaagcg agagaaacag gcaaagggat taatgccaat gatcctctta     420
   cggttcatgc aggtagctat atcaatcaat ttaacgtaca aaggcatacg gcatatcaag     480
   ccctcaaaga tgcttgtaaa gacttgtttg cccgtcaatt cagttaccaa gaaaagcgag     540
   aacgaggacg aattaatatt acaagtcgat gggtttcgca aattggctat atggacgata     600
   cagcaaccgt tgagattatt tttgcccctg cggttgttcc tctgattaca cggctagagg     660
   aacagttcac ccagtacgat attgagcaaa ttagcggttt atcgagtgca tatgctgttc     720
   gtatgtacga actgctgatt tgttggcgta gcacaggcaa aacaccaatt attgagctag     780
   acgagtttag aaagcgaata ggtgttttag atactgaata cactagaaca gataatttaa     840
   agatgcaagt tattgaatta gccctaaaac aaatcaacga acatactgac atcacagcaa     900
   gctatgaaca acacaaaaaa gggcgagtga ttaccggatt ctcattcatg tttaagcaca     960
   agaaacaaaa cagcgataaa acgcctgata ctaacgcttc tagcccacgt atcgtaaaac    1020
   atagtcaaat ccctaccaac attgtaaaac agcctgaaaa cgccaaaatg agcgatttag    1080
   aacatagagc gagccgtgtt acaggggaaa taatgcgaaa tcgtctgtca gatcggttta    1140
   aacaaggcga tgaatcagca atcgacatga tgaaacgtat tcaaagtgaa ataataaccg    1200
   atgcaatagc agaccagtgg gaaagcaaac tggaggagtt tggcgtggtt ttttagtcat    1260
   gacgatttcc cgaagggcgc acttagccat tgagaaaaat cttcgatttt ttcaatggaa    1320
   gtccgtgggg gtaaacccct caaccccaaa agcaaaaaca ctgtaatcag ggaaaaaaca    1380
   tttttgattt tgatccttgt ttgcactcgt agacactcgt tttgttttgc tctt          1434
<210> 27
   <211> 325
   <212> DNA
   <213> Artificial Sequence
<220>
   <221> source
   <222> 1..325
   <223> /mol_type="DNA"
   /note="query"
   /organism="Artificial Sequence"
<400> 27
   tctagaattc acaaaaaaga tattagcgag tgtctacgag cgactcaatg aaagttcgat      60
   tattccccct ctggaaaacc gcttttaaaa atattggctg ctagatggtt tttactatag     120
   tgaggttttg cttttaaaaa aacacgagca aagcgagttc atagttgctt ttgcttgttt     180
   tcgggtctta ggggaaatcc cctaacaagt cctcgaatat caaaatgtgg ctacattttg     240
   tatatacggg taggcttgct tatttgattt tttttcttct aaacctttga cttcttcccc     300
   attgtttgca gaaattgccc ctcga                                           325
<210> 28
   <211> 325
   <212> DNA
   <213> Artificial Sequence
<220>
   <221> source
   <222> 1..325
   <223> /mol_type="DNA"
   /note="subject"
   /organism="Artificial Sequence"
<400> 28
   tctagaattc acaaaaaaga tattagcgag tgtctacgag cgactcaatg aaagttcgat      60
   tattccccct ctggaaaacc gcttttaaaa atattggctg ctagatggtt tttactatag     120
   tgaggttttg cttttaaaaa aacacgagca aagcgagttc atagttgctt ttgcttgttt     180
   tcgggtctta ggggaaatcc cctaacaagt cctcgaatat caaaatgtgg ctacattttg     240
   tatatacggg taggcttgct tatttgattt tttttcttct aaacctttga cttcttcccc     300
   attgtttgca gaaattgccc ctcga                                           325
<210> 29
   <211> 324
   <212> PRT
   <213> Artificial Sequence
<220>
   <221> SOURCE
   <222> 1..324
   <223> /mol_type="protein"
   /note="query"
   /organism="Artificial Sequence"
<400> 29
   Met Ser Asp Leu Ile Val Lys Asp Asn Ala Leu Met Asn Ala Ser Tyr
   1               5                   10                  15
   Asn Leu Ala Leu Val Glu Gln Arg Leu Ile Leu Leu Ala Ile Ile Glu
               20                  25                  30
   Ala Arg Glu Thr Gly Lys Gly Ile Asn Ala Asn Asp Pro Leu Thr Val
           35                  40                  45
   His Ala Ser Ser Tyr Ile Asn Gln Phe Asn Val Glu Arg His Thr Ala
       50                  55                  60
   Tyr Gln Ala Leu Lys Asp Ala Cys Lys Asp Leu Phe Ala Arg Gln Phe
   65                  70                  75                  80
   Ser Tyr Gln Glu Lys Arg Glu Arg Gly Arg Ile Asn Ile Thr Ser Arg
                   85                  90                  95
   Trp Val Ser Gln Ile Gly Tyr Met Asp Asp Thr Ala Thr Val Glu Ile
               100                 105                 110
   Ile Phe Ala Pro Ala Val Val Pro Leu Ile Thr Arg Leu Glu Glu Gln
           115                 120                 125
   Phe Thr Gln Tyr Asp Ile Glu Gln Ile Ser Gly Leu Ser Ser Ala Tyr
       130                 135                 140
   Ala Val Arg Met Tyr Glu Leu Leu Ile Cys Trp Arg Ser Thr Gly Lys
   145                 150                 155                 160
   Thr Pro Ile Ile Glu Leu Asp Glu Phe Arg Lys Arg Ile Gly Val Leu
                   165                 170                 175
   Asp Thr Glu Tyr Thr Arg Thr Asp Asn Leu Lys Met Arg Val Ile Glu
               180                 185                 190
   Leu Ala Leu Lys Gln Ile Asn Glu His Thr Asp Ile Thr Ala Ser Tyr
           195                 200                 205
   Glu Gln His Lys Lys Gly Arg Val Ile Thr Gly Phe Ser Phe Lys Phe
       210                 215                 220
   Lys His Lys Lys Gln Asn Ser Asp Lys Thr Pro Lys Asn Ser Asp Ser
   225                 230                 235                 240
   Ser Pro Arg Ile Val Lys His Ser Gln Ile Pro Thr Asn Ile Val Lys
                   245                 250                 255
   Gln Pro Glu Asn Ala Lys Met Ser Asp Leu Glu His Arg Ala Ser Arg
               260                 265                 270
   Val Thr Gly Glu Ile Met Arg Asn Arg Leu Ser Asp Arg Phe Lys Gln
           275                 280                 285
   Gly Asp Glu Ser Ala Ile Asp Met Met Lys Arg Ile Gln Ser Glu Ile
       290                 295                 300
   Ile Thr Asp Ala Ile Ala Asp Gln Trp Glu Ser Lys Leu Glu Glu Phe
   305                 310                 315                 320
   Gly Val Val Phe
<210> 30
   <211> 324
   <212> PRT
   <213> Artificial Sequence
<220>
   <221> SOURCE
   <222> 1..324
   <223> /mol_type="protein"
   /note="subject"
   /organism="Artificial Sequence"
<400> 30
   Met Ser Asp Leu Ile Val Lys Asp Asn Ala Leu Met Asn Ala Ser Tyr
   1               5                   10                  15
   Asn Leu Ala Leu Val Glu Gln Arg Leu Ile Leu Leu Ala Ile Ile Glu
               20                  25                  30
   Ala Arg Glu Thr Gly Lys Gly Ile Asn Ala Asn Asp Pro Leu Thr Val
           35                  40                  45
   His Ala Gly Ser Tyr Ile Asn Gln Phe Asn Val Gln Arg His Thr Ala
       50                  55                  60
   Tyr Gln Ala Leu Lys Asp Ala Cys Lys Asp Leu Phe Ala Arg Gln Phe
   65                  70                  75                  80
   Ser Tyr Gln Glu Lys Arg Glu Arg Gly Arg Ile Asn Ile Thr Ser Arg
                   85                  90                  95
   Trp Val Ser Gln Ile Gly Tyr Met Asp Asp Thr Ala Thr Val Glu Ile
               100                 105                 110
   Ile Phe Ala Pro Ala Val Val Pro Leu Ile Thr Arg Leu Glu Glu Gln
           115                 120                 125
   Phe Thr Gln Tyr Asp Ile Glu Gln Ile Ser Gly Leu Ser Ser Ala Tyr
       130                 135                 140
   Ala Val Arg Met Tyr Glu Leu Leu Ile Cys Trp Arg Ser Thr Gly Lys
   145                 150                 155                 160
   Thr Pro Ile Ile Glu Leu Asp Glu Phe Arg Lys Arg Ile Gly Val Leu
                   165                 170                 175
   Asp Thr Glu Tyr Thr Arg Thr Asp Asn Leu Lys Met Gln Val Ile Glu
               180                 185                 190
   Leu Ala Leu Lys Gln Ile Asn Glu His Thr Asp Ile Thr Ala Ser Tyr
           195                 200                 205
   Glu Gln His Lys Lys Gly Arg Val Ile Thr Gly Phe Ser Phe Met Phe
       210                 215                 220
   Lys His Lys Lys Gln Asn Ser Asp Lys Thr Pro Asp Thr Asn Ala Ser
   225                 230                 235                 240
   Ser Pro Arg Ile Val Lys His Ser Gln Ile Pro Thr Asn Ile Val Lys
                   245                 250                 255
   Gln Pro Glu Asn Ala Lys Met Ser Asp Leu Glu His Arg Ala Ser Arg
               260                 265                 270
   Val Thr Gly Glu Ile Met Arg Asn Arg Leu Ser Asp Arg Phe Lys Gln
           275                 280                 285
   Gly Asp Glu Ser Ala Ile Asp Met Met Lys Arg Ile Gln Ser Glu Ile
       290                 295                 300
   Ile Thr Asp Ala Ile Ala Asp Gln Trp Glu Ser Lys Leu Glu Glu Phe
   305                 310                 315                 320
   Gly Val Val Phe
<210> 31
   <211> 324
   <212> PRT
   <213> Artificial Sequence
<220>
   <221> SOURCE
   <222> 1..324
   <223> /mol_type="protein"
   /note="query"
   /organism="Artificial Sequence"
<400> 31
   Met Ser Asp Leu Ile Val Lys Asp Asn Ala Leu Met Asn Ala Ser Tyr
   1               5                   10                  15
   Asn Leu Ala Leu Val Glu Gln Arg Leu Ile Leu Leu Ala Ile Ile Glu
               20                  25                  30
   Ala Arg Glu Thr Gly Lys Gly Ile Asn Ala Asn Asp Pro Leu Thr Val
           35                  40                  45
   His Ala Ser Ser Tyr Ile Asn Gln Phe Asn Val Glu Arg His Thr Ala
       50                  55                  60
   Tyr Gln Ala Leu Lys Asp Ala Cys Lys Asp Leu Phe Ala Arg Gln Phe
   65                  70                  75                  80
   Ser Tyr Gln Glu Lys Arg Glu Arg Gly Arg Ile Asn Ile Thr Ser Arg
                   85                  90                  95
   Trp Val Ser Gln Ile Gly Tyr Met Asp Asp Thr Ala Thr Val Glu Ile
               100                 105                 110
   Ile Phe Ala Pro Ala Val Val Pro Leu Ile Thr Arg Leu Glu Glu Gln
           115                 120                 125
   Phe Thr Gln Tyr Asp Ile Glu Gln Ile Ser Gly Leu Ser Ser Ala Tyr
       130                 135                 140
   Ala Val Arg Met Tyr Glu Leu Leu Ile Cys Trp Arg Ser Thr Gly Lys
   145                 150                 155                 160
   Thr Pro Ile Ile Glu Leu Asp Glu Phe Arg Lys Arg Ile Gly Val Leu
                   165                 170                 175
   Asp Thr Glu Tyr Thr Arg Thr Asp Asn Leu Lys Met Arg Val Ile Glu
               180                 185                 190
   Leu Ala Leu Lys Gln Ile Asn Glu His Thr Asp Ile Thr Ala Ser Tyr
           195                 200                 205
   Glu Gln His Lys Lys Gly Arg Val Ile Thr Gly Phe Ser Phe Lys Phe
       210                 215                 220
   Lys His Lys Lys Gln Asn Ser Asp Lys Thr Pro Lys Asn Ser Asp Ser
   225                 230                 235                 240
   Ser Pro Arg Ile Val Lys His Ser Gln Ile Pro Thr Asn Ile Val Lys
                   245                 250                 255
   Gln Pro Glu Asn Ala Lys Met Ser Asp Leu Glu His Arg Ala Ser Arg
               260                 265                 270
   Val Thr Gly Glu Ile Met Arg Asn Arg Leu Ser Asp Arg Phe Lys Gln
           275                 280                 285
   Gly Asp Glu Ser Ala Ile Asp Met Met Lys Arg Ile Gln Ser Glu Ile
       290                 295                 300
   Ile Thr Asp Ala Ile Ala Asp Gln Trp Glu Ser Lys Leu Glu Glu Phe
   305                 310                 315                 320
   Gly Val Val Phe
<210> 32
   <211> 324
   <212> PRT
   <213> Artificial Sequence
<220>
   <221> SOURCE
   <222> 1..324
   <223> /mol_type="protein"
   /note="subject"
   /organism="Artificial Sequence"
<400> 32
   Met Ser Glu Leu Ile Val Lys Asp Asn Ala Leu Met Asn Ala Ser Tyr
   1               5                   10                  15
   Asn Leu Asp Leu Val Glu Gln Arg Leu Ile Leu Leu Ala Ile Val Glu
               20                  25                  30
   Ala Arg Glu Ser Gly Lys Gly Ile Asn Ala Asn Asp Pro Leu Thr Val
           35                  40                  45
   His Ala Glu Ser Tyr Ile Asn Gln Phe Gly Val His Arg Thr Thr Ala
       50                  55                  60
   Tyr Gln Ala Leu Lys Asp Ala Cys Lys Asp Leu Phe Ala Arg Gln Phe
   65                  70                  75                  80
   Ser Tyr Gln Glu Lys Arg Glu Arg Gly Arg Ala Asn Ile Thr Ser Arg
                   85                  90                  95
   Trp Val Ser Gln Ile Ala Tyr Ile Asp Glu Thr Ala Thr Val Glu Ile
               100                 105                 110
   Ile Phe Ala Pro Ala Val Val Pro Leu Ile Thr Arg Leu Glu Lys Gln
           115                 120                 125
   Phe Thr Gln Tyr Asp Ile Glu Gln Ile Ser Gly Leu Ser Ser Ala Tyr
       130                 135                 140
   Ala Val Arg Met Tyr Glu Leu Leu Ile Cys Trp Arg Ser Thr Gly Lys
   145                 150                 155                 160
   Thr Pro Val Ile Glu Leu Gly Glu Phe Arg Lys Arg Ile Gly Val Leu
                   165                 170                 175
   Asp Thr Glu Tyr Ile Arg Thr Asp Asn Leu Lys Met Arg Val Ile Glu
               180                 185                 190
   Leu Ala Leu Lys Gln Ile Asn Asp His Thr Asp Ile Thr Ala Thr Tyr
           195                 200                 205
   Glu Gln His Lys Lys Gly Arg Thr Ile Thr Gly Phe Ser Phe Lys Phe
       210                 215                 220
   Lys Gln Lys Arg Lys Thr Glu Leu Glu Thr Pro Lys Asn Ser Asp Ser
   225                 230                 235                 240
   Asp Val Pro Lys Gln Lys Ser Val Glu Ile Pro Thr Asn Leu Val Lys
                   245                 250                 255
   Gln Pro Glu Asn Ala Asn Met Ser Asp Leu Gln His Arg Ala Ser Lys
               260                 265                 270
   Ile Thr Gly Leu Ile Met Ser Asn Arg Leu Ser Asp Arg Phe Lys Gln
           275                 280                 285
   Ser Asp Glu Ser Ile Met Gln Met Met Ala Arg Ile Gln Ser Glu Ile
       290                 295                 300
   Thr Asp Glu Ala Ile Ala Asn Gln Trp Glu Asn Lys Leu Glu Glu Phe
   305                 310                 315                 320
   Gly Val Ile Phe
<210> 33
   <211> 1766
   <212> DNA
   <213> Artificial Sequence
<220>
   <221> source
   <222> 1..1766
   <223> /mol_type="DNA"
   /note="MSBI2.176"
   /organism="Artificial Sequence"
<400> 33
   aagcttgctt agtcaaaaaa gtttgagcaa agcgaaaaca tagggcaatt ttcgtgatga      60
   aaatgggctt ttaatgtttt taaatgcttt taaatgcttt tagacatgct gaaacgctta     120
   tatagcaatg catacagagg acatatgtcc acgtttacct atcaatatgt ccacgtttac     180
   ctatcaatat gtccacgttt acctatcaat atgtccacgt ttaccttgcg tatttacaca     240
   catttaaata gtatggatat atccaatgaa attataatgt gtacttatga gcaaattagt     300
   agtgaaagac aatgccctaa tgaacgctag ttacaacttg gatctcgttg aacagcgttt     360
   aattctattg gcaatcatcg aagcaagaga atcaggcaaa ggaattaatg caaatgaccc     420
   gcttacggtt catgcagaga gttatatcaa tcaatttggt gttcatcgag taactgcata     480
   tcaagctctc aaagatgctt gtgataactt gtttgcacgt caattcagct accaatccaa     540
   aagtgaaaaa gggaacatac aaaatcatcg ttcacgttgg gttagtgaaa ttatttacat     600
   tgatacagaa gcaacagtaa aaataatatt tgcacctgct attgtcccac tgattacaag     660
   gctagaagaa cagttcacca agtatgatat tgagcaaatt agtgatttat cgagtgctta     720
   tgcaattcgc ttatacgagt tattgatttg ctggcgtagc acagggaaaa caccaattat     780
   tgggctaggc gaatttagaa atcgggttgg tgtgttagat agtgaatatc atcgaattgc     840
   acacttgaaa gaacgagtta ttgaacattc aattaaacag attaacgagc ataccgacat     900
   cacagccacc tacgaacagc acaaaaaagg gcggacaatc acaggatttt cattcaagtt     960
   taagcagaag aagcccaaac aagccgaaat tgctacagaa acgccaaaaa cagccacgaa    1020
   tgacccagac acgacaaaac cccttacaga gcctcagatc gcaaaataca gcatgattct    1080
   gtgcaaacta ggcagtattt cagacttgag taacttccca gactatccag cttttgcaaa    1140
   ttggattggg aacattttga ggaaccctga aaaagcagat gaacaaatag caaaacggat    1200
   tttcacagca ttgaaaacag aaaccgacta cagcaagaaa aactaatttt tagttgtgat    1260
   gggttttccc gaaataacat gaagggcgca cttacgcaaa atttttgcta cgccaaattt    1320
   tgcaagtacg gtcagggaaa ccccgacacc ccaaaagcaa aaacactgta atcagggaaa    1380
   aaacattttt gattttgatc cttgtttgtc actcgtagac actcgttttg ttttgctctt    1440
   tctagaattc acaaaaaaga tattagcgag tgtctacgag cgactcaatg aaagttcgat    1500
   tattccccct ctggaaaacc gcttttaaaa atattggctg ctagatggtt tttactatag    1560
   tgaggttttg cttttaaaaa aacacgagca aagcgagttc atagttgctt ttgcttgttt    1620
   tcgggtctta ggggaaatcc cctaacaagt cctcgaatat caaaatgtgg ctacattttg    1680
   tatatacggg taggcttgct tatttgattt tttttcttct aaacctttga cttcttcccc    1740
   attgtttgca gaaatgcccc ccgacc                                         1766
<210> 34
   <211> 1284
   <212> DNA
   <213> Artificial Sequence
<220>
   <221> source
   <222> 1..1284
   <223> /mol_type="DNA"
   /note="query"
   /organism="Artificial Sequence"
<400> 34
   agtcaaaaaa gtttgagcaa agcgaaaaca tagggcaatt ttcgtgatga aaatgggctt      60
   ttaatgtttt taaatgcttt taaatgcttt tagacatgct gaaacgctta tatagcaatg     120
   catacagagg acatatgtcc acgtttacct atcaatatgt ccacgtttac ctatcaatat     180
   gtccacgttt acctatcaat atgtccacgt ttaccttgcg tatttacaca catttaaata     240
   gtatggatat atccaatgaa attataatgt gtacttatga gcaaattagt agtgaaagac     300
   aatgccctaa tgaacgctag ttacaacttg gatctcgttg aacagcgttt aattctattg     360
   gcaatcatcg aagcaagaga atcaggcaaa ggaattaatg caaatgaccc gcttacggtt     420
   catgcagaga gttatatcaa tcaatttggt gttcatcgag taactgcata tcaagctctc     480
   aaagatgctt gtgataactt gtttgcacgt caattcagct accaatccaa aagtgaaaaa     540
   gggaacatac aaaatcatcg ttcacgttgg gttagtgaaa ttatttacat tgatacagaa     600
   gcaacagtaa aaataatatt tgcacctgct attgtcccac tgattacaag gctagaagaa     660
   cagttcacca agtatgatat tgagcaaatt agtgatttat cgagtgctta tgcaattcgc     720
   ttatacgagt tattgatttg ctggcgtagc acagggaaaa caccaattat tgggctaggc     780
   gaatttagaa atcgggttgg tgtgttagat agtgaatatc atcgaattgc acacttgaaa     840
   gaacgagtta ttgaacattc aattaaacag attaacgagc ataccgacat cacagccacc     900
   tacgaacagc acaaaaaagg gcggacaatc acaggatttt cattcaagtt taagcagaag     960
   aagcccaaac aagccgaaat tgctacagaa acgccaaaaa cagccacgaa tgacccagac    1020
   acgacaaaac cccttacaga gcctcagatc gcaaaataca gcatgattct gtgcaaacta    1080
   ggcagtattt cagacttgag taacttccca gactatccag cttttgcaaa ttggattggg    1140
   aacattttga ggaaccctga aaaagcagat gaacaaatag caaaacggat tttcacagca    1200
   ttgaaaacag aaaccgacta cagcaagaaa aactaatttt tagttgtgat gggttttccc    1260
   gaaataacat gaagggcgca ctta                                          1284
<210> 35
   <211> 1283
   <212> DNA
   <213> Artificial Sequence
<220>
   <221> source
   <222> 1..1283
   <223> /mol_type="DNA"
   /note="subject"
   /organism="Artificial Sequence"
<400> 35
   agtcaaaaaa gtttgagaga agcgaaaaca tagggcaatt tcatggtgag aatgggcttt      60
   taaggttttt aaaagctttt aaatgctttt agacatgctg aaacgaccac acagcaaggc     120
   atacagaggg catatgtcca cgtttaccta tcaatatgtc cacgtttacc tatcaatatg     180
   tccacgttta cctatcaata tgtccacgtt taccttgcat agtcacacat atttaaataa     240
   tatggatata tccaataaaa ttataatgtg tacttatgag taggttagta gtgaaagaca     300
   atgccctaat gaacgctagt tataacttgg atctcgttga acagcgttta atcctattgg     360
   caatcatcga agcgagggag tcaggcaaag gaattaacgc caatgaccct ctaacaatcc     420
   atgcagagag ctacatcaat caatttggtg ttcatcgagt aactgcatat caagctctca     480
   aagatgcttg tgataacctg tttgcacgtc aattcagcta tcagtccaaa agtgaaaaag     540
   gaaacataca aaatcatcgt tcacgctggg ttagtgaaat tatttacatc gacacagaag     600
   caacagtaaa aataatcttt gcacctgcta tcgtcccact gattacaaga ttagaagaac     660
   agttcaccaa gtatgatatt cagcaaatta gtgatttatc gagtgcttat gcaatccgct     720
   tatacgaatt attgatttgt tggcgtagca cagggaaaac gccaattatt gaattagctg     780
   aatttaggaa tcgggttggt gtgttagata ctgaatatca tcgaattgcc cacttgaaag     840
   agcgagttat tgaacattca attaaacaaa ttaacgaaca taccgacatc acagcgacct     900
   acgaacagca taaaaaaggg cgagtgatta cagggttctc attcaaattt aagcagaaga     960
   agcccaaaca agccgagatt gccacagaaa cgcccaaaac agccacgaat gacctagata    1020
   cgataaaacc ccttacagag ccacagatcg caaaatacag catgattctg tgcaaactag    1080
   gcagtatttc agacttgagt aacttcccag actatccagc ttttgcaaat tggattggga    1140
   acattttgag gaaccctgaa aaagcagatg aacaaatagc aaaacggatt ttcacagcat    1200
   tgaaaacaga aaccgactac agcaagaaaa actaattttt agttgtgatg ggttttcccg    1260
   aaataacatg aagggcgcac tta                                            1283
<210> 36
   <211> 125
   <212> DNA
   <213> Artificial Sequence
<220>
   <221> source
   <222> 1..125
   <223> /mol_type="DNA"
   /note="query"
   /organism="Artificial Sequence"
<400> 36
   cctaacaagt cctcgaatat caaaatgtgg ctacattttg tatatacggg taggcttgct     60
   tatttgattt tttttcttct aaacctttga cttcttcccc attgtttgca gaaatgcccc    120
   ccgac                                                                125
<210> 37
   <211> 125
   <212> DNA
   <213> Artificial Sequence
<220>
   <221> source
   <222> 1..125
   <223> /mol_type="DNA"
   /note="subject"
   /organism="Artificial Sequence"
<400> 37
   cctaacaagt ccccgaatat caaaatgtgg ctacattttg tatatacggg taggcttgct     60
   tattgaatct aattcctttt taaacctttc gactttccct atcctttgca taaattgccc    120
   ctgac                                                                125
<210> 38
   <211> 885
   <212> DNA
   <213> Artificial Sequence
<220>
   <221> source
   <222> 1..885
   <223> /mol_type="DNA"
   /note="query"
   /organism="Artificial Sequence"
<400> 38
   attaacgagc ataccgacat cacagccacc tacgaacagc acaaaaaagg gcggacaatc      60
   acaggatttt cattcaagtt taagcagaag aagcccaaac aagccgaaat tgctacagaa     120
   acgccaaaaa cagccacgaa tgacccagac acgacaaaac cccttacaga gcctcagatc     180
   gcaaaataca gcatgattct gtgcaaacta ggcagtattt cagacttgag taacttccca     240
   gactatccag cttttgcaaa ttggattggg aacattttga ggaaccctga aaaagcagat     300
   gaacaaatag caaaacggat tttcacagca ttgaaaacag aaaccgacta cagcaagaaa     360
   aactaatttt tagttgtgat gggttttccc gaaataacat gaagggcgca cttacgcaaa     420
   atttttgcta cgccaaattt tgcaagtacg gtcagggaaa ccccgacacc ccaaaagcaa     480
   aaacactgta atcagggaaa aaacattttt gattttgatc cttgtttgtc actcgtagac     540
   actcgttttg ttttgctctt tctagaattc acaaaaaaga tattagcgag tgtctacgag     600
   cgactcaatg aaagttcgat tattccccct ctggaaaacc gcttttaaaa atattggctg     660
   ctagatggtt tttactatag tgaggttttg cttttaaaaa aacacgagca aagcgagttc     720
   atagttgctt ttgcttgttt tcgggtctta ggggaaatcc cctaacaagt cctcgaatat     780
   caaaatgtgg ctacattttg tatatacggg taggcttgct tatttgattt tttttcttct     840
   aaacctttga cttcttcccc attgtttgca gaaatgcccc ccgac                     885
<210> 39
   <211> 890
   <212> DNA
   <213> Artificial Sequence
<220>
   <221> source
   <222> 1..890
   <223> /mol_type="DNA"
   /note="subject"
   /organism="Artificial Sequence"
<400> 39
   atcaacgagc acaccgacat cacagccacc tacgaacagc acaaaaaagg gcggacaatc 60
   acaggatttt cattcaagtt taagcagaag aagcccaaac aagccgaaat tgctacagaa 120
   acgccaaaaa cagccacgaa tgacccagac acgacaaaac cccttacaga gcctcagatc 180
   gcaaaataca gcatgattct gtgcaaacta ggcagtattt cagacttgag taacttccca 240
   gactatccag cttttgcaaa ttggattggg aacattttga ggaaccctga aaaagcagat 300
   gaacaaatag caaaacggat tttcacagca ttgaaaacag aaaccgacta cagcaagaaa 360
   aactaatttt tagttgtgat gggttttccc gaaataacat gaagggcgca cttagccatt 420
   gagaaaaatc ttcgattttt tcaatggaag tccgtggggg taaacccctc aaccccaaaa 480
   gcaaaaacac tgtaatcagg gaaaaaacat ttttgatttt gatccttgtt tgtcactcgt 540
   agacactcgt tttgttttgc tctttctaga attcacaaaa aagatattag cgagtgtcta 600
   cgagcgactc aatgaaagtt cgattattcc ccctctggaa aaccgctttt aaaaatattg 660
   gctgctagat ggtttttact atagtgaggt tttgctttta aaaaaacacg agcaaagcga 720
   gttcatagtt gcttttgctt gttttcgggt cttaggggaa atcccctaac aagtcctcga 780
   atatcaaaat gtggctacat tttgtatata cgggtaggct tgcttatttg attttttttc 840
   ttctaaacct ttgacttctt ccccattgtt tgcagaaatt gcccctcgac 890
<210> 40
   <211> 324
   <212> DNA
   <213> Artificial Sequence
<220>
   <221> source
   <222> 1..324
   <223> /mol_type="DNA"
   /note="query"
   /organism="Artificial Sequence"
<400> 40
   aagcttgctt agtcaaaaaa gtttgagcaa agcgaaaaca tagggcaatt ttcgtgatga 60
   aaatgggctt ttaatgtttt taaatgcttt taaatgcttt tagacatgct gaaacgctta 120
   tatagcaatg catacagagg acatatgtcc acgtttacct atcaatatgt ccacgtttac 180
   ctatcaatat gtccacgttt acctatcaat atgtccacgt ttaccttgcg tatttacaca 240
   catttaaata gtatggatat atccaatgaa attataatgt gtacttatga gcaaattagt 300
   agtgaaagac aatgccctaa tgaa 324
<210> 41
   <211> 324
   <212> DNA
   <213> Artificial Sequence
<220>
   <221> source
   <222> 1..324
   <223> /mol_type="DNA"
   /note="subject"
   /organism="Artificial Sequence"
<400> 41
   aagcttgctt agtcaaaaaa gtttgagcaa agcgaaaaca tagggcaatt ttcgtgatga 60
   aaatgggctt ttaatgtttt taaatgcttt taaatgcttt tagacatgct gaaacgctta 120
   tatagcaatg catacagagg acatatgtcc acgtttacct atcaatatgt ccacgtttac 180
   ctatcaatat gtccacgttt acctatcaat atgtccacgt ttaccttgcg tatttacaca 240
   catttaaata gtatggatat atccaatgag attataatgt gtacttatga gcaaattagt 300
   agtgaaagac aatgccctaa tcaa 324
<210> 42
   <211> 319
   <212> PRT
   <213> Artificial Sequence
<220>
   <221> SOURCE
   <222> 1..319
   <223> /mol_type="protein"
   /note="query"
   /organism="Artificial Sequence"
<400> 42
   Met Ser Lys Leu Val Val Lys Asp Asn Ala Leu Met Asn Ala Ser Tyr
   1               5                   10                  15
   Asn Leu Asp Leu Val Glu Gln Arg Leu Ile Leu Leu Ala Ile Ile Glu
               20                  25                  30
   Ala Arg Glu Ser Gly Lys Gly Ile Asn Ala Asn Asp Pro Leu Thr Val
           35                  40                  45
   His Ala Glu Ser Tyr Ile Asn Gln Phe Gly Val His Arg Val Thr Ala
       50                  55                  60
   Tyr Gln Ala Leu Lys Asp Ala Cys Asp Asn Leu Phe Ala Arg Gln Phe
   65                  70                  75                  80
   Ser Tyr Gln Ser Lys Ser Glu Lys Gly Asn Ile Gln Asn His Arg Ser
                   85                  90                  95
   Arg Trp Val Ser Glu Ile Ile Tyr Ile Asp Thr Glu Ala Thr Val Lys
               100                 105                 110
   Ile Ile Phe Ala Pro Ala Ile Val Pro Leu Ile Thr Arg Leu Glu Glu
           115                 120                 125
   Gln Phe Thr Lys Tyr Asp Ile Glu Gln Ile Ser Asp Leu Ser Ser Ala
       130                 135                 140
   Tyr Ala Ile Arg Leu Tyr Glu Leu Leu Ile Cys Trp Arg Ser Thr Gly
   145                 150                 155                 160
   Lys Thr Pro Ile Ile Gly Leu Gly Glu Phe Arg Asn Arg Val Gly Val
                   165                 170                 175
   Leu Asp Ser Glu Tyr His Arg Ile Ala His Leu Lys Glu Arg Val Ile
               180                 185                 190
   Glu His Ser Ile Lys Gln Ile Asn Glu His Thr Asp Ile Thr Ala Thr
           195                 200                 205
   Tyr Glu Gln His Lys Lys Gly Arg Thr Ile Thr Gly Phe Ser Phe Lys
       210                 215                 220
   Phe Lys Gln Lys Lys Pro Lys Gln Ala Glu Ile Ala Thr Glu Thr Pro
   225                 230                 235                 240
   Lys Thr Ala Thr Asn Asp Pro Asp Thr Thr Lys Pro Leu Thr Glu Pro
                   245                 250                 255
   Gln Ile Ala Lys Tyr Ser Met Ile Leu Cys Lys Leu Gly Ser Ile Ser
               260                 265                 270
   Asp Leu Ser Asn Phe Pro Asp Tyr Pro Ala Phe Ala Asn Trp Ile Gly
           275                 280                 285
   Asn Ile Leu Arg Asn Pro Glu Lys Ala Asp Glu Gln Ile Ala Lys Arg
       290                 295                 300
   Ile Phe Thr Ala Leu Lys Thr Glu Thr Asp Tyr Ser Lys Lys Asn
   305                 310                 315
<210> 43
   <211> 312
   <212> PRT
   <213> Artificial Sequence
<220>
   <221> SOURCE
   <222> 1..312
   <223> /mol_type="protein"
   /note="subject"
   /organism="Artificial Sequence"
<400> 43
   Met Arg Glu Leu Val Val Lys Asp Asn Ala Leu Ile Asn Ala Ser Tyr
   1 5 10 15
   Asn Leu Asp Leu Val Glu Gln Arg Leu Ile Leu Leu Ala Ile Val Glu
   20 25 30
   Ala Arg Glu Ser Gly Lys Gly Ile Asn Ala Asn Asp Pro Leu Val Val
   35 40 45
   His Ala Glu Ser Tyr Ile Asn Gln Phe Asn Val His Arg Asn Thr Ala
   50 55 60
   Tyr Gln Ala Leu Lys Asp Ala Cys His Asp Leu Phe Val Arg Gln Phe
   65 70 75 80
   Ser Tyr Gln Lys Val Asn Asp Arg Gly Asn Val Glu His Tyr Arg Ser
   85 90 95
   Arg Trp Val Ser Glu Ile Gly Tyr Val Asp Asn Glu Ala Ile Val Lys
   100 105 110
   Leu Ile Phe Ala Pro Ala Ile Val Pro Leu Ile Thr Arg Leu Glu Glu
   115 120 125
   Gln Phe Thr Lys Tyr Glu Leu Gln Gln Val Ser Gln Leu Thr Ser Ala
   130 135 140
   Tyr Ala Val Arg Leu Tyr Glu Leu Leu Ile Ala Trp Arg Ser Thr Gly
   145 150 155 160
   Lys Thr Pro Val Ile Glu Leu Ala Asp Phe Arg Lys Arg Ile Gly Ile
   165 170 175
   Leu Glu Thr Glu Tyr Lys Arg Met Glu Arg Phe Lys Thr Ser Val Leu
   180 185 190
   Glu Leu Ala Ile Asn Gln Ile Asn Glu His Thr Asp Ile Asn Val Ala
   195 200 205
   Tyr Glu Gln His Lys Lys Gly Arg Ser Ile Val Gly Phe Ser Phe Asn
   210 215 220
   Phe Ser Gln Lys Glu Lys Lys Lys Ile Leu Glu Lys Ala Gln Val Ser
   225 230 235 240
   Glu Gly Phe Lys Lys Leu Thr Glu Ala Gln Ile Thr Lys Tyr Ser Thr
   245 250 255
   Val Leu Ser Lys Leu His Glu Leu Ser Asp Leu Ser Thr Phe Gln Asp
   260 265 270
   Tyr Gln Ser Phe Ser Ile Trp Ile Gly Asn Ile Leu Arg Glu Pro Glu
   275 280 285
   Ser Val Arg Phe Glu Thr Ala Glu Arg Ile Phe Ser Ser Leu Phe Lys
   290 295 300
   Arg Thr Asp Phe Ala Ser Pro Asn
   305 310
<210> 44
   <211> 319
   <212> PRT
   <213> Artificial Sequence
<220>
   <221> SOURCE
   <222> 1..319
   <223> /mol_type="protein"
   /note="query"
   /organism="Artificial Sequence"
<400> 44
   Met Ser Lys Leu Val Val Lys Asp Asn Ala Leu Met Asn Ala Ser Tyr
   1 5 10 15
   Asn Leu Asp Leu Val Glu Gln Arg Leu Ile Leu Leu Ala Ile Ile Glu
               20                  25                  30
   Ala Arg Glu Ser Gly Lys Gly Ile Asn Ala Asn Asp Pro Leu Thr Val
           35                  40                  45
   His Ala Glu Ser Tyr Ile Asn Gln Phe Gly Val His Arg Val Thr Ala
       50                  55                  60
   Tyr Gln Ala Leu Lys Asp Ala Cys Asp Asn Leu Phe Ala Arg Gln Phe
   65                  70                  75                  80
   Ser Tyr Gln Ser Lys Ser Glu Lys Gly Asn Ile Gln Asn His Arg Ser
                   85                  90                  95
   Arg Trp Val Ser Glu Ile Ile Tyr Ile Asp Thr Glu Ala Thr Val Lys
               100                 105                 110
   Ile Ile Phe Ala Pro Ala Ile Val Pro Leu Ile Thr Arg Leu Glu Glu
           115                 120                 125
   Gln Phe Thr Lys Tyr Asp Ile Glu Gln Ile Ser Asp Leu Ser Ser Ala
       130                 135                 140
   Tyr Ala Ile Arg Leu Tyr Glu Leu Leu Ile Cys Trp Arg Ser Thr Gly
   145                 150                 155                 160
   Lys Thr Pro Ile Ile Gly Leu Gly Glu Phe Arg Asn Arg Val Gly Val
                   165                 170                 175
   Leu Asp Ser Glu Tyr His Arg Ile Ala His Leu Lys Glu Arg Val Ile
               180                 185                 190
   Glu His Ser Ile Lys Gln Ile Asn Glu His Thr Asp Ile Thr Ala Thr
           195                 200                 205
   Tyr Glu Gln His Lys Lys Gly Arg Thr Ile Thr Gly Phe Ser Phe Lys
       210                 215                 220
   Phe Lys Gln Lys Lys Pro Lys Gln Ala Glu Ile Ala Thr Glu Thr Pro
   225                 230                 235                 240
   Lys Thr Ala Thr Asn Asp Pro Asp Thr Thr Lys Pro Leu Thr Glu Pro
                   245                 250                 255
   Gln Ile Ala Lys Tyr Ser Met Ile Leu Cys Lys Leu Gly Ser Ile Ser
               260                 265                 270
   Asp Leu Ser Asn Phe Pro Asp Tyr Pro Ala Phe Ala Asn Trp Ile Gly
           275                 280                 285
   Asn Ile Leu Arg Asn Pro Glu Lys Ala Asp Glu Gln Ile Ala Lys Arg
       290                 295                 300
   Ile Phe Thr Ala Leu Lys Thr Glu Thr Asp Tyr Ser Lys Lys Asn
   305                 310                 315
<210> 45
   <211> 312
   <212> PRT
   <213> Artificial Sequence
<220>
   <221> SOURCE
   <222> 1..312
   <223> /mol_type="protein"
   /note="subject"
   /organism="Artificial Sequence"
<400> 45
   Met Arg Glu Leu Val Val Lys Asp Asn Ala Leu Ile Asn Ala Ser Tyr
   1               5                   10                  15
   Asn Leu Asp Leu Val Glu Gln Arg Leu Ile Leu Leu Ala Ile Val Glu
               20                  25                  30
   Ala Arg Glu Ser Gly Lys Gly Ile Asn Ala Asn Asp Pro Leu Val Val
           35                  40                  45
   His Ala Glu Ser Tyr Ile Asn Gln Phe Asn Val His Arg Asn Thr Ala
       50                  55                  60
   Tyr Gln Ala Leu Lys Asp Ala Cys His Asp Leu Phe Val Arg Gln Phe
   65                  70                  75                  80
   Ser Tyr Gln Lys Val Asn Asp Arg Gly Asn Val Glu His Tyr Arg Ser
                   85                  90                  95
   Arg Trp Val Ser Glu Ile Gly Tyr Val Asp Asn Glu Ala Ile Val Lys
               100                 105                 110
   Leu Ile Phe Ala Pro Ala Ile Val Pro Leu Ile Thr Arg Leu Glu Glu
           115                 120                 125
   Gln Phe Thr Lys Tyr Glu Leu Gln Gln Val Ser Gln Leu Thr Ser Ala
       130                 135                 140
   Tyr Ala Val Arg Leu Tyr Glu Leu Leu Ile Ala Trp Arg Ser Thr Gly
   145                 150                 155                 160
   Lys Thr Pro Val Ile Glu Leu Ala Asp Phe Arg Lys Arg Ile Gly Ile
                   165                 170                 175
   Leu Glu Thr Glu Tyr Lys Arg Met Glu Arg Phe Lys Thr Ser Val Leu
               180                 185                 190
   Glu Leu Ala Ile Asn Gln Ile Asn Glu His Thr Asp Ile Asn Val Ala
           195                 200                 205
   Tyr Glu Gln His Lys Lys Gly Arg Ser Ile Val Gly Phe Ser Phe Asn
       210                 215                 220
   Phe Ser Gln Lys Glu Lys Lys Lys Ile Leu Glu Lys Ala Gln Val Ser
   225                 230                 235                 240
   Glu Gly Phe Lys Lys Leu Thr Glu Ala Gln Ile Thr Lys Tyr Ser Thr
                   245                 250                 255
   Val Leu Ser Lys Leu His Glu Leu Ser Asp Leu Ser Thr Phe Gln Asp
               260                 265                 270
   Tyr Gln Ser Phe Ser Ile Trp Ile Gly Asn Ile Leu Arg Glu Pro Glu
           275                 280                 285
   Ser Val Arg Phe Glu Thr Ala Glu Arg Ile Phe Ser Ser Leu Phe Lys
       290                 295                 300
   Arg Thr Asp Phe Ala Ser Pro Asn
   305                 310
<210> 46
   <211> 2521
   <212> DNA
   <213> Artificial Sequence
<220>
   <221> source
   <222> 1..2521
   <223> /mol_type="DNA"
   /note="CMI1.252"
   /organism="Artificial Sequence"
<400> 46
   ttaaaaatat tggttgctag atggttttaa ctatcgtgga gcttttgctt ttaaaaatac      60
   acgaagcaaa gcgagttcat agtcgctttt gatctgctct tactaacagc tcatttttaa     120
   tgggccgtta gtaagagatg cttttggggt ttcggggctt gccctgaaca aggtcacaaa     180
   gtgggaattt atcacggtag tgaaaaagta ccctttgtgt atccttgctt tatgcttttt     240
   aaacctttgg acttttccct taatttgcag aaattgcccc tcgactaagc aagcttagtc     300
   aaaaaagttt gagcaaagcg aaaacatagg gcaattttca tgatgaaaat gggcttttaa     360
   agtttttaaa tgcttttaaa tgcttttaga catgttgaaa tgcaagccta gcaaggcata     420
   cagaggacat acccctacgt ttaccgatca atacccctac gtttaccgat caatacccct   480
   acgtttaccg atcaataccc ctacgtttac cttgcatatc actacaaaca atattaatgt     540
   agtaataact tcaaaataat aaatgtaggt tatgagcgat ttaatagtaa aggataacgc     600
   cctaatgaac gctagttaca acttagcttt ggttgaacag cgtttaatcc ttttagctat     660
   attggaagcg agagaaacag gaaaagggat taatgccaat gaccccttaa cagttcatgc      720
   aagtagctat atcaatcaat ttaacgtgga aagacatacg gcatatcaag cattaaaaga     780
   tgcttgcaaa gacttgtttg cacgtcaatt tagttaccaa gaaaagcgag aacgaggacg     840
   aattaatatt acaagtcgat gggtttcaca aattggttat atggacgata cagcaaccgt     900
   tgaaattatt tttgcccctg cggttgttcc tttgattaca cgtttagagg aacaattcac     960
   tcaatacgat attgaacaaa ttagcgagtt atcgagtgct tatgctgtcc gcctatatga    1020
   gttactaatt tgttggcgta gcacaggaaa aaccccaatt attgatctaa cagagtttag    1080
   aaagcgtcta ggcgttttag ataccgaata cactaggaca gataacttga agatgcgagt    1140
   gattgagcta ggacttaaac aaatcaacga gcatacggac attacagcaa gctacgaaca    1200
   gcacaaaaaa gggcggacaa tcacaggatt ctcattcaag tttaagcaga agaagaaaac    1260
   aggggcggaa atgccaaaaa atagcgattc tagcccacat atcgaaaaac ctagtcaaat    1320
   ccctgccaac atcgcaaaac agcctgaaaa tgccaaaaaa gatgatttag ggcacagagc    1380
   aagcaagatt acagggttaa ttatgtcgaa tggtttagca gatcggttta aacgtggtga    1440
   tgagtcagta atagacatga tgaaacgcat cgaggaaatt acaactgata caacagcaga    1500
   ccaatgggaa aacaagctag aagaatttgg cgtgattttt cagtcatgat caatttcccg    1560
   aaggacatat aaaaaataat gtagttaaat atagtagtta taatattgac tacaatattt    1620
   aactactata ttgtagtctt aaatcaactt acatagaatt taaattatgt ctcttcgcca    1680
   aattttagaa caatctctct ttgatcaaac tgatgaaagc tctactgtgc cactatcaat    1740
   tagacttcca aacaacttga ataatgagct tgaagaactg actataacac tagataaatc    1800
   caagagtttt ctactcctag agttcattaa ggctggaatt agtgagacta gcaatatgtt    1860
   agaagacaaa gcgaagaacc ctatacttga aagtccaaga gattctagtg actttacaac    1920
   tcataagaag tttatgttga acactaattt caataacaat aaagcatcac acatgactat    1980
   gttggagaat caagaagctg ctgcatttcg taaaggttgg aaagaatata tctgtaattt    2040
   agaagaaggc gataaggttt acctttatca aagtggcgtt ggttttgtag catctggtat    2100
   tgtcactggt gacttaatca aaagtgagta tcgtggacaa ccagaagaaa aatattctaa    2160
   aaaattaaag gattttaaaa taggatttaa agcaatttct gcaaaagagt ttaagacctt    2220
   aactggcggt ggtgctagct ttcgtacaac aatgagcgag ctaaagcctg aacaatgtag    2280
   aaaattagat atggaaatag aaaaacggat gaagtcagta agttaaaaat atgagttgtt    2340
   gttttagtgt gtcacgtagc aatcacttag aagttccctt tgtattactt gtctagcaat    2400
   ggttttaaaa agcgtcacta ctgatagtga cgcttttttt gtttttcaaa ggaagtaatt    2460
   agcgagtgtc tacgagcgac acaatgaaag ttcgattatt ccctcctcta aaaaaccgct    2520
   t 2521
<210> 47
   <211> 1328
   <212> DNA
   <213> Artificial Sequence
<220>
   <221> source
   <222> 1..1328
   <223> /mol_type="DNA"
   /note="query"
   /organism="Artificial Sequence"
<400> 47
   ttttaaacct ttggactttt cccttaattt gcagaaattg cccctcgact aagcaagctt      60
   agtcaaaaaa gtttgagcaa agcgaaaaca tagggcaatt ttcatgatga aaatgggctt     120
   ttaaagtttt taaatgcttt taaatgcttt tagacatgtt gaaatgcaag cctagcaagg     180
   catacagagg acatacccct acgtttaccg atcaataccc ctacgtttac cgatcaatac     240
   ccctacgttt accgatcaat acccctacgt ttaccttgca tatcactaca aacaatatta     300
   atgtagtaat aacttcaaaa taataaatgt aggttatgag cgatttaata gtaaaggata     360
   acgccctaat gaacgctagt tacaacttag ctttggttga acagcgttta atccttttag     420
   ctatattgga agcgagagaa acaggaaaag ggattaatgc caatgacccc ttaacagttc     480
   atgcaagtag ctatatcaat caatttaacg tggaaagaca tacggcatat caagcattaa     540
   aagatgcttg caaagacttg tttgcacgtc aatttagtta ccaagaaaag cgagaacgag     600
   gacgaattaa tattacaagt cgatgggttt cacaaattgg ttatatggac gatacagcaa     660
   ccgttgaaat tatttttgcc cctgcggttg ttcctttgat tacacgttta gaggaacaat     720
   tcactcaata cgatattgaa caaattagcg agttatcgag tgcttatgct gtccgcctat     780
   atgagttact aatttgttgg cgtagcacag gaaaaacccc aattattgat ctaacagagt     840
   ttagaaagcg tctaggcgtt ttagataccg aatacactag gacagataac ttgaagatgc     900
   gagtgattga gctaggactt aaacaaatca acgagcatac ggacattaca gcaagctacg     960
   aacagcacaa aaaagggcgg acaatcacag gattctcatt caagtttaag cagaagaaga    1020
   aaacaggggc ggaaatgcca aaaaatagcg attctagccc acatatcgaa aaacctagtc    1080
   aaatccctgc caacatcgca aaacagcctg aaaatgccaa aaaagatgat ttagggcaca    1140
   gagcaagcaa gattacaggg ttaattatgt cgaatggttt agcagatcgg tttaaacgtg    1200
   gtgatgagtc agtaatagac atgatgaaac gcatcgagga aattacaact gatacaacag    1260
   cagaccaatg ggaaaacaag ctagaagaat ttggcgtgat ttttcagtca tgatcaattt    1320
   cccgaagg                                                             1328
<210> 48
   <211> 1324
   <212> DNA
   <213> Artificial Sequence
<220>
   <221> source
   <222> 1..1324
   <223> /mol_type="DNA"
   /note="subject"
   /organism="Artificial Sequence"
<400> 48
   ttctaaacct ttgacttctt ccccattgtt tgcagaaatt gcccctcgaa gcttgcttag 60
   tcaaaaaagt ttgagcaaag cgaaaacata gggcaatttt catgaagaaa ttgggctttt 120
   aaagttttta aatgttttaa atgcttttag acatgctaag aagcccacac agcaaggcat     180
   acagaggaca ttctcctacg tttaccgatc aatacccccg tttaccgatc aataccccta     240
   cgtttaccga tcaatacccc tacgtttacc ttgcgtataa ctacaaagaa tactagtgta 300
   gtataacttc aaaagaataa ttgtaggtta tgagcgattt aatagtaaaa gataacgccc 360
   taatgaatgc tagttataac ttagctttgg ttgaacagag gttaattcta ttagcaatca 420
   tagaagcgag agaaacaggc aaagggatta atgccaatga tcctcttacg gttcatgcag 480
   gtagctatat caatcaattt aacgtacaaa ggcatacggc atatcaagcc ctcaaagatg 540
   cttgtaaaga cttgtttgcc cgtcaattca gttaccaaga aaagcgagaa cgaggacgaa 600
   ttaatattac aagtcgatgg gtttcgcaaa ttggctatat ggacgataca gcaaccgttg 660
   agattatttt tgcccctgcg gttgttcctc tgattacacg gctagaggaa cagttcaccc 720
   agtacgatat tgagcaaatt agcggtttat cgagtgcata tgctgttcgt atgtacgaac 780
   tgctgatttg ttggcgtagc acaggcaaaa caccaattat tgagctagac gagtttagaa 840
   agcgaatagg tgttttagat actgaataca ctagaacaga taatttaaag atgcaagtta 900
   ttgaattagc cctaaaacaa atcaacgaac atactgacat cacagcaagc tatgaacaac 960
   acaaaaaagg gcgagtgatt accggattct cattcatgtt taagcacaag aaacaaaaca 1020
   gcgataaaac gcctgatact aacgcttcta gcccacgtat cgtaaaacat agtcaaatcc 1080
   ctaccaacat tgtaaaacag cctgaaaacg ccaaaatgag cgatttagaa catagagcga 1140
   gccgtgttac aggggaaata atgcgaaatc gtctgtcaga tcggtttaaa caaggcgatg 1200
   aatcagcaat cgacatgatg aaacgtattc aaagtgaaat aataaccgat gcaatagcag 1260
   accagtggga aagcaaactg gaggagtttg gcgtggtttt ttagtcatga cgatttcccg 1320
   aagg 1324
<210> 49
   <211> 91
   <212> DNA
   <213> Artificial Sequence
<220>
   <221> source
   <222> 1..91
   <223> /mol_type="DNA"
   /note="query"
   /organism="Artificial Sequence"
<400> 49
   ttaaaaatat tggttgctag atggttttaa ctatcgtgga gcttttgctt ttaaaaatac 60
   acgaagcaaa gcgagttcat agtcgctttt g 91
<210> 50
   <211> 89
   <212> DNA
   <213> Artificial Sequence
<220>
   <221> source
   <222> 1..89
   <223> /mol_type="DNA"
   /note="subject"
   /organism="Artificial Sequence"
<400> 50
   ttaaaaatat tggctgctag atggttttta ctatagtgag gttttgcttt taaaaaaaca 60
   cgagcaaagc gagttcatag ttgcttttg 89
<210> 51
   <211> 64
   <212> DNA
   <213> Artificial Sequence
<220>
   <221> source
   <222> 1..64
   <223> /mol_type="DNA"
   /note="query"
   /organism="Artificial Sequence"
<400> 51
   attagcgagt gtctacgagc gacacaatga aagttcgatt attccctcct ctaaaaaacc 60
   gctt 64
<210> 52
   <211> 63
   <212> DNA
   <213> Artificial Sequence
<220>
   <221> source
   <222> 1..63
   <223> /mol_type="DNA"
   /note="subject"
   /organism="Artificial Sequence"
<400> 52
   attagcgagt gtctacgagc gactcaatga aagttcgatt attccccctc tggaaaaccg 60
   ctt 63
<210> 53
   <211> 304
   <212> PRT
   <213> Artificial Sequence
<220>
   <221> SOURCE
   <222> 1..304
   <223> /mol_type="protein"
   /note="query"
   /organism="Artificial Sequence"
<220>
   <221> VARIANT
   <222> 301
   <223> unknown or other
<400> 53
   Met Ser Asp Leu Ile Val Lys Asp Asn Ala Leu Met Asn Ala Ser Tyr
   1 5 10 15
   Asn Leu Ala Leu Val Glu Gln Arg Leu Ile Leu Leu Ala Ile Leu Glu
   20 25 30
   Ala Arg Glu Thr Gly Lys Gly Ile Asn Ala Asn Asp Pro Leu Thr Val
   35 40 45
   His Ala Ser Ser Tyr Ile Asn Gln Phe Asn Val Glu Arg His Thr Ala
   50 55 60
   Tyr Gln Ala Leu Lys Asp Ala Cys Lys Asp Leu Phe Ala Arg Gln Phe
   65 70 75 80
   Ser Tyr Gln Glu Lys Arg Glu Arg Gly Arg Ile Asn Ile Thr Ser Arg
   85 90 95
   Trp Val Ser Gln Ile Gly Tyr Met Asp Asp Thr Ala Thr Val Glu Ile
   100 105 110
   Ile Phe Ala Pro Ala Val Val Pro Leu Ile Thr Arg Leu Glu Glu Gln
   115 120 125
   Phe Thr Gln Tyr Asp Ile Glu Gln Ile Ser Glu Leu Ser Ser Ala Tyr
   130 135 140
   Ala Val Arg Leu Tyr Glu Leu Leu Ile Cys Trp Arg Ser Thr Gly Lys
   145 150 155 160
   Thr Pro Ile Ile Asp Leu Thr Glu Phe Arg Lys Arg Leu Gly Val Leu
   165 170 175
   Asp Thr Glu Tyr Thr Arg Thr Asp Asn Leu Lys Met Arg Val Ile Glu
   180 185 190
   Leu Gly Leu Lys Gln Ile Asn Glu His Thr Asp Ile Thr Ala Ser Tyr
   195 200 205
   Glu Gln His Lys Lys Gly Arg Thr Ile Thr Gly Phe Ser Phe Lys Phe
   210 215 220
   Lys Gln Lys Lys Lys Thr Gly Ala Glu Met Pro Lys Asn Ser Asp Ser
   225 230 235 240
   Ser Pro His Ile Glu Lys Pro Ser Gln Ile Pro Ala Asn Ile Ala Lys
   245 250 255
   Gln Pro Glu Asn Ala Lys Lys Asp Asp Leu Gly His Arg Ala Ser Lys
   260 265 270
   Ile Thr Gly Leu Ile Met Ser Asn Gly Leu Ala Asp Arg Phe Lys Arg
   275 280 285
   Gly Asp Glu Ser Val Ile Asp Met Met Lys Arg Ile Xaa Arg Lys Leu
   290 295 300
<210> 54
   <211> 304
   <212> PRT
   <213> Artificial Sequence
<220>
   <221> SOURCE
   <222> 1..304
   <223> /mol_type="protein"
   /note="subject"
   /organism="Artificial Sequence"
<400> 54
   Met Ser Asp Leu Ile Val Lys Asp Asn Ala Leu Met Asn Ala Ser Tyr
   1               5                   10                  15
   Asn Leu Ala Leu Val Glu Gln Arg Leu Ile Leu Leu Ala Ile Ile Glu
               20                  25                  30
   Ala Arg Glu Thr Gly Lys Gly Ile Asn Ala Asn Asp Pro Leu Thr Val
           35                  40                  45
   His Ala Gly Ser Tyr Ile Asn Gln Phe Asn Val Gln Arg His Thr Ala
       50                  55                  60
   Tyr Gln Ala Leu Lys Asp Ala Cys Lys Asp Leu Phe Ala Arg Gln Phe
   65                  70                  75                  80
   Ser Tyr Gln Glu Lys Arg Glu Arg Gly Arg Ile Asn Ile Thr Ser Arg
                   85                  90                  95
   Trp Val Ser Gln Ile Gly Tyr Met Asp Asp Thr Ala Thr Val Glu Ile
               100                 105                 110
   Ile Phe Ala Pro Ala Val Val Pro Leu Ile Thr Arg Leu Glu Glu Gln
           115                 120                 125
   Phe Thr Gln Tyr Asp Ile Glu Gln Ile Ser Gly Leu Ser Ser Ala Tyr
       130                 135                 140
   Ala Val Arg Met Tyr Glu Leu Leu Ile Cys Trp Arg Ser Thr Gly Lys
   145                 150                 155                 160
   Thr Pro Ile Ile Glu Leu Asp Glu Phe Arg Lys Arg Ile Gly Val Leu
                   165                 170                 175
   Asp Thr Glu Tyr Thr Arg Thr Asp Asn Leu Lys Met Gln Val Ile Glu
               180                 185                 190
   Leu Ala Leu Lys Gln Ile Asn Glu His Thr Asp Ile Thr Ala Ser Tyr
           195                 200                 205
   Glu Gln His Lys Lys Gly Arg Val Ile Thr Gly Phe Ser Phe Met Phe
       210                 215                 220
   Lys His Lys Lys Gln Asn Ser Asp Lys Thr Pro Asp Thr Asn Ala Ser
   225                 230                 235                 240
   Ser Pro Arg Ile Val Lys His Ser Gln Ile Pro Thr Asn Ile Val Lys
                   245                 250                 255
   Gln Pro Glu Asn Ala Lys Met Ser Asp Leu Glu His Arg Ala Ser Arg
               260                 265                 270
   Val Thr Gly Glu Ile Met Arg Asn Arg Leu Ser Asp Arg Phe Lys Gln
           275                 280                 285
   Gly Asp Glu Ser Ala Ile Asp Met Met Lys Arg Ile Gln Ser Glu Ile
       290                 295                 300
<210> 55
   <211> 304
   <212> PRT
   <213> Artificial Sequence
<220>
   <221> SOURCE
   <222> 1..304
   <223> /mol_type="protein"
   /note="query"
   /organism="Artificial Sequence"
<220>
   <221> UNSURE
   <222> 301
   <223> unknown or other
<400> 55
   Met Ser Asp Leu Ile Val Lys Asp Asn Ala Leu Met Asn Ala Ser Tyr
   1               5                   10                  15
   Asn Leu Ala Leu Val Glu Gln Arg Leu Ile Leu Leu Ala Ile Leu Glu
               20                  25                  30
   Ala Arg Glu Thr Gly Lys Gly Ile Asn Ala Asn Asp Pro Leu Thr Val
           35                  40                  45
   His Ala Ser Ser Tyr Ile Asn Gln Phe Asn Val Glu Arg His Thr Ala
       50                  55                  60
   Tyr Gln Ala Leu Lys Asp Ala Cys Lys Asp Leu Phe Ala Arg Gln Phe
   65                  70                  75                  80
   Ser Tyr Gln Glu Lys Arg Glu Arg Gly Arg Ile Asn Ile Thr Ser Arg
                   85                  90                  95
   Trp Val Ser Gln Ile Gly Tyr Met Asp Asp Thr Ala Thr Val Glu Ile
               100                 105                 110
   Ile Phe Ala Pro Ala Val Val Pro Leu Ile Thr Arg Leu Glu Glu Gln
           115                 120                 125
   Phe Thr Gln Tyr Asp Ile Glu Gln Ile Ser Glu Leu Ser Ser Ala Tyr
       130                 135                 140
   Ala Val Arg Leu Tyr Glu Leu Leu Ile Cys Trp Arg Ser Thr Gly Lys
   145                 150                 155                 160
   Thr Pro Ile Ile Asp Leu Thr Glu Phe Arg Lys Arg Leu Gly Val Leu
                   165                 170                 175
   Asp Thr Glu Tyr Thr Arg Thr Asp Asn Leu Lys Met Arg Val Ile Glu
               180                 185                 190
   Leu Gly Leu Lys Gln Ile Asn Glu His Thr Asp Ile Thr Ala Ser Tyr
           195                 200                 205
   Glu Gln His Lys Lys Gly Arg Thr Ile Thr Gly Phe Ser Phe Lys Phe
       210                 215                 220
   Lys Gln Lys Lys Lys Thr Gly Ala Glu Met Pro Lys Asn Ser Asp Ser
   225                 230                 235                 240
   Ser Pro His Ile Glu Lys Pro Ser Gln Ile Pro Ala Asn Ile Ala Lys
                   245                 250                 255
   Gln Pro Glu Asn Ala Lys Lys Asp Asp Leu Gly His Arg Ala Ser Lys
               260                 265                 270
   Ile Thr Gly Leu Ile Met Ser Asn Gly Leu Ala Asp Arg Phe Lys Arg
           275                 280                 285
   Gly Asp Glu Ser Val Ile Asp Met Met Lys Arg Ile Xaa Arg Lys Leu
       290                 295                 300
<210> 56
   <211> 304
   <212> PRT
   <213> Artificial Sequence
<220>
   <221> SOURCE
   <222> 1..304
   <223> /mol_type="protein"
   /note="subject"
   /organism="Artificial Sequence"
<400> 56
   Met Ser Glu Leu Ile Val Lys Asp Asn Ala Leu Met Asn Ala Ser Tyr
   1               5                   10                  15
   Asn Leu Asp Leu Val Glu Gln Arg Leu Ile Leu Leu Ala Ile Val Glu
               20                  25                  30
   Ala Arg Glu Ser Gly Lys Gly Ile Asn Ala Asn Asp Pro Leu Thr Val
           35                  40                  45
   His Ala Glu Ser Tyr Ile Asn Gln Phe Gly Val His Arg Thr Thr Ala
       50                  55                  60
   Tyr Gln Ala Leu Lys Asp Ala Cys Lys Asp Leu Phe Ala Arg Gln Phe
   65                  70                  75                  80
   Ser Tyr Gln Glu Lys Arg Glu Arg Gly Arg Ala Asn Ile Thr Ser Arg
                   85                  90                  95
   Trp Val Ser Gln Ile Ala Tyr Ile Asp Glu Thr Ala Thr Val Glu Ile
               100                 105                 110
   Ile Phe Ala Pro Ala Val Val Pro Leu Ile Thr Arg Leu Glu Lys Gln
           115                 120                 125
   Phe Thr Gln Tyr Asp Ile Glu Gln Ile Ser Gly Leu Ser Ser Ala Tyr
       130                 135                 140
   Ala Val Arg Met Tyr Glu Leu Leu Ile Cys Trp Arg Ser Thr Gly Lys
   145                 150                 155                 160
   Thr Pro Val Ile Glu Leu Gly Glu Phe Arg Lys Arg Ile Gly Val Leu
                   165                 170                 175
   Asp Thr Glu Tyr Ile Arg Thr Asp Asn Leu Lys Met Arg Val Ile Glu
               180                 185                 190
   Leu Ala Leu Lys Gln Ile Asn Asp His Thr Asp Ile Thr Ala Thr Tyr
           195                 200                 205
   Glu Gln His Lys Lys Gly Arg Thr Ile Thr Gly Phe Ser Phe Lys Phe
       210                 215                 220
   Lys Gln Lys Arg Lys Thr Glu Leu Glu Thr Pro Lys Asn Ser Asp Ser
   225                 230                 235                 240
   Asp Val Pro Lys Gln Lys Ser Val Glu Ile Pro Thr Asn Leu Val Lys
                   245                 250                 255
   Gln Pro Glu Asn Ala Asn Met Ser Asp Leu Gln His Arg Ala Ser Lys
               260                 265                 270
   Ile Thr Gly Leu Ile Met Ser Asn Arg Leu Ser Asp Arg Phe Lys Gln
           275                 280                 285
   Ser Asp Glu Ser Ile Met Gln Met Met Ala Arg Ile Gln Ser Glu Ile
       290                 295                 300
<210> 57
   <211> 2148
   <212> DNA
   <213> Artificial Sequence
<220>
   <221> source
   <222> 1..2148
   <223> /mol_type="DNA"
   /note="CMI2.214"
   /organism="Artificial Sequence"
<400> 57
   ttgtactgaa ttaatgaacg agtgtctacg agtggcataa agaaagttcg cttattgccc      60
   ctctgaaaaa ccgctttttc tatatctaat ttcatgaaaa ttttaactac tgtgagcttt     120
   tgcttttgaa aagacacgag cgaagcgagt gccatttttt gctcttgctt ttggggtttg     180
   gggcttgccc caacagggca cagagtagga attttacacg gtagtgaaaa agtcccctct     240
   gtgtgtcctt gccctatgct ttttaaacct ttgaattttt ccctgtaatt tgaagaaatt     300
   gcccctcgac taagcttgct tagtcaaaaa agtttgagca aagcgaaaac atagggcaat     360
   tttcataata aaaatgggct tttaaagttt ttaaatgctt ttaaatgctt ttagacatgc     420
   tgaaatgcct acacagcaag gcatacagag gacatactcc taggtttacc caccaatact     480
   cctaggttta cctaccaata ctcctaggtt tacctaccaa tactcctagg tttaccttgc     540
   gtaatcctaa aagatataat acaaaggaac tactaggttt aatattaata agtaaaatga     600
   gcgatttaat agtaaaagat aatgccttaa tgaacgctag ttataaccta gacttagttg     660
   agcagcgttt aattctcttg gctatccttg aggcgagaga aacaggcaaa gggattaatg     720
   ccaatgatcc tctaaccgtt catgctgaaa gctatatcaa tcaatttggt gtagctagac     780
   agactgcata tcaagcatta aaagatgctt gtaaggactt atttgcacgc caatttagtt     840
   accaagaaaa acgtgagcgt gggcgagcta atattacaag tcgttgggta tcgcaaattg     900
   cttacattga tgaaacggca acagttgagg ttatttttgc ccctgccgtt gttccactga     960
   ttacacgatt agaggaacaa ttcacccaat acgatattga acaaattagc ggtttatcga    1020
   gtgcctatgc cgtccgtcta tatgaattat tgatttgttg gcgtagtaca gggaaaacac    1080
   ctgttattga actagcagag tttagaaagc gtttaggtgt tttaaatgat gaatatacta    1140
   gatcggataa tttcaaaaaa tggattattg aaaaccctat taaacaaata aacgagcata    1200
   ccgacatcac agcaagctac gaacagcaca aaaaagggcg gacaatcaca gggttttcat    1260
   tcaagtttaa gcagaagaaa aaaacagaac ctgaaacgcc aaaaaacagc gattctagcc    1320
   aacgtatcga aaagcctagt caaatccctg ccaacatcgt aaaacaacct gaaaacgcaa    1380
   atttgagcga tttacagcat cgagcaagca agattacagg gttaatcatg tctaaccgtc    1440
   tatcagatcg gtttaaacaa ggtgatgaat caatcatgca gatgatggca cgtattcaaa    1500
   gtgagattac aaccgattca atagccgacc aatggcaatc aaaattagaa gaatttggcg    1560
   tagtttttta gtctgtcacg tagcaatcac gtagaaaatt atgcttgaaa ggcttgctgt    1620
   tattacattt caaaaagcgt cactactgat agtgacgctt ttcttagttt gcctgttgaa    1680
   ttaaacagtg agtgatagac ctcttttggt taggacttca ttaacctttt catgccattc    1740
   tttaattatt tttgtcgttt ctgcattttc actagaacaa ataactaatg tttttcttgg    1800
   ctgaggtagt gttttccccc aaatgtccca atccaataca cgaatagggc gtctgaaaac    1860
   aatccaatcg gtttcacttt catcaccatt gacttcataa gctccaccaa ttacctgaac    1920
   taatgcaatt aatttgccac catttttaat agcaacaata tcattcactt tcattctgtt    1980
   aaaaaagtcc tctttttcaa cctgtcctcc agcccatttt cctaaaccta taattttttt    2040
   gttctctaaa attgaatgta aaacttccga aaagctttga tcatcagggt gcatctgcat    2100
   atgccaatat tgagtcatgt tttttttcca taggtaatta tgtttgtt                 2148
<210> 58
   <211> 960
   <212> DNA
   <213> Artificial Sequence
<220>
   <221> source
   <222> 1..960
   <223> /mol_type="DNA"
   /note="query"
   /organism="Artificial Sequence"
<400> 58
   ataaacgagc ataccgacat cacagcaagc tacgaacagc acaaaaaagg gcggacaatc      60
   acagggtttt cattcaagtt taagcagaag aaaaaaacag aacctgaaac gccaaaaaac     120
   agcgattcta gccaacgtat cgaaaagcct agtcaaatcc ctgccaacat cgtaaaacaa     180
   cctgaaaacg caaatttgag cgatttacag catcgagcaa gcaagattac agggttaatc     240
   atgtctaacc gtctatcaga tcggtttaaa caaggtgatg aatcaatcat gcagatgatg     300
   gcacgtattc aaagtgagat tacaaccgat tcaatagccg accaatggca atcaaaatta     360
   gaagaatttg gcgtagtttt ttagtctgtc acgtagcaat cacgtagaaa attatgcttg     420
   aaaggcttgc tgttattaca tttcaaaaag cgtcactact gatagtgacg cttttcttag     480
   tttgcctgtt gaattaaaca gtgagtgata gacctctttt ggttaggact tcattaacct     540
   tttcatgcca ttctttaatt atttttgtcg tttctgcatt ttcactagaa caaataacta     600
   atgtttttct tggctgaggt agtgttttcc cccaaatgtc ccaatccaat acacgaatag     660
   ggcgtctgaa aacaatccaa tcggtttcac tttcatcacc attgacttca taagctccac     720
   caattacctg aactaatgca attaatttgc caccattttt aatagcaaca atatcattca     780
   ctttcattct gttaaaaaag tcctcttttt caacctgtcc tccagcccat tttcctaaac     840
   ctataatttt tttgttctct aaaattgaat gtaaaacttc cgaaaagctt tgatcatcag     900
   ggtgcatctg catatgccaa tattgagtca tgtttttttt ccataggtaa ttatgtttgt     960
<210> 59
   <211> 959
   <212> DNA
   <213> Artificial Sequence
<220>
   <221> source
   <222> 1..959
   <223> /mol_type="DNA"
   /note="subject"
   /organism="Artificial Sequence"
<400> 59
   ataaacgagt acaccgacat catagcaagc tacgaacagc acaaaaaagg gcggacaatc 60
   acagggtttt cattcaagtt taagcagaag aaaaaaacag aacctgaaac gccaaaaaat 120
   agcgattcta gccaacgtat cgaaaagcct agtcaaatcc ctgccaacat cgtaaaacaa 180
   cctgaaaacg caaatttgag cgatttacag catcgagcaa gcaagattac agggttaatc 240
   atgtctaacc gtctatcaga tcggtttaaa caaggtgatg aatcaatcat gcagatgatg 300
   gcacgtattc aaagtgagat tacaaccgat tcaatagccg accaatggca atcaaaatta 360
   gaagaatttg gcgtagtttt ttagtctgtc acgtggcaat cacgtagaaa attatgtttg 420
   aaaggcttgc tgttattaca tttcaaaaag cgtcactact gatagtgacg cttttcttag 480
   tttgcctgtt gaattaaaca gtgagtgata gacctctttt tgttaggact tcattaacct 540
   tttcatgcca ttctttaatt atttttgtcg tttctgcatt ttcactagaa caaataacta 600
   atgtttttct tggctgaggt agtgttttcc cccaaatgtc ccaatccaat atacgaatag 660
   ggcgtctgaa aacaatccaa tcggtttcac tttcatcatc attgacttca taagctccac 720
   caattacctg aactaatgca attaatttgc caccattttt aatagcaaca atatcattca 780
   ctttcattct gttagaaaag tcctcttttt caacctgtcc tccagcccat tttcctaaac 840
   ctataatttt tttgttctct aaaattgaat gtaaaacttc tgaaaagttt tgatcatcag 900
   ggtgcatctg catatgccaa tattgagtca tgtttttttc cataggtaat tatgtttgt 959
<210> 60
   <211> 150
   <212> DNA
   <213> Artificial Sequence
<220>
   <221> source
   <222> 1..150
   <223> /mol_type="DNA"
   /note="query"
   /organism="Artificial Sequence"
<400> 60
   ttgtactgaa ttaatgaacg agtgtctacg agtggcataa agaaagttcg cttattgccc 60
   ctctgaaaaa ccgctttttc tatatctaat ttcatgaaaa ttttaactac tgtgagcttt 120
   tgcttttgaa aagacacgag cgaagcgagt 150
<210> 61
   <211> 151
   <212> DNA
   <213> Artificial Sequence
<220>
   <221> source
   <222> 1..151
   <223> /mol_type="DNA"
   /note="subject"
   /organism="Artificial Sequence"
<400> 61
   ttgtactgaa ttaatgaacg agtgtctacg agtggcataa agaaagttcg ctcattgccc 60
   tctttgaaaa accgctttta aaaatactgg ctgctcgata atttttacta tcgagtgctt 120
   ttgcttttga aaaaatacga gcgaagcgag t 151
<210> 62
   <211> 48
   <212> DNA
   <213> Artificial Sequence
<220>
   <221> source
   <222> 1..48
   <223> /mol_type="DNA"
   /note="query"
   /organism="Artificial Sequence"
<400> 62
   ataaacgagc ataccgacat cacagcaagc tacgaacagc acaaaaaa 48
<210> 63
   <211> 48
   <212> DNA
   <213> Artificial Sequence
<220>
   <221> source
   <222> 1..48
   <223> /mol_type="DNA"
   /note="subject"
   /organism="Artificial Sequence"
<400> 63
   ataaacgagt acaccgacat cacagccacc tacgaacagc acaaaaaa 48
<210> 64
   <211> 1607
   <212> DNA
   <213> Artificial Sequence
<220>
   <221> source
   <222> 1..1607
   <223> /mol_type="DNA"
   /note="query"
   /organism="Artificial Sequence"
<400> 64
   cgagtgtcta cgagtggcat aaagaaagtt cgcttattgc ccctctgaaa aaccgctttt 60
   tctatatcta atttcatgaa aattttaact actgtgagct tttgcttttg aaaagacacg 120
   agcgaagcga gtgccatttt ttgctcttgc ttttggggtt tggggcttgc cccaacaggg 180
   cacagagtag gaattttaca cggtagtgaa aaagtcccct ctgtgtgtaa caagtcctcg 240
   aatatcaaaa tgtggctaca ttttgtatat acgggtaggc ttgcttattt gatttttttt 300
   cttctaaacc tttgacttct tccccattgt ttgcagaaat tgcccctcga agcttgctta 360
   gtcaaaaaag tttgagcaaa gcgaaaacat agggcaattt tcatgaagaa attgggcttt 420
   taaagttttt aaatgtttta aatgctttta gacatgctaa gaagcccaca cagcaaggca 480
   tacagaggac attctcctac gtttaccgat caataccccc gtttaccgat caatacccct 540
   acgtttaccg atcaataccc ctacgtttac cttgcgtata actacaaaga atactagtgt 600
   agtataactt caaaagaata attgtaggtt atgagcgatt taatagtaaa agataacgcc 660
   ctaatgaatg ctagttataa cttagctttg gttgaacaga ggttaattct attagcaatc 720
   atagaagcga gagaaacagg caaagggatt aatgccaatg atcctcttac ggttcatgca 780
   ggtagctata tcaatcaatt taacgtacaa aggcatacgg catatcaagc cctcaaagat 840
   gcttgtaaag acttgtttgc ccgtcaattc agttaccaag aaaagcgaga acgaggacga 900
   attaatatta caagtcgatg ggtttcgcaa attggctata tggacgatac agcaaccgtt 960
   gagattattt ttgcccctgc ggttgttcct ctgattacac ggctagagga acagttcacc 1020
   cagtacgata ttgagcaaat tagcggttta tcgagtgcat atgctgttcg tatgtacgaa 1080
   ctgctgattt gttggcgtag cacaggcaaa acaccaatta ttgagctaga cgagtttaga 1140
   aagcgaatag gtgttttaga tactgaatac actagaacag ataatttaaa gatgcaagtt 1200
   attgaattag ccctaaaaca aatcaacgaa catactgaca tcacagcaag ctatgaacaa 1260
   cacaaaaaag ggcgagtgat taccggattc tcattcatgt ttaagcacaa gaaacaaaac 1320
   agcgataaaa cgcctgatac taacgcttct agcccacgta tcgtaaaaca tagtcaaatc 1380
   cctaccaaca ttgtaaaaca gcctgaaaac gccaaaatga gcgatttaga acatagagcg 1440
   agccgtgtta caggggaaat aatgcgaaat cgtctgtcag atcggtttaa acaaggcgat 1500
   gaatcagcaa tcgacatgat gaaacgtatt caaagtgaaa taataaccga tgcaatagca 1560
   gaccagtggg aaagcaaact ggaggagttt ggcgtggttt tttagtc 1607
<210> 65
   <211> 1556
   <212> DNA
   <213> Artificial Sequence
<220>
   <221> source
   <222> 1..1556
   <223> /mol_type="DNA"
   /note="subject"
   /organism="Artificial Sequence"
<400> 65
   cgagtgtcta cgagcgactc aatgaaagtt cgattattcc ccctctggaa aaccgctttt      60
   aaaaatattg gctgctagat ggtttttact atagtgaggt tttgctttta aaaaaacacg     120
   agcaaagcga gttcatagtt gcttttgctt gttttcgggt cttaggggaa atcccctaac     180
   aagtcctcga atatcaaaat gtggctacat tttgtatata cgggtaggct tgcttatttg     240
   attttttttc ttctaaacct ttgacttctt ccccattgtt tgcagaaatt gcccctcgaa      300
   gcttgcttag tcaaaaaagt ttgagcaaag cgaaaacata gggcaatttt catgaagaaa     360
   ttgggctttt aaagttttta aatgttttaa atgcttttag acatgctaag aagcccacac     420
   agcaaggcat acagaggaca ttctcctacg tttaccgatc aatacccccg tttaccgatc     480
   aataccccta cgtttaccga tcaatacccc tacgtttacc ttgcgtataa ctacaaagaa     540
   tactagtgta gtataacttc aaaagaataa ttgtaggtta tgagcgattt aatagtaaaa     600
   gataacgccc taatgaatgc tagttataac ttagctttgg ttgaacagag gttaattcta     660
   ttagcaatca tagaagcgag agaaacaggc aaagggatta atgccaatga tcctcttacg     720
   gttcatgcag gtagctatat caatcaattt aacgtacaaa ggcatacggc atatcaagcc     780
   ctcaaagatg cttgtaaaga cttgtttgcc cgtcaattca gttaccaaga aaagcgagaa     840
   cgaggacgaa ttaatattac aagtcgatgg gtttcgcaaa ttggctatat ggacgataca     900
   gcaaccgttg agattatttt tgcccctgcg gttgttcctc tgattacacg gctagaggaa     960
   cagttcaccc agtacgatat tgagcaaatt agcggtttat cgagtgcata tgctgttcgt    1020
   atgtacgaac tgctgatttg ttggcgtagc acaggcaaaa caccaattat tgagctagac    1080
   gagtttagaa agcgaatagg tgttttagat actgaataca ctagaacaga taatttaaag    1140
   atgcaagtta ttgaattagc cctaaaacaa atcaacgaac atactgacat cacagcaagc    1200
   tatgaacaac acaaaaaagg gcgagtgatt accggattct cattcatgtt taagcacaag    1260
   aaacaaaaca gcgataaaac gcctgatact aacgcttcta gcccacgtat cgtaaaacat    1320
   agtcaaatcc ctaccaacat tgtaaaacag cctgaaaacg ccaaaatgag cgatttagaa    1380
   catagagcga gccgtgttac aggggaaata atgcgaaatc gtctgtcaga tcggtttaaa    1440
   caaggcgatg aatcagcaat cgacatgatg aaacgtattc aaagtgaaat aataaccgat    1500
   gcaatagcag accagtggga aagcaaactg gaggagtttg gcgtggtttt ttagtc        1556
<210> 66
   <211> 324
   <212> PRT
   <213> Artificial Sequence
<220>
   <221> SOURCE
   <222> 1..324
   <223> /mol_type="protein"
   /note="query"
   /organism="Artificial Sequence"
<400> 66
   Met Ser Asp Leu Ile Val Lys Asp Asn Ala Leu Met Asn Ala Ser Tyr
   1               5                   10                  15
   Asn Leu Asp Leu Val Glu Gln Arg Leu Ile Leu Leu Ala Ile Leu Glu
               20                  25                  30
   Ala Arg Glu Thr Gly Lys Gly Ile Asn Ala Asn Asp Pro Leu Thr Val
           35                  40                  45
   His Ala Glu Ser Tyr Ile Asn Gln Phe Gly Val Ala Arg Gln Thr Ala
       50                  55                  60
   Tyr Gln Ala Leu Lys Asp Ala Cys Lys Asp Leu Phe Ala Arg Gln Phe
   65                  70                  75                  80
   Ser Tyr Gln Glu Lys Arg Glu Arg Gly Arg Ala Asn Ile Thr Ser Arg
                   85                  90                  95
   Trp Val Ser Gln Ile Ala Tyr Ile Asp Glu Thr Ala Thr Val Glu Val
               100                 105                 110
   Ile Phe Ala Pro Ala Val Val Pro Leu Ile Thr Arg Leu Glu Glu Gln
           115                 120                 125
   Phe Thr Gln Tyr Asp Ile Glu Gln Ile Ser Gly Leu Ser Ser Ala Tyr
       130                 135                 140
   Ala Val Arg Leu Tyr Glu Leu Leu Ile Cys Trp Arg Ser Thr Gly Lys
   145                 150                 155                 160
   Thr Pro Val Ile Glu Leu Ala Glu Phe Arg Lys Arg Leu Gly Val Leu
                   165                 170                 175
   Asn Asp Glu Tyr Thr Arg Ser Asp Asn Phe Lys Lys Trp Ile Ile Glu
               180                 185                 190
   Asn Pro Ile Lys Gln Ile Asn Glu His Thr Asp Ile Thr Ala Ser Tyr
           195                 200                 205
   Glu Gln His Lys Lys Gly Arg Thr Ile Thr Gly Phe Ser Phe Lys Phe
       210                 215                 220
   Lys Gln Lys Lys Lys Thr Glu Pro Glu Thr Pro Lys Asn Ser Asp Ser
   225                 230                 235                 240
   Ser Gln Arg Ile Glu Lys Pro Ser Gln Ile Pro Ala Asn Ile Val Lys
                   245                 250                 255
   Gln Pro Glu Asn Ala Asn Leu Ser Asp Leu Gln His Arg Ala Ser Lys
               260                 265                 270
   Ile Thr Gly Leu Ile Met Ser Asn Arg Leu Ser Asp Arg Phe Lys Gln
           275                 280                 285
   Gly Asp Glu Ser Ile Met Gln Met Met Ala Arg Ile Gln Ser Glu Ile
       290                 295                 300
   Thr Thr Asp Ser Ile Ala Asp Gln Trp Gln Ser Lys Leu Glu Glu Phe
   305                 310                 315                 320
   Gly Val Val Phe
<210> 67
   <211> 324
   <212> PRT
   <213> Artificial Sequence
<220>
   <221> SOURCE
   <222> 1..324
   <223> /mol_type="protein"
   /note="subject"
   /organism="Artificial Sequence"
<400> 67
   Met Ser Glu Leu Ile Val Lys Asp Asn Ala Leu Met Asn Ala Ser Tyr
   1               5                   10                  15
   Asn Leu Asp Leu Val Glu Gln Arg Leu Ile Leu Leu Ala Ile Val Glu
               20                  25                  30
   Ala Arg Glu Ser Gly Lys Gly Ile Asn Ala Asn Asp Pro Leu Thr Val
           35                  40                  45
   His Ala Glu Ser Tyr Ile Asn Gln Phe Gly Val His Arg Thr Thr Ala
       50                  55                  60
   Tyr Gln Ala Leu Lys Asp Ala Cys Lys Asp Leu Phe Ala Arg Gln Phe
   65                  70                  75                  80
   Ser Tyr Gln Glu Lys Arg Glu Arg Gly Arg Ala Asn Ile Thr Ser Arg
                   85                  90                  95
   Trp Val Ser Gln Ile Ala Tyr Ile Asp Glu Thr Ala Thr Val Glu Ile
               100                 105                 110
   Ile Phe Ala Pro Ala Val Val Pro Leu Ile Thr Arg Leu Glu Lys Gln
           115                 120                 125
   Phe Thr Gln Tyr Asp Ile Glu Gln Ile Ser Gly Leu Ser Ser Ala Tyr
       130                 135                 140
   Ala Val Arg Met Tyr Glu Leu Leu Ile Cys Trp Arg Ser Thr Gly Lys
   145                 150                 155                 160
   Thr Pro Val Ile Glu Leu Gly Glu Phe Arg Lys Arg Ile Gly Val Leu
                   165                 170                 175
   Asp Thr Glu Tyr Ile Arg Thr Asp Asn Leu Lys Met Arg Val Ile Glu
               180                 185                 190
   Leu Ala Leu Lys Gln Ile Asn Asp His Thr Asp Ile Thr Ala Thr Tyr
           195                 200                 205
   Glu Gln His Lys Lys Gly Arg Thr Ile Thr Gly Phe Ser Phe Lys Phe
       210                 215                 220
   Lys Gln Lys Arg Lys Thr Glu Leu Glu Thr Pro Lys Asn Ser Asp Ser
   225                 230                 235                 240
   Asp Val Pro Lys Gln Lys Ser Val Glu Ile Pro Thr Asn Leu Val Lys
                   245                 250                 255
   Gln Pro Glu Asn Ala Asn Met Ser Asp Leu Gln His Arg Ala Ser Lys
               260                 265                 270
   Ile Thr Gly Leu Ile Met Ser Asn Arg Leu Ser Asp Arg Phe Lys Gln
           275                 280                 285
   Ser Asp Glu Ser Ile Met Gln Met Met Ala Arg Ile Gln Ser Glu Ile
       290                 295                 300
   Thr Asp Glu Ala Ile Ala Asn Gln Trp Glu Asn Lys Leu Glu Glu Phe
   305                 310                 315                 320
   Gly Val Ile Phe
<210> 68
   <211> 324
   <212> PRT
   <213> Artificial Sequence
<220>
   <221> SOURCE
   <222> 1..324
   <223> /mol_type="protein"
   /note="query"
   /organism="Artificial Sequence"
<400> 68
   Met Ser Asp Leu Ile Val Lys Asp Asn Ala Leu Met Asn Ala Ser Tyr
   1               5                   10                  15
   Asn Leu Asp Leu Val Glu Gln Arg Leu Ile Leu Leu Ala Ile Leu Glu
               20                  25                  30
   Ala Arg Glu Thr Gly Lys Gly Ile Asn Ala Asn Asp Pro Leu Thr Val
           35                  40                  45
   His Ala Glu Ser Tyr Ile Asn Gln Phe Gly Val Ala Arg Gln Thr Ala
       50                  55                  60
   Tyr Gln Ala Leu Lys Asp Ala Cys Lys Asp Leu Phe Ala Arg Gln Phe
   65                  70                  75                  80
   Ser Tyr Gln Glu Lys Arg Glu Arg Gly Arg Ala Asn Ile Thr Ser Arg
                   85                  90                  95
   Trp Val Ser Gln Ile Ala Tyr Ile Asp Glu Thr Ala Thr Val Glu Val
               100                 105                 110
   Ile Phe Ala Pro Ala Val Val Pro Leu Ile Thr Arg Leu Glu Glu Gln
           115                 120                 125
   Phe Thr Gln Tyr Asp Ile Glu Gln Ile Ser Gly Leu Ser Ser Ala Tyr
       130                 135                 140
   Ala Val Arg Leu Tyr Glu Leu Leu Ile Cys Trp Arg Ser Thr Gly Lys
   145                 150                 155                 160
   Thr Pro Val Ile Glu Leu Ala Glu Phe Arg Lys Arg Leu Gly Val Leu
                   165                 170                 175
   Asn Asp Glu Tyr Thr Arg Ser Asp Asn Phe Lys Lys Trp Ile Ile Glu
               180                 185                 190
   Asn Pro Ile Lys Gln Ile Asn Glu His Thr Asp Ile Thr Ala Ser Tyr
           195                 200                 205
   Glu Gln His Lys Lys Gly Arg Thr Ile Thr Gly Phe Ser Phe Lys Phe
       210                 215                 220
   Lys Gln Lys Lys Lys Thr Glu Pro Glu Thr Pro Lys Asn Ser Asp Ser
   225                 230                 235                 240
   Ser Gln Arg Ile Glu Lys Pro Ser Gln Ile Pro Ala Asn Ile Val Lys
                   245                 250                 255
   Gln Pro Glu Asn Ala Asn Leu Ser Asp Leu Gln His Arg Ala Ser Lys
               260                 265                 270
   Ile Thr Gly Leu Ile Met Ser Asn Arg Leu Ser Asp Arg Phe Lys Gln
           275                 280                 285
   Gly Asp Glu Ser Ile Met Gln Met Met Ala Arg Ile Gln Ser Glu Ile
       290                 295                 300
   Thr Thr Asp Ser Ile Ala Asp Gln Trp Gln Ser Lys Leu Glu Glu Phe
   305                 310                 315                 320
   Gly Val Val Phe
<210> 69
   <211> 324
   <212> PRT
   <213> Artificial Sequence
<220>
   <221> SOURCE
   <222> 1..324
   <223> /mol_type="protein"
   /note="subject"
   /organism="Artificial Sequence"
<400> 69
   Met Ser Asp Leu Ile Val Lys Asp Asn Ala Leu Met Asn Ala Ser Tyr
   1               5                   10                  15
   Asn Leu Ala Leu Val Glu Gln Arg Leu Ile Leu Leu Ala Ile Ile Glu
               20                  25                  30
   Ala Arg Glu Thr Gly Lys Gly Ile Asn Ala Asn Asp Pro Leu Thr Val
           35                  40                  45
   His Ala Gly Ser Tyr Ile Asn Gln Phe Asn Val Gln Arg His Thr Ala
       50                  55                  60
   Tyr Gln Ala Leu Lys Asp Ala Cys Lys Asp Leu Phe Ala Arg Gln Phe
   65                  70                  75                  80
   Ser Tyr Gln Glu Lys Arg Glu Arg Gly Arg Ile Asn Ile Thr Ser Arg
                   85                  90                  95
   Trp Val Ser Gln Ile Gly Tyr Met Asp Asp Thr Ala Thr Val Glu Ile
               100                 105                 110
   Ile Phe Ala Pro Ala Val Val Pro Leu Ile Thr Arg Leu Glu Glu Gln
           115                 120                 125
   Phe Thr Gln Tyr Asp Ile Glu Gln Ile Ser Gly Leu Ser Ser Ala Tyr
       130                 135                 140
   Ala Val Arg Met Tyr Glu Leu Leu Ile Cys Trp Arg Ser Thr Gly Lys
   145                 150                 155                 160
   Thr Pro Ile Ile Glu Leu Asp Glu Phe Arg Lys Arg Ile Gly Val Leu
                   165                 170                 175
   Asp Thr Glu Tyr Thr Arg Thr Asp Asn Leu Lys Met Gln Val Ile Glu
               180                 185                 190
   Leu Ala Leu Lys Gln Ile Asn Glu His Thr Asp Ile Thr Ala Ser Tyr
           195                 200                 205
   Glu Gln His Lys Lys Gly Arg Val Ile Thr Gly Phe Ser Phe Met Phe
       210                 215                 220
   Lys His Lys Lys Gln Asn Ser Asp Lys Thr Pro Asp Thr Asn Ala Ser
   225                 230                 235                 240
   Ser Pro Arg Ile Val Lys His Ser Gln Ile Pro Thr Asn Ile Val Lys
                   245                 250                 255
   Gln Pro Glu Asn Ala Lys Met Ser Asp Leu Glu His Arg Ala Ser Arg
               260                 265                 270
   Val Thr Gly Glu Ile Met Arg Asn Arg Leu Ser Asp Arg Phe Lys Gln
           275                 280                 285
   Gly Asp Glu Ser Ala Ile Asp Met Met Lys Arg Ile Gln Ser Glu Ile
       290                 295                 300
   Ile Thr Asp Ala Ile Ala Asp Gln Trp Glu Ser Lys Leu Glu Glu Phe
   305                 310                 315                 320
   Gly Val Val Phe
<210> 70
   <211> 1687
   <212> DNA
   <213> Artificial Sequence
<220>
   <221> source
   <222> 1..1687
   <223> /mol_type="DNA"
   /note="CMI3.168"
   /organism="Artificial Sequence"
<400> 70
   ggattaatgc caatgatccc ttaacagttc atgcaagtag ctatatcaat caatttaacg      60
   ttgaaagaca tacggcatat caagcattaa aagatgcttg taaggactta tttgcacgcc     120
   aatttagtta ccaagaaaaa cgtgagcgtg ggcgagctaa tattacaagt cgttgggtat     180
   cgcaaattgc ttacattgat gaaacggcaa cagttgaggt tatttttgcc cctgccgttg     240
   ttccactgat tacacgatta gaggaacaat tcacccaata cgatattgaa caaattagcg     300
   gtttatcgag tgcctatgca gttcgcttat atgagttatt gatctgctgg agaacaacag     360
   gaaagacacc agttcttgac ttaacagagt ttagaaagcg tctaggtgtt ttagataccg     420
   aatacactag aacagataac ttgaagatgc gagtgattga gcagtcactc aagcaaataa     480
   acaagcatac cgacatcaca gcgagctacg aacagcacaa aaaagggcgg acaatcacag     540
   gattctcatt caagtttaag cagaagaaga aaacagagcc agaaacgcca aaaaacaacg     600
   attctggcgt ttctaagccg aaaacagtag aaatccctgc cgaagtcgta aaacagccta     660
   aaaacaccaa tttgagcgat ttagagaaac gagtaaggat gatcacaggg gcaattgcta     720
   aaaataacct ggcaagtcgt tttcaacatg ggaatgaatc accactggat atgatgaaac     780
   gtattcaaag tgaaatcaca tcagacgaaa ctgccgatct atggcaaaac aaacttgaat     840
   caatgggtgt ggttttttag tcatgacgaa tttcccgaag ggcgcacttt cccaaactat      900
   cgtttgagtg cgtttttcta tcgaaaaagt aaaaagacaa agaaaatttt ctttgtcttt     960
   ttttctgttt ttcaaaaaaa gggattagcg agtgtctacg agcgacacaa tgaaagttcg    1020
   attatttccc cctctgaaaa accgcttttt tgtatctgat ttcaagaaaa ttttaactat    1080
   cgtgcgcttt tgcttttgag aagacacgag cgaagcgagt gccatttttg cctttgcttt    1140
   tgctttttgg ggtttggggc ttgccccaac aaggcacaga gtaggaattt tacacggtag 1200
   tggaaaagtc ccctctgtgt gtccttgctc taatcttttt aaaacctttg attcttcacc 1260
   gtaatttgct aaaaattgcc ccgactgagt gaagcgaagt caaaaaagtt tgagcaaagc 1320
   gaaaacatag ggcaattttc gtgatgaaaa tgggctttta aggtttttaa atgcttttaa 1380
   atgcttttag acacgctgaa acgcaagcct agcaaggtat acagagggca tttacctacg 1440
   tttaccgatc aataccccta cgtttaccga tcaatacccc tacgtttacc gatcaatacc 1500
   cctacgttta ccttgcatat aactacaaaa tataataatg tagtaataac ttcaaaataa 1560
   taaacgtagg ttatgagcga tttaatagta aaagataacg ccctaatgaa cgctagttac 1620
   aacttagctt tggttgaaca gcgtttaatt ctattggcta tcctagaggc gagagaaaca 1680
   ggcaaag 1687
<210> 71
   <211> 22
   <212> DNA
   <213> Artificial Sequence
<220>
   <221> source
   <222> 1..22
   <223> /mol_type="DNA"
   /note="repeat"
   /organism="Artificial Sequence"
<400> 71
   atacccctac gtttaccgat ca 22
<210> 72
   <211> 889
   <212> DNA
   <213> Artificial Sequence
<220>
   <221> source
   <222> 1..889
   <223> /mol_type="DNA"
   /note="query"
   /organism="Artificial Sequence"
<400> 72
   ggattaatgc caatgatccc ttaacagttc atgcaagtag ctatatcaat caatttaacg      60
   ttgaaagaca tacggcatat caagcattaa aagatgcttg taaggactta tttgcacgcc     120
   aatttagtta ccaagaaaaa cgtgagcgtg ggcgagctaa tattacaagt cgttgggtat     180
   cgcaaattgc ttacattgat gaaacggcaa cagttgaggt tatttttgcc cctgccgttg     240
   ttccactgat tacacgatta gaggaacaat tcacccaata cgatattgaa caaattagcg     300
   gtttatcgag tgcctatgca gttcgcttat atgagttatt gatctgctgg agaacaacag     360
   gaaagacacc agttcttgac ttaacagagt ttagaaagcg tctaggtgtt ttagataccg     420
   aatacactag aacagataac ttgaagatgc gagtgattga gcagtcactc aagcaaataa     480
   acaagcatac cgacatcaca gcgagctacg aacagcacaa aaaagggcgg acaatcacag     540
   gattctcatt caagtttaag cagaagaaga aaacagagcc agaaacgcca aaaaacaacg     600
   attctggcgt ttctaagccg aaaacagtag aaatccctgc cgaagtcgta aaacagccta     660
   aaaacaccaa tttgagcgat ttagagaaac gagtaaggat gatcacaggg gcaattgcta     720
   aaaataacct ggcaagtcgt tttcaacatg ggaatgaatc accactggat atgatgaaac     780
   gtattcaaag tgaaatcaca tcagacgaaa ctgccgatct atggcaaaac aaacttgaat     840
   caatgggtgt ggttttttag tcatgacgaa tttcccgaag ggcgcactt                  889
<210> 73
   <211> 888
   <212> DNA
   <213> Artificial Sequence
<220>
   <221> source
   <222> 1..888
   <223> /mol_type="DNA"
   /note="subject"
   /organism="Artificial Sequence"
<400> 73
   ggattaatgc caatgatcct cttacggttc atgcaggtag ctatatcaat caatttaacg 60
   tacaaaggca tacggcatat caagccctca aagatgcttg taaagacttg tttgcccgtc 120
   aattcagtta ccaagaaaag cgagaacgag gacgaattaa tattacaagt cgatgggttt 180
   cgcaaattgg ctatatggac gatacagcaa ccgttgagat tatttttgcc cctgcggttg 240
   ttcctctgat tacacggcta gaggaacagt tcacccagta cgatattgag caaattagcg 300
   gtttatcgag tgcatatgct gttcgtatgt acgaactgct gatttgttgg cgtagcacag 360
   gcaaaacacc aattattgag ctagacgagt ttagaaagcg aataggtgtt ttagatactg 420
   aatacactag aacagataat ttaaagatgc aagttattga attagcccta aaacaaatca 480
   acgaacatac tgacatcaca gcaagctatg aacaacacaa aaaagggcga gtgattaccg 540
   gattctcatt catgtttaag cacaagaaac aaaacagcga taaaacgcct gatactaacg 600
   cttctagccc acgtatcgta aaacatagtc aaatccctac caacattgta aaacagcctg 660
   aaaacgccaa aatgagcgat ttagaacata gagcgagccg tgttacaggg gaaataatgc 720
   gaaatcgtct gtcagatcgg tttaaacaag gcgatgaatc agcaatcgac atgatgaaac 780
   gtattcaaag tgaaataata accgatgcaa tagcagacca gtgggaaagc aaactggagg 840
   agtttggcgt ggttttttag tcatgacgat ttcccgaagg gcgcactt 888
<210> 74
   <211> 717
   <212> DNA
   <213> Artificial Sequence
<220>
   <221> source
   <222> 1..717
   <223> /mol_type="DNA"
   /note="query"
   /organism="Artificial Sequence"
<400> 74
   ttcaaaaaaa gggattagcg agtgtctacg agcgacacaa tgaaagttcg attatttccc      60
   cctctgaaaa accgcttttt tgtatctgat ttcaagaaaa ttttaactat cgtgcgcttt     120
   tgcttttgag aagacacgag cgaagcgagt gccatttttg cctttgcttt tgctttttgg     180
   ggtttggggc ttgccccaac aaggcacaga gtaggaattt tacacggtag tggaaaagtc     240
   ccctctgtgt gtccttgctc taatcttttt aaaacctttg attcttcacc gtaatttgct     300
   aaaaattgcc ccgactgagt gaagcgaagt caaaaaagtt tgagcaaagc gaaaacatag     360
   ggcaattttc gtgatgaaaa tgggctttta aggtttttaa atgcttttaa atgcttttag     420
   acacgctgaa acgcaagcct agcaaggtat acagagggca tttacctacg tttaccgatc     480
   aataccccta cgtttaccga tcaatacccc tacgtttacc gatcaatacc cctacgttta     540
   ccttgcatat aactacaaaa tataataatg tagtaataac ttcaaaataa taaacgtagg     600
   ttatgagcga tttaatagta aaagataacg ccctaatgaa cgctagttac aacttagctt     660
   tggttgaaca gcgtttaatt ctattggcta tcctagaggc gagagaaaca ggcaaag        717
<210> 75
   <211> 713
   <212> DNA
   <213> Artificial Sequence
<220>
   <221> source
   <222> 1..713
   <223> /mol_type="DNA"
   /note="subject"
   /organism="Artificial Sequence"
<400> 75
   ttcacaaaaa agatattagc gagtgtctac gagcgactca atgaaagttc gattattccc      60
   cctctggaaa accgctttta aaaatattgg ctgctagatg gtttttacta tagtgaggtt     120
   ttgcttttaa aaaaacacga gcaaagcgag ttcatagttg cttttgcttg ttttcgggtc     180
   ttaggggaaa tcccctaaca agtcctcgaa tatcaaaatg tggctacatt ttgtatatac     240
   gggtaggctt gcttatttga ttttttttct tctaaacctt tgacttcttc cccattgttt     300
   gcagaaattg cccctcgaag cttgcttagt caaaaaagtt tgagcaaagc gaaaacatag     360
   ggcaattttc atgaagaaat tgggctttta aagtttttaa atgttttaaa tgcttttaga     420
   catgctaaga agcccacaca gcaaggcata cagaggacat tctcctacgt ttaccgatca     480
   atacccccgt ttaccgatca atacccctac gtttaccgat caatacccct acgtttacct     540
   tgcgtataac tacaaagaat actagtgtag tataacttca aaagaataat tgtaggttat     600
   gagcgattta atagtaaaag ataacgccct aatgaatgct agttataact tagctttggt     660
   tgaacagagg ttaattctat tagcaatcat agaagcgaga gaaacaggca aag            713
<210> 76
   <211> 324
   <212> PRT
   <213> Artificial Sequence
<220>
   <221> SOURCE
   <222> 1..324
   <223> /mol_type="protein"
   /note="query"
   /organism="Artificial Sequence"
<400> 76
   Met Ser Asp Leu Ile Val Lys Asp Asn Ala Leu Met Asn Ala Ser Tyr
   1               5                   10                  15
   Asn Leu Ala Leu Val Glu Gln Arg Leu Ile Leu Leu Ala Ile Leu Glu
               20                  25                  30
   Ala Arg Glu Thr Gly Lys Gly Ile Asn Ala Asn Asp Pro Leu Thr Val
           35                  40                  45
   His Ala Ser Ser Tyr Ile Asn Gln Phe Asn Val Glu Arg His Thr Ala
       50                  55                  60
   Tyr Gln Ala Leu Lys Asp Ala Cys Lys Asp Leu Phe Ala Arg Gln Phe
   65                  70                  75                  80
   Ser Tyr Gln Glu Lys Arg Glu Arg Gly Arg Ala Asn Ile Thr Ser Arg
                   85                  90                  95
   Trp Val Ser Gln Ile Ala Tyr Ile Asp Glu Thr Ala Thr Val Glu Val
               100                 105                 110
   Ile Phe Ala Pro Ala Val Val Pro Leu Ile Thr Arg Leu Glu Glu Gln
           115                 120                 125
   Phe Thr Gln Tyr Asp Ile Glu Gln Ile Ser Gly Leu Ser Ser Ala Tyr
       130                 135                 140
   Ala Val Arg Leu Tyr Glu Leu Leu Ile Cys Trp Arg Thr Thr Gly Lys
   145                 150                 155                 160
   Thr Pro Val Leu Asp Leu Thr Glu Phe Arg Lys Arg Leu Gly Val Leu
                   165                 170                 175
   Asp Thr Glu Tyr Thr Arg Thr Asp Asn Leu Lys Met Arg Val Ile Glu
               180                 185                 190
   Gln Ser Leu Lys Gln Ile Asn Lys His Thr Asp Ile Thr Ala Ser Tyr
           195                 200                 205
   Glu Gln His Lys Lys Gly Arg Thr Ile Thr Gly Phe Ser Phe Lys Phe
       210                 215                 220
   Lys Gln Lys Lys Lys Thr Glu Pro Glu Thr Pro Lys Asn Asn Asp Ser
   225                 230                 235                 240
   Gly Val Ser Lys Pro Lys Thr Val Glu Ile Pro Ala Glu Val Val Lys
                   245                 250                 255
   Gln Pro Lys Asn Thr Asn Leu Ser Asp Leu Glu Lys Arg Val Arg Met
               260                 265                 270
   Ile Thr Gly Ala Ile Ala Lys Asn Asn Leu Ala Ser Arg Phe Gln His
           275                 280                 285
   Gly Asn Glu Ser Pro Leu Asp Met Met Lys Arg Ile Gln Ser Glu Ile
       290                 295                 300
   Thr Ser Asp Glu Thr Ala Asp Leu Trp Gln Asn Lys Leu Glu Ser Met
   305                 310                 315                 320
   Gly Val Val Phe
<210> 77
   <211> 324
   <212> PRT
   <213> Artificial Sequence
<220>
   <221> SOURCE
   <222> 1..324
   <223> /mol_type="protein"
   /note="subject"
   /organism="Artificial Sequence"
<400> 77
   Met Ser Glu Leu Ile Val Lys Asp Asn Ala Leu Met Asn Ala Ser Tyr
   1               5                   10                  15
   Asn Leu Asp Leu Val Glu Gln Arg Leu Ile Leu Leu Ala Ile Val Glu
               20                  25                  30
   Ala Arg Glu Ser Gly Lys Gly Ile Asn Ala Asn Asp Pro Leu Thr Val
           35                  40                  45
   His Ala Glu Ser Tyr Ile Asn Gln Phe Gly Val His Arg Thr Thr Ala
       50                  55                  60
   Tyr Gln Ala Leu Lys Asp Ala Cys Lys Asp Leu Phe Ala Arg Gln Phe
   65                  70                  75                  80
   Ser Tyr Gln Glu Lys Arg Glu Arg Gly Arg Ala Asn Ile Thr Ser Arg
                   85                  90                  95
   Trp Val Ser Gln Ile Ala Tyr Ile Asp Glu Thr Ala Thr Val Glu Ile
               100                 105                 110
   Ile Phe Ala Pro Ala Val Val Pro Leu Ile Thr Arg Leu Glu Lys Gln
           115                 120                 125
   Phe Thr Gln Tyr Asp Ile Glu Gln Ile Ser Gly Leu Ser Ser Ala Tyr
       130                 135                 140
   Ala Val Arg Met Tyr Glu Leu Leu Ile Cys Trp Arg Ser Thr Gly Lys
   145                 150                 155                 160
   Thr Pro Val Ile Glu Leu Gly Glu Phe Arg Lys Arg Ile Gly Val Leu
                   165                 170                 175
   Asp Thr Glu Tyr Ile Arg Thr Asp Asn Leu Lys Met Arg Val Ile Glu
               180                 185                 190
   Leu Ala Leu Lys Gln Ile Asn Asp His Thr Asp Ile Thr Ala Thr Tyr
           195                 200                 205
   Glu Gln His Lys Lys Gly Arg Thr Ile Thr Gly Phe Ser Phe Lys Phe
       210                 215                 220
   Lys Gln Lys Arg Lys Thr Glu Leu Glu Thr Pro Lys Asn Ser Asp Ser
   225                 230                 235                 240
   Asp Val Pro Lys Gln Lys Ser Val Glu Ile Pro Thr Asn Leu Val Lys
                   245                 250                 255
   Gln Pro Glu Asn Ala Asn Met Ser Asp Leu Gln His Arg Ala Ser Lys
               260                 265                 270
   Ile Thr Gly Leu Ile Met Ser Asn Arg Leu Ser Asp Arg Phe Lys Gln
           275                 280                 285
   Ser Asp Glu Ser Ile Met Gln Met Met Ala Arg Ile Gln Ser Glu Ile
       290                 295                 300
   Thr Asp Glu Ala Ile Ala Asn Gln Trp Glu Asn Lys Leu Glu Glu Phe
   305                 310                 315                 320
   Gly Val Ile Phe
<210> 78
   <211> 264
   <212> PRT
   <213> Artificial Sequence
<220>
   <221> SOURCE
   <222> 1..264
   <223> /mol_type="protein"
   /note="query"
   /organism="Artificial Sequence"
<400> 78
   Met Ser Asp Leu Ile Val Lys Asp Asn Ala Leu Met Asn Ala Ser Tyr
   1               5                   10                  15
   Asn Leu Ala Leu Val Glu Gln Arg Leu Ile Leu Leu Ala Ile Leu Glu
               20                  25                  30
   Ala Arg Glu Thr Gly Lys Gly Ile Asn Ala Asn Asp Pro Leu Thr Val
           35                  40                  45
   His Ala Ser Ser Tyr Ile Asn Gln Phe Asn Val Glu Arg His Thr Ala
       50                  55                  60
   Tyr Gln Ala Leu Lys Asp Ala Cys Lys Asp Leu Phe Ala Arg Gln Phe
   65                  70                  75                  80
   Ser Tyr Gln Glu Lys Arg Glu Arg Gly Arg Ala Asn Ile Thr Ser Arg
                   85                  90                  95
   Trp Val Ser Gln Ile Ala Tyr Ile Asp Glu Thr Ala Thr Val Glu Val
               100                 105                 110
   Ile Phe Ala Pro Ala Val Val Pro Leu Ile Thr Arg Leu Glu Glu Gln
           115                 120                 125
   Phe Thr Gln Tyr Asp Ile Glu Gln Ile Ser Gly Leu Ser Ser Ala Tyr
       130                 135                 140
   Ala Val Arg Leu Tyr Glu Leu Leu Ile Cys Trp Arg Thr Thr Gly Lys
   145                 150                 155                 160
   Thr Pro Val Leu Asp Leu Thr Glu Phe Arg Lys Arg Leu Gly Val Leu
                   165                 170                 175
   Asp Thr Glu Tyr Thr Arg Thr Asp Asn Leu Lys Met Arg Val Ile Glu
               180                 185                 190
   Gln Ser Leu Lys Gln Ile Asn Lys His Thr Asp Ile Thr Ala Ser Tyr
           195                 200                 205
   Glu Gln His Lys Lys Gly Arg Thr Ile Thr Gly Phe Ser Phe Lys Phe
       210                 215                 220
   Lys Gln Lys Lys Lys Thr Glu Pro Glu Thr Pro Lys Asn Asn Asp Ser
   225                 230                 235                 240
   Gln Ser Glu Ile Thr Ser Asp Glu Thr Ala Asp Leu Trp Gln Asn Lys
                   245                 250                 255
   Leu Glu Ser Met Gly Val Val Phe
               260
<210> 79
   <211> 324
   <212> PRT
   <213> Artificial Sequence
<220>
   <221> SOURCE
   <222> 1..324
   <223> /mol_type="protein"
   /note="subject"
   /organism="Artificial Sequence"
<400> 79
   Met Ser Asp Leu Ile Val Lys Asp Asn Ala Leu Met Asn Ala Ser Tyr
   1               5                   10                  15
   Asn Leu Ala Leu Val Glu Gln Arg Leu Ile Leu Leu Ala Ile Ile Glu
                   20                  25                  30
   Ala Arg Glu Thr Gly Lys Gly Ile Asn Ala Asn Asp Pro Leu Thr Val
           35                  40                  45
   His Ala Gly Ser Tyr Ile Asn Gln Phe Asn Val Gln Arg His Thr Ala
       50                  55                  60
   Tyr Gln Ala Leu Lys Asp Ala Cys Lys Asp Leu Phe Ala Arg Gln Phe
   65                  70                  75                  80
   Ser Tyr Gln Glu Lys Arg Glu Arg Gly Arg Ile Asn Ile Thr Ser Arg
                   85                  90                  95
   Trp Val Ser Gln Ile Gly Tyr Met Asp Asp Thr Ala Thr Val Glu Ile
               100                 105                 110
   Ile Phe Ala Pro Ala Val Val Pro Leu Ile Thr Arg Leu Glu Glu Gln
           115                 120                 125
   Phe Thr Gln Tyr Asp Ile Glu Gln Ile Ser Gly Leu Ser Ser Ala Tyr
       130                 135                 140
   Ala Val Arg Met Tyr Glu Leu Leu Ile Cys Trp Arg Ser Thr Gly Lys
   145                 150                 155                 160
   Thr Pro Ile Ile Glu Leu Asp Glu Phe Arg Lys Arg Ile Gly Val Leu
                   165                 170                 175
   Asp Thr Glu Tyr Thr Arg Thr Asp Asn Leu Lys Met Gln Val Ile Glu
               180                 185                 190
   Leu Ala Leu Lys Gln Ile Asn Glu His Thr Asp Ile Thr Ala Ser Tyr
           195                 200                 205
   Glu Gln His Lys Lys Gly Arg Val Ile Thr Gly Phe Ser Phe Met Phe
       210                 215                 220
   Lys His Lys Lys Gln Asn Ser Asp Lys Thr Pro Asp Thr Asn Ala Ser
   225                 230                 235                 240
   Ser Pro Arg Ile Val Lys His Ser Gln Ile Pro Thr Asn Ile Val Lys
                   245                 250                 255
   Gln Pro Glu Asn Ala Lys Met Ser Asp Leu Glu His Arg Ala Ser Arg
               260                 265                 270
   Val Thr Gly Glu Ile Met Arg Asn Arg Leu Ser Asp Arg Phe Lys Gln
           275                 280                 285
   Gly Asp Glu Ser Ala Ile Asp Met Met Lys Arg Ile Gln Ser Glu Ile
       290                 295                 300
   Ile Thr Asp Ala Ile Ala Asp Gln Trp Glu Ser Lys Leu Glu Glu Phe
   305                 310                 315                 320
   Gly Val Val Phe
<210> 80
   <211> 137
   <212> PRT
   <213> Artificial Sequence
<220>
   <221> SOURCE
   <222> 1..137
   <223> /mol_type="protein"
   /note="query"
   /organism="Artificial Sequence"
<400> 80
   Met Arg Val Ile Glu Gln Ser Leu Lys Gln Ile Asn Lys His Thr Asp
   1               5                   10                  15
   Ile Thr Ala Ser Tyr Glu Gln His Lys Lys Gly Arg Thr Ile Thr Gly
               20                  25                  30
   Phe Ser Phe Lys Phe Lys Gln Lys Lys Lys Thr Glu Pro Glu Thr Pro
           35                  40                  45
   Lys Asn Asn Asp Ser Gly Val Ser Lys Pro Lys Thr Val Glu Ile Pro
       50                  55                  60
   Ala Glu Val Val Lys Gln Pro Lys Asn Thr Asn Leu Ser Asp Leu Glu
   65                  70                  75                  80
   Lys Arg Val Arg Met Ile Thr Gly Ala Ile Ala Lys Asn Asn Leu Ala
                   85                  90                  95
   Ser Arg Phe Gln His Gly Asn Glu Ser Pro Leu Asp Met Met Lys Arg
               100                 105                 110
   Ile Gln Ser Glu Ile Thr Ser Asp Glu Thr Ala Asp Leu Trp Gln Asn
           115                 120                 125
   Lys Leu Glu Ser Met Gly Val Val Phe
       130                 135
<210> 81
   <211> 137
   <212> PRT
   <213> Artificial Sequence
<220>
   <221> SOURCE
   <222> 1..137
   <223> /mol_type="protein"
   /note="subject"
   /organism="Artificial Sequence"
<400> 81
   Met Arg Val Ile Glu Leu Ala Leu Lys Gln Ile Asn Asp His Thr Asp
   1               5                   10                  15
   Ile Thr Ala Thr Tyr Glu Gln His Lys Lys Gly Arg Thr Ile Thr Gly
               20                  25                  30
   Phe Ser Phe Lys Phe Lys Gln Lys Arg Lys Thr Glu Leu Glu Thr Pro
           35                  40                  45
   Lys Asn Ser Asp Ser Asp Val Pro Lys Gln Lys Ser Val Glu Ile Pro
       50                  55                  60
   Thr Asn Leu Val Lys Gln Pro Glu Asn Ala Asn Met Ser Asp Leu Gln
   65                  70                  75                  80
   His Arg Ala Ser Lys Ile Thr Gly Leu Ile Met Ser Asn Arg Leu Ser
                   85                  90                  95
   Asp Arg Phe Lys Gln Ser Asp Glu Ser Ile Met Gln Met Met Ala Arg
               100                 105                 110
   Ile Gln Ser Glu Ile Thr Asp Glu Ala Ile Ala Asn Gln Trp Glu Asn
           115                 120                 125
   Lys Leu Glu Glu Phe Gly Val Ile Phe
       130                 135
<210> 82
   <211> 137
   <212> PRT
   <213> Artificial Sequence
<220>
   <221> SOURCE
   <222> 1..137
   <223> /mol_type="protein"
   /note="query"
   /organism="Artificial Sequence"
<400> 82
   Met Arg Val Ile Glu Gln Ser Leu Lys Gln Ile Asn Lys His Thr Asp
   1               5                   10                  15
   Ile Thr Ala Ser Tyr Glu Gln His Lys Lys Gly Arg Thr Ile Thr Gly
               20                  25                  30
   Phe Ser Phe Lys Phe Lys Gln Lys Lys Lys Thr Glu Pro Glu Thr Pro
           35                  40                  45
   Lys Asn Asn Asp Ser Gly Val Ser Lys Pro Lys Thr Val Glu Ile Pro
       50                  55                  60
   Ala Glu Val Val Lys Gln Pro Lys Asn Thr Asn Leu Ser Asp Leu Glu
   65                  70                  75                  80
   Lys Arg Val Arg Met Ile Thr Gly Ala Ile Ala Lys Asn Asn Leu Ala
                   85                  90                  95
   Ser Arg Phe Gln His Gly Asn Glu Ser Pro Leu Asp Met Met Lys Arg
               100                 105                 110
   Ile Gln Ser Glu Ile Thr Ser Asp Glu Thr Ala Asp Leu Trp Gln Asn
           115                 120                 125
   Lys Leu Glu Ser Met Gly Val Val Phe
       130                 135
<210> 83
   <211> 137
   <212> PRT
   <213> Artificial Sequence
<220>
   <221> SOURCE
   <222> 1..137
   <223> /mol_type="protein"
   /note="subject"
   /organism="Artificial Sequence"
<400> 83
   Met Arg Val Ile Glu Leu Ala Leu Lys Gln Ile Asn Asp Tyr Thr Asp
   1               5                   10                  15
   Ile Thr Ala Ser Tyr Glu Gln His Lys Lys Gly Arg Val Ile Thr Gly
               20                  25                  30
   Phe Ser Phe Lys Phe Lys Gln Lys Lys Lys Thr Glu Ala Glu Thr Pro
           35                  40                  45
   Lys Asn Ser Asp Ser Gly Val Ser Lys Gln Lys Pro Ile Glu Ile Pro
       50                  55                  60
   Thr Glu Val Val Lys Gln Pro Lys Asn Ala Asn Leu Ser Asp Leu Glu
   65                  70                  75                  80
   His Arg Ala Ser Arg Ile Thr Gly Glu Ile Met Arg Asn Arg Leu Thr
                   85                  90                  95
   Asp Arg Phe Lys Gln Ala Asp Glu Ser Pro Leu Glu Met Met Lys Arg
               100                 105                 110
   Ile Gln Ser Glu Ile Thr Thr Asp Thr Ile Ala Asp Gln Trp Glu Asn
           115                 120                 125
   Lys Leu Glu Glu Phe Gly Val Val Phe
       130                 135
<210> 84
   <211> 137
   <212> PRT
   <213> Artificial Sequence
<220>
   <221> SOURCE
   <222> 1..137
   <223> /mol_type="protein"
   /note="query"
   /organism="Artificial Sequence"
<400> 84
   Met Arg Val Ile Glu Gln Ser Leu Lys Gln Ile Asn Lys His Thr Asp
   1               5                   10                  15
   Ile Thr Ala Ser Tyr Glu Gln His Lys Lys Gly Arg Thr Ile Thr Gly
               20                  25                  30
   Phe Ser Phe Lys Phe Lys Gln Lys Lys Lys Thr Glu Pro Glu Thr Pro
           35                  40                  45
   Lys Asn Asn Asp Ser Gly Val Ser Lys Pro Lys Thr Val Glu Ile Pro
       50                  55                  60
   Ala Glu Val Val Lys Gln Pro Lys Asn Thr Asn Leu Ser Asp Leu Glu
   65                  70                  75                  80
   Lys Arg Val Arg Met Ile Thr Gly Ala Ile Ala Lys Asn Asn Leu Ala
                   85                  90                  95
   Ser Arg Phe Gln His Gly Asn Glu Ser Pro Leu Asp Met Met Lys Arg
               100                 105                 110
   Ile Gln Ser Glu Ile Thr Ser Asp Glu Thr Ala Asp Leu Trp Gln Asn
           115                 120                 125
   Lys Leu Glu Ser Met Gly Val Val Phe
       130                 135
<210> 85
   <211> 137
   <212> PRT
   <213> Artificial Sequence
<220>
   <221> SOURCE
   <222> 1..137
   <223> /mol_type="protein"
   /note="subject"
   /organism="Artificial Sequence"
<400> 85
   Met Arg Val Ile Glu Leu Ala Leu Lys Gln Ile Asn Asp Tyr Thr Asp
   1               5                   10                  15
   Ile Thr Ala Ser Tyr Glu Gln His Lys Lys Gly Arg Val Ile Thr Gly
               20                  25                  30
   Phe Ser Phe Lys Phe Lys Gln Lys Lys Lys Thr Glu Ala Glu Thr Pro
           35                  40                  45
   Lys Asn Ser Asp Ser Gly Val Ser Lys Gln Lys Pro Ile Glu Ile Pro
       50                  55                  60
   Thr Glu Val Val Lys Gln Pro Lys Asn Ala Asn Leu Ser Asp Leu Glu
   65                  70                  75                  80
   His Arg Ala Ser Arg Ile Thr Gly Glu Ile Met Arg Asn Arg Leu Thr
                   85                  90                  95
   Asp Arg Phe Lys Gln Ala Asp Glu Ser Pro Leu Glu Met Met Lys Arg
               100                 105                 110
   Ile Gln Ser Glu Ile Thr Thr Asp Thr Ile Ala Asp Gln Trp Glu Asn
           115                 120                 125
   Lys Leu Glu Glu Phe Gly Val Val Phe
       130                 135
<210> 86
   <211> 137
   <212> PRT
   <213> Artificial Sequence
<220>
   <221> SOURCE
   <222> 1..137
   <223> /mol_type="protein"
   /note="query"
   /organism="Artificial Sequence"
<400> 86
   Met Arg Val Ile Glu Gln Ser Leu Lys Gln Ile Asn Lys His Thr Asp
   1               5                   10                  15
   Ile Thr Ala Ser Tyr Glu Gln His Lys Lys Gly Arg Thr Ile Thr Gly
               20                  25                  30
   Phe Ser Phe Lys Phe Lys Gln Lys Lys Lys Thr Glu Pro Glu Thr Pro
           35                  40                  45
   Lys Asn Asn Asp Ser Gly Val Ser Lys Pro Lys Thr Val Glu Ile Pro
       50                  55                  60
   Ala Glu Val Val Lys Gln Pro Lys Asn Thr Asn Leu Ser Asp Leu Glu
   65                  70                  75                  80
   Lys Arg Val Arg Met Ile Thr Gly Ala Ile Ala Lys Asn Asn Leu Ala
                   85                  90                  95
   Ser Arg Phe Gln His Gly Asn Glu Ser Pro Leu Asp Met Met Lys Arg
               100                 105                 110
   Ile Gln Ser Glu Ile Thr Ser Asp Glu Thr Ala Asp Leu Trp Gln Asn
           115                 120                 125
   Lys Leu Glu Ser Met Gly Val Val Phe
       130                 135
<210> 87
   <211> 137
   <212> PRT
   <213> Artificial Sequence
<220>
   <221> SOURCE
   <222> 1..137
   <223> /mol_type="protein"
   /note="subject"
   /organism="Artificial Sequence"
<400> 87
   Met Arg Val Ile Glu Leu Ala Leu Lys Gln Ile Asn Asp Tyr Thr Asp
   1               5                   10                  15
   Ile Thr Ala Ser Tyr Glu Gln His Lys Lys Gly Arg Val Ile Thr Gly
               20                  25                  30
   Phe Ser Phe Lys Phe Lys Gln Lys Lys Lys Thr Glu Ala Glu Thr Pro
           35                  40                  45
   Lys Asn Ser Asp Ser Gly Val Ser Lys Gln Lys Pro Ile Glu Ile Pro
       50                  55                  60
   Thr Glu Val Val Lys Gln Pro Lys Asn Ala Asn Leu Ser Asp Leu Glu
   65                  70                  75                  80
   His Arg Ala Ser Arg Ile Thr Gly Glu Ile Met Arg Asn Arg Leu Thr
                   85                  90                  95
   Asp Arg Phe Lys Gln Ala Asp Glu Ser Pro Leu Glu Met Met Lys Arg
               100                 105                 110
   Ile Gln Ser Glu Ile Thr Thr Asp Thr Ile Ala Asp Gln Trp Glu Asn
           115                 120                 125
   Lys Leu Glu Glu Phe Gly Val Val Phe
       130                 135
<210> 88
   <211> 137
   <212> PRT
   <213> Artificial Sequence
<220>
   <221> SOURCE
   <222> 1..137
   <223> /mol_type="protein"
   /note="query"
   /organism="Artificial Sequence"
<400> 88
   Met Arg Val Ile Glu Gln Ser Leu Lys Gln Ile Asn Lys His Thr Asp
   1               5                   10                  15
   Ile Thr Ala Ser Tyr Glu Gln His Lys Lys Gly Arg Thr Ile Thr Gly
               20                  25                  30
   Phe Ser Phe Lys Phe Lys Gln Lys Lys Lys Thr Glu Pro Glu Thr Pro
           35                  40                  45
   Lys Asn Asn Asp Ser Gly Val Ser Lys Pro Lys Thr Val Glu Ile Pro
       50                  55                  60
   Ala Glu Val Val Lys Gln Pro Lys Asn Thr Asn Leu Ser Asp Leu Glu
   65                  70                  75                  80
   Lys Arg Val Arg Met Ile Thr Gly Ala Ile Ala Lys Asn Asn Leu Ala
                   85                  90                  95
   Ser Arg Phe Gln His Gly Asn Glu Ser Pro Leu Asp Met Met Lys Arg
               100                 105                 110
   Ile Gln Ser Glu Ile Thr Ser Asp Glu Thr Ala Asp Leu Trp Gln Asn
           115                 120                 125
   Lys Leu Glu Ser Met Gly Val Val Phe
       130                 135
<210> 89
   <211> 137
   <212> PRT
   <213> Artificial Sequence
<220>
   <221> SOURCE
   <222> 1..137
   <223> /mol_type="protein"
   /note="subject"
   /organism="Artificial Sequence"
<400> 89
   Met Gln Val Ile Glu Leu Ala Leu Lys Gln Ile Asn Glu His Thr Asp
   1               5                   10                  15
   Ile Thr Ala Ser Tyr Glu Gln His Lys Lys Gly Arg Val Ile Thr Gly
               20                  25                  30
   Phe Ser Phe Met Phe Lys His Lys Lys Gln Asn Ser Asp Lys Thr Pro
           35                  40                  45
   Asp Thr Asn Ala Ser Ser Pro Arg Ile Val Lys His Ser Gln Ile Pro
       50                  55                  60
   Thr Asn Ile Val Lys Gln Pro Glu Asn Ala Lys Met Ser Asp Leu Glu
   65                  70                  75                  80
   His Arg Ala Ser Arg Val Thr Gly Glu Ile Met Arg Asn Arg Leu Ser
                   85                  90                  95
   Asp Arg Phe Lys Gln Gly Asp Glu Ser Ala Ile Asp Met Met Lys Arg
               100                 105                 110
   Ile Gln Ser Glu Ile Ile Thr Asp Ala Ile Ala Asp Gln Trp Glu Ser
           115                 120                 125
   Lys Leu Glu Glu Phe Gly Val Val Phe
       130                 135
<210> 90
   <211> 1583
   <212> DNA
   <213> Artificial Sequence
<220>
   <221> source
   <222> 1..1583
   <223> /mol_type="DNA"
   /note="CMI4.158"
   /organism="Artificial Sequence"
<400> 90
   gaggacgaat taatattaca agtcgttggg tttcacaaat tggttatatg gacgatacag      60
   caaccgttga ggttattttt gcccctgcgg ttgttccatt gattacaaga ctggaggaac     120
   aattcaccca gtacgatatt gagcaaatta gcggtttatc tagtgcttat gccgtccgtc     180
   tgtatgaatt attgatctgt tggcgtagca caggaaaaac gcccgtcata gaactagcag 240
   agtttagaaa acgaataggt gttttagata ctgaatacac gagaacagat aacttaaaga 300
   tgcgagttat tgagctagca ctcaagcaga tcaacgaaca tacagacatc acagcaagct 360
   atgaacagca caaaaaaggg cggacaatca ctgggttctc attcaagttt aagcagaaga 420
   agaaaacaga gctagaaacg ccaaaaaaca gcgattctag cccacatatc gaaaaaccta 480
   gtcaaatccc taccaacatc gtaaaacagc ctgaaaacgc caaaaaagac gatttagggc 540
   atagagcaag taagattaca ggtttaatca tgtccaatgg tttagcagat cggtttaaac 600
   gtggtgatga gtccgtcatg aatatgatga aacgcatcaa agaagaaatt acaaccgatg 660
   caacagccga tctatggcaa aacaaacttg aatcaatggt gtggtttttt agtcatgact 720
   aaattggcta tgcaatgtct ccgctctttt tctaagaacc gtttttgctt tttcaaaatc 780
   gcggctcgcg ggtgtcttta ctggtatatg cgctttttcg tgctgctttt caaaaaatgt 840
   ggcgatagcc aacattctcg attttgatct tgattttggg gttttagggt ctccctaaca 900
   agacatagag acacaactcg ctagaggggt ctctatgtgt ccttgccctt aattaaaaaa 960
   ctaaataaat ttttacccgt ttttgcttta aaaaaggcag gtttttcttt gtccttactt 1020
   ttgactttga ttttctcctg tttttttgag ttacccacaa gaattttcat gtggttttgg 1080
   gcttttaagg tttttaaatg cttttaaatg cttttaaaca tgctgaaacg taagcatagc 1140
   aaggtataca gagttcattc tcctacgttt accgatcaat acccctacgt ttaccgatca 1200
   atacccctac gtttaccgat caatacccct acgtttaccg atcaataccc ctacgtttac 1260
   cttgcatata actacaaaaa acaataatgt agtaataact tcaaaataat aagagtaggt 1320
   tatgagcgat ttaatagtaa aagataacgc cctaatgaac gctagttaca acttagcttt 1380
   ggttgaacag cgtttaattc ttttagctat attggaagcg agagaaacag gaaaagggat 1440
   taatgccaat gatcccttaa cagttcatgc aaatagctat atcaatcaat ttaacgtgga 1500
   aagacatacg gcatatcaag cattaaaaga tgcctgcaaa gacttgtttg cacgtcaatt 1560
   cagttaccaa gaaaagcgag aac 1583
<210> 91
   <211> 1002
   <212> DNA
   <213> Artificial Sequence
<220>
   <221> source
   <222> 1..1002
   <223> /mol_type="DNA"
   /note="query"
   /organism="Artificial Sequence"
<400> 91
   tcaaaataat aagagtaggt tatgagcgat ttaatagtaa aagataacgc cctaatgaac      60
   gctagttaca acttagcttt ggttgaacag cgtttaattc ttttagctat attggaagcg     120
   agagaaacag gaaaagggat taatgccaat gatcccttaa cagttcatgc aaatagctat     180
   atcaatcaat ttaacgtgga aagacatacg gcatatcaag cattaaaaga tgcctgcaaa     240
   gacttgtttg cacgtcaatt cagttaccaa gaaaagcgag aacgaggacg aattaatatt     300
   acaagtcgtt gggtttcaca aattggttat atggacgata cagcaaccgt tgaggttatt     360
   tttgcccctg cggttgttcc attgattaca agactggagg aacaattcac ccagtacgat     420
   attgagcaaa ttagcggttt atctagtgct tatgccgtcc gtctgtatga attattgatc     480
   tgttggcgta gcacaggaaa aacgcccgtc atagaactag cagagtttag aaaacgaata     540
   ggtgttttag atactgaata cacgagaaca gataacttaa agatgcgagt tattgagcta     600
   gcactcaagc agatcaacga acatacagac atcacagcaa gctatgaaca gcacaaaaaa     660
   gggcggacaa tcactgggtt ctcattcaag tttaagcaga agaagaaaac agagctagaa     720
   acgccaaaaa acagcgattc tagcccacat atcgaaaaac ctagtcaaat ccctaccaac     780
   atcgtaaaac agcctgaaaa cgccaaaaaa gacgatttag ggcatagagc aagtaagatt     840
   acaggtttaa tcatgtccaa tggtttagca gatcggttta aacgtggtga tgagtccgtc     900
   atgaatatga tgaaacgcat caaagaagaa attacaaccg atgcaacagc cgatctatgg     960
   caaaacaaac ttgaatcaat ggtgtggttt tttagtcatg ac                       1002
<210> 92
   <211> 1003
   <212> DNA
   <213> Artificial Sequence
<220>
   <221> source
   <222> 1..1003
   <223> /mol_type="DNA"
   /note="subject"
   /organism="Artificial Sequence"
<400> 92
   tcaaaagaat aattgtaggt tatgagcgat ttaatagtaa aagataacgc cctaatgaat 60
   gctagttata acttagcttt ggttgaacag aggttaattc tattagcaat catagaagcg 120
   agagaaacag gcaaagggat taatgccaat gatcctctta cggttcatgc aggtagctat 180
   atcaatcaat ttaacgtaca aaggcatacg gcatatcaag ccctcaaaga tgcttgtaaa 240
   gacttgtttg cccgtcaatt cagttaccaa gaaaagcgag aacgaggacg aattaatatt 300
   acaagtcgat gggtttcgca aattggctat atggacgata cagcaaccgt tgagattatt 360
   tttgcccctg cggttgttcc tctgattaca cggctagagg aacagttcac ccagtacgat 420
   attgagcaaa ttagcggttt atcgagtgca tatgctgttc gtatgtacga actgctgatt 480
   tgttggcgta gcacaggcaa aacaccaatt attgagctag acgagtttag aaagcgaata 540
   ggtgttttag atactgaata cactagaaca gataatttaa agatgcaagt tattgaatta 600
   gccctaaaac aaatcaacga acatactgac atcacagcaa gctatgaaca acacaaaaaa 660
   gggcgagtga ttaccggatt ctcattcatg tttaagcaca agaaacaaaa cagcgataaa 720
   acgcctgata ctaacgcttc tagcccacgt atcgtaaaac atagtcaaat ccctaccaac 780
   attgtaaaac agcctgaaaa cgccaaaatg agcgatttag aacatagagc gagccgtgtt 840
   acaggggaaa taatgcgaaa tcgtctgtca gatcggttta aacaaggcga tgaatcagca 900
   atcgacatga tgaaacgtat tcaaagtgaa ataataaccg atgcaatagc agaccagtgg 960
   gaaagcaaac tggaggagtt tggcgtggtt ttttagtcat gac 1003
<210> 93
   <211> 178
   <212> DNA
   <213> Artificial Sequence
<220>
   <221> source
   <222> 1..178
   <223> /mol_type="DNA"
   /note="query"
   /organism="Artificial Sequence"
<400> 93
   aattttcatg tggttttggg cttttaaggt ttttaaatgc ttttaaatgc ttttaaacat 60
   gctgaaacgt aagcatagca aggtatacag agttcattct cctacgttta ccgatcaata     120
   cccctacgtt taccgatcaa tacccctacg tttaccgatc aataccccta cgtttacc       178
<210> 94
   <211> 176
   <212> DNA
   <213> Artificial Sequence
<220>
   <221> source
   <222> 1..176
   <223> /mol_type="DNA"
   /note="subject"
   /organism="Artificial Sequence"
<400> 94
   aattttcatg aagaaattgg gcttttaaag tttttaaatg ttttaaatgc ttttagacat      60
   gctaagaagc ccacacagca aggcatacag aggacattct cctacgttta ccgatcaata     120
   cccccgttta ccgatcaata cccctacgtt taccgatcaa tacccctacg tttacc         176
<210> 95
   <211> 324
   <212> PRT
   <213> Artificial Sequence
<220>
   <221> SOURCE
   <222> 1..324
   <223> /mol_type="protein"
   /note="query"
   /organism="Artificial Sequence"
<400> 95
   Met Ser Asp Leu Ile Val Lys Asp Asn Ala Leu Met Asn Ala Ser Tyr
   1               5                   10                  15
   Asn Leu Ala Leu Val Glu Gln Arg Leu Ile Leu Leu Ala Ile Leu Glu
               20                  25                  30
   Ala Arg Glu Thr Gly Lys Gly Ile Asn Ala Asn Asp Pro Leu Thr Val
           35                  40                  45
   His Ala Asn Ser Tyr Ile Asn Gln Phe Asn Val Glu Arg His Thr Ala
       50                  55                  60
   Tyr Gln Ala Leu Lys Asp Ala Cys Lys Asp Leu Phe Ala Arg Gln Phe
   65                  70                  75                  80
   Ser Tyr Gln Glu Lys Arg Glu Arg Gly Arg Ile Asn Ile Thr Ser Arg
                   85                  90                  95
   Trp Val Ser Gln Ile Gly Tyr Met Asp Asp Thr Ala Thr Val Glu Val
               100                 105                 110
   Ile Phe Ala Pro Ala Val Val Pro Leu Ile Thr Arg Leu Glu Glu Gln
           115                 120                 125
   Phe Thr Gln Tyr Asp Ile Glu Gln Ile Ser Gly Leu Ser Ser Ala Tyr
       130                 135                 140
   Ala Val Arg Leu Tyr Glu Leu Leu Ile Cys Trp Arg Ser Thr Gly Lys
   145                 150                 155                 160
   Thr Pro Val Ile Glu Leu Ala Glu Phe Arg Lys Arg Ile Gly Val Leu
                   165                 170                 175
   Asp Thr Glu Tyr Thr Arg Thr Asp Asn Leu Lys Met Arg Val Ile Glu
               180                 185                 190
   Leu Ala Leu Lys Gln Ile Asn Glu His Thr Asp Ile Thr Ala Ser Tyr
           195                 200                 205
   Glu Gln His Lys Lys Gly Arg Thr Ile Thr Gly Phe Ser Phe Lys Phe
       210                 215                 220
   Lys Gln Lys Lys Lys Thr Glu Leu Glu Thr Pro Lys Asn Ser Asp Ser
   225                 230                 235                 240
   Ser Pro His Ile Glu Lys Pro Ser Gln Ile Pro Thr Asn Ile Val Lys
                   245                 250                 255
   Gln Pro Glu Asn Ala Lys Lys Asp Asp Leu Gly His Arg Ala Ser Lys
               260                 265                 270
   Ile Thr Gly Leu Ile Met Ser Asn Gly Leu Ala Asp Arg Phe Lys Arg
           275                 280                 285
   Gly Asp Glu Ser Val Met Asn Met Met Lys Arg Ile Lys Glu Glu Ile
       290                 295                 300
   Thr Thr Asp Ala Thr Ala Asp Leu Trp Gln Asn Lys Leu Glu Ser Met
   305                 310                 315                 320
   Val Trp Phe Phe
<210> 96
   <211> 324
   <212> PRT
   <213> Artificial Sequence
<220>
   <221> SOURCE
   <222> 1..324
   <223> /mol_type="protein"
   /note="subject"
   /organism="Artificial Sequence"
<400> 96
   Met Ser Asp Leu Ile Val Lys Asp Asn Ala Leu Met Asn Ala Ser Tyr
   1               5                   10                  15
   Asn Leu Ala Leu Val Glu Gln Arg Leu Ile Leu Leu Ala Ile Ile Glu
               20                  25                  30
   Ala Arg Glu Thr Gly Lys Gly Ile Asn Ala Asn Asp Pro Leu Thr Val
           35                  40                  45
   His Ala Gly Ser Tyr Ile Asn Gln Phe Asn Val Gln Arg His Thr Ala
       50                  55                  60
   Tyr Gln Ala Leu Lys Asp Ala Cys Lys Asp Leu Phe Ala Arg Gln Phe
   65                  70                  75                  80
   Ser Tyr Gln Glu Lys Arg Glu Arg Gly Arg Ile Asn Ile Thr Ser Arg
                   85                  90                  95
   Trp Val Ser Gln Ile Gly Tyr Met Asp Asp Thr Ala Thr Val Glu Ile
               100                 105                 110
   Ile Phe Ala Pro Ala Val Val Pro Leu Ile Thr Arg Leu Glu Glu Gln
           115                 120                 125
   Phe Thr Gln Tyr Asp Ile Glu Gln Ile Ser Gly Leu Ser Ser Ala Tyr
       130                 135                 140
   Ala Val Arg Met Tyr Glu Leu Leu Ile Cys Trp Arg Ser Thr Gly Lys
   145                 150                 155                 160
   Thr Pro Ile Ile Glu Leu Asp Glu Phe Arg Lys Arg Ile Gly Val Leu
                   165                 170                 175
   Asp Thr Glu Tyr Thr Arg Thr Asp Asn Leu Lys Met Gln Val Ile Glu
               180                 185                 190
   Leu Ala Leu Lys Gln Ile Asn Glu His Thr Asp Ile Thr Ala Ser Tyr
           195                 200                 205
   Glu Gln His Lys Lys Gly Arg Val Ile Thr Gly Phe Ser Phe Met Phe
       210                 215                 220
   Lys His Lys Lys Gln Asn Ser Asp Lys Thr Pro Asp Thr Asn Ala Ser
   225                 230                 235                 240
   Ser Pro Arg Ile Val Lys His Ser Gln Ile Pro Thr Asn Ile Val Lys
                   245                 250                 255
   Gln Pro Glu Asn Ala Lys Met Ser Asp Leu Glu His Arg Ala Ser Arg
               260                 265                 270
   Val Thr Gly Glu Ile Met Arg Asn Arg Leu Ser Asp Arg Phe Lys Gln
           275                 280                 285
   Gly Asp Glu Ser Ala Ile Asp Met Met Lys Arg Ile Gln Ser Glu Ile
       290                 295                 300
   Ile Thr Asp Ala Ile Ala Asp Gln Trp Glu Ser Lys Leu Glu Glu Phe
   305                 310                 315                 320
   Gly Val Val Phe
<210> 97
   <211> 324
   <212> PRT
   <213> Artificial Sequence
<220>
   <221> SOURCE
   <222> 1..324
   <223> /mol_type="protein"
   /note="query"
   /organism="Artificial Sequence"
<400> 97
   Met Ser Asp Leu Ile Val Lys Asp Asn Ala Leu Met Asn Ala Ser Tyr
   1               5                   10                  15
   Asn Leu Ala Leu Val Glu Gln Arg Leu Ile Leu Leu Ala Ile Leu Glu
               20                  25                  30
   Ala Arg Glu Thr Gly Lys Gly Ile Asn Ala Asn Asp Pro Leu Thr Val
           35                  40                  45
   His Ala Asn Ser Tyr Ile Asn Gln Phe Asn Val Glu Arg His Thr Ala
       50                  55                  60
   Tyr Gln Ala Leu Lys Asp Ala Cys Lys Asp Leu Phe Ala Arg Gln Phe
   65                  70                  75                  80
   Ser Tyr Gln Glu Lys Arg Glu Arg Gly Arg Ile Asn Ile Thr Ser Arg
                   85                  90                  95
   Trp Val Ser Gln Ile Gly Tyr Met Asp Asp Thr Ala Thr Val Glu Val
               100                 105                 110
   Ile Phe Ala Pro Ala Val Val Pro Leu Ile Thr Arg Leu Glu Glu Gln
           115                 120                 125
   Phe Thr Gln Tyr Asp Ile Glu Gln Ile Ser Gly Leu Ser Ser Ala Tyr
       130                 135                 140
   Ala Val Arg Leu Tyr Glu Leu Leu Ile Cys Trp Arg Ser Thr Gly Lys
   145                 150                 155                 160
   Thr Pro Val Ile Glu Leu Ala Glu Phe Arg Lys Arg Ile Gly Val Leu
                   165                 170                 175
   Asp Thr Glu Tyr Thr Arg Thr Asp Asn Leu Lys Met Arg Val Ile Glu
               180                 185                 190
   Leu Ala Leu Lys Gln Ile Asn Glu His Thr Asp Ile Thr Ala Ser Tyr
           195                 200                 205
   Glu Gln His Lys Lys Gly Arg Thr Ile Thr Gly Phe Ser Phe Lys Phe
       210                 215                 220
   Lys Gln Lys Lys Lys Thr Glu Leu Glu Thr Pro Lys Asn Ser Asp Ser
   225                 230                 235                 240
   Ser Pro His Ile Glu Lys Pro Ser Gln Ile Pro Thr Asn Ile Val Lys
                   245                 250                 255
   Gln Pro Glu Asn Ala Lys Lys Asp Asp Leu Gly His Arg Ala Ser Lys
               260                 265                 270
   Ile Thr Gly Leu Ile Met Ser Asn Gly Leu Ala Asp Arg Phe Lys Arg
           275                 280                 285
   Gly Asp Glu Ser Val Met Asn Met Met Lys Arg Ile Lys Glu Glu Ile
       290                 295                 300
   Thr Thr Asp Ala Thr Ala Asp Leu Trp Gln Asn Lys Leu Glu Ser Met
   305                 310                 315                 320
   Val Trp Phe Phe
<210> 98
   <211> 324
   <212> PRT
   <213> Artificial Sequence
<220>
   <221> SOURCE
   <222> 1..324
   <223> /mol_type="protein"
   /note="subject"
   /organism="Artificial Sequence"
<400> 98
   Met Ser Glu Leu Ile Val Lys Asp Asn Ala Leu Met Asn Ala Ser Tyr
   1               5                   10                  15
   Asn Leu Asp Leu Val Glu Gln Arg Leu Ile Leu Leu Ala Ile Val Glu
               20                  25                  30
   Ala Arg Glu Ser Gly Lys Gly Ile Asn Ala Asn Asp Pro Leu Thr Val
           35                  40                  45
   His Ala Glu Ser Tyr Ile Asn Gln Phe Gly Val His Arg Thr Thr Ala
       50                  55                  60
   Tyr Gln Ala Leu Lys Asp Ala Cys Lys Asp Leu Phe Ala Arg Gln Phe
   65                  70                  75                  80
   Ser Tyr Gln Glu Lys Arg Glu Arg Gly Arg Ala Asn Ile Thr Ser Arg
                   85                  90                  95
   Trp Val Ser Gln Ile Ala Tyr Ile Asp Glu Thr Ala Thr Val Glu Ile
               100                 105                 110
   Ile Phe Ala Pro Ala Val Val Pro Leu Ile Thr Arg Leu Glu Lys Gln
           115                 120                 125
   Phe Thr Gln Tyr Asp Ile Glu Gln Ile Ser Gly Leu Ser Ser Ala Tyr
       130                 135                 140
   Ala Val Arg Met Tyr Glu Leu Leu Ile Cys Trp Arg Ser Thr Gly Lys
   145                 150                 155                 160
   Thr Pro Val Ile Glu Leu Gly Glu Phe Arg Lys Arg Ile Gly Val Leu
                   165                 170                 175
   Asp Thr Glu Tyr Ile Arg Thr Asp Asn Leu Lys Met Arg Val Ile Glu
               180                 185                 190
   Leu Ala Leu Lys Gln Ile Asn Asp His Thr Asp Ile Thr Ala Thr Tyr
           195                 200                 205
   Glu Gln His Lys Lys Gly Arg Thr Ile Thr Gly Phe Ser Phe Lys Phe
       210                 215                 220
   Lys Gln Lys Arg Lys Thr Glu Leu Glu Thr Pro Lys Asn Ser Asp Ser
   225                 230                 235                 240
   Asp Val Pro Lys Gln Lys Ser Val Glu Ile Pro Thr Asn Leu Val Lys
                   245                 250                 255
   Gln Pro Glu Asn Ala Asn Met Ser Asp Leu Gln His Arg Ala Ser Lys
               260                 265                 270
   Ile Thr Gly Leu Ile Met Ser Asn Arg Leu Ser Asp Arg Phe Lys Gln
           275                 280                 285
   Ser Asp Glu Ser Ile Met Gln Met Met Ala Arg Ile Gln Ser Glu Ile
       290                 295                 300
   Thr Asp Glu Ala Ile Ala Asn Gln Trp Glu Asn Lys Leu Glu Glu Phe
   305                 310                 315                 320
   Gly Val Ile Phe
<210> 99
   <211> 2280
   <212> DNA
   <213> Artificial Sequence
<220>
   <221> source
   <222> 1..2280
   <223> /mol_type="DNA"
   /note="HCBI7.228"
   /organism="Artificial Sequence"
<400> 99
   ctaaggcaga tcaacacagg gatagaataa cacgttttgg gattttgaaa catagagcga      60
   agctacaaga acaatatttg tggacgcagg ttgatttcaa atctgaaggc gaagacgatc     120
   tatccaataa ggctcttaag gctgcaacaa aattaaaagg ttgtggccag tttcttatat     180
   tcagaaatta ctacactatc gaccaagtta agctcgaaaa attccacgta tgtggacagc     240
   atttgctatg cccaatgtgt gcaggtattc gcgctgctcg ctcaatgaaa cgctatttag     300
   atcgtatcca tgaattaatg cgtcagaatc cgcgtttaaa gcctgttttg atcactttaa     360
   cggtcaagaa cggggaagac ttagaagaac gctataatca ccttacaagc tcatttagaa     420
   agcttctatc gcgttatagg gattacaaga aaaagggttg ggggtttaac caattctgta      480
   aaattgatgg tgctttctat accactgaat acacctacaa cgaaaagaca aaacaatggc     540
   atccgcatat tcatattttc gctttactca atgaatggat agaccaggaa gaattatctg 600
   agacctggca tgacattacc ctggattctt atatcatcga tattcgtaga gttaaaaaaa 660
   ccaaagaaca cggctatagc aaagctgttg ctgaggtatg taaatacgct cttaagtttg 720
   gcgatctatc agttgaaaac acttgggatg catttttaac gcttaagggt aaacgtctta 780
   caggctcttt cggttctatg catggagtta aaattccaga taaggccacg gatgatatgc 840
   cactggagga gctgccgtac ttagaaatgt tctatcgttt cgtgtttgat aaaaaatcct 900
   actacaacct agaaataaca aaagacgtaa cgccacaaac gaattctcga cagagtgagg 960
   aggcggcacc aacgaccgac acgactggag agaatgagtt gaaggcgcag gatgaagcga 1020
   ggacgttgcc ggtgcgaggg cacgcagtca cggaacgacc gcagcaagtc catgaatatt 1080
   ttgacccatt tgatattgag agagaagaat acgacttcta cccttaaggg gttcgacccc 1140
   ttaaacccca aagggcgcac ttatgcaaac ttttcaagtt cgccagtgct cccaccagta 1200
   acagagggcg cggagtgcgc ccgaactgac atcatctaaa tttcaaaacc ttctctccct 1260
   gattacaagc tcccttttgc ctttctccgg aaagttttag ccatcccaaa cgggtggcga 1320
   cgcattgaca attataattt aatctgcaat attcatcgga cattcataaa taatctgatt 1380
   ggaatattaa atatgctttt taaatctcta ggcgttgcaa tcgctacaaa taatgcttct 1440
   aactctgacg aagacttgaa accaattact atccgtttag ctgatagtga aattcaggcg 1500
   tatgacactg ttgcaaaatc aatgggtcta actcgtcaag attttctatc acacgttatt 1560
   cgttccaact tccgacaggc tttaaaagag tttgtagtgg gttatacaag ttcaaatcct 1620
   tctacaccct tatctgtttt gtttaattca aatacggaca atgacgaagt taagctcaaa 1680
   ctttccaacc ttctctcatc tatttctcaa gaattaatgg acgaagaaga aaaagaaatt 1740
   catgaagcaa tcgaaaacca tatgaatact tcctatgcat atgcaactcg tccaaaaggc 1800
   atctttgttg agaaggatga ttcaaaatga ttaagcttga agggatcgtt ttaaacgtat 1860
   ttacccagca aggtggtaaa gataaaaaaa caggtgatca atttgctgac cgtgacaagg 1920
   tgcaaatttt aggtgctatg gatctgccta acggtgacgt taaaaatgag ttatttacgt 1980
   tatctgtaga aaactatcgg gactttaaag acttcttaaa tcagaaaatc tgtattgctg 2040
   ttggtgcaat ggcaagtggt cgcaatgtta ttttttacgt tgctaaaggc gcaaagcctg 2100
   ttttagcaga aatgatgtga aacacgaaaa aaaacagtgg gttagccttg gcttctcgct 2160
   gttttttctt tctcttgata ctattaacta aagtggggat gaaattgact aaaaaagaca 2220
   ataaaaaagc ccataattct gacactttgg cgggtgatga attacaggct ttgcaatctg 2280
<210> 100
   <211> 874
   <212> DNA
   <213> Artificial Sequence
<220>
   <221> source
   <222> 1..874
   <223> /mol_type="DNA"
   /note="query"
   /organism="Artificial Sequence"
<400> 100
   caagaacaat atttgtggac gcaggttgat ttcaaatctg aaggcgaaga cgatctatcc      60
   aataaggctc ttaaggctgc aacaaaatta aaaggttgtg gccagtttct tatattcaga     120
   aattactaca ctatcgacca agttaagctc gaaaaattcc acgtatgtgg acagcatttg     180
   ctatgcccaa tgtgtgcagg tattcgcgct gctcgctcaa tgaaacgcta tttagatcgt     240
   atccatgaat taatgcgtca gaatccgcgt ttaaagcctg ttttgatcac tttaacggtc     300
   aagaacgggg aagacttaga agaacgctat aatcacctta caagctcatt tagaaagctt     360
   ctatcgcgtt atagggatta caagaaaaag ggttgggggt ttaaccaatt ctgtaaaatt     420
   gatggtgctt tctataccac tgaatacacc tacaacgaaa agacaaaaca atggcatccg     480
   catattcata ttttcgcttt actcaatgaa tggatagacc aggaagaatt atctgagacc     540
   tggcatgaca ttaccctgga ttcttatatc atcgatattc gtagagttaa aaaaaccaaa     600
   gaacacggct atagcaaagc tgttgctgag gtatgtaaat acgctcttaa gtttggcgat     660
   ctatcagttg aaaacacttg ggatgcattt ttaacgctta agggtaaacg tcttacaggc     720
   tctttcggtt ctatgcatgg agttaaaatt ccagataagg ccacggatga tatgccactg     780
   gaggagctgc cgtacttaga aatgttctat cgtttcgtgt ttgataaaaa atcctactac     840
   aacctagaaa taacaaaaga cgtaacgcca caaa                                 874
<210> 101
   <211> 871
   <212> DNA
   <213> Artificial Sequence
<220>
   <221> source
   <222> 1..871
   <223> /mol_type="DNA"
   /note="subject"
   /organism="Artificial Sequence"
<400> 101
   caagaacaat atttgtggac tcaggttgat ttcaaggttg gaagtgaaac atctatcaaa 60
   gctcttaagg ctgcaaccaa attaaaggga tgcggtcaat ttctgctttt tcgtaattac 120
   tacaccatag atcaaatcaa gctcgaaaaa ttccacgtat gcggacagca tttgctatgt 180
   ccaatgtgtg ctggtattcg tgctgcccgt tcaatgaatc ggtatattca acgcatcgaa 240
   gaaataatgc gtcagaatcg caagctaaag cccgtattga tcactttgac cgttaagaac 300
   ggtgaagacc tacaggaacg ctttgaacac ctcacaggct catttaagac gcttttacag 360
   cgttaccgtg attttaagaa aaagggtcga gggtttaatc aattttgcaa aattgatggc 420
   ggtttttata cgaccgaata cacctacaac gaaacaaccc aacaatggca tccgcatatt 480
   catatttttg cgttagtgac tgaccggatt gaccaggagg aactagcaga aacttggcac 540
   gatataacgc ttgattcata cattgtggac atccgcaggg tcaaaaaaac taaagaacac 600
   ggatatgcaa aggctgttgc cgaagtctgc aaatacgctc ttaagtttag cgatctatcc 660
   actgagaaaa cctttcaagc attttttgac ccttaagggc aaaaggctta caggttcatt 720
   cggctctatg catggtgtaa aaattcctga aagcggaccc gatgaaatgc ctaaagagga 780
   acttccatat cttgagctgc tctatcgttt cgtttttggt gaaaggtctt attacaacct 840
   agagttaact aaggacgtaa agccgaacaa a 871
<210> 102
   <211> 370
   <212> DNA
   <213> Artificial Sequence
<220>
   <221> source
   <222> 1..370
   <223> /mol_type="DNA"
   /note="query"
   /organism="Artificial Sequence"
<400> 102
   cgtgacaagg tgcaaatttt aggtgctatg gatctgccta acggtgacgt taaaaatgag 60
   ttatttacgt tatctgtaga aaactatcgg gactttaaag acttcttaaa tcagaaaatc 120
   tgtattgctg ttggtgcaat ggcaagtggt cgcaatgtta ttttttacgt tgctaaaggc 180
   gcaaagcctg ttttagcaga aatgatgtga aacacgaaaa aaaacagtgg gttagccttg 240
   gcttctcgct gttttttctt tctcttgata ctattaacta aagtggggat gaaattgact 300
   aaaaaagaca ataaaaaagc ccataattct gacactttgg cgggtgatga attacaggct 360
   ttgcaatctg 370
<210> 103
   <211> 370
   <212> DNA
   <213> Artificial Sequence
<220>
   <221> source
   <222> 1..370
   <223> /mol_type="DNA"
   /note="subject"
   /organism="Artificial Sequence"
<400> 103
   cgtgacaagg tgcaaattct tggttcgctg gagttgccca acggtgagat caaacatgag      60
   cttgttgact taacggttga agatgctcgc atctttgagc cattcaaaca caaggtaatt     120
   agcatttcat gcggtgctat ggctatcggt cgaaatgttg ttttttatgt tcgaaaaggt     180
   gcaaagcctg ttttagcaga tgtaatgtaa ttcatgaaaa aaaacagtga gttaggcttg     240
   ccgactcgct gttttttctt ttacttgata ctattaacta aagtggggac aaaattgact     300
   aaaaaagaca ataaaaaagc ccataattct gatgctttgg cgggcgacga attacaggct     360
   ttgcaatctg                                                            370
<210> 104
   <211> 52
   <212> DNA
   <213> Artificial Sequence
<220>
   <221> source
   <222> 1..52
   <223> /mol_type="DNA"
   /note="query"
   /organism="Artificial Sequence"
<400> 104
   ctaaggcaga tcaacacagg gatagaataa cacgttttgg gattttgaaa ca 52
<210> 105
   <211> 52
   <212> RNA
   <213> Artificial Sequence
<220>
   <221> source
   <222> 1..52
   <223> /mol_type="RNA"
   /note="subject"
   /organism="Artificial Sequence"
<400> 105
   cuaaugcaga ucaacacagg gauagaauau cacguuuugg acuuuugaaa ca 52
<210> 106
   <211> 112
   <212> DNA
   <213> Artificial Sequence
<220>
   <221> source
   <222> 1..112
   <223> /mol_type="DNA"
   /note="query"
   /organism="Artificial Sequence"
<400> 106
   aaggcagatc aacacaggga tagaataaca cgttttggga ttttgaaaca tagagcgaag      60
   ctacaagaac aatatttgtg gacgcaggtt gatttcaaat ctgaaggcga ag             112
<210> 107
   <211> 119
   <212> DNA
   <213> Artificial Sequence
<220>
   <221> source
   <222> 1..119
   <223> /mol_type="DNA"
   /note="subject"
   /organism="Artificial Sequence"
<400> 107
   aaggagatca acatagggat agaataacac gttttggcat tttgaaacat agatcgaagc      60
   aacaagaaaa ctatttattt tcgttagcta agattaaaga aaattatcat gccgatgta      119
<210> 108
   <211> 338
   <212> PRT
   <213> Artificial Sequence
<220>
   <221> SOURCE
   <222> 1..338
   <223> /mol_type="protein"
   /note="query"
   /organism="Artificial Sequence"
<400> 108
   Leu Thr Lys Lys Asp Asn Lys Lys Ala His Asn Ser Asp Thr Leu Ala
   1               5                   10                  15
   Gly Asp Glu Leu Gln Ala Leu Gln Ser Ala Lys Ala Asp Gln His Arg
               20                  25                  30
   Asp Arg Ile Thr Arg Phe Gly Ile Leu Lys His Arg Ala Lys Leu Gln
           35                  40                  45
   Glu Gln Tyr Leu Trp Thr Gln Val Asp Phe Lys Ser Glu Gly Glu Asp
       50                  55                  60
   Asp Leu Ser Asn Lys Ala Leu Lys Ala Ala Thr Lys Leu Lys Gly Cys
   65                  70                  75                  80
   Gly Gln Phe Leu Ile Phe Arg Asn Tyr Tyr Thr Ile Asp Gln Val Lys
                   85                  90                  95
   Leu Glu Lys Phe His Val Cys Gly Gln His Leu Leu Cys Pro Met Cys
               100                 105                 110
   Ala Gly Ile Arg Ala Ala Arg Ser Met Lys Arg Tyr Leu Asp Arg Ile
           115                 120                 125
   His Glu Leu Met Arg Gln Asn Pro Arg Leu Lys Pro Val Leu Ile Thr
       130                 135                 140
   Leu Thr Val Lys Asn Gly Glu Asp Leu Glu Glu Arg Tyr Asn His Leu
   145                 150                 155                 160
   Thr Ser Ser Phe Arg Lys Leu Leu Ser Arg Tyr Arg Asp Tyr Lys Lys
                   165                 170                 175
   Lys Gly Trp Gly Phe Asn Gln Phe Cys Lys Ile Asp Gly Ala Phe Tyr
               180                 185                 190
   Thr Thr Glu Tyr Thr Tyr Asn Glu Lys Thr Lys Gln Trp His Pro His
           195                 200                 205
   Ile His Ile Phe Ala Leu Leu Asn Glu Trp Ile Asp Gln Glu Glu Leu
       210                 215                 220
   Ser Glu Thr Trp His Asp Ile Thr Leu Asp Ser Tyr Ile Ile Asp Ile
   225                 230                 235                 240
   Arg Arg Val Lys Lys Thr Lys Glu His Gly Tyr Ser Lys Ala Val Ala
                   245                 250                 255
   Glu Val Cys Lys Tyr Ala Leu Lys Phe Gly Asp Leu Ser Val Glu Asn
               260                 265                 270
   Thr Trp Asp Ala Phe Leu Thr Leu Lys Gly Lys Arg Leu Thr Gly Ser
           275                 280                 285
   Phe Gly Ser Met His Gly Val Lys Ile Pro Asp Lys Ala Thr Asp Asp
       290                 295                 300
   Met Pro Leu Glu Glu Leu Pro Tyr Leu Glu Met Phe Tyr Arg Phe Val
   305                 310                 315                 320
   Phe Asp Lys Lys Ser Tyr Tyr Asn Leu Glu Ile Thr Lys Asp Val Thr
                   325                 330                 335
   Pro Gln
<210> 109
   <211> 342
   <212> PRT
   <213> Artificial Sequence
<220>
   <221> SOURCE
   <222> 1..342
   <223> /mol_type="protein"
   /note="subject"
   /organism="Artificial Sequence"
<400> 109
   Met Leu Lys Lys Asp Asn Lys Lys Ala His Ile Ser Glu Ala Leu Ala
   1               5                   10                  15
   Gly Gly Glu Ile Gln Ala Leu Gln Ser Ala Asn Ala Asp Gln His Arg
               20                  25                  30
   Asp Arg Ile Thr Arg Phe Ala Thr Leu Lys His Arg Ala Lys Asn Gln
           35                  40                  45
   Glu Asn Tyr Leu Phe Thr Leu Ala Lys Phe Lys Glu Asn Tyr Glu Lys
       50                  55                  60
   Asp Val Lys Asn Glu Glu Ser Ile Lys Ala Leu Lys Ser Ala Gln Lys
   65                  70                  75                  80
   Leu Asn Glu Cys Gly Asn Tyr Leu Leu Phe Lys Asn Phe Tyr Thr Ile
                   85                  90                  95
   Gly Glu Val Lys Leu Ser Lys Leu Arg Thr Cys Gly Gln His Leu Leu
               100                 105                 110
   Cys Pro Phe Cys Ala Ala Ile Arg Ala Ser Arg Ala Ile Gln Lys Tyr
           115                 120                 125
   Val Glu Arg Ile Asp Gln Val Leu Gln Glu Asn Arg Lys Leu Lys Pro
       130                 135                 140
   Val Leu Ile Thr Leu Thr Val Lys Asn Gly Ser Asp Leu Ala Glu Arg
   145                 150                 155                 160
   Ser Glu His Leu Met Lys Ser Phe Arg Thr Leu Leu Glu Arg Arg Arg
                   165                 170                 175
   Asp Tyr Glu Lys Lys Gly Arg Gly Phe Asn Glu Phe Cys Lys Val Gln
               180                 185                 190
   Gly Ala Met Tyr Ser Tyr Glu Asn Thr Phe Asn Glu Lys Thr Gly Glu
           195                 200                 205
   Trp His Pro His Ile His Met Phe Ala Leu Val Asp Gln Trp Ile Asp
       210                 215                 220
   Gln Gln Glu Phe Ser Glu Tyr Trp His Ser Leu Thr Gly Asp Ser Met
   225                 230                 235                 240
   Val Val Asp Val Arg Arg Ala Arg Lys Glu Lys Gly Tyr Gly Tyr Ser
                   245                 250                 255
   Lys Ala Ala Ala Glu Val Cys Lys Tyr Ala Leu Lys Phe Gly Asp Leu
               260                 265                 270
   Ser Val Glu Lys Thr Trp Glu Ala Phe Lys Val Leu Lys Gly Lys Arg
           275                 280                 285
   Leu Thr Gly Ser Phe Gly Leu Leu Trp Gly Val Lys Ile Pro Asp Ser
       290                 295                 300
   Met Thr Asp Asp Met Pro Ser Glu Asp Leu Pro Tyr Leu Glu Met Leu
   305                 310                 315                 320
   Tyr Lys Phe Ala Tyr Ser Lys Lys Ser Tyr Tyr Asp Leu Leu Ile Thr
                   325                 330                 335
   Arg His Val Glu Pro Gln
               340
<210> 110
   <211> 355
   <212> PRT
   <213> Artificial Sequence
<220>
   <221> SOURCE
   <222> 1..355
   <223> /mol_type="protein"
   /note="query"
   /organism="Artificial Sequence"
<400> 110
   Lys Lys Asp Asn Lys Lys Ala His Asn Ser Asp Thr Leu Ala Gly Asp
   1               5                   10                  15
   Glu Leu Gln Ala Leu Gln Ser Ala Lys Ala Asp Gln His Arg Asp Arg
               20                  25                  30
   Ile Thr Arg Phe Gly Ile Leu Lys His Arg Ala Lys Leu Gln Glu Gln
           35                  40                  45
   Tyr Leu Trp Thr Gln Val Asp Phe Lys Ser Glu Gly Glu Asp Asp Leu
       50                  55                  60
   Ser Asn Lys Ala Leu Lys Ala Ala Thr Lys Leu Lys Gly Cys Gly Gln
   65                  70                  75                  80
   Phe Leu Ile Phe Arg Asn Tyr Tyr Thr Ile Asp Gln Val Lys Leu Glu
                   85                  90                  95
   Lys Phe His Val Cys Gly Gln His Leu Leu Cys Pro Met Cys Ala Gly
               100                 105                 110
   Ile Arg Ala Ala Arg Ser Met Lys Arg Tyr Leu Asp Arg Ile His Glu
           115                 120                 125
   Leu Met Arg Gln Asn Pro Arg Leu Lys Pro Val Leu Ile Thr Leu Thr
       130                 135                 140
   Val Lys Asn Gly Glu Asp Leu Glu Glu Arg Tyr Asn His Leu Thr Ser
   145                 150                 155                 160
   Ser Phe Arg Lys Leu Leu Ser Arg Tyr Arg Asp Tyr Lys Lys Lys Gly
                   165                 170                 175
   Trp Gly Phe Asn Gln Phe Cys Lys Ile Asp Gly Ala Phe Tyr Thr Thr
               180                 185                 190
   Glu Tyr Thr Tyr Asn Glu Lys Thr Lys Gln Trp His Pro His Ile His
           195                 200                 205
   Ile Phe Ala Leu Leu Asn Glu Trp Ile Asp Gln Glu Glu Leu Ser Glu
       210                 215                 220
   Thr Trp His Asp Ile Thr Leu Asp Ser Tyr Ile Ile Asp Ile Arg Arg
   225                 230                 235                 240
   Val Lys Lys Thr Lys Glu His Gly Tyr Ser Lys Ala Val Ala Glu Val
                   245                 250                 255
   Cys Lys Tyr Ala Leu Lys Phe Gly Asp Leu Ser Val Glu Asn Thr Trp
               260                 265                 270
   Asp Ala Phe Leu Thr Leu Lys Gly Lys Arg Leu Thr Gly Ser Phe Gly
           275                 280                 285
   Ser Met His Gly Val Lys Ile Pro Asp Lys Ala Thr Asp Asp Met Pro
       290                 295                 300
   Leu Glu Glu Leu Pro Tyr Leu Glu Met Phe Tyr Arg Phe Val Phe Asp
   305                 310                 315                 320
   Lys Lys Ser Tyr Tyr Asn Leu Glu Ile Thr Lys Asp Val Thr Pro Gln
                   325                 330                 335
   Thr Asn Ser Arg Gln Ser Glu Glu Ala Ala Pro Thr Thr Asp Thr Thr
               340                 345                 350
   Gly Glu Asn
           355
<210> 111
   <211> 360
   <212> PRT
   <213> Artificial Sequence
<220>
   <221> SOURCE
   <222> 1..360
   <223> /mol_type="protein"
   /note="subject"
   /organism="Artificial Sequence"
<400> 111
   Gln Lys Asp Ile Lys Lys Pro Leu Leu Ser Ser Thr Leu Val Gly Gly
   1               5                   10                  15
   Asp Asn Lys Gly Ser Asn Ser Val Glu Ala Asp Gln His Arg Asp Arg
               20                  25                  30
   Ile Thr Arg Phe Gly Ile Leu Lys His Arg Ser Lys Gln Gln Glu Asn
           35                  40                  45
   Tyr Leu Trp Thr Leu Ala Lys Tyr Lys Glu Asn Tyr Gln Asn Asp Gln
       50                  55                  60
   Pro Asn Glu Glu Ser Val Arg Ala Thr Lys Ser Ala Gln Lys Leu Leu
   65                  70                  75                  80
   Gly Cys Gly Asn Phe Leu Leu Phe Lys Asn Phe Tyr Thr Ile Asp Gln
                   85                  90                  95
   Val Lys Leu Ser Lys Phe His Val Cys Asn Gln His Leu Leu Cys Pro
               100                 105                 110
   Phe Cys Ala Gly Ile Arg Ala Ser Lys Ala Ile Gln Lys Tyr Thr Glu
           115                 120                 125
   Arg Val Asp Glu Val Leu Ser Lys Lys Arg Thr Leu Lys Pro Val Leu
       130                 135                 140
   Ile Thr Phe Thr Val Lys Asn Gly Thr Asp Leu Gly Glu Arg Ser Ser
   145                 150                 155                 160
   His Leu Met Lys Ser Phe Arg Thr Leu Met Glu Arg Arg Arg Asp Tyr
                   165                 170                 175
   Leu Lys Lys Gly Arg Gly Phe Asn Glu Phe Cys Lys Ile Asn Gly Ala
               180                 185                 190
   Met Tyr Ser Tyr Glu Asn Thr Tyr Asn Glu Gln Thr Lys Glu Trp His
           195                 200                 205
   Pro His Leu His Met Phe Ala Leu Leu Asp Asp Trp Ile Asp Gln Glu
       210                 215                 220
   Glu Leu Ser Gln Tyr Trp His Ser Ile Thr Gly Asp Ser Met Ile Val
   225                 230                 235                 240
   Asp Ile Arg Lys Val Lys Lys Glu Lys Gly Leu Gly Tyr Ser Lys Ala
                   245                 250                 255
   Ala Ala Glu Val Cys Lys Tyr Ala Leu Lys Phe Gly Asp Leu Ser Val
               260                 265                 270
   Glu Asn Thr Trp Glu Ala Phe Lys Ile Leu Lys Gly Lys Arg Leu Ser
           275                 280                 285
   Gly Ala Phe Gly Ser Leu Tyr Gly Val Lys Ile Pro Glu Asn Leu Ala
       290                 295                 300
   Asp Glu Met Pro Asp Glu Asn Asp Leu Pro Tyr Leu Glu Met Leu Tyr
   305                 310                 315                 320
   Lys Phe Val Phe Gly Lys Lys Ser Tyr Tyr Asp Leu Thr Met Thr Arg
                   325                 330                 335
   His Val Glu Pro Arg Ala Gln Asp Asp Arg Pro Met Glu Lys Glu Leu
               340                 345                 350
   Arg Arg Pro Thr Glu Gly Glu Ser
           355                 360
<210> 112
   <211> 232
   <212> PRT
   <213> Artificial Sequence
<220>
   <221> SOURCE
   <222> 1..232
   <223> /mol_type="protein"
   /note="query"
   /organism="Artificial Sequence"
<400> 112
   Leu Gln Glu Gln Tyr Leu Trp Thr Gln Val Asp Phe Lys Ser Glu Gly
   1               5                   10                  15
   Glu Asp Asp Leu Ser Asn Lys Ala Leu Lys Ala Ala Thr Lys Leu Lys
               20                  25                  30
   Gly Cys Gly Gln Phe Leu Ile Phe Arg Asn Tyr Tyr Thr Ile Asp Gln
           35                  40                  45
   Val Lys Leu Glu Lys Phe His Val Cys Gly Gln His Leu Leu Cys Pro
       50                  55                  60
   Met Cys Ala Gly Ile Arg Ala Ala Arg Ser Met Lys Arg Tyr Leu Asp
   65                  70                  75                  80
   Arg Ile His Glu Leu Met Arg Gln Asn Pro Arg Leu Lys Pro Val Leu
                   85                  90                  95
   Ile Thr Leu Thr Val Lys Asn Gly Glu Asp Leu Glu Glu Arg Tyr Asn
               100                 105                 110
   His Leu Thr Ser Ser Phe Arg Lys Leu Leu Ser Arg Tyr Arg Asp Tyr
           115                 120                 125
   Lys Lys Lys Gly Trp Gly Phe Asn Gln Phe Cys Lys Ile Asp Gly Ala
       130                 135                 140
   Phe Tyr Thr Thr Glu Tyr Thr Tyr Asn Glu Lys Thr Lys Gln Trp His
   145                 150                 155                 160
   Pro His Ile His Ile Phe Ala Leu Leu Asn Glu Trp Ile Asp Gln Glu
                   165                 170                 175
   Glu Leu Ser Glu Thr Trp His Asp Ile Thr Leu Asp Ser Tyr Ile Ile
               180                 185                 190
   Asp Ile Arg Arg Val Lys Lys Thr Lys Glu His Gly Tyr Ser Lys Ala
           195                 200                 205
   Val Ala Glu Val Cys Lys Tyr Ala Leu Lys Phe Gly Asp Leu Ser Val
       210                 215                 220
   Glu Asn Thr Trp Asp Ala Phe Leu
   225                 230
<210> 113
   <211> 230
   <212> PRT
   <213> Artificial Sequence
<220>
   <221> SOURCE
   <222> 1..230
   <223> /mol_type="protein"
   /note="subject"
   /organism="Artificial Sequence"
<400> 113
   Met Gln Glu Gln Tyr Leu Trp Thr Gln Val Asp Phe Lys Val Gly Ser
   1               5                   10                  15
   Glu Thr Ser Ile Lys Ala Leu Lys Ala Ala Thr Lys Leu Lys Gly Cys
               20                  25                  30
   Gly Gln Phe Leu Leu Phe Arg Asn Tyr Tyr Thr Ile Asp Gln Ile Lys
           35                  40                  45
   Leu Glu Lys Phe His Val Cys Gly Gln His Leu Leu Cys Pro Met Cys
       50                  55                  60
   Ala Gly Ile Arg Ala Ala Arg Ser Met Asn Arg Tyr Ile Gln Arg Ile
   65                  70                  75                  80
   Glu Glu Ile Met Arg Gln Asn Arg Lys Leu Lys Pro Val Leu Ile Thr
                   85                  90                  95
   Leu Thr Val Lys Asn Gly Glu Asp Leu Gln Glu Arg Phe Glu His Leu
               100                 105                 110
   Thr Gly Ser Phe Lys Thr Leu Leu Gln Arg Tyr Arg Asp Phe Lys Lys
           115                 120                 125
   Lys Gly Arg Gly Phe Asn Gln Phe Cys Lys Ile Asp Gly Gly Phe Tyr
       130                 135                 140
   Thr Thr Glu Tyr Thr Tyr Asn Glu Thr Thr Gln Gln Trp His Pro His
   145                 150                 155                 160
   Ile His Ile Phe Ala Leu Val Thr Asp Arg Ile Asp Gln Glu Glu Leu
                   165                 170                 175
   Ala Glu Thr Trp His Asp Ile Thr Leu Asp Ser Tyr Ile Val Asp Ile
               180                 185                 190
   Arg Arg Val Lys Lys Thr Lys Glu His Gly Tyr Ala Lys Ala Val Ala
           195                 200                 205
   Glu Val Cys Lys Tyr Ala Leu Lys Phe Ser Asp Leu Ser Thr Glu Lys
       210                 215                 220
   Thr Phe Gln Ala Phe Phe
   225                 230
<210> 114
   <211> 245
   <212> PRT
   <213> Artificial Sequence
<220>
   <221> SOURCE
   <222> 1..245
   <223> /mol_type="protein"
   /note="query"
   /organism="Artificial Sequence"
<400> 114
   Met Cys Ala Gly Ile Arg Ala Ala Arg Ser Met Lys Arg Tyr Leu Asp
   1               5                   10                  15
   Arg Ile His Glu Leu Met Arg Gln Asn Pro Arg Leu Lys Pro Val Leu
               20                  25                  30
   Ile Thr Leu Thr Val Lys Asn Gly Glu Asp Leu Glu Glu Arg Tyr Asn
           35                  40                  45
   His Leu Thr Ser Ser Phe Arg Lys Leu Leu Ser Arg Tyr Arg Asp Tyr
       50                  55                  60
   Lys Lys Lys Gly Trp Gly Phe Asn Gln Phe Cys Lys Ile Asp Gly Ala
   65                  70                  75                  80
   Phe Tyr Thr Thr Glu Tyr Thr Tyr Asn Glu Lys Thr Lys Gln Trp His
                   85                  90                  95
   Pro His Ile His Ile Phe Ala Leu Leu Asn Glu Trp Ile Asp Gln Glu
               100                 105                 110
   Glu Leu Ser Glu Thr Trp His Asp Ile Thr Leu Asp Ser Tyr Ile Ile
           115                 120                 125
   Asp Ile Arg Arg Val Lys Lys Thr Lys Glu His Gly Tyr Ser Lys Ala
       130                 135                 140
   Val Ala Glu Val Cys Lys Tyr Ala Leu Lys Phe Gly Asp Leu Ser Val
   145                 150                 155                 160
   Glu Asn Thr Trp Asp Ala Phe Leu Thr Leu Lys Gly Lys Arg Leu Thr
                   165                 170                 175
   Gly Ser Phe Gly Ser Met His Gly Val Lys Ile Pro Asp Lys Ala Thr
               180                 185                 190
   Asp Asp Met Pro Leu Glu Glu Leu Pro Tyr Leu Glu Met Phe Tyr Arg
           195                 200                 205
   Phe Val Phe Asp Lys Lys Ser Tyr Tyr Asn Leu Glu Ile Thr Lys Asp
       210                 215                 220
   Val Thr Pro Gln Thr Asn Ser Arg Gln Ser Glu Glu Ala Ala Pro Thr
   225                 230                 235                 240
   Thr Asp Thr Thr Gly
                   245
<210> 115
   <211> 247
   <212> PRT
   <213> Artificial Sequence
<220>
   <221> SOURCE
   <222> 1..247
   <223> /mol_type="protein"
   /note="subject"
   /organism="Artificial Sequence"
<400> 115
   Phe Cys Ala Gly Ile Arg Ala Ser Lys Ser Ile Gln Lys Tyr Thr Asp
   1               5                   10                  15
   Arg Val Asp Glu Val Leu Ser Lys Asn Arg Lys Leu Lys Pro Val Leu
               20                  25                  30
   Ile Thr Phe Thr Val Lys Asn Gly Thr Asp Leu Gly Glu Arg Ser Ser
           35                  40                  45
   His Leu Ile Lys Ser Phe Arg Thr Leu Leu Glu Arg Arg Arg Asp Tyr
       50                  55                  60
   Leu Lys Lys Gly Arg Gly Phe Asn Glu Phe Cys Lys Ile Asp Gly Ala
   65                  70                  75                  80
   Met Tyr Ser Tyr Glu Asn Thr Phe Asn Glu Ser Thr Lys Glu Trp His
                   85                  90                  95
   Pro His Leu His Leu Phe Ala Leu Leu Asp Glu Trp Ile Asp Gln Glu
               100                 105                 110
   Gln Leu Ser Glu Leu Trp His Asp Ile Thr Gly Asp Ser Tyr Ile Val
           115                 120                 125
   Asp Val Arg Lys Val Lys Lys Ser Lys Asp Thr Gly Tyr Ala Lys Ala
       130                 135                 140
   Ala Ala Glu Val Cys Lys Tyr Ala Leu Lys Phe Gly Asp Leu Thr Val
   145                 150                 155                 160
   Glu Asn Thr Trp Glu Ala Phe Lys Ile Leu Lys Gly Lys Arg Leu Ser
                   165                 170                 175
   Gly Ala Phe Gly Ser Leu Tyr Gly Val Lys Ile Pro Asp Asn Pro Ala
               180                 185                 190
   Asp Asp Ile Asp Asp Ser Asp Leu Pro Tyr Leu Glu Met Val Tyr Lys
           195                 200                 205
   Phe Val Phe Gly Lys Lys Ser Tyr Tyr Asp Leu Thr Met Thr Arg His
       210                 215                 220
   Val Glu Pro Gln Gly Gln Asp Asn Asp Asp Asn Glu Glu Lys Glu Gly
   225                 230                 235                 240
   Ala Thr Ser Asp Arg Gly Gly
                   245
<210> 116
   <211> 245
   <212> PRT
   <213> Artificial Sequence
<220>
   <221> SOURCE
   <222> 1..245
   <223> /mol_type="protein"
   /note="query"
   /organism="Artificial Sequence"
<400> 116
   Cys Ala Gly Ile Arg Ala Ala Arg Ser Met Lys Arg Tyr Leu Asp Arg
   1 5 10 15
   Ile His Glu Leu Met Arg Gln Asn Pro Arg Leu Lys Pro Val Leu Ile
   20 25 30
   Thr Leu Thr Val Lys Asn Gly Glu Asp Leu Glu Glu Arg Tyr Asn His
   35 40 45
   Leu Thr Ser Ser Phe Arg Lys Leu Leu Ser Arg Tyr Arg Asp Tyr Lys
   50 55 60
   Lys Lys Gly Trp Gly Phe Asn Gln Phe Cys Lys Ile Asp Gly Ala Phe
   65 70 75 80
   Tyr Thr Thr Glu Tyr Thr Tyr Asn Glu Lys Thr Lys Gln Trp His Pro
   85 90 95
   His Ile His Ile Phe Ala Leu Leu Asn Glu Trp Ile Asp Gln Glu Glu
   100 105 110
   Leu Ser Glu Thr Trp His Asp Ile Thr Leu Asp Ser Tyr Ile Ile Asp
   115 120 125
   Ile Arg Arg Val Lys Lys Thr Lys Glu His Gly Tyr Ser Lys Ala Val
   130 135 140
   Ala Glu Val Cys Lys Tyr Ala Leu Lys Phe Gly Asp Leu Ser Val Glu
   145 150 155 160
   Asn Thr Trp Asp Ala Phe Leu Thr Leu Lys Gly Lys Arg Leu Thr Gly
   165 170 175
   Ser Phe Gly Ser Met His Gly Val Lys Ile Pro Asp Lys Ala Thr Asp
   180 185 190
   Asp Met Pro Leu Glu Glu Leu Pro Tyr Leu Glu Met Phe Tyr Arg Phe
   195 200 205
   Val Phe Asp Lys Lys Ser Tyr Tyr Asn Leu Glu Ile Thr Lys Asp Val
   210 215 220
   Thr Pro Gln Thr Asn Ser Arg Gln Ser Glu Glu Ala Ala Pro Thr Thr
   225 230 235 240
   Asp Thr Thr Gly Glu
   245
<210> 117
   <211> 245
   <212> PRT
   <213> Artificial Sequence
<220>
   <221> SOURCE
   <222> 1..245
   <223> /mol_type="protein"
   /note="subject"
   /organism="Artificial Sequence"
<400> 117
   Cys Ala Ala Ile Arg Ala Ser Arg Ala Ile Gln Lys Tyr Val Glu Arg
   1 5 10 15
   Ile Asp Gln Val Leu Gln Glu Asn Arg Lys Leu Lys Pro Val Leu Ile
   20 25 30
   Thr Leu Thr Val Lys Asn Gly Ser Asp Leu Ala Glu Arg Ser Glu His
   35 40 45
   Leu Met Lys Ser Phe Arg Lys Leu Leu Asp Arg Arg Arg Asp Tyr Glu
   50 55 60
   Lys Lys Gly Arg Gly Phe Asn Glu Phe Cys Lys Val Gln Gly Ala Met
   65 70 75 80
   Tyr Ser Tyr Glu Asn Thr Phe Asn Glu Lys Thr Gly Glu Trp His Pro
                   85                  90                  95
   His Ile His Met Phe Ala Leu Val Asp Gln Trp Ile Asp Gln Gln Glu
               100                 105                 110
   Phe Ser Glu Tyr Trp His Ser Leu Thr Gly Asp Ser Met Val Val Asp
           115                 120                 125
   Val Arg Arg Ala Lys Lys Glu Lys Gly His Gly Tyr Ser Lys Ala Ala
       130                 135                 140
   Ala Glu Val Cys Lys Tyr Ala Leu Lys Phe Gly Asp Leu Ser Val Glu
   145                 150                 155                 160
   Lys Thr Trp Glu Ala Phe Lys Val Leu Lys Gly Lys Arg Leu Thr Gly
                   165                 170                 175
   Ser Phe Gly Leu Leu Trp Gly Val Lys Ile Pro Asp Thr Met Thr Asp
               180                 185                 190
   Asp Met Pro Glu Glu Asp Leu Pro Tyr Leu Glu Met Leu Tyr Lys Phe
           195                 200                 205
   Ala Tyr Gly Lys Lys Ser Tyr Tyr Asp Leu Leu Ile Thr Arg His Val
       210                 215                 220
   Glu Pro Lys Ala Lys Asp Asp Asn Asp Glu Lys Glu Glu Pro Arg Gln
   225                 230                 235                 240
   Arg Arg Gly Gly Glu
                   245
<210> 118
   <211> 228
   <212> PRT
   <213> Artificial Sequence
<220>
   <221> SOURCE
   <222> 1..228
   <223> /mol_type="protein"
   /note="query"
   /organism="Artificial Sequence"
<400> 118 210 215 220 Val Thr Pro Gln 225
<210> 119
   <211> 228
   <212> PRT
   <213> Artificial Sequence
<220>
   <221> SOURCE
   <222> 1..228
   <223> /mol_type="protein"
   /note="subject"
   /organism="Artificial Sequence"
<400> 119
   Phe Cys Ala Ala Ile Arg Ala Ser Arg Ala Ile Gln Lys Tyr Val Glu
   1               5                   10                  15
   Arg Ile Asp Gln Val Leu Gln Glu Asn Arg Lys Leu Lys Pro Val Leu
               20                  25                  30
   Ile Thr Leu Thr Val Lys Asn Gly Ser Asp Leu Ala Glu Arg Ser Glu
           35                  40                  45
   His Leu Met Lys Ser Phe Arg Thr Leu Leu Glu Arg Arg Arg Asp Tyr
       50                  55                  60
   Glu Lys Lys Gly Arg Gly Phe Asn Glu Phe Cys Lys Val Gln Gly Ala
   65                  70                  75                  80
   Met Tyr Ser Tyr Glu Asn Thr Phe Asn Glu Lys Thr Gly Glu Trp His
                   85                  90                  95
   Pro His Ile His Met Phe Ala Leu Val Asp Gln Trp Ile Asp Gln Gln
               100                 105                 110
   Glu Phe Ser Glu Tyr Trp His Ser Leu Thr Gly Asp Ser Met Val Val
           115                 120                 125
   Asp Val Arg Arg Ala Arg Lys Glu Lys Gly Tyr Gly Tyr Ser Lys Ala
       130                 135                 140
   Ala Ala Glu Val Cys Lys Tyr Ala Leu Lys Phe Gly Asp Leu Ser Val
   145                 150                 155                 160
   Glu Lys Thr Trp Glu Ala Phe Lys Val Leu Lys Gly Lys Arg Leu Thr
                   165                 170                 175
   Gly Ser Phe Gly Leu Leu Trp Gly Val Lys Ile Pro Asp Ser Met Thr
               180                 185                 190
   Asp Asp Met Pro Ser Glu Asp Leu Pro Tyr Leu Glu Met Leu Tyr Lys
           195                 200                 205
   Phe Ala Tyr Ser Lys Lys Ser Tyr Tyr Asp Leu Leu Ile Thr Arg His
       210                 215                 220
   Val Glu Pro Gln
   225
<210> 120
   <211> 168
   <212> PRT
   <213> Artificial Sequence
<220>
   <221> SOURCE
   <222> 1..168
   <223> /mol_type="protein"
   /note="query"
   /organism="Artificial Sequence"
<400> 120
   Met Cys Ala Gly Ile Arg Ala Ala Arg Ser Met Lys Arg Tyr Leu Asp
   1               5                   10                  15
   Arg Ile His Glu Leu Met Arg Gln Asn Pro Arg Leu Lys Pro Val Leu
               20                  25                  30
   Ile Thr Leu Thr Val Lys Asn Gly Glu Asp Leu Glu Glu Arg Tyr Asn
           35                  40                  45
   His Leu Thr Ser Ser Phe Arg Lys Leu Leu Ser Arg Tyr Arg Asp Tyr
       50                  55                  60
   Lys Lys Lys Gly Trp Gly Phe Asn Gln Phe Cys Lys Ile Asp Gly Ala
   65                  70                  75                  80
   Phe Tyr Thr Thr Glu Tyr Thr Tyr Asn Glu Lys Thr Lys Gln Trp His
                   85                  90                  95
   Pro His Ile His Ile Phe Ala Leu Leu Asn Glu Trp Ile Asp Gln Glu
               100                 105                 110
   Glu Leu Ser Glu Thr Trp His Asp Ile Thr Leu Asp Ser Tyr Ile Ile
           115                 120                 125
   Asp Ile Arg Arg Val Lys Lys Thr Lys Glu His Gly Tyr Ser Lys Ala
       130                 135                 140
   Val Ala Glu Val Cys Lys Tyr Ala Leu Lys Phe Gly Asp Leu Ser Val
   145                 150                 155                 160
   Glu Asn Thr Trp Asp Ala Phe Leu
                   165
<210> 121
   <211> 168
   <212> PRT
   <213> Artificial Sequence
<220>
   <221> SOURCE
   <222> 1..168
   <223> /mol_type="protein"
   /note="subject"
   /organism="Artificial Sequence"
<400> 121
   Met Cys Ala Gly Ile Arg Ala Ala Arg Ser Met Asn Arg Tyr Ile Gln
   1               5                   10                  15
   Arg Ile Glu Glu Ile Met Arg Gln Asn Arg Lys Leu Lys Pro Val Leu
               20                  25                  30
   Ile Thr Leu Thr Val Lys Asn Gly Glu Asp Leu Gln Glu Arg Phe Glu
           35                  40                  45
   His Leu Thr Gly Ser Phe Lys Thr Leu Leu Gln Arg Tyr Arg Asp Phe
       50                  55                  60
   Lys Lys Lys Gly Arg Gly Phe Asn Gln Phe Cys Lys Ile Asp Gly Gly
   65                  70                  75                  80
   Phe Tyr Thr Thr Glu Tyr Thr Tyr Asn Glu Thr Thr Gln Gln Trp His
                   85                  90                  95
   Pro His Ile His Ile Phe Ala Leu Val Thr Asp Arg Ile Asp Gln Glu
               100                 105                 110
   Glu Leu Ala Glu Thr Trp His Asp Ile Thr Leu Asp Ser Tyr Ile Val
           115                 120                 125
   Asp Ile Arg Arg Val Lys Lys Thr Lys Glu His Gly Tyr Ala Lys Ala
       130                 135                 140
   Val Ala Glu Val Cys Lys Tyr Ala Leu Lys Phe Ser Asp Leu Ser Thr
   145                 150                 155                 160
   Glu Lys Thr Phe Gln Ala Phe Phe
                   165
<210> 122
   <211> 78
   <212> PRT
   <213> Artificial Sequence
<220>
   <221> SOURCE
   <222> 1..78
   <223> /mol_type="protein"
   /note="query"
   /organism="Artificial Sequence"
<400> 122
   Ser Asp Glu Asp Leu Lys Pro Ile Thr Ile Arg Leu Ala Asp Ser Glu
   1               5                   10                  15
   Ile Gln Ala Tyr Asp Thr Val Ala Lys Ser Met Gly Leu Thr Arg Gln
               20                  25                  30
   Asp Phe Leu Ser His Val Ile Arg Ser Asn Phe Arg Gln Ala Leu Lys
           35                  40                  45
   Glu Phe Val Val Gly Tyr Thr Ser Ser Asn Pro Ser Thr Pro Leu Ser
       50                  55                  60
   Val Leu Phe Asn Ser Asn Thr Asp Asn Asp Glu Val Lys Leu
   65                  70                  75
<210> 123
   <211> 70
   <212> PRT
   <213> Artificial Sequence
<220>
   <221> SOURCE
   <222> 1..70
   <223> /mol_type="protein"
   /note="subject"
   /organism="Artificial Sequence"
<400> 123
   Ala Asp Gly Leu Ile Arg Pro Pro Ser Gln Arg Glu Ile Gln Ala His
   1               5                   10                  15
   Ser Thr Ala Lys Tyr Gly Ala Leu Ala Arg Gln Arg Tyr Arg Met Glu
               20                  25                  30
   Thr Ser Phe Pro Pro Trp Leu Lys Ser Leu Thr Val Gly Ser Ala Val
           35                  40                  45
   Ser Thr Pro Cys Thr Pro Leu Lys His Leu Gln Asn Cys Asn Arg Asn
       50                  55                  60
   Thr Ser Lys Leu Lys Leu
   65                  70
<210> 124
   <211> 60
   <212> PRT
   <213> Artificial Sequence
<220>
   <221> SOURCE
   <222> 1..60
   <223> /mol_type="protein"
   /note="subject"
   /organism="Artificial Sequence"
<400> 124
   Phe Leu Phe Arg Ser Ile Ala Val Leu Ile Thr Thr Asn Asn Trp Asp
   1               5                   10                  15
   Met Thr Glu Ile Phe Leu Phe Ala Leu Ser Val Leu Leu Leu Ile Ser
               20                  25                  30
   Cys Ile Val Tyr Tyr Tyr Thr Val Pro Asp Lys Glu Met Ile Leu Asn
           35                  40                  45
   Arg Arg Asp Gly Leu Val Ser Phe Pro Ala Phe Thr
       50                  55                  60
<210> 125
   <211> 79
   <212> PRT
   <213> Artificial Sequence
<220>
   <221> SOURCE
   <222> 1..79
   <223> /mol_type="protein"
   /note="query"
   /organism="Artificial Sequence"
<400> 125
   Met Leu Phe Lys Ser Leu Gly Val Ala Ile Ala Thr Asn Asn Ala Ser
   1               5                   10                  15
   Asn Ser Asp Glu Asp Leu Lys Pro Ile Thr Ile Arg Leu Ala Asp Ser
               20                  25                  30
   Glu Ile Gln Ala Tyr Asp Thr Val Ala Lys Ser Met Gly Leu Thr Arg
           35                  40                  45
   Gln Asp Phe Leu Ser His Val Ile Arg Ser Asn Phe Arg Gln Ala Leu
       50                  55                  60
   Lys Glu Phe Val Val Gly Tyr Thr Ser Ser Asn Pro Ser Thr Pro
   65                  70                  75
<210> 126
   <211> 19
   <212> DNA
   <213> Artificial Sequence
<220>
   <221> source
   <222> 1..19
   <223> /mol_type="DNA"
   /note="Nn forward primer"
   /organism="Artificial Sequence"
<400> 126
   ggattaatgc caatgatcc 19
<210> 127
   <211> 18
   <212> DNA
   <213> Artificial Sequence
<220>
   <221> source
   <222> 1..18
   <223> /mol_type="DNA"
   /note="Xn reverse primer"
   /organism="Artificial Sequence"
<400> 127
   ctttgcctgt ttctctcg 18
<210> 128
   <211> 24
   <212> DNA
   <213> Artificial Sequence
<220>
   <221> source
   <222> 1..24
   <223> /mol_type="DNA"
   /note="No forward primer"
   /organism="Artificial Sequence"
<400> 128
   gaggacgaat taatattaca agtc 24
<210> 129
   <211> 19
   <212> DNA
   <213> Artificial Sequence
<220>
   <221> source
   <222> 1..19
   <223> /mol_type="DNA"
   /note="Xo reverse primer"
   /organism="Artificial Sequence"
<400> 129
   gttctcgttt tcttggtaa 19
<210> 130
   <211> 24
   <212> DNA
   <213> Artificial Sequence
<220>
   <221> source
   <222> 1..24
   <223> /mol_type="DNA"
   /note="Nd forward primer"
   /organism="Artificial Sequence"
<400> 130
   cagattgcaa agcctgtaat tcat 24
<210> 131
   <211> 24
   <212> DNA
   <213> Artificial Sequence
<220>
   <221> source
   <222> 1..24
   <223> /mol_type="DNA"
   /note="Xd reverse primer"
   /organism="Artificial Sequence"
<400> 131
   ctaaggcaga tcaacacagg gata 24
<210> 132
   <211> 19
   <212> DNA
   <213> Artificial Sequence
<220>
   <221> source
   <222> 1..19
   <223> /mol_type="DNA"
   /note="forward primer"
   /organism="Artificial Sequence"
<400> 132
   ggattaatgc caatgatcc 19
<210> 133
   <211> 18
   <212> DNA
   <213> Artificial Sequence
<220>
   <221> source
   <222> 1..18
   <223> /mol_type="DNA"
   /note="reverse primer"
   /organism="Artificial Sequence"
<400> 133
   cgagagaaac aggcaaag 18
<210> 134
   <211> 24
   <212> DNA
   <213> Artificial Sequence
<220>
   <221> source
   <222> 1..24
   <223> /mol_type="DNA"
   /note="forward primer"
   /organism="Artificial Sequence"
<400> 134
   gaggacgaat taatattaca agtc 24
<210> 135
   <211> 20
   <212> DNA
   <213> Artificial Sequence
<220>
   <221> source
   <222> 1..20
   <223> /mol_type="DNA"
   /note="reverse primer"
   /organism="Artificial Sequence"
<400> 135
   ttaccaagaa aagcgagaac 20
<210> 136
   <211> 24
   <212> DNA
   <213> Artificial Sequence
<220>
   <221> source
   <222> 1..24
   <223> /mol_type="DNA"
   /note="forward primer"
   /organism="Artificial Sequence"
<400> 136
   ctaatgcaga tcaacacagg gata 24
<210> 137
   <211> 22
   <212> DNA
   <213> Artificial Sequence
<220>
   <221> source
   <222> 1..22
   <223> /mol_type="DNA"
   /note="reverse primer"
   /organism="Artificial Sequence"
<400> 137
   gaattacagg ctttgcaatc tg 22
<210> 138
   <211> 5
   <212> PRT
   <213> Artificial Sequence
<220>
   <221> SOURCE
   <222> 1..5
   <223> /mol_type="protein"
   /note="motif"
   /organism="Artificial Sequence"
<400> 138
   Leu Leu Thr Tyr Ala
   1 5
<210> 139
   <211> 7
   <212> PRT
   <213> Artificial Sequence
<220>
   <221> SOURCE
   <222> 1..7
   <223> /mol_type="protein"
   /note="motif"
   /organism="Artificial Sequence"
<400> 139
   His Leu His Ala Phe Val Asp
   1 5
<210> 140
   <211> 5
   <212> PRT
   <213> Artificial Sequence
<220>
   <221> SOURCE
   <222> 1..5
   <223> /mol_type="protein"
   /note="motif"
   /organism="Artificial Sequence"
<400> 140
   Tyr Ala Ile Lys Asp
   1 5
<210> 141
   <211> 5
   <212> PRT
   <213> Artificial Sequence
<220>
   <221> SOURCE
   <222> 1..5
   <223> /mol_type="protein"
   /note="motif"
   /organism="Artificial Sequence"
<400> 141
   Val Phe Asp Asp Ile
   1 5
<210> 142
   <211> 5
   <212> PRT
   <213> Artificial Sequence
<220>
   <221> SOURCE
   <222> 1..5
   <223> /mol_type="protein"
   /note="motif"
   /organism="Artificial Sequence"
<400> 142
   Leu Leu Lys Met Pro
   1 5
<210> 143
   <211> 7
   <212> PRT
   <213> Artificial Sequence
<220>
   <221> SOURCE
   <222> 1..7
   <223> /mol_type="protein"
   /note="motif"
   /organism="Artificial Sequence"
<400> 143
   His Tyr His Ile Tyr Leu Gly
   1 5
<210> 144
   <211> 5
   <212> PRT
   <213> Artificial Sequence
<220>
   <221> SOURCE
   <222> 1..5
   <223> /mol_type="protein"
   /note="motif"
   /organism="Artificial Sequence"
<400> 144
   Tyr Val Gly Lys Asp
   1 5
<210> 145
   <211> 5
   <212> PRT
   <213> Artificial Sequence
<220>
   <221> SOURCE
   <222> 1..5
   <223> /mol_type="protein"
   /note="motif"
   /organism="Artificial Sequence"
<400> 145
   Val Phe Asp Asp Ile
   1 5
<210> 146
   <211> 5
   <212> PRT
   <213> Artificial Sequence
<220>
   <221> SOURCE
   <222> 1..5
   <223> /mol_type="protein"
   /note="motif"
   /organism="Artificial Sequence"
<400> 146
   Leu Leu Thr Tyr Pro
   1 5
<210> 147
   <211> 7
   <212> PRT
   <213> Artificial Sequence
<220>
   <221> SOURCE
   <222> 1..7
   <223> /mol_type="protein"
   /note="motif"
   /organism="Artificial Sequence"
<400> 147
   His Leu His Ala Phe Val Asp
   1 5
<210> 148
   <211> 5
   <212> PRT
   <213> Artificial Sequence
<220>
   <221> SOURCE
   <222> 1..5
   <223> /mol_type="protein"
   /note="motif"
   /organism="Artificial Sequence"
<400> 148
   Tyr Ala Ile Lys Asp
   1 5
<210> 149
   <211> 5
   <212> PRT
   <213> Artificial Sequence
<220>
   <221> SOURCE
   <222> 1..5
   <223> /mol_type="protein"
   /note="motif"
   /organism="Artificial Sequence"
<400> 149
   Ile Phe Asp Asp Phe
   1               5
<210> 150
   <211> 16
   <212> PRT
   <213> Artificial Sequence
<220>
   <221> SOURCE
   <222> 1..16
   <223> /mol_type="protein"
   /note="motif"
   /organism="Artificial Sequence"
<400> 150
   Ala Val Phe Asp Val Gly Gly Phe His Pro Asn Ile Ser Ile Thr Lys
   1               5                   10                  15
<210> 151
   <211> 16
   <212> PRT
   <213> Artificial Sequence
<220>
   <221> SOURCE
   <222> 1..16
   <223> /mol_type="protein"
   /note="motif"
   /organism="Artificial Sequence"
<400> 151
   Thr Ala Phe Asp Tyr Phe Gly Ala His Gly Asn Ile Lys Ser Ile Arg
   1               5                   10                  15
<210> 152
   <211> 16
   <212> PRT
   <213> Artificial Sequence
<220>
   <221> SOURCE
   <222> 1..16
   <223> /mol_type="protein"
   /note="motif"
   /organism="Artificial Sequence"
<400> 152
   Arg Ala Phe Asp Val Glu Gly Cys His Pro Asn Val Ser Pro Ser Arg
   1               5                   10                  15
<210> 153
   <211> 23
   <212> DNA
   <213> Artificial Sequence
<220>
   <221> source
   <222> 1..23
   <223> /mol_type="DNA"
   /note="forward primer"
   /organism="Artificial Sequence"
<400> 153
   gacttctgat tgattgatgc ctg 23
<210> 154
   <211> 24
   <212> DNA
   <213> Artificial Sequence
<220>
   <221> source
   <222> 1..24
   <223> /mol_type="DNA"
   /note="reverse primer"
   /organism="Artificial Sequence"
<400> 154
   cctgttgaat accgcttaaa tact 24
<210> 155
   <211> 2149
   <212> DNA
   <213> Artificial Sequence
<220>
   <221> source
   <222> 1..2149
   <223> /mol_type="DNA"
   /note="HCBI8.215"
   /organism="Artificial Sequence"
<400> 155
   taatgttatc tacctttctg ttgtgtcggt gtcactttac ttggtatata taggcggttg 60
   tcccgcggag aaatttattt cgcaaagagg ggtatgttgc atttacaaaa tggcgttccg 120
   gaatcggcgt tttcaggcgg ggcgcaggcg tcgttttact ccacggaggt ccacttttcg 180
   ccgacgatct ttccgcaatg gtcggtcacg tacgacaagg cggtattcac ggccacgcac 240
   gaatgtgcgt cgtgtgcgca atctcgcgtc tcggaagtgt cgcgacaata tgatttgtac 300
   tcctattgac gacacgggta ctcctcagac tcccggaccc gtatctatgg atgcatcatc 360
   tgtttacggg ttcatctttt caccaactgc acggaccgca ggtactcaac ttgagagcgg 420
   tgagcgggtc aatgcaccta gctcggccca gcggaggaag agcaaatgtt acgttcgggg 480
   ttacggagaa actgtcgaaa ttcagacgga tgacgcgtcg ccatgggtat ggcgtcgtat 540
   tgtgttcagc actattggct tggcgtctca gtttgacccc ggtcttttgt acaacaatga 600
   cgaaacccgt ggctggggtc gcagcctgtt caatatccgg agtggtacgc agcaggcatc 660
   cattcttgag ggcgttgttt ttaggtacct attccagggt accgagggtg tcgattgggc 720
   caatgtcatg actgctacta cggcccctcg gcatgtcagg gtttattccg atcggcgccg 780
   tgttattcaa tcccccaact cggagggggg tactattcga ctatcgaagc attacgatgg 840
   tattaatcgg agcattattt acgacgatgt ggaaattgga gcgggagcga aggctacgaa 900
   tgccattgcg agtggaactc catatgggaa tatgggagat ttgtttgttc ttgatttctt 960
   ttcgagtgca tcggatgatg agaccagcgg agcgtctttc cagcctcatg ggaagtttta 1020
   ttggcatgaa gggcaatctg cttagctata gaggggagtc gtgacttcta caaaaataca 1080
   attcttgtcc atccagtcct gatccacccc ttctgtgatg ggatactcat ttgatatcca 1140
   aatacatggt ttcccccact taattgcctt cttcttcttg tacttgtctg tagaatagaa 1200
   gtctagctgg tttcccagcc atgacttgta ctgtgggaag aattgagtcc tccggcgatg 1260
   tcatcaaaga cggcatactt gacatcctca agggattcct ccagtgagaa gagccgccaa 1320
   aataggcgtg cttggcgaga gatcttgccc acatagtctt gccagttctt gagggtccat 1380
   agaggaccag tgactttccc ctgcctaaca cattagccgt tagggttagg gttacccttt 1440
   agggttaggg ttagggttag ggttagggtt agggttaggg ttagggttac cctttagggt 1500
   tagaggatta tgaaaatgtc gtggtgtgtg tgtactaacc ccttactgtg gcatcccatc 1560
   caatgtagtc ctcagcccac tgattgagct caggaaactg cgaagtgtcg aattcatact 1620
   ctgggtcgtg tcgatatggg tctggatcca cacgatagtg ccagtcggca tatttctcga 1680
   gtgacgggaa gctggtgagt agaagtcttg ggtccaaatc acggacagct tcgaaaaact 1740
   ccgctcgagt cggtgccaag acgatttgat ggtgcttatt ctcaactcca gcgcttgcat 1800
   ctgctgctgg tcgttcgagt cctccagcga caacatccca tccttgatcg cgtaatcata 1860
   atggacttga gggtcgcgct tagtaattga gatgttaggg tggaaacctc caacatcaaa 1920
   gacagcagca cgtcttgatt gaaatcgtcg tccaaaatcc acaaaagcgt gcagatgagt 1980
   tccgccatca gagtgggctt ctcttccaat gatgcattct cctccaaggt caccaatgtg 2040
   tgaaacaata gtgaatgggt cgaggccgcc gcattgagcg taggtgagaa gcgcatatcg 2100
   ggcatagaag cgaaatgtcg acattgaggg tgacacagaa aggtaggtt 2149
<210> 156
   <211> 286
   <212> DNA
   <213> Artificial Sequence
<220>
   <221> source
   <222> 1..286
   <223> /mol_type="DNA"
   /note="query"
   /organism="Artificial Sequence"
<400> 156
   cctccagcga caacatcacc atccttgatc gcgtaatcat aatggacttg agggtcgcgc 60
   ttagtaattg agatgttagg gtggaaacct ccaacatcaa agacagcagc acgtcttgat 120
   tgaaatcgtc gtccaaaatc cacaaaagcg tgcagatgag ttccgccatc agagtgggct 180
   tctcttccaa tgatgcattc tcctccaagg tcaccaatgt gtgaaacaat agtgaatggg 240
   tcgaggccgc cgcattgagc gtaggtgaga agcgcatatc gggcat 286
<210> 157
   <211> 286
   <212> DNA
   <213> Artificial Sequence
<220>
   <221> source
   <222> 1..286
   <223> /mol_type="DNA"
   /note="subject"
   /organism="Artificial Sequence"
<400> 157
   cctccagcga ccacgtctcc atctttgatc gcatagtcgt aaccgccttc cggctttccg 60
   taagatggaa caaggttcgg gtggcggcct ccaacatcaa agacatcgga tcgtcgggat 120
   cgaaatcggc gggagaaatc acagaaagca tggagatgag tacctccatc agcgtgcaac 180
   tctcgtccaa tgatacactc agctccaagg aacgccaaat ggtcattaac ggtccatgca 240
   tcaagagtgc cgcactgtgc gtacgtgaga agaaagtatc ggccat 286
<210> 158
   <211> 300
   <212> DNA
   <213> Artificial Sequence
<220>
   <221> source
   <222> 1..300
   <223> /mol_type="DNA"
   /note="query"
   /organism="Artificial Sequence"
<400> 158
   agtcctccag cgacaacatc accatccttg atcgcgtaat cataatggac ttgagggtcg 60
   cgcttagtaa ttgagatgtt agggtggaaa cctccaacat caaagacagc agcacgtctt 120
   gattgaaatc gtcgtccaaa atccacaaaa gcgtgcagat gagttccgcc atcagagtgg 180
   gcttctcttc caatgatgca ttctcctcca aggtcaccaa tgtgtgaaac aatagtgaat 240
   gggtcgaggc cgccgcattg agcgtaggtg agaagcgcat atcgggcata gaagcgaaat 300
<210> 159
   <211> 300
   <212> DNA
   <213> Artificial Sequence
<220>
   <221> source
   <222> 1..300
   <223> /mol_type="DNA"
   /note="query"
   /organism="Artificial Sequence"
<400> 159
   agccctccag cgaccacgtc tccatctttg atcgcatagt cataaccgcc ttccggcctt 60
   cctcgagatg cttcaatgtt agggtggaag cctccaacat caaacacatc gggtcttcgg 120
   gatcgaaact ttcgggagaa atcgcagaaa gcatggagat gagtaccccc atcagcgtga 180
   tgctctcgtc caatgataca ctcaccgcca aggaatgcca gatggtcatt aacggcccat 240
   gcatcaaggt cgccgcactg tgcgtaggta aggaggacat agcgtgcttg gaattgaaat 300
<210> 160
   <211> 282
   <212> DNA
   <213> Artificial Sequence
<220>
   <221> source
   <222> 1..282
   <223> /mol_type="DNA"
   /note="query"
   /organism="Artificial Sequence"
<400> 160
   cctccagcga caacatcacc atccttgatc gcgtaatcat aatggacttg agggtcgcgc 60
   ttagtaattg agatgttagg gtggaaacct ccaacatcaa agacagcagc acgtcttgat 120
   tgaaatcgtc gtccaaaatc cacaaaagcg tgcagatgag ttccgccatc agagtgggct 180
   tctcttccaa tgatgcattc tcctccaagg tcaccaatgt gtgaaacaat agtgaatggg 240
   tcgaggccgc cgcattgagc gtaggtgaga agcgcatatc gg 282
<210> 161
   <211> 282
   <212> DNA
   <213> Artificial Sequence
<220>
   <221> source
   <222> 1..282
   <223> /mol_type="DNA"
   /note="subject"
   /organism="Artificial Sequence"
<400> 161
   cctccagcga ccacgtctcc atctttgatc gcatagtcat aaccgccttc cgggcttccg 60
   taagatggaa caaggttcgg atggcggcct ccaacatcaa agacatcgga tcttctggat 120
   cgaaacttcc gggagaaatc gacgaaagca tggagatgag tacctccatc agcatgcaat 180
   tctcgtccaa tgatacactc accgccaaga aatgccatat ggtcattaac ggcccatgca 240
   tcaaggtcgc cgcactgtgc gtaggtaagg agggcataac gg 282
<210> 162
   <211> 287
   <212> PRT
   <213> Artificial Sequence
<220>
   <221> SOURCE
   <222> 1..287
   <223> /mol_type="protein"
   /note="query"
   /organism="Artificial Sequence"
<400> 162
   Ser Thr Phe Arg Arg Arg Ser Phe Arg Asn Gly Arg Ser Arg Thr Thr
   1               5                   10                  15
   Arg Arg Tyr Ser Arg Pro Arg Thr Asn Val Arg Arg Val Arg Asn Leu
               20                  25                  30
   Ala Ser Arg Lys Cys Arg Asp Asn Met Ile Cys Thr Pro Ile Asp Asp
           35                  40                  45
   Thr Gly Thr Pro Gln Thr Pro Gly Pro Val Ser Met Asp Ala Ser Ser
       50                  55                  60
   Val Tyr Gly Phe Ile Phe Ser Pro Thr Ala Arg Thr Ala Gly Thr Gln
   65                  70                  75                  80
   Leu Glu Ser Gly Glu Arg Val Asn Ala Pro Ser Ser Ala Gln Arg Arg
                   85                  90                  95
   Lys Ser Lys Cys Tyr Val Arg Gly Tyr Gly Glu Thr Val Glu Ile Gln
               100                 105                 110
   Thr Asp Asp Ala Ser Pro Trp Val Trp Arg Arg Ile Val Phe Ser Thr
           115                 120                 125
   Ile Gly Leu Ala Ser Gln Phe Asp Pro Gly Leu Leu Tyr Asn Asn Asp
       130                 135                 140
   Glu Thr Arg Gly Trp Gly Arg Ser Leu Phe Asn Ile Arg Ser Gly Thr
   145                 150                 155                 160
   Gln Gln Ala Ser Ile Leu Glu Gly Val Val Phe Arg Tyr Leu Phe Gln
                   165                 170                 175
   Gly Thr Glu Gly Val Asp Trp Ala Asn Val Met Thr Ala Thr Thr Ala
               180                 185                 190
   Pro Arg His Val Arg Val Tyr Ser Asp Arg Arg Arg Val Ile Gln Ser
           195                 200                 205
   Pro Asn Ser Glu Gly Gly Thr Ile Arg Leu Ser Lys His Tyr Asp Gly
       210                 215                 220
   Ile Asn Arg Ser Ile Ile Tyr Asp Asp Val Glu Ile Gly Ala Gly Ala
   225                 230                 235                 240
   Lys Ala Thr Asn Ala Ile Ala Ser Gly Thr Pro Tyr Gly Asn Met Gly
                   245                 250                 255
   Asp Leu Phe Val Leu Asp Phe Phe Ser Ser Ala Ser Asp Asp Glu Thr
               260                 265                 270
   Ser Gly Ala Ser Phe Gln Pro His Gly Lys Phe Tyr Trp His Glu
           275                 280                 285
<210> 163
   <211> 273
   <212> PRT
   <213> Artificial Sequence
<220>
   <221> SOURCE
   <222> 1..273
   <223> /mol_type="protein"
   /note="subject"
   /organism="Artificial Sequence"
<400> 163
   Ser Ser Lys Lys Arg Arg Ser Tyr Val Gly Arg Arg Leu Ser Ser Arg
   1               5                   10                  15
   Arg Thr Thr Lys Thr Val Met Lys Arg Thr Ser Arg Arg Arg Ile Leu
               20                  25                  30
   Asn Ile Ser Ser Gln Lys Lys Arg Asn Asn Ala Gln Pro Val Asn Phe
           35                  40                  45
   Asn Tyr Leu Gly Glu Ser Pro Ile Pro Gly Ser Lys Val Met Gly Ser
       50                  55                  60
   Asp Arg Thr Thr Tyr Leu Leu Trp Arg Pro Thr Ala Leu Thr Asp Asp
   65                  70                  75                  80
   Thr Asp Ile Tyr Ser Ser Ala Ser Arg Gly Ala Gln Gln Ile Tyr Trp
                   85                  90                  95
   Arg Gly Ile Lys Glu Arg Ala Glu Val Gly Thr Ser Ser Gly Ala Ala
               100                 105                 110
   Trp Arg Trp Arg Arg Ile Val Phe Glu Ala Lys Ser Leu Asn Ile Asn
           115                 120                 125
   Gly Leu Ile Ser Asn Val Glu Thr Ser Ala Gly Phe Asn Arg Ala Met
       130                 135                 140
   Val Glu Leu Ser Gly Asp Pro Pro Ala Asn Leu Arg Asn Ser Leu Glu
   145                 150                 155                 160
   Ser Phe Leu Phe Gln Gly Ser Val Gly Val Asp Trp Asn Thr Val Phe
                   165                 170                 175
   Asn Ala Lys Val Asp Asn Asn Arg Cys Arg Leu Leu Ser Asp Lys Val
               180                 185                 190
   Arg His Leu Gln Ser Gly Asn Asp Arg Gly Arg Phe Tyr Thr Phe Arg
           195                 200                 205
   Gln Trp Ile Pro Leu Asn Lys Thr Met Val Tyr Asn Asp Asp Glu Val
       210                 215                 220
   Gly Asp Asp Lys Gln Ser Asn Thr Phe Ala Ser Pro Gly Arg Lys Gly
   225                 230                 235                 240
   Met Gly Asp Val Tyr Val Tyr Asp Met Phe Gln Cys Leu Thr Ser Asp
                   245                 250                 255
   Ala Ile Asn Thr Leu Asn Phe Asn Pro Gln Ala Thr Leu Tyr Trp His
               260                 265                 270
   Glu
<210> 164
   <211> 283
   <212> PRT
   <213> Artificial Sequence
<220>
   <221> SOURCE
   <222> 1..283
   <223> /mol_type="protein"
   /note="query"
   /organism="Artificial Sequence"
<400> 164
   Arg Arg Arg Ser Phe Arg Asn Gly Arg Ser Arg Thr Thr Arg Arg Tyr
   1               5                   10                  15
   Ser Arg Pro Arg Thr Asn Val Arg Arg Val Arg Asn Leu Ala Ser Arg
               20                  25                  30
   Lys Cys Arg Asp Asn Met Ile Cys Thr Pro Ile Asp Asp Thr Gly Thr
           35                  40                  45
   Pro Gln Thr Pro Gly Pro Val Ser Met Asp Ala Ser Ser Val Tyr Gly
       50                  55                  60
   Phe Ile Phe Ser Pro Thr Ala Arg Thr Ala Gly Thr Gln Leu Glu Ser
   65                  70                  75                  80
   Gly Glu Arg Val Asn Ala Pro Ser Ala Gln Arg Arg Lys Ser Lys Cys
                   85                  90                  95
   Tyr Val Arg Gly Tyr Gly Glu Thr Val Glu Ile Gln Thr Asp Asp Ala
               100                 105                 110
   Ser Pro Trp Val Trp Arg Arg Ile Val Phe Ser Thr Ile Gly Leu Ala
           115                 120                 125
   Ser Gln Phe Asp Pro Gly Leu Leu Tyr Asn Asn Asp Glu Thr Arg Gly
       130                 135                 140
   Trp Gly Arg Ser Leu Phe Asn Ile Arg Ser Gly Thr Gln Gln Ala Ser
   145                 150                 155                 160
   Ile Leu Glu Gly Val Val Phe Arg Tyr Leu Phe Gln Gly Thr Glu Gly
                   165                 170                 175
   Val Asp Trp Ala Asn Val Met Thr Ala Thr Thr Ala Pro Arg His Val
               180                 185                 190
   Arg Val Tyr Ser Asp Arg Arg Arg Val Ile Gln Ser Pro Asn Ser Glu
           195                 200                 205
   Gly Gly Thr Ile Arg Leu Ser Lys His Tyr Asp Gly Ile Asn Arg Ser
       210                 215                 220
   Ile Ile Tyr Asp Asp Val Glu Ile Gly Ala Gly Ala Lys Ala Thr Asn
   225                 230                 235                 240
   Ala Ile Ala Ser Gly Thr Pro Tyr Gly Asn Met Gly Asp Leu Phe Val
                   245                 250                 255
   Leu Asp Phe Phe Ser Ser Ala Ser Asp Asp Glu Thr Ser Gly Ala Ser
               260                 265                 270
   Phe Gln Pro His Gly Lys Phe Tyr Trp His Glu
           275                 280
<210> 165
   <211> 277
   <212> PRT
   <213> Artificial Sequence
<220>
   <221> SOURCE
   <222> 1..277
   <223> /mol_type="protein"
   /note="subject"
   /organism="Artificial Sequence"
<400> 165
   Arg Arg Arg Ser Tyr Pro Thr Thr Arg Arg Arg Tyr Pro Arg Lys Thr
   1               5                   10                  15
   Leu Ala Lys Arg Ile Leu Asn Ile Ser Ser Thr Lys Lys Arg Asp Asn
               20                  25                  30
   Met Met Ser Tyr Thr Asn Val Thr Val Ala Asn Pro Ile Asn Gly Gln
           35                  40                  45
   Thr Tyr Ala Ala Gly Pro Ala Val Leu Arg Gly Asp Gln Thr Tyr Ile
       50                  55                  60
   Phe Ala Trp Ile Pro Thr Ala Arg Asp Leu Ile Arg Ala Asp Gly Glu
   65                  70                  75                  80
   Pro Asn Thr Pro Val Trp Leu Ala Gly Arg Thr Arg Gln Thr Cys Tyr
                   85                  90                  95
   Met Arg Gly Leu Lys Glu Ala Met Asn Val Arg Thr Asn Ser Gly Ser
               100                 105                 110
   Ser Trp Gln Trp Arg Arg Ile Cys Phe Thr Met Lys Gly Pro Asn Leu
           115                 120                 125
   Ile Gly Tyr Ala Thr Thr Gly Ser Asn Leu Trp Gln Glu Thr Ser Asn
       130                 135                 140
   Gly Met Val Arg Thr Met Asn Asn Val Asn Thr Lys Pro Leu Gly Asn
   145                 150                 155                 160
   Glu Ile Leu Ala Gln Leu Phe Arg Gly Ala Gln Asn Gln Asp Trp Leu
                   165                 170                 175
   Asn Val Phe Asn Ala Gln Thr Asp Thr Thr Lys Phe Thr Ile Lys Tyr
               180                 185                 190
   Asp Arg Thr Ile Ser Ile Ala Ser Gly Asn Glu Gln Gly Leu Val Arg
           195                 200                 205
   Thr Tyr Lys His Trp Tyr Pro Met Asn Lys Asn Leu Val Tyr Asn Asp
       210                 215                 220
   Asp Glu Asn Ala Asp Gly Val Leu Thr Ser Tyr Thr Ser Thr Asn Gly
   225                 230                 235                 240
   Lys Pro Gly Met Gly Asp Tyr Tyr Val Val Asp Phe Phe Gln Pro Ala
                   245                 250                 255
   Asp Gly Ala Asp Ala Ser Asp Thr Leu Ala Leu Leu Pro Glu Ala Thr
               260                 265                 270
   Leu Tyr Trp His Glu
           275
<210> 166
   <211> 261
   <212> PRT
   <213> Artificial Sequence
<220>
   <221> SOURCE
   <222> 1..261
   <223> /mol_type="protein"
   /note="query"
   /organism="Artificial Sequence"
<400> 166
   Arg Arg Val Arg Asn Leu Ala Ser Arg Lys Cys Arg Asp Asn Met Ile
   1 5 10 15
   Cys Thr Pro Ile Asp Asp Thr Gly Thr Pro Gln Thr Pro Gly Pro Val
   20 25 30
   Ser Met Asp Ala Ser Ser Val Tyr Gly Phe Ile Phe Ser Pro Thr Ala
   35 40 45
   Arg Thr Ala Gly Thr Gln Leu Glu Ser Gly Glu Arg Val Asn Ala Pro
   50 55 60
   Ser Ser Ala Gln Arg Arg Lys Ser Lys Cys Tyr Val Arg Gly Tyr Gly
   65 70 75 80
   Glu Thr Val Glu Ile Gln Thr Asp Asp Ala Ser Pro Trp Val Trp Arg
   85 90 95
   Arg Ile Val Phe Ser Thr Ile Gly Leu Ala Ser Gln Phe Asp Pro Gly
   100 105 110
   Leu Leu Tyr Asn Asn Asp Glu Thr Arg Gly Trp Gly Arg Ser Leu Phe
   115 120 125
   Asn Ile Arg Ser Gly Thr Gln Gln Ala Ser Ile Leu Glu Gly Val Val
   130 135 140
   Phe Arg Tyr Leu Phe Gln Gly Thr Glu Gly Val Asp Trp Ala Asn Val
   145 150 155 160
   Met Thr Ala Thr Thr Ala Pro Arg His Val Arg Val Tyr Ser Asp Arg
   165 170 175
   Arg Arg Val Ile Gln Ser Pro Asn Ser Glu Gly Gly Thr Ile Arg Leu
   180 185 190
   Ser Lys His Tyr Asp Gly Ile Asn Arg Ser Ile Ile Tyr Asp Asp Val
   195 200 205
   Glu Ile Gly Ala Gly Ala Lys Ala Thr Asn Ala Ile Ala Ser Gly Thr
   210 215 220
   Pro Tyr Gly Asn Met Gly Asp Leu Phe Val Leu Asp Phe Phe Ser Ser
   225 230 235 240
   Ala Ser Asp Asp Glu Thr Ser Gly Ala Ser Phe Gln Pro His Gly Lys
   245 250 255
   Phe Tyr Trp His Glu
   260
<210> 167
   <211> 246
   <212> PRT
   <213> Artificial Sequence
<220>
   <221> SOURCE
   <222> 1..246
   <223> /mol_type="protein"
   /note="subject"
   /organism="Artificial Sequence"
<400> 167
   Arg Arg Ile Leu Asn Val Ser Ser Lys Lys Lys Arg Asp Asn Met Ile
   1               5                   10                  15
   Asn Val Ser Phe Ser Pro Thr Gly Ile Asn Pro Ala Val Arg Gly Phe
               20                  25                  30
   Ile Ala Thr Gly Ala Ala Pro Thr Met Leu Val Trp Cys Ala Thr Ala
           35                  40                  45
   Arg Asp Arg Val Ser Asn Ala Ser Asn Pro Thr Ala Asp Ser Val Arg
       50                  55                  60
   Glu Ser Gln Thr Cys Tyr Met Arg Gly Leu Lys Glu His Ile Arg Ile
   65                  70                  75                  80
   Asn Thr Asn Asn Pro Thr Ser Trp Leu Trp Arg Arg Ile Cys Phe Thr
                   85                  90                  95
   Ala Lys Gly Leu Phe Ser Gly Leu Gly Thr Ser Ala Asp Ser Val Glu
               100                 105                 110
   Thr Ser Asn Gly Trp Val Arg Ala Leu Met Asp Gln Thr Gly Thr Ser
           115                 120                 125
   Phe Gly Thr Ser Leu Thr Asn Leu Leu Phe Ala Gly Thr Asn Gly Val
       130                 135                 140
   Asp Trp Asn Asp Thr Phe Thr Ala Asn Thr Asp Ser Thr Asn Val Lys
   145                 150                 155                 160
   Ile Met Tyr Asp Lys Thr Phe Thr Ile Asn Ser Gly Val Ser Gly Val
                   165                 170                 175
   Ile Arg Asp Tyr Lys Met Trp His Gly Met Asn Lys Asn Ile Val Tyr
               180                 185                 190
   Gly Glu Asp Glu Asn Gly Glu Gly Glu Ile Asn Asn Arg Tyr Ser Thr
           195                 200                 205
   Ile Gly Arg Pro Gly Met Gly Asp Tyr Tyr Val Val Asp Leu Leu Ala
       210                 215                 220
   Ser Asn Ser Ser Ala Ala Ala Asp Thr Leu Thr Phe Ser Thr Glu Ala
   225                 230                 235                     240
   Thr Leu Tyr Trp His Glu
                   245
<210> 168
   <211> 287
   <212> PRT
   <213> Artificial Sequence
<220>
   <221> SOURCE
   <222> 1..287
   <223> /mol_type="protein"
   /note="query"
   /organism="Artificial Sequence"
<400> 168
   Ser Thr Phe Arg Arg Arg Ser Phe Arg Asn Gly Arg Ser Arg Thr Thr
   1               5                   10                  15
   Arg Arg Tyr Ser Arg Pro Arg Thr Asn Val Arg Arg Val Arg Asn Leu
               20                  25                  30
   Ala Ser Arg Lys Cys Arg Asp Asn Met Ile Cys Thr Pro Ile Asp Asp
           35                  40                  45
   Thr Gly Thr Pro Gln Thr Pro Gly Pro Val Ser Met Asp Ala Ser Ser
       50                  55                  60
   Val Tyr Gly Phe Ile Phe Ser Pro Thr Ala Arg Thr Ala Gly Thr Gln
   65                  70                  75                  80
   Leu Glu Ser Gly Glu Arg Val Asn Ala Pro Ser Ser Ala Gln Arg Arg
                   85                  90                  95
   Lys Ser Lys Cys Tyr Val Arg Gly Tyr Gly Glu Thr Val Glu Ile Gln
               100                 105                 110
   Thr Asp Asp Ala Ser Pro Trp Val Trp Arg Arg Ile Val Phe Ser Thr
           115                 120                 125
   Ile Gly Leu Ala Ser Gln Phe Asp Pro Gly Leu Leu Tyr Asn Asn Asp
       130                 135                 140
   Glu Thr Arg Gly Trp Gly Arg Ser Leu Phe Asn Ile Arg Ser Gly Thr
   145                 150                 155                 160
   Gln Gln Ala Ser Ile Leu Glu Gly Val Val Phe Arg Tyr Leu Phe Gln
                   165                 170                 175
   Gly Thr Glu Gly Val Asp Trp Ala Asn Val Met Thr Ala Thr Thr Ala
               180                 185                 190
   Pro Arg His Val Arg Val Tyr Ser Asp Arg Arg Arg Val Ile Gln Ser
           195                 200                 205
   Pro Asn Ser Glu Gly Gly Thr Ile Arg Leu Ser Lys His Tyr Asp Gly
       210                 215                 220
   Ile Asn Arg Ser Ile Ile Tyr Asp Asp Val Glu Ile Gly Ala Gly Ala
   225                 230                 235                 240
   Lys Ala Thr Asn Ala Ile Ala Ser Gly Thr Pro Tyr Gly Asn Met Gly
                   245                 250                 255
   Asp Leu Phe Val Leu Asp Phe Phe Ser Ser Ala Ser Asp Asp Glu Thr
               260                 265                 270
   Ser Gly Ala Ser Phe Gln Pro His Gly Lys Phe Tyr Trp His Glu
           275                 280                 285
<210> 169
   <211> 273
   <212> PRT
   <213> Artificial Sequence
<220>
   <221> SOURCE
   <222> 1..273
   <223> /mol_type="protein"
   /note="subject"
   /organism="Artificial Sequence"
<400> 169
   Ser Ser Lys Lys Arg Arg Ser Tyr Val Gly Arg Arg Leu Ser Ser Arg
   1               5                   10                  15
   Arg Thr Thr Lys Thr Val Met Lys Arg Thr Ser Arg Arg Arg Ile Leu
               20                  25                  30
   Asn Ile Ser Ser Gln Lys Lys Arg Asn Asn Ala Gln Pro Val Asn Phe
           35                  40                  45
   Asn Tyr Leu Gly Glu Ser Pro Ile Pro Gly Ser Lys Val Met Gly Ser
       50                  55                  60
   Asp Arg Thr Thr Tyr Leu Leu Trp Arg Pro Thr Ala Leu Thr Asp Asp
   65                  70                  75                  80
   Thr Asp Ile Tyr Ser Ser Ala Ser Arg Gly Ala Gln Gln Ile Tyr Trp
                   85                  90                  95
   Arg Gly Ile Lys Glu Arg Ala Glu Val Gly Thr Ser Ser Gly Ala Ala
               100                 105                 110
   Trp Arg Trp Arg Arg Ile Val Phe Glu Ala Lys Ser Leu Asn Ile Asn
           115                 120                 125
   Gly Leu Ile Ser Asn Val Glu Thr Ser Ala Gly Phe Asn Arg Ala Met
       130                 135                 140
   Val Glu Leu Ser Gly Asp Pro Pro Ala Asn Leu Arg Asn Ser Leu Glu
   145                 150                 155                 160
   Ser Phe Leu Phe Gln Gly Ser Val Gly Val Asp Trp Asn Thr Val Phe
                   165                 170                 175
   Asn Ala Lys Val Asp Asn Asn Arg Cys Arg Leu Leu Ser Asp Lys Val
               180                 185                 190
   Arg His Leu Gln Ser Gly Asn Asp Arg Gly Arg Phe Tyr Thr Phe Arg
           195                 200                 205
   Gln Trp Ile Pro Leu Asn Lys Thr Met Val Tyr Asn Asp Asp Glu Val
       210                 215                 220
   Gly Asp Asp Lys Gln Ser Asn Thr Phe Ala Ser Pro Gly Arg Lys Gly
   225                 230                 235                 240
   Met Gly Asp Val Tyr Val Tyr Asp Met Phe Gln Cys Leu Thr Ser Asp
                   245                 250                 255
   Ala Ile Asn Thr Leu Asn Phe Asn Pro Gln Ala Thr Leu Tyr Trp His
               260                 265                 270
   Glu
<210> 170
   <211> 284
   <212> PRT
   <213> Artificial Sequence
<220>
   <221> SOURCE
   <222> 1..284
   <223> /mol_type="protein"
   /note="query"
   /organism="Artificial Sequence"
<400> 170
   Arg Arg Arg Ser Phe Arg Asn Gly Arg Ser Arg Thr Thr Arg Arg Tyr
   1               5                   10                  15
   Ser Arg Pro Arg Thr Asn Val Arg Arg Val Arg Asn Leu Ala Ser Arg
               20                  25                  30
   Lys Cys Arg Asp Asn Met Ile Cys Thr Pro Ile Asp Asp Thr Gly Thr
           35                  40                  45
   Pro Gln Thr Pro Gly Pro Val Ser Met Asp Ala Ser Ser Val Tyr Gly
       50                  55                  60
   Phe Ile Phe Ser Pro Thr Ala Arg Thr Ala Gly Thr Gln Leu Glu Ser
   65                  70                  75                  80
   Gly Glu Arg Val Asn Ala Pro Ser Ser Ala Gln Arg Arg Lys Ser Lys
                   85                  90                  95
   Cys Tyr Val Arg Gly Tyr Gly Glu Thr Val Glu Ile Gln Thr Asp Asp
               100                 105                 110
   Ala Ser Pro Trp Val Trp Arg Arg Ile Val Phe Ser Thr Ile Gly Leu
           115                 120                 125
   Ala Ser Gln Phe Asp Pro Gly Leu Leu Tyr Asn Asn Asp Glu Thr Arg
       130                 135                 140
   Gly Trp Gly Arg Ser Leu Phe Asn Ile Arg Ser Gly Thr Gln Gln Ala
   145                 150                 155                 160
   Ser Ile Leu Glu Gly Val Val Phe Arg Tyr Leu Phe Gln Gly Thr Glu
                   165                 170                 175
   Gly Val Asp Trp Ala Asn Val Met Thr Ala Thr Thr Ala Pro Arg His
               180                 185                 190
   Val Arg Val Tyr Ser Asp Arg Arg Arg Val Ile Gln Ser Pro Asn Ser
           195                 200                 205
   Glu Gly Gly Thr Ile Arg Leu Ser Lys His Tyr Asp Gly Ile Asn Arg
       210                 215                 220
   Ser Ile Ile Tyr Asp Asp Val Glu Ile Gly Ala Gly Ala Lys Ala Thr
   225                 230                 235                 240
   Asn Ala Ile Ala Ser Gly Thr Pro Tyr Gly Asn Met Gly Asp Leu Phe
                   245                 250                 255
   Val Leu Asp Phe Phe Ser Ser Ala Ser Asp Asp Glu Thr Ser Gly Ala
               260                 265                 270
   Ser Phe Gln Pro His Gly Lys Phe Tyr Trp His Glu
           275                 280
<210> 171
   <211> 277
   <212> PRT
   <213> Artificial Sequence
<220>
   <221> SOURCE
   <222> 1..277
   <223> /mol_type="protein"
   /note="subject"
   /organism="Artificial Sequence"
<400> 171
   Arg Arg Arg Ser Tyr Pro Thr Thr Arg Arg Arg Tyr Pro Arg Lys Thr
   1               5                   10                  15
   Leu Ala Lys Arg Ile Leu Asn Ile Ser Ser Thr Lys Lys Arg Asp Asn
               20                  25                  30
   Met Met Ser Tyr Thr Asn Val Thr Val Ala Asn Pro Ile Asn Gly Gln
           35                  40                  45
   Thr Tyr Ala Ala Gly Pro Ala Val Leu Arg Gly Asp Gln Thr Tyr Ile
       50                  55                  60
   Phe Ala Trp Ile Pro Thr Ala Arg Asp Leu Ile Arg Ala Asp Gly Glu
   65                  70                  75                  80
   Pro Asn Thr Pro Val Trp Leu Ala Gly Arg Thr Arg Gln Thr Cys Tyr
                   85                  90                  95
   Met Arg Gly Leu Lys Glu Ala Met Asn Val Arg Thr Asn Ser Gly Ser
               100                 105                 110
   Ser Trp Gln Trp Arg Arg Ile Cys Phe Thr Met Lys Gly Pro Asn Leu
           115                 120                 125
   Ile Gly Tyr Ala Thr Thr Gly Ser Asn Leu Trp Gln Glu Thr Ser Asn
       130                 135                 140
   Gly Met Val Arg Thr Met Asn Asn Val Asn Thr Lys Pro Leu Gly Asn
   145                 150                 155                 160
   Glu Ile Leu Ala Gln Leu Phe Arg Gly Ala Gln Asn Gln Asp Trp Leu
                   165                 170                 175
   Asn Val Phe Asn Ala Gln Thr Asp Thr Thr Lys Phe Thr Ile Lys Tyr
               180                 185                 190
   Asp Arg Thr Ile Ser Ile Ala Ser Gly Asn Glu Gln Gly Leu Val Arg
           195                 200                 205
   Thr Tyr Lys His Trp Tyr Pro Met Asn Lys Asn Leu Val Tyr Asn Asp
       210                 215                 220
   Asp Glu Asn Ala Asp Gly Val Leu Thr Ser Tyr Thr Ser Thr Asn Gly
   225                 230                 235                 240
   Lys Pro Gly Met Gly Asp Tyr Tyr Val Val Asp Phe Phe Gln Pro Ala
                   245                 250                 255
   Asp Gly Ala Asp Ala Ser Asp Thr Leu Ala Leu Leu Pro Glu Ala Thr
               260                 265                 270
   Leu Tyr Trp His Glu
           275
<210> 172
   <211> 261
   <212> PRT
   <213> Artificial Sequence
<220>
   <221> SOURCE
   <222> 1..261
   <223> /mol_type="protein"
   /note="query"
   /organism="Artificial Sequence"
<400> 172
   Arg Arg Val Arg Asn Leu Ala Ser Arg Lys Cys Arg Asp Asn Met Ile
   1               5                   10                  15
   Cys Thr Pro Ile Asp Asp Thr Gly Thr Pro Gln Thr Pro Gly Pro Val
               20                  25                  30
   Ser Met Asp Ala Ser Ser Val Tyr Gly Phe Ile Phe Ser Pro Thr Ala
           35                  40                  45
   Arg Thr Ala Gly Thr Gln Leu Glu Ser Gly Glu Arg Val Asn Ala Pro
       50                  55                  60
   Ser Ser Ala Gln Arg Arg Lys Ser Lys Cys Tyr Val Arg Gly Tyr Gly
   65                  70                  75                  80
   Glu Thr Val Glu Ile Gln Thr Asp Asp Ala Ser Pro Trp Val Trp Arg
                   85                  90                  95
   Arg Ile Val Phe Ser Thr Ile Gly Leu Ala Ser Gln Phe Asp Pro Gly
               100                 105                 110
   Leu Leu Tyr Asn Asn Asp Glu Thr Arg Gly Trp Gly Arg Ser Leu Phe
           115                 120                 125
   Asn Ile Arg Ser Gly Thr Gln Gln Ala Ser Ile Leu Glu Gly Val Val
       130                 135                 140
   Phe Arg Tyr Leu Phe Gln Gly Thr Glu Gly Val Asp Trp Ala Asn Val
   145                 150                 155                 160
   Met Thr Ala Thr Thr Ala Pro Arg His Val Arg Val Tyr Ser Asp Arg
                   165                 170                 175
   Arg Arg Val Ile Gln Ser Pro Asn Ser Glu Gly Gly Thr Ile Arg Leu
               180                 185                 190
   Ser Lys His Tyr Asp Gly Ile Asn Arg Ser Ile Ile Tyr Asp Asp Val
           195                 200                 205
   Glu Ile Gly Ala Gly Ala Lys Ala Thr Asn Ala Ile Ala Ser Gly Thr
       210                 215                 220
   Pro Tyr Gly Asn Met Gly Asp Leu Phe Val Leu Asp Phe Phe Ser Ser
   225                 230                 235                 240
   Ala Ser Asp Asp Glu Thr Ser Gly Ala Ser Phe Gln Pro His Gly Lys
                   245                 250                 255
   Phe Tyr Trp His Glu
               260
<210> 173
   <211> 246
   <212> PRT
   <213> Artificial Sequence
<220>
   <221> SOURCE
   <222> 1..246
   <223> /mol_type="protein"
   /note="subject"
   /organism="Artificial Sequence"
<400> 173
   Arg Arg Ile Leu Asn Val Ser Ser Lys Lys Lys Arg Asp Asn Met Ile
   1               5                   10                  15
   Asn Val Ser Phe Ser Pro Thr Gly Ile Asn Pro Ala Val Arg Gly Phe
               20                  25                  30
   Ile Ala Thr Gly Ala Ala Pro Thr Met Leu Val Trp Cys Ala Thr Ala
           35                  40                  45
   Arg Asp Arg Val Ser Asn Ala Ser Asn Pro Thr Ala Asp Ser Val Arg
       50                  55                  60
   Glu Ser Gln Thr Cys Tyr Met Arg Gly Leu Lys Glu His Ile Arg Ile
   65                  70                  75                  80
   Asn Thr Asn Asn Pro Thr Ser Trp Leu Trp Arg Arg Ile Cys Phe Thr
                   85                  90                  95
   Ala Lys Gly Leu Phe Ser Gly Leu Gly Thr Ser Ala Asp Ser Val Glu
               100                 105                 110
   Thr Ser Asn Gly Trp Val Arg Ala Leu Met Asp Gln Thr Gly Thr Ser
           115                 120                 125
   Phe Gly Thr Ser Leu Thr Asn Leu Leu Phe Ala Gly Thr Asn Gly Val
       130                 135                 140
   Asp Trp Asn Asp Thr Phe Thr Ala Asn Thr Asp Ser Thr Asn Val Lys
   145                 150                 155                 160
   Ile Met Tyr Asp Lys Thr Phe Thr Ile Asn Ser Gly Val Ser Gly Val
                   165                 170                 175
   Ile Arg Asp Tyr Lys Met Trp His Gly Met Asn Lys Asn Ile Val Tyr
               180                 185                 190
   Gly Glu Asp Glu Asn Gly Glu Gly Glu Ile Asn Asn Arg Tyr Ser Thr
           195                 200                 205
   Ile Gly Arg Pro Gly Met Gly Asp Tyr Tyr Val Val Asp Leu Leu Ala
       210                 215                 220
   Ser Asn Ser Ser Ala Ala Ala Asp Thr Leu Thr Phe Ser Thr Glu Ala
   225                 230                 235                 240
   Thr Leu Tyr Trp His Glu
                   245
<210> 174
   <211> 186
   <212> PRT
   <213> Artificial Sequence
<220>
   <221> SOURCE
   <222> 1..186
   <223> /mol_type="protein"
   /note="query"
   /organism="Artificial Sequence"
<400> 174
   Phe Arg Phe Tyr Ala Arg Tyr Ala Leu Leu Thr Tyr Ala Gln Cys Gly
   1               5                   10                  15
   Gly Leu Asp Pro Phe Thr Ile Val Ser His Ile Gly Asp Leu Gly Gly
                   20                  25                  30
   Glu Cys Ile Ile Gly Arg Glu Ala His Ser Asp Gly Gly Thr His Leu
           35                  40                  45
   His Ala Phe Val Asp Phe Gly Arg Arg Phe Gln Ser Arg Arg Ala Ala
       50                  55                  60
   Val Phe Asp Val Gly Gly Phe His Pro Asn Ile Ser Ile Thr Lys Arg
   65                  70                  75                  80
   Asp Pro Gln Val His Tyr Asp Tyr Ala Ile Lys Asp Gly Asp Val Val
                   85                  90                  95
   Ala Gly Gly Leu Glu Arg Pro Ala Ala Asp Ala Ser Ala Gly Val Glu
               100                 105                 110
   Asn Lys His His Gln Ile Val Leu Ala Pro Thr Arg Ala Glu Phe Phe
           115                 120                 125
   Glu Ala Val Arg Asp Leu Asp Pro Arg Leu Leu Leu Thr Ser Phe Pro
       130                 135                 140
   Ser Leu Glu Lys Tyr Ala Asp Trp His Tyr Arg Val Asp Pro Asp Pro
   145                 150                 155                 160
   Tyr Arg His Asp Pro Glu Tyr Glu Phe Asp Thr Ser Gln Phe Pro Glu
                   165                 170                 175
   Leu Asn Gln Trp Ala Glu Asp Tyr Ile Gly
               180                 185
<210> 175
   <211> 186
   <212> PRT
   <213> Artificial Sequence
<220>
   <221> SOURCE
   <222> 1..186
   <223> /mol_type="protein"
   /note="subject"
   /organism="Artificial Sequence"
<400> 175
   Phe Gln Phe Gln Ala Arg Tyr Val Leu Leu Thr Tyr Ala Gln Cys Gly
   1               5                   10                  15
   Asp Leu Asp Ala Trp Ala Val Asn Asp His Leu Ala Phe Leu Gly Gly
               20                  25                  30
   Glu Cys Ile Ile Gly Arg Glu His His Ala Asp Gly Gly Thr His Leu
           35                  40                  45
   His Ala Phe Cys Asp Phe Ser Arg Lys Phe Arg Ser Arg Arg Pro Asp
       50                  55                  60
   Val Phe Asp Val Gly Gly Phe His Pro Asn Ile Glu Ala Ser Arg Gly
   65                  70                  75                  80
   Arg Pro Glu Gly Gly Tyr Asp Tyr Ala Ile Lys Asp Gly Asp Val Val
                   85                  90                  95
   Ala Gly Gly Leu Ser Arg Pro Gly Gly Arg Gly Val Tyr Glu Asp Val
               100                 105                 110
   Ser Ser Trp Ser Ile Ile Val Ser Gln Glu Ser Glu Gly Asp Phe Trp
           115                 120                 125
   Glu Cys Val Ala Arg Leu Asp Pro Arg Ser Leu Cys Thr Asn Tyr Asn
       130                 135                 140
   Ser Leu Arg Ala Tyr Ala Asn Trp Lys Tyr Arg Pro Thr Pro Val Gln
   145                 150                 155                 160
   Tyr Val His Pro Ala Gly Val Glu Phe Glu Leu Gly Met Val Pro Glu
                   165                 170                 175
   Leu Ala Val Trp Arg Glu Ile Ala Leu Gly
               180                 185
<210> 176
   <211> 188
   <212> PRT
   <213> Artificial Sequence
<220>
   <221> SOURCE
   <222> 1..188
   <223> /mol_type="protein"
   /note="query"
   /organism="Artificial Sequence"
<400> 176
   Met Ser Thr Phe Arg Phe Tyr Ala Arg Tyr Ala Leu Leu Thr Tyr Ala
   1               5                   10                  15
   Gln Cys Gly Gly Leu Asp Pro Phe Thr Ile Val Ser His Ile Gly Asp
               20                  25                  30
   Leu Gly Gly Glu Cys Ile Ile Gly Arg Glu Ala His Ser Asp Gly Gly
           35                  40                  45
   Thr His Leu His Ala Phe Val Asp Phe Gly Arg Arg Phe Gln Ser Arg
       50                  55                  60
   Arg Ala Ala Val Phe Asp Val Gly Gly Phe His Pro Asn Ile Ser Ile
   65                  70                  75                  80
   Thr Lys Arg Asp Pro Gln Val His Tyr Asp Tyr Ala Ile Lys Asp Gly
                   85                  90                  95
   Asp Val Val Ala Gly Gly Leu Glu Arg Pro Ala Ala Asp Ala Ser Ala
               100                 105                 110
   Gly Val Glu Asn Lys His His Gln Ile Val Leu Ala Pro Thr Arg Ala
           115                 120                 125
   Glu Phe Phe Glu Ala Val Arg Asp Leu Asp Pro Arg Leu Leu Leu Thr
       130                 135                 140
   Ser Phe Pro Ser Leu Glu Lys Tyr Ala Asp Trp His Tyr Arg Val Asp
   145                 150                 155                 160
   Pro Asp Pro Tyr Arg His Asp Pro Glu Tyr Glu Phe Asp Thr Ser Gln
                   165                 170                 175
   Phe Pro Glu Leu Asn Gln Trp Ala Glu Asp Tyr Ile
               180                 185
<210> 177
   <211> 191
   <212> PRT
   <213> Artificial Sequence
<220>
   <221> SOURCE
   <222> 1..191
   <223> /mol_type="protein"
   /note="subject"
   /organism="Artificial Sequence"
<400> 177
   Met Ser Ser Phe Arg Phe Gln Ala Arg Tyr Ala Leu Leu Thr Tyr Ala
   1               5                   10                  15
   Gln Cys Gly Asp Leu Asp Pro Phe Ala Val Ser Asp His Leu Ser Gly
               20                  25                  30
   Leu Arg Ala Glu Cys Ile Ile Gly Arg Glu Thr His Ala Asp Glu Gly
           35                  40                  45
   Ile His Leu His Ala Phe Val Asp Phe Gly Val Lys Phe Ser Thr Arg
       50                  55                  60
   Asn Val Arg Val Phe Asp Val Gln Gly His His Pro Asn Val Glu Pro
   65                  70                  75                  80
   Ser Arg Gly Arg Pro Ala Gly Gly Tyr Asp Tyr Ala Ile Lys Asp Gly
                   85                  90                  95
   Asp Val Val Ala Gly Gly Leu Asp Arg Pro Ala Asp Asp Gly Gly Val
               100                 105                 110
   Gly Val Ser Lys Thr Thr Gln Lys Trp Ala Thr Ile Val Gly Ala Gln
           115                 120                 125
   Thr Arg Asp Glu Phe Trp Ala Leu Leu Ala Glu Leu Asp Pro Gly Ala
       130                 135                 140
   Met Val Arg Ser Phe Thr Gln Cys Arg Ala Tyr Ala Asp His Arg Phe
   145                 150                 155                 160
   Arg Glu Ser Pro Glu Arg Tyr Val Thr Pro Glu Ser Ile Ile Ile Asp
                   165                 170                 175
   Thr Gly His Val Asp Gly Leu Asn Glu Trp Ala Leu Ser Asn Ile
               180                 185                 190
<210> 178
   <211> 183
   <212> PRT
   <213> Artificial Sequence
<220>
   <221> SOURCE
   <222> 1..183
   <223> /mol_type="protein"
   /note="query"
   /organism="Artificial Sequence"
<400> 178
   Arg Tyr Ala Leu Leu Thr Tyr Ala Gln Cys Gly Gly Leu Asp Pro Phe
   1               5                   10                  15
   Thr Ile Val Ser His Ile Gly Asp Leu Gly Gly Glu Cys Ile Ile Gly
               20                  25                  30
   Arg Glu Ala His Ser Asp Gly Gly Thr His Leu His Ala Phe Val Asp
           35                  40                  45
   Phe Gly Arg Arg Phe Gln Ser Arg Arg Ala Ala Val Phe Asp Val Gly
       50                  55                  60
   Gly Phe His Pro Asn Ile Ser Ile Thr Lys Arg Asp Pro Gln Val His
   65                  70                  75                  80
   Tyr Asp Tyr Ala Ile Lys Asp Gly Asp Val Val Ala Gly Gly Leu Glu
                   85                  90                  95
   Arg Pro Ala Ala Asp Ala Ser Ala Gly Val Glu Asn Lys His His Gln
               100                 105                 110
   Ile Val Leu Ala Pro Thr Arg Ala Glu Phe Phe Glu Ala Val Arg Asp
           115                 120                 125
   Leu Asp Pro Arg Leu Leu Leu Thr Ser Phe Pro Ser Leu Glu Lys Tyr
       130                 135                 140
   Ala Asp Trp His Tyr Arg Val Asp Pro Asp Pro Tyr Arg His Asp Pro
   145                 150                 155                 160
   Glu Tyr Glu Phe Asp Thr Ser Gln Phe Pro Glu Leu Asn Gln Trp Ala
                   165                 170                 175
   Glu Asp Tyr Ile Gly Trp Asp
               180
<210> 179
   <211> 183
   <212> PRT
   <213> Artificial Sequence
<220>
   <221> SOURCE
   <222> 1..183
   <223> /mol_type="protein"
   /note="subject"
   /organism="Artificial Sequence"
<400> 179
   Arg Tyr Ala Leu Leu Thr Tyr Ala Gln Cys Gly Asp Leu Asp Ala Trp
   1               5                   10                  15
   Ala Val Asn Asp His Met Ala Phe Leu Gly Gly Glu Cys Ile Ile Gly
               20                  25                  30
   Arg Glu Leu His Ala Asp Gly Gly Thr His Leu His Ala Phe Val Asp
           35                  40                  45
   Phe Ser Arg Lys Phe Arg Ser Arg Arg Ser Asp Val Phe Asp Val Gly
       50                  55                  60
   Gly Arg His Pro Asn Leu Val Pro Ser Tyr Gly Ser Pro Glu Gly Gly
   65                  70                  75                  80
   Tyr Asp Tyr Ala Ile Lys Asp Gly Asp Val Val Ala Gly Gly Leu Ser
                   85                  90                  95
   Arg Pro Ser Gly Arg Gly Val Tyr Glu Asp Val Ser Ser Trp Ser Ile
               100                 105                 110
   Ile Val Ser Gln Glu Ser Glu Gly Asp Phe Trp Glu Cys Val Ala Arg
           115                 120                 125
   Leu Asp Pro Arg Ser Leu Cys Thr Asn Tyr Asn Ser Leu Arg Ala Tyr
       130                 135                 140
   Ala Asn Trp Lys Tyr Gln Pro Leu Pro Val Arg Tyr Glu His Pro Ala
   145                 150                 155                 160
   Gly Val Lys Phe Glu Leu Gly Met Val Pro Glu Leu Ala Val Trp Arg
                   165                 170                 175
   Glu Val Ala Leu Gly Val Asp
               180
<210> 180
   <211> 102
   <212> PRT
   <213> Artificial Sequence
<220>
   <221> SOURCE
   <222> 1..102
   <223> /mol_type="protein"
   /note="query"
   /organism="Artificial Sequence"
<400> 180
   Met Trp Ala Arg Ser Leu Ala Lys His Ala Tyr Phe Gly Gly Leu Phe
   1               5                   10                  15
   Ser Leu Glu Glu Ser Leu Glu Asp Val Lys Tyr Ala Val Phe Asp Asp
               20                  25                  30
   Ile Ala Gly Gly Leu Lys Phe Phe Pro Gln Tyr Lys Ser Trp Leu Gly
           35                  40                  45
   Asn Gln Leu Asp Phe Tyr Ser Thr Asp Lys Tyr Lys Lys Lys Lys Ala
       50                  55                  60
   Ile Lys Trp Gly Lys Pro Cys Ile Trp Ile Ser Asn Glu Tyr Pro Ile
   65                  70                  75                  80
   Thr Glu Gly Val Asp Gln Asp Trp Met Asp Lys Asn Cys Ile Phe Val
                   85                  90                  95
   Glu Val Thr Thr Pro Leu
               100
<210> 181
   <211> 103
   <212> PRT
   <213> Artificial Sequence
<220>
   <221> SOURCE
   <222> 1..103
   <223> /mol_type="protein"
   /note="subject"
   /organism="Artificial Sequence"
<400> 181
   Leu Trp Ala Arg Ser Leu Gly Asn His Ala Tyr Phe Gly Gly Leu Phe
   1               5                   10                  15
   Ser Leu Glu Glu Glu Leu Glu Gly Val Asp Tyr Ala Ile Phe Asp Asp
               20                  25                  30
   Met Gln Gly Gly Leu Lys Phe Phe His Ser Tyr Lys Phe Trp Leu Gly
           35                  40                  45
   Ala Gln Ser Gln Phe Trp Ala Thr Asp Lys Tyr Arg Gly Lys Lys Leu
       50                  55                  60
   Ile His Trp Gly Arg Pro Ser Ile Tyr Ile Cys Asn Gln Asn Pro Leu
   65                  70                  75                  80
   Cys Asp Glu Gly Val Asp His Asp Trp Leu Ile Gly Asn Cys Asp Phe
                   85                  90                  95
   Val Glu Ile Thr Glu Ser Leu
               100
<210> 182
   <211> 103
   <212> PRT
   <213> Artificial Sequence
<220>
   <221> SOURCE
   <222> 1..103
   <223> /mol_type="protein"
   /note="query"
   /organism="Artificial Sequence"
<400> 182
   Met Trp Ala Arg Ser Leu Ala Lys His Ala Tyr Phe Gly Gly Leu Phe
   1               5                   10                  15
   Ser Leu Glu Glu Ser Leu Glu Asp Val Lys Tyr Ala Val Phe Asp Asp
               20                  25                  30
   Ile Ala Gly Gly Leu Lys Phe Phe Pro Gln Tyr Lys Ser Trp Leu Gly
           35                  40                  45
   Asn Gln Leu Asp Phe Tyr Ser Thr Asp Lys Tyr Lys Lys Lys Lys Ala
       50                  55                  60
   Ile Lys Trp Gly Lys Pro Cys Ile Trp Ile Ser Asn Glu Tyr Pro Ile
   65                  70                  75                  80
   Thr Glu Gly Val Asp Gln Asp Trp Met Asp Lys Asn Cys Ile Phe Val
                   85                  90                  95
   Glu Val Thr Thr Pro Leu Tyr
               100
<210> 183
   <211> 104
   <212> PRT
   <213> Artificial Sequence
<220>
   <221> SOURCE
   <222> 1..104
   <223> /mol_type="protein"
   /note="subject"
   /organism="Artificial Sequence"
<400> 183
   Leu Trp Ala Arg Ser Leu Gly Arg His Ala Tyr Phe Gly Gly Leu Phe
   1               5                   10                  15
   Cys Leu Asp Glu Ser Leu Glu Asp Val Asp Tyr Ala Val Phe Asp Asp
               20                  25                  30
   Met Gln Gly Gly Leu Glu Phe Phe His Ala Tyr Lys Phe Trp Leu Gly
           35                  40                  45
   Ala Gln Lys Gln Phe Tyr Ala Thr Asp Lys Tyr Arg Gly Lys Gln Leu
       50                  55                  60
   Val Asn Trp Gly Arg Pro Ser Ile Tyr Ile Ser Asn Thr Asn Pro Leu
   65                  70                  75                  80
   Ala Asp Lys Gly Ala Asp Val Asp Trp Leu Met Gly Asn Cys Val Ile
                   85                  90                  95
   Val His Val Asp Arg Pro Ile Phe
               100
<210> 184
   <211> 102
   <212> PRT
   <213> Artificial Sequence
<220>
   <221> SOURCE
   <222> 1..102
   <223> /mol_type="protein"
   /note="query"
   /organism="Artificial Sequence"
<400> 184
   Met Trp Ala Arg Ser Leu Ala Lys His Ala Tyr Phe Gly Gly Leu Phe
   1               5                   10                  15
   Ser Leu Glu Glu Ser Leu Glu Asp Val Lys Tyr Ala Val Phe Asp Asp
               20                  25                  30
   Ile Ala Gly Gly Leu Lys Phe Phe Pro Gln Tyr Lys Ser Trp Leu Gly
           35                  40                  45
   Asn Gln Leu Asp Phe Tyr Ser Thr Asp Lys Tyr Lys Lys Lys Lys Ala
       50                  55                  60
   Ile Lys Trp Gly Lys Pro Cys Ile Trp Ile Ser Asn Glu Tyr Pro Ile
   65                  70                  75                  80
   Thr Glu Gly Val Asp Gln Asp Trp Met Asp Lys Asn Cys Ile Phe Val
                   85                  90                  95
   Glu Val Thr Thr Pro Leu
               100
<210> 185
   <211> 105
   <212> PRT
   <213> Artificial Sequence
<220>
   <221> SOURCE
   <222> 1..105
   <223> /mol_type="protein"
   /note="subject"
   /organism="Artificial Sequence"
<400> 185
   Val Trp Ala Arg Ser Leu Gly Asp His Ala Tyr Phe Gly Gly Leu Phe
   1               5                   10                  15
   Ser Met Asp Glu Asp Ile Ser Ser Ala Thr Tyr Ala Val Phe Asp Asp
               20                  25                  30
   Phe Gly Gly Leu Lys Phe Leu Pro Ser Tyr Lys Phe Trp Met Gly His
           35                  40                  45
   Gln Lys Gln Phe Tyr Val Thr Asp Lys Tyr Lys Gly Lys Lys Leu Val
       50                  55                  60
   Glu Trp Gly Arg Pro Ser Ile Trp Ile Asn Asn Glu Asp Pro Arg Glu
   65                  70                  75                  80
   Glu His Gly Val Arg Cys Asp Glu Ile Glu Trp Leu Glu Gln Asn Cys
                   85                  90                  95
   Ile Phe Ile Arg Leu Asp Ser Thr Ile
               100                 105
<210> 186
   <211> 2120
   <212> DNA
   <213> Artificial Sequence
<220>
   <221> source
   <222> 1..2120
   <223> /mol_type="DNA"
   /note="HCBI9.212"
   /organism="Artificial Sequence"
<400> 186
   atttttcgac cagtaaaaaa tggcttacgg attccgccgc cgctttggtc gctcccgtcg 60
   ctcgtaccga cgaaggcccg ttcgccgcta ttcccgaagg ggtagaagcg ggtatggtcg 120
   tggcggttac aaccgtcggc gttatcgtcg tcgtcgccgc tcttggatta gtccttacaa 180
   caaaaagcat gacgtgatta agggggcgca tgattcgaat gataattcgt tcgcccctat 240
   aggtgccccc aacacctttt ttgggttctg cccaacctac atgcccaacg ccgttgctgg 300
   ggatactggt cacaatcctc aaaaggttcg gaccagtaag aatgttgggt tcagtggata 360
   tcaggaacgt gttcatattg ctacggctga atctttgatt tggcgtcgca tggttatttg 420
   gtcatataac cggcttgaga atacggccgg tccgacgaag acggatgatc agggtacttc 480
   gtacacgaca cgtcagctta ccccgctcca attaaacgaa ggggtccgtg cgtttttgtt 540
   ccgaggtact cagggtatcg attacactga gaacaccttg catgaggctc cgctgaattc 600
   cgatcacttt accgttgcgt atgatcgtac acgtgtgtta cagcctcgtc gtcctactgg 660
   tgacgaatac ggtcatattt ttaatatgaa gttctggaac cctggtggaa ggattattta 720
   ctcggatgat gaggatggtt tacagaaggg tcctcttgtg aacggatggt cttctcttgg 780
   acgtgtgtct aagggaaaca tgtatatttt tgatgtcttt tctacggggt attctttgag 840
   tagtaatact ccttctattg ctagatttgc tccgactggg actcgttact ggacggaggg 900
   ttagatacaa tacaaattgg atccaccaca ttgattatgt gacagttgtt cataagccaa 960
   tcgtagtcca cccctttagc gaagtgtggg tcctcgttca tgcacatgat tgagggtttg 1020
   ccccagtaga tcgttcgctt ctttcggtac ttgtccgtga tgacgaattg tttttgtgcg 1080
   cctagccacc ccttgtatga gtggaagtat tcgaatcctc cctggatgtc gtcgaagacg 1140
   gcgtattgac aatcgtcgga gaattcgtcc atgttgaatt gtaggttgaa gtacgcatgg 1200
   tgtcccaacg atctggcgta tagcgtcttt ccggttcttg ttggtcccca taaaatgagt 1260
   gatttaactc tgcccaagta aagatcagta atggggttcg gggcgaagcc ctgaagcgga 1320
   caagatttga tcgtgcgagg acgctacgtc ggccgcagcg ccagcagact tacctctcgg 1380
   gtctacagtc cccaatggct gcctgattct cccactcgga gatctcagga tagcgttctc 1440
   tctccacaat gatgggcggc gcctcgtatt ccggtttctc gtcggggtaa taaaaattgg 1500
   cgtattcgat gatgcgctga ttggtagcaa ccaatctctt ggagcttgtt gacggacagc 1560
   cgacagaaat gattctttag tgtcgcagct gatgatatca gcccacttat ttgaattatc 1620
   cccgtcacat gagcgagcat ctgctggggg ctcgccgtgc tcaagtcgga tgtctccatc 1680
   cttgccgaca taatcgaaga ccttgtgtgg agttcgacga atcgatttaa tattgccgtg 1740
   agctccaaag tagtcgaacg cagttgccga gttgacagtg acaatcttgt cgaagccaag 1800
   atatatgtgg taatgaattc ctccatccga atggcgctct cgtccgagtc gacagcataa 1860
   aggatttcca aaattcgctg tgacacgttc aaggaaagga gttcctgcgt cgaattcatg 1920
   ttggaggtgt tcggggactt gtggaaacgt aatgataaga tttcttgctc tgacttggaa 1980
   tcgtcgaggc attttaagga gtcgttcacg tgacgtgtgc aaatagagag agacttggct 2040
   aatattatct ctctctattt gcagtttgca cattgcacat ggctgcctat aaatacccct 2100
   catcccccct gcggtgagtc 2120
<210> 187
   <211> 146
   <212> DNA
   <213> Artificial Sequence
<220>
   <221> source
   <222> 1..146
   <223> /mol_type="DNA"
   /note="query"
   /organism="Artificial Sequence"
<400> 187
   gggtcctcgt tcatgcacat gattgagggt ttgccccagt agatcgttcg cttctttcgg 60
   tacttgtccg tgatgacgaa ttgtttttgt gcgcctagcc accccttgta tgagtggaag 120
   tattcgaatc ctccctggat gtcgtc 146
<210> 188
   <211> 146
   <212> DNA
   <213> Artificial Sequence
<220>
   <221> source
   <222> 1..146
   <223> /mol_type="DNA"
   /note="subject"
   /organism="Artificial Sequence"
<400> 188
   gggtcctcgt tcataagcca gatagtgggg cgcccccact ggatggttgt cttccctcgg 60
   tayttgtcgg tgacggtgaa tgtttgttgt gcaccyagcc acccyttgta ggaaggaaag 120
   aattggaagt ttccttgcat atcgtc 146
<210> 189
   <211> 71
   <212> DNA
   <213> Artificial Sequence
<220>
   <221> source
   <222> 1..71
   <223> /mol_type="DNA"
   /note="query"
   /organism="Artificial Sequence"
<400> 189
   tgtgcgccta gccacccctt gtatgagtgg aagtattcga atcctccctg gatgtcgtcg 60
   aagacggcgt a 71
<210> 190
   <211> 71
   <212> DNA
   <213> Artificial Sequence
<220>
   <221> source
   <222> 1..71
   <223> /mol_type="DNA"
   /note="subject"
   /organism="Artificial Sequence"
<400> 190
   tgagcaccga gccactcctt gtatccgtga aagaatccaa aaccacctct gatgtcgtcg 60
   aagacggcgt a 71
<210> 191
   <211> 102
   <212> DNA
   <213> Artificial Sequence
<220>
   <221> source
   <222> 1..102
   <223> /mol_type="DNA"
   /note="query"
   /organism="Artificial Sequence"
<400> 191
   tcggtacttg tccgtgatga cgaattgttt ttgtgcgcct agccacccct tgtatgagtg 60
   gaagtattcg aatcctccct ggatgtcgtc gaagacggcg ta 102
<210> 192
   <211> 102
   <212> DNA
   <213> Artificial Sequence
<220>
   <221> source
   <222> 1..102
   <223> /mol_type="DNA"
   /note="subject"
   /organism="Artificial Sequence"
<400> 192
   tctgtacttg tccgtaactg taaacttgga ctgcgccccc aaccacccct tgtatgacgg 60
   gaaatacttg aatccgccct gcatatcatc aaatactgca ta 102
<210> 193
   <211> 276
   <212> PRT
   <213> Artificial Sequence
<220>
   <221> SOURCE
   <222> 1..276
   <223> /mol_type="protein"
   /note="query"
   /organism="Artificial Sequence"
<400> 193
   Arg Arg Pro Val Arg Arg Tyr Ser Arg Arg Gly Arg Ser Gly Tyr Gly
   1               5                   10                  15
   Arg Gly Gly Tyr Asn Arg Arg Arg Tyr Arg Arg Arg Arg Arg Ser Trp
               20                  25                  30
   Ile Ser Pro Tyr Asn Lys Lys His Asp Val Ile Lys Gly Ala His Asp
           35                  40                  45
   Ser Asn Asp Asn Ser Phe Ala Pro Ile Gly Ala Pro Asn Thr Phe Phe
       50                  55                  60
   Gly Phe Cys Pro Thr Tyr Met Pro Asn Ala Val Ala Gly Asp Thr Gly
   65                  70                  75                  80
   His Asn Pro Gln Lys Val Arg Thr Ser Lys Asn Val Gly Phe Ser Gly
                   85                  90                  95
   Tyr Gln Glu Arg Val His Ile Ala Thr Ala Glu Ser Leu Ile Trp Arg
               100                 105                 110
   Arg Met Val Ile Trp Ser Tyr Asn Arg Leu Glu Asn Thr Ala Gly Pro
           115                 120                 125
   Thr Lys Thr Asp Asp Gln Gly Thr Ser Tyr Thr Thr Arg Gln Leu Thr
       130                 135                 140
   Pro Leu Gln Leu Asn Glu Gly Val Arg Ala Phe Leu Phe Arg Gly Thr
   145                 150                 155                 160
   Gln Gly Ile Asp Tyr Thr Glu Asn Thr Leu His Glu Ala Pro Leu Asn
                   165                 170                 175
   Ser Asp His Phe Thr Val Ala Tyr Asp Arg Thr Arg Val Leu Gln Pro
               180                 185                 190
   Arg Arg Pro Thr Gly Asp Glu Tyr Gly His Ile Phe Asn Met Lys Phe
           195                 200                 205
   Trp Asn Pro Gly Gly Arg Ile Ile Tyr Ser Asp Asp Glu Asp Gly Leu
       210                 215                 220
   Gln Lys Gly Pro Leu Val Asn Gly Trp Ser Ser Leu Gly Arg Val Ser
   225                 230                 235                 240
   Lys Gly Asn Met Tyr Ile Phe Asp Val Phe Ser Thr Gly Tyr Ser Leu
                   245                 250                 255
   Ser Ser Asn Thr Pro Ser Ile Ala Arg Phe Ala Pro Thr Gly Thr Arg
               260                 265                 270
   Tyr Trp Thr Glu
           275
<210> 194
   <211> 287
   <212> PRT
   <213> Artificial Sequence
<220>
   <221> SOURCE
   <222> 1..287
   <223> /mol_type="protein"
   /note="subject"
   /organism="Artificial Sequence"
<400> 194
   Arg Arg Lys Tyr Thr Arg Thr Ile Arg Arg Gly Arg Ala Gly Ser Ser
   1               5                   10                  15
   Lys Lys Arg Arg Ser Tyr Val Gly Arg Arg Leu Ser Ser Arg Arg Thr
               20                  25                  30
   Thr Lys Thr Val Met Lys Arg Thr Ser Arg Arg Arg Ile Leu Asn Ile
           35                  40                  45
   Ser Ser Gln Lys Lys Arg Asn Asn Ala Gln Pro Val Asn Phe Asn Tyr
       50                  55                  60
   Leu Gly Glu Ser Pro Ile Pro Gly Ser Lys Val Met Gly Ser Asp Arg
   65                  70                  75                  80
   Thr Thr Tyr Leu Leu Trp Arg Pro Thr Ala Leu Thr Asp Asp Thr Asp
                   85                  90                  95
   Ile Tyr Ser Ser Ala Ser Arg Gly Ala Gln Gln Ile Tyr Trp Arg Gly
               100                 105                 110
   Ile Lys Glu Arg Ala Glu Val Gly Thr Ser Ser Gly Ala Ala Trp Arg
           115                 120                 125
   Trp Arg Arg Ile Val Phe Glu Ala Lys Ser Leu Asn Ile Asn Gly Leu
       130                 135                 140
   Ile Ser Asn Val Glu Thr Ser Ala Gly Phe Asn Arg Ala Met Val Glu
   145                 150                 155                 160
   Leu Ser Gly Asp Pro Pro Ala Asn Leu Arg Asn Ser Leu Glu Ser Phe
                   165                 170                 175
   Leu Phe Gln Gly Ser Val Gly Val Asp Trp Asn Thr Val Phe Asn Ala
               180                 185                 190
   Lys Val Asp Asn Asn Arg Cys Arg Leu Leu Ser Asp Lys Val Arg His
           195                 200                 205
   Leu Gln Ser Gly Asn Asp Arg Gly Arg Phe Tyr Thr Phe Arg Gln Trp
       210                 215                 220
   Ile Pro Leu Asn Lys Thr Met Val Tyr Asn Asp Asp Glu Val Gly Asp
   225                 230                 235                 240
   Asp Lys Gln Ser Asn Thr Phe Ala Ser Pro Gly Arg Lys Gly Met Gly
                   245                 250                 255
   Asp Val Tyr Val Tyr Asp Met Phe Gln Cys Leu Thr Ser Asp Ala Ile
               260                 265                 270
   Asn Thr Leu Asn Phe Asn Pro Gln Ala Thr Leu Tyr Trp His Glu
           275                 280                 285
<210> 195
   <211> 293
   <212> PRT
   <213> Artificial Sequence
<220>
   <221> SOURCE
   <222> 1..293
   <223> /mol_type="protein"
   /note="query"
   /organism="Artificial Sequence"
<400> 195
   Met Ala Tyr Gly Phe Arg Arg Arg Phe Gly Arg Ser Arg Arg Ser Tyr
   1               5                   10                  15
   Arg Arg Arg Pro Val Arg Arg Tyr Ser Arg Arg Gly Arg Ser Gly Tyr
               20                  25                  30
   Gly Arg Gly Gly Tyr Asn Arg Arg Arg Tyr Arg Arg Arg Arg Arg Ser
           35                  40                  45
   Trp Ile Ser Pro Tyr Asn Lys Lys His Asp Val Ile Lys Gly Ala His
       50                  55                  60
   Asp Ser Asn Asp Asn Ser Phe Ala Pro Ile Gly Ala Pro Asn Thr Phe
   65                  70                  75                  80
   Phe Gly Phe Cys Pro Thr Tyr Met Pro Asn Ala Val Ala Gly Asp Thr
                   85                  90                  95
   Gly His Asn Pro Gln Lys Val Arg Thr Ser Lys Asn Val Gly Phe Ser
               100                 105                 110
   Gly Tyr Gln Glu Arg Val His Ile Ala Thr Ala Glu Ser Leu Ile Trp
           115                 120                 125
   Arg Arg Met Val Ile Trp Ser Tyr Asn Arg Leu Glu Asn Thr Ala Gly
       130                 135                 140
   Pro Thr Lys Thr Asp Asp Gln Gly Thr Ser Tyr Thr Thr Arg Gln Leu
   145                 150                 155                 160
   Thr Pro Leu Gln Leu Asn Glu Gly Val Arg Ala Phe Leu Phe Arg Gly
                   165                 170                 175
   Thr Gln Gly Ile Asp Tyr Thr Glu Asn Thr Leu His Glu Ala Pro Leu
               180                 185                 190
   Asn Ser Asp His Phe Thr Val Ala Tyr Asp Arg Thr Arg Val Leu Gln
           195                 200                 205
   Pro Arg Arg Pro Thr Gly Asp Glu Tyr Gly His Ile Phe Asn Met Lys
       210                 215                 220
   Phe Trp Asn Pro Gly Gly Arg Ile Ile Tyr Ser Asp Asp Glu Asp Gly
   225                 230                 235                 240
   Leu Gln Lys Gly Pro Leu Val Asn Gly Trp Ser Ser Leu Gly Arg Val
                   245                 250                 255
   Ser Lys Gly Asn Met Tyr Ile Phe Asp Val Phe Ser Thr Gly Tyr Ser
               260                 265                 270
   Leu Ser Ser Asn Thr Pro Ser Ile Ala Arg Phe Ala Pro Thr Gly Thr
           275                 280                 285
   Arg Tyr Trp Thr Glu
       290
<210> 196
   <211> 302
   <212> PRT
   <213> Artificial Sequence
<220>
   <221> SOURCE
   <222> 1..302
   <223> /mol_type="protein"
   /note="subject"
   /organism="Artificial Sequence"
<400> 196
   Ser Met Ala Tyr Ala Arg Arg Arg Phe Thr Ser Ser Arg Ala Arg Lys
   1               5                   10                  15
   Pro Ile Arg Arg Ser Arg Arg Pro Ala Gly Lys Arg Arg Ser Tyr Arg
               20                  25                  30
   Arg Ser Thr Arg Lys Arg Gly Met Ser Thr Arg Thr Leu Leu Asn Lys
           35                  40                  45
   Thr Ser Gln Lys Lys Arg Asp Asn Met Leu Ser Phe Ser Asn Thr Thr
       50                  55                  60
   Ala Glu Asp Pro Phe Ser Thr Asp Tyr Thr Ile Gly Pro Ala Val Met
   65                  70                  75                  80
   Arg Arg Pro Val Gly Ala Asp Leu Pro Ala Glu Phe Ile Tyr Val Trp
                   85                  90                  95
   Asn Ala Thr Gly Arg Pro His Glu Leu Ser Thr Gly Phe Arg Gly Ser
               100                 105                 110
   Lys Ile Asp Thr Ser Leu Arg Thr Asn Thr Thr Ile Phe Ala Val Gly
           115                 120                 125
   Leu Lys Glu Arg Ile Lys Leu Glu Thr Asn Asn Ser Ala Pro Trp Arg
       130                 135                 140
   Trp Arg Arg Ile Cys Phe Thr Ser Lys Asn Asp Tyr Gly Glu Ala Asp
   145                 150                 155                 160
   Pro Asp Glu Ser Asp Tyr Phe Arg Arg Thr Ser Asn Gly Met Val Arg
                   165                 170                 175
   Leu Leu Arg Ala Gln Ser Gln Ala Glu Glu Ile Asn Asp Asp Val Phe
               180                 185                 190
   Asp Gly Gln Arg Asn Val Asp Trp Leu Ser Ala Ile Thr Ala Pro Ile
           195                 200                 205
   Asn Arg Arg His Val Ser Val His Tyr Asp Arg Thr Arg Thr Ile Gln
       210                 215                 220
   Ser Gly Asn Gln Ser Gly Val Thr Arg Thr Tyr Arg Gln Trp His Pro
   225                 230                 235                 240
   Met Arg Lys Asn Val Val Tyr Ala Asp Glu Gln Ala Gly Glu Gly Met
                   245                 250                 255
   Val Asp Ser Gly Val Ser Val Thr Gly Lys Val Gly Met Gly Asn Tyr
               260                 265                 270
   Tyr Val Met Asp Ile Phe Thr Lys His Gly Leu Asn Asp Asp Gln Ser
           275                 280                 285
   Thr Leu Ser Tyr Glu Pro Glu Ser Thr Phe Phe Trp His Glu
       290                 295                 300
<210> 197
   <211> 283
   <212> PRT
   <213> Artificial Sequence
<220>
   <221> SOURCE
   <222> 1..283
   <223> /mol_type="protein"
   /note="query"
   /organism="Artificial Sequence"
<400> 197
   Arg Ser Arg Arg Ser Tyr Arg Arg Arg Pro Val Arg Arg Tyr Ser Arg
   1               5                   10                  15
   Arg Gly Arg Ser Gly Tyr Gly Arg Gly Gly Tyr Asn Arg Arg Arg Tyr
               20                  25                  30
   Arg Arg Arg Arg Arg Ser Trp Ile Ser Pro Tyr Asn Lys Lys His Asp
           35                  40                  45
   Val Ile Lys Gly Ala His Asp Ser Asn Asp Asn Ser Phe Ala Pro Ile
       50                  55                  60
   Gly Ala Pro Asn Thr Phe Phe Gly Phe Cys Pro Thr Tyr Met Pro Asn
   65                  70                  75                  80
   Ala Val Ala Gly Asp Thr Gly His Asn Pro Gln Lys Val Arg Thr Ser
                   85                  90                  95
   Lys Asn Val Gly Phe Ser Gly Tyr Gln Glu Arg Val His Ile Ala Thr
               100                 105                 110
   Ala Glu Ser Leu Ile Trp Arg Arg Met Val Ile Trp Ser Tyr Asn Arg
           115                 120                 125
   Leu Glu Asn Thr Ala Gly Pro Thr Lys Thr Asp Asp Gln Gly Thr Ser
       130                 135                 140
   Tyr Thr Thr Arg Gln Leu Thr Pro Leu Gln Leu Asn Glu Gly Val Arg
   145                 150                 155                 160
   Ala Phe Leu Phe Arg Gly Thr Gln Gly Ile Asp Tyr Thr Glu Asn Thr
                   165                 170                 175
   Leu His Glu Ala Pro Leu Asn Ser Asp His Phe Thr Val Ala Tyr Asp
               180                 185                 190
   Arg Thr Arg Val Leu Gln Pro Arg Arg Pro Thr Gly Asp Glu Tyr Gly
           195                 200                 205
   His Ile Phe Asn Met Lys Phe Trp Asn Pro Gly Gly Arg Ile Ile Tyr
       210                 215                 220
   Ser Asp Asp Glu Asp Gly Leu Gln Lys Gly Pro Leu Val Asn Gly Trp
   225                 230                 235                 240
   Ser Ser Leu Gly Arg Val Ser Lys Gly Asn Met Tyr Ile Phe Asp Val
                   245                 250                 255
   Phe Ser Thr Gly Tyr Ser Leu Ser Ser Asn Thr Pro Ser Ile Ala Arg
               260                 265                 270
   Phe Ala Pro Thr Gly Thr Arg Tyr Trp Thr Glu
           275                 280
<210> 198
   <211> 305
   <212> PRT
   <213> Artificial Sequence
<220>
   <221> SOURCE
   <222> 1..305
   <223> /mol_type="protein"
   /note="subject"
   /organism="Artificial Sequence"
<400> 198
   Arg Ser Ser Arg Phe Arg Ala Arg Arg Pro Ile Lys Thr Ser Arg Arg
   1               5                   10                  15
   Gly Gly Val Ser Lys Pro Arg Arg Arg Ser Tyr Ser Ala Lys Lys Arg
               20                  25                  30
   Thr Tyr Arg Lys Lys Ala Ser Met Ser Lys Arg Ser Ile Leu Asn Thr
           35                  40                  45
   Thr Ser Arg Lys Lys Arg Asn Gly Met Leu Thr Phe Ser Asn Thr Thr
       50                  55                  60
   Asn Ser Gly Ala Ser Thr Ala Pro Ala Ala Ala Pro Val Tyr Val Asn
   65                  70                  75                  80
   Ala Val Leu Gly Gly Thr Phe Ile Trp Cys Pro Thr Ala Met Asn Leu
                   85                  90                  95
   Val Ala Gln Ser Leu Ile Ser Asn Met Ser Ser His Thr Ala Thr Thr
               100                 105                 110
   Cys Tyr Met Arg Gly Leu Ser Glu His Ile Arg Ile Gln Thr Ser Ser
           115                 120                 125
   Gly Ile Pro Trp Phe His Arg Arg Val Cys Phe Thr Trp Lys Gly Leu
       130                 135                 140
   Gly Pro Phe Asn Val Met Ala Thr Glu Ser Pro Thr Ser Asp Phe Pro
   145                 150                 155                 160
   Trp Leu Pro Tyr Gln Asp Ser Ser Asn Gly Val Gln Arg Leu Phe Phe
                   165                 170                 175
   Asn Glu Asn Val Asn Asn Met Pro Gln Thr Ile Asn Arg Gln Arg Asn
               180                 185                 190
   Ile Leu Phe Lys Gly Ala Glu Asn Val Asp Trp Thr Asp Pro Ile Ile
           195                 200                 205
   Ala Pro Leu Asp Thr Gly Arg Ile Thr Val Lys Phe Asp Lys Thr Trp
       210                 215                 220
   Thr Met Gln Ser Gly Asn Thr Asn Gly Ile Val Arg Glu Arg Lys Leu
   225                 230                 235                 240
   Trp His Pro Met Asn His Asn Leu Val Tyr Asp Asp Asp Glu Tyr Gly
                   245                 250                 255
   Asp Ile Glu Ser Gly Ser Trp Phe Ser Thr Asp Ser Lys Ala Gly Met
               260                 265                 270
   Gly Asp Tyr Tyr Val Met Asp Ile Ile Gln Gly Gly Gln Gly Gly Thr
           275                 280                 285
   Ala Ser Asp Leu Leu Arg Met Thr Ala Asn Ser Thr Met Tyr Trp His
               290                 295                 300
   Glu
   305
<210> 199
   <211> 151
   <212> PRT
   <213> Artificial Sequence
<220>
   <221> SOURCE
   <222> 1..151
   <223> /mol_type="protein"
   /note="query"
   /organism="Artificial Sequence"
<400> 199
   Pro Leu Cys Cys Arg Leu Gly Arg Glu Arg His Ser Asp Gly Gly Ile
   1 5 10 15
   His Tyr His Ile Tyr Leu Gly Phe Asp Lys Ile Val Thr Val Asn Ser
   20 25 30
   Ala Thr Ala Phe Asp Tyr Phe Gly Ala His Gly Asn Ile Lys Ser Ile
   35 40 45
   Arg Arg Thr Pro His Lys Val Phe Asp Tyr Val Gly Lys Asp Gly Asp
   50 55 60
   Ile Arg Leu Glu His Gly Glu Pro Pro Ala Asp Ala Arg Ser Cys Asp
   65 70 75 80
   Gly Asp Asn Ser Asn Lys Trp Ala Asp Ile Ile Ser Cys Asp Thr Lys
   85 90 95
   Glu Ser Phe Leu Ser Ala Val Arg Gln Gln Ala Pro Arg Asp Trp Leu
   100 105 110
   Leu Ser Asn Gln Arg Ile Ile Glu Tyr Ala Asn Phe Tyr Tyr Pro Asp
   115 120 125
   Glu Lys Pro Glu Tyr Glu Ala Pro Pro Ile Ile Val Glu Arg Glu Arg
   130 135 140
   Tyr Pro Glu Ile Ser Glu Trp
   145 150
<210> 200
   <211> 150
   <212> PRT
   <213> Artificial Sequence
<220>
   <221> SOURCE
   <222> 1..150
   <223> /mol_type="protein"
   /note="subject"
   /organism="Artificial Sequence"
<400> 200
   Pro Ala Glu Cys Ile Ile Gly Arg Glu Asn His Ala Asp Gly Gly Ile
   1 5 10 15
   His Leu His Ala Phe Val Asp Phe Gly Lys Arg Val Asp Ile Arg Asp
   20 25 30
   Asn Arg Arg Phe Asp Val Glu Gly Phe His Pro Asn Ile Ala Pro Cys
   35 40 45
   Gly Arg Thr Pro Gln Lys Met Leu Asp Tyr Ala Ile Lys Asp Gly Asp
   50 55 60
   Val Val Ala Gly Gly Leu Asn Pro Ile Leu Asp Leu Pro Asp Ala Glu
   65 70 75 80
   Thr Asp Ser Ile Trp His Arg Ile Ala Asn Ala Pro Ser Val Asp Glu
   85 90 95
   Phe Trp Ala Ile Thr Arg Glu Leu Ala Pro Arg Ala Leu Leu Cys Asn
   100 105 110
   His Gln Ser Leu Arg Ala Tyr Ala Glu Trp His Tyr Arg Pro Val Ala
   115 120 125
   Thr Arg Tyr Glu His Pro His Gly Leu Ser Leu Arg Thr Asp His Val
   130 135 140
   Pro Gln Leu Asp Glu Trp
   145 150
<210> 201
   <211> 179
   <212> PRT
   <213> Artificial Sequence
<220>
   <221> SOURCE
   <222> 1..179
   <223> /mol_type="protein"
   /note="query"
   /organism="Artificial Sequence"
<400> 201
   Met Pro Arg Arg Phe Gln Val Arg Ala Arg Asn Leu Ile Ile Thr Phe
   1               5                   10                  15
   Pro Gln Val Pro Glu His Leu Gln His Glu Phe Asp Ala Gly Thr Pro
               20                  25                  30
   Phe Leu Glu Arg Val Thr Ala Asn Phe Gly Asn Pro Leu Cys Cys Arg
           35                  40                  45
   Leu Gly Arg Glu Arg His Ser Asp Gly Gly Ile His Tyr His Ile Tyr
       50                  55                  60
   Leu Gly Phe Asp Lys Ile Val Thr Val Asn Ser Ala Thr Ala Phe Asp
   65                  70                  75                  80
   Tyr Phe Gly Ala His Gly Asn Ile Lys Ser Ile Arg Arg Thr Pro His
                   85                  90                  95
   Lys Val Phe Asp Tyr Val Gly Lys Asp Gly Asp Ile Arg Leu Glu His
               100                 105                 110
   Gly Glu Pro Pro Ala Asp Ala Arg Ser Cys Asp Gly Asp Asn Ser Asn
           115                 120                 125
   Lys Trp Ala Asp Ile Ile Ser Cys Asp Thr Lys Glu Ser Phe Leu Ser
       130                 135                 140
   Ala Val Arg Gln Gln Ala Pro Arg Asp Trp Leu Leu Ser Asn Gln Arg
   145                 150                 155                 160
   Ile Ile Glu Tyr Ala Asn Phe Tyr Tyr Pro Asp Glu Lys Pro Glu Tyr
                   165                 170                 175
   Glu Ala Pro
<210> 202
   <211> 184
   <212> PRT
   <213> Artificial Sequence
<220>
   <221> SOURCE
   <222> 1..184
   <223> /mol_type="protein"
   /note="subject"
   /organism="Artificial Sequence"
<400> 202
   Met Ser Arg Gln Phe Cys Leu Lys Asp Ala Lys Tyr Cys Leu Leu Thr
   1               5                   10                  15
   Tyr Pro Gln Ile Pro Glu Thr Glu Ala Tyr Glu Phe Pro Glu Leu Ala
               20                  25                  30
   Ser Glu Leu Phe Ser Ser Glu Arg Val Asp Ala Asn Tyr Val Ile Gly
           35                  40                  45
   Arg Glu Leu His Ala Asp Gly Gly Tyr Thr Ser His Cys Phe Leu Asp
       50                  55                  60
   Phe Gly Arg Lys Phe Ser Ser Arg Asp Thr Arg Ile Phe Asp Ile Gln
   65                  70                  75                  80
   Gly His His Pro Asn Ile Glu Arg Val Gly Arg Thr Pro Arg Thr Ala
                   85                  90                  95
   Tyr Asn Tyr Thr Ile Lys Asp Asn Asp Val Val Ala Arg Ser Gly Glu
               100                 105                 110
   Tyr Ser Gly Pro Pro Glu Asn Asn Asn Ser Arg Glu Arg Gln Ser Gly
           115                 120                 125
   Asn Asp Thr Ser Ser Asp Trp Ala Thr Ile Leu Cys Ala Glu Ser Arg
       130                 135                 140
   Asp Glu Phe Phe Asp Leu Cys Lys Ser Leu Gln Pro Arg Ser Leu Ala
   145                 150                 155                 160
   Cys Ser Phe Leu Ser Leu Thr Arg Tyr Ala Asp Trp Arg Tyr Arg Pro
                   165                 170                 175
   Val Pro Thr Pro Tyr Gln His Pro
               180
<210> 203
   <211> 151
   <212> PRT
   <213> Artificial Sequence
<220>
   <221> SOURCE
   <222> 1..151
   <223> /mol_type="protein"
   /note="query"
   /organism="Artificial Sequence"
<400> 203
   Pro Leu Cys Cys Arg Leu Gly Arg Glu Arg His Ser Asp Gly Gly Ile
   1               5                   10                  15
   His Tyr His Ile Tyr Leu Gly Phe Asp Lys Ile Val Thr Val Asn Ser
               20                  25                  30
   Ala Thr Ala Phe Asp Tyr Phe Gly Ala His Gly Asn Ile Lys Ser Ile
           35                  40                  45
   Arg Arg Thr Pro His Lys Val Phe Asp Tyr Val Gly Lys Asp Gly Asp
       50                  55                  60
   Ile Arg Leu Glu His Gly Glu Pro Pro Ala Asp Ala Arg Ser Cys Asp
   65                  70                  75                  80
   Gly Asp Asn Ser Asn Lys Trp Ala Asp Ile Ile Ser Cys Asp Thr Lys
                   85                  90                  95
   Glu Ser Phe Leu Ser Ala Val Arg Gln Gln Ala Pro Arg Asp Trp Leu
               100                 105                 110
   Leu Ser Asn Gln Arg Ile Ile Glu Tyr Ala Asn Phe Tyr Tyr Pro Asp
           115                 120                 125
   Glu Lys Pro Glu Tyr Glu Ala Pro Pro Ile Ile Val Glu Arg Glu Arg
       130                 135                 140
   Tyr Pro Glu Ile Ser Glu Trp
   145                 150
<210> 204
   <211> 153
   <212> PRT
   <213> Artificial Sequence
<220>
   <221> SOURCE
   <222> 1..153
   <223> /mol_type="protein"
   /note="subject"
   /organism="Artificial Sequence"
<400> 204
   Pro Ala Glu Cys Ile Ile Gly Arg Glu Ala His Ala Asp Gly Gly Thr
   1               5                   10                  15
   His Leu His Ala Phe Cys Asp Phe Gly Glu Arg Phe Tyr Thr Ala Asp
               20                  25                  30
   Pro Arg Arg Phe Asp Val Asp Gly Tyr His Pro Asn Val Gln Pro Phe
           35                  40                  45
   Gly Arg Thr Pro Tyr Lys Gly Trp Asp Tyr Ala Ile Lys Asp Gly Asp
       50                  55                  60
   Val Val Cys Gly Gly Leu Glu Arg Pro Gly Thr Gly Pro Gly Thr Asp
   65                  70                  75                  80
   Val Ser Gly Ala Gly Ser Gln Trp Asp Arg Ile Val Gly Ala Glu Thr
                   85                  90                  95
   Pro Asp Glu Phe Trp Arg Leu Ile Arg Glu Leu Ala Pro Arg Thr Leu
               100                 105                 110
   Leu Thr Asn Phe Asn Ser Val Arg Ala Tyr Ala Asp Trp His Tyr Arg
           115                 120                 125
   Pro Val Val Ala Pro Tyr Glu Ser Pro Pro Glu Ile Glu Phe Asp Leu
       130                 135                 140
   Ser Gly Val Glu Glu Leu Arg Glu Trp
   145                 150
<210> 205
   <211> 86
   <212> PRT
   <213> Artificial Sequence
<220>
   <221> SOURCE
   <222> 1..86
   <223> /mol_type="protein"
   /note="query"
   /organism="Artificial Sequence"
<400> 205
   Met Asp Glu Phe Ser Asp Asp Cys Gln Tyr Ala Val Phe Asp Asp Ile
   1               5                   10                  15
   Gln Gly Gly Phe Glu Tyr Phe His Ser Tyr Lys Gly Trp Leu Gly Ala
               20                  25                  30
   Gln Lys Gln Phe Val Ile Thr Asp Lys Tyr Arg Lys Lys Arg Thr Ile
           35                  40                  45
   Tyr Trp Gly Lys Pro Ser Ile Met Cys Met Asn Glu Asp Pro His Phe
       50                  55                  60
   Ala Lys Gly Val Asp Tyr Asp Trp Leu Met Asn Asn Cys His Ile Ile
   65                  70                  75                  80
   Asn Val Val Asp Pro Ile
                   85
<210> 206
   <211> 86
   <212> PRT
   <213> Artificial Sequence
<220>
   <221> SOURCE
   <222> 1..86
   <223> /mol_type="protein"
   /note="subject"
<210> 207
   <211> 88
   <212> PRT
   <213> Artificial Sequence
<220>
   <221> SOURCE
   <222> 1..88
   <223> /mol_type="protein"
   /note="query"
   /organism="Artificial Sequence"
<400> 207
   Met Asp Glu Phe Ser Asp Asp Cys Gln Tyr Ala Val Phe Asp Asp Ile
   1               5                   10                  15
   Gln Gly Gly Phe Glu Tyr Phe His Ser Tyr Lys Gly Trp Leu Gly Ala
               20                  25                  30
   Gln Lys Gln Phe Val Ile Thr Asp Lys Tyr Arg Lys Lys Arg Thr Ile
           35                  40                  45
   Tyr Trp Gly Lys Pro Ser Ile Met Cys Met Asn Glu Asp Pro His Phe
       50                  55                  60
   Ala Lys Gly Val Asp Tyr Asp Trp Leu Met Asn Asn Cys His Ile Ile
   65                  70                  75                  80
   Asn Val Val Asp Pro Ile Cys Ile
                   85
<210> 208
   <211> 88
   <212> PRT
   <213> Artificial Sequence
<220>
   <221> SOURCE
   <222> 1..88
   <223> /mol_type="protein"
   /note="subject"
   /organism="Artificial Sequence"
<400> 208
   Leu Glu Glu Glu Leu Glu Gly Val Asp Tyr Ala Ile Phe Asp Asp Met
   1 5 10 15
   Gln Gly Gly Leu Lys Phe Phe His Ser Tyr Lys Phe Trp Leu Gly Ala
   20 25 30
   Gln Ser Gln Phe Trp Ala Thr Asp Lys Tyr Arg Gly Lys Lys Leu Ile
   35 40 45
   His Trp Gly Arg Pro Ser Ile Tyr Ile Cys Asn Gln Asn Pro Leu Cys
   50 55 60
   Asp Glu Gly Val Asp His Asp Trp Leu Ile Gly Asn Cys Asp Phe Val
   65 70 75 80
   Glu Ile Thr Glu Ser Leu Leu Val
   85
<210> 209
   <211> 81
   <212> PRT
   <213> Artificial Sequence
<220>
   <221> SOURCE
   <222> 1..81
   <223> /mol_type="protein"
   /note="query"
   /organism="Artificial Sequence"
<400> 209
   Asp Asp Cys Gln Tyr Ala Val Phe Asp Asp Ile Gln Gly Gly Phe Glu
   1 5 10 15
   Tyr Phe His Ser Tyr Lys Gly Trp Leu Gly Ala Gln Lys Gln Phe Val
   20 25 30
   Ile Thr Asp Lys Tyr Arg Lys Lys Arg Thr Ile Tyr Trp Gly Lys Pro
   35 40 45
   Ser Ile Met Cys Met Asn Glu Asp Pro His Phe Ala Lys Gly Val Asp
   50 55 60
   Tyr Asp Trp Leu Met Asn Asn Cys His Ile Ile Asn Val Val Asp Pro
   65 70 75 80
   Ile
<210> 210
   <211> 81
   <212> PRT
   <213> Artificial Sequence
<220>
   <221> SOURCE
   <222> 1..81
   <223> /mol_type="protein"
   /note="subject"
   /organism="Artificial Sequence"
<400> 210
   Asp Asp Ala Gln Tyr Ala Val Leu Asp Asp Ile Gln Gly Asn Phe Gln
   1               5                   10                  15
   Phe Phe Pro Ala Tyr Lys Gly Trp Leu Gly Ala Gln Glu Ser Phe Thr
               20                  25                  30
   Val Thr Asp Lys Tyr Arg Gly Lys Thr Thr Ile Thr Trp Gly Arg Pro
           35                  40                  45
   Thr Ile Trp Leu Met Asn Glu Asp Pro Glu Glu Val Gly His Val Asp
       50                  55                  60
   Leu Asn Trp Leu Arg Gly Asn Cys Thr Ile Val His Leu Thr Gln Ser
   65                  70                  75                  80
   Leu
<210> 211
   <211> 2259
   <212> DNA
   <213> Artificial Sequence
<220>
   <221> source
   <222> 1..2259
   <223> /mol_type="DNA"
   /note="MSSI2.225"
   /organism="Artificial Sequence"
<400> 211
   ttcctaatca gctgttcgat cagaccaccg aaggtggatg tcgacgcgtc atcggcccac 60
   ttctcggggt cactaatgcc cgagatgagc tgcaatctta cgtcttccgg ggtcaggaag 120
   attctgattg ggcagatcag tttactgccc cccttgatac tcgtcgcatt acggttaagt 180
   ccgacaaact ccgcgtcatc cgccccggta atgaaacagg tgcttcgcgc ttgtttaagt 240
   tttggtaccc cattcgtcgt accatctctt acgaagatga ccttgagtcc gacgttgtcg 300
   gtgatcggcc cttttctacc gcaggtttac gaggtgttgg ggatatgtac gtcatggata 360
   taatgggtat tacgaatttg gtgaatgatg cacctccaac gtcctacagg ttcggtcctg 420
   agggcagctt ttattggcat gagcggtaaa ttaggagact atggggctat ctaggtacac 480
   gaaatcgcag ttggcattaa gccactcggt gtcaacacca agctcgtcac gtgggtcgga 540
   gttagatagc caaattgagg gccgagccca gtgtaccaac tttttcccct tgtacttatc 600
   cgtgacgtag aactgctttt gatgacctaa ccagaatttg tagctgggaa ggaatttaat 660
   tcctccgaag tcgtcaaaga tggcgtactc aaccccatca aggttttcgt ccaagctaaa 720
   gaggcctccg aagtaagcat ggtttcccag gcttctagcc caaatggttt ttcccattct 780
   ggaaggcccg tataccacaa gtgattttct tcgttctgta tatcgaagtc agcatccaga 840
   tttgttgatg cgcactcgag cgaggcgctg cgcgaggccc ccgccgagca tggaggcgcg 900
   cgtagccgcc gactgcagcc gctgcgacgt gcatgataac ttacctccag tttcacctcg 960
   tccaagattt tgttgtaccc attgattgag ttcagccacc caagacgtat cgatgttaac 1020
   tccctccggt gtctcatacg ggactctgat tggtgggaat ttccaggctg cgtatgccct 1080
   gagctgggtg aaggaggtga ccaatgagcg tggagccagg cgtttgcata ggtcccaaaa 1140
   ctcttgctca ctctcagcat tgatgatttc agcccacacc ccactagttg catccactct 1200
   gcctgcaaca ggtcgctcga gtcccccagc aacaacatct ccatctttga ttgcataatc 1260
   aaacccctct tctggtgttc cgcgagaagg cgatacattt gggtggcatc cttcaacatc 1320
   gaatgcacgc gcgttccggg tcctatattt gactccgaag tcgacaaaag cgtgcagatg 1380
   aagaccccca tctgcgtgat tctctcggcc gatgatgcat tcagctccaa gtcccgcaag 1440
   atgattgacc actgcaaaag ggtcgaggtc tccacattgt ggataggtga gcaaggcgta 1500
   ccgtgcttga aatcgaaaag aggacatagt tcgttgttgc acattttcct tgggtccaag 1560
   tctgggcagt taatgttata tgcccagacg gacccagacc cgggtccttc gaactataaa 1620
   tacccctcgc tcgtccccaa cctcctcgtt tttttgtatg cgcgatttat catgtctgcc 1680
   ttgatccacc ccgttgcctg caatcggact tgccaagact acgtcaacca cgctagacac 1740
   gaatgttgtt tagtgtgtaa ccctccgcca cccccttcat ctcctactgt cgcacattgt 1800
   tcagaatgtc ggtcgcccgt cgatctggtc gaagaactcg caaagctttc cgccgcggtg 1860
   gtagagctac aagatctcgt ggccgaagta cacgccgctc ttctaggcga agagtcagac 1920
   ggcgaactat gactacccga cgtgtcctca acataaccac tcgtaagaag gtggataaca 1980
   tgttgcctgt tgttgttgcc gaggataaca cgactactgt ggggccttac acgtccgtgt 2040
   cacccctcct ttcccttttc gttcccaatg cccgcacgac ccgaacccct gttaccaatc 2100
   ctgccgtccg aaattcctcg gatattttcg cggtcggata tagggagcgc gtccgcattg 2160
   atcttatggg tggtggaact tttatgtggc gccgcatcgt cttcatgttg aagggatccg 2220
   atttgagagt tgctatgaat tccagtgatt caggcaaca 2259
<210> 212
   <211> 335
   <212> DNA
   <213> Artificial Sequence
<220>
   <221> source
   <222> 1..335
   <223> /mol_type="DNA"
   /note="query"
   /organism="Artificial Sequence"
<400> 212
   ccactctgcc tgcaacaggt cgctcgagtc ccccagcaac aacatctcca tctttgattg 60
   cataatcaaa cccctcttct ggtgttccgc gagaaggcga tacatttggg tggcatcctt 120
   caacatcgaa tgcacgcgcg ttccgggtcc tatatttgac tccgaagtcg acaaaagcgt 180
   gcagatgaag acccccatct gcgtgattct ctcggccgat gatgcattca gctccaagtc 240
   ccgcaagatg attgaccact gcaaaagggt cgaggtctcc acattgtgga taggtgagca 300
   aggcgtaccg tgcttgaaat cgaaaagagg acata 335
<210> 213
   <211> 338
   <212> DNA
   <213> Artificial Sequence
<220>
   <221> source
   <222> 1..338
   <223> /mol_type="DNA"
   /note="subject"
   /organism="Artificial Sequence"
<400> 213
   ccactccgcc atcatcagcg ggtctgtcca gtcctccagc aacaacatcc ccatctttga 60
   ttgcatagtc gtatccgcca gctgggcgtc cccgagatgg ttcaacattc gggtggtgtc 120
   cttgcacatc gaagacgcgt acgttccgcg tactaaattt gactccgaag tcgacgaaag 180
   catggagatg aattccttca tcagcgtgag tctctcgtcc aatgatgcac tctgctctaa 240
   ggcctgatag gtggtcagac acagcaaacg ggtcgaggtc tccacactgg gcgtaagtga 300
   gtaaggcata tctggcctgg aatctgaatg atgacata 338
<210> 214
   <211> 307
   <212> DNA
   <213> Artificial Sequence
<220>
   <221> source
   <222> 1..307
   <223> /mol_type="DNA"
   /note="query"
   /organism="Artificial Sequence"
<400> 214
   acaccaagct cgtcacgtgg gtcggagtta gatagccaaa ttgagggccg agcccagtgt 60
   accaactttt tccccttgta cttatccgtg acgtagaact gcttttgatg acctaaccag 120
   aatttgtagc tgggaaggaa tttaattcct ccgaagtcgt caaagatggc gtactcaacc 180
   ccatcaaggt tttcgtccaa gctaaagagg cctccgaagt aagcatggtt tcccaggctt 240
   ctagcccaaa tggtttttcc cattctggaa ggcccgtata ccacaagtga ttttcttcgt 300
   tctgtat 307
<210> 215
   <211> 307
   <212> DNA
   <213> Artificial Sequence
<220>
   <221> source
   <222> 1..307
   <223> /mol_type="DNA"
   /note="subject"
   /organism="Artificial Sequence"
<400> 215
   accccatgct cctcgcgtgg gtcctcattg ttgatccata ttgatggtct gccccattca 60
   accaactttt tccccttgta cttgtctgtg acgtaaaact gcttctggtg tcccatccaa 120
   aatttgtaag atgggaggaa ttttaaacct ccaaaatcat caaacaccgc atatgtggcc 180
   gagcttatgt cctcgtccat tgagaaaagc cctccgaaat aggcatggtc tcctagtgac 240
   ctggcccaca cagtcttacc cgttcgggtt ggaccatata gcactagtga tttccctctt 300
   actatat 307
<210> 216
   <211> 277
   <212> DNA
   <213> Artificial Sequence
<220>
   <221> source
   <222> 1..277
   <223> /mol_type="DNA"
   /note="query"
   /organism="Artificial Sequence"
<400> 216
   tcgagtcccc cagcaacaac atctccatct ttgattgcat aatcaaaccc ctcttctggt 60
   gttccgcgag aaggcgatac atttgggtgg catccttcaa catcgaatgc acgcgcgttc 120
   cgggtcctat atttgactcc gaagtcgaca aaagcgtgca gatgaagacc cccatctgcg 180
   tgattctctc ggccgatgat gcattcagct ccaagtcccg caagatgatt gaccactgca 240
   aaagggtcga ggtctccaca ttgtggatag gtgagca 277
<210> 217
   <211> 277
   <212> DNA
   <213> Artificial Sequence
<220>
   <221> source
   <222> 1..277
   <223> /mol_type="DNA"
   /note="subject"
   /organism="Artificial Sequence"
<400> 217
   tcgagtcctc cagcaacaac atctccgtcc ttgatcgcat aagttgcacc cttttccggt 60
   gttgagtaac cgcgtacgac atttgggtgg cgtccgtcca catcgaatac gcggacattt 120
   cttgactcaa acttccgttc gaacatgaag aaagcgtgca ggtgaagtcc tccatcagcg 180
   tgctcttctc ggccaatgat gcactcagct cccaggcgtc caagatggtc gttaacttcc 240
   caggggtcca ggtctccgga ctgggggaac gtgagca 277
<210> 218
   <211> 276
   <212> DNA
   <213> Artificial Sequence
<220>
   <221> source
   <222> 1..276
   <223> /mol_type="DNA"
   /note="query"
   /organism="Artificial Sequence"
<400> 218
   agtcccccag caacaacatc tccatctttg attgcataat caaacccctc ttctggtgtt 60
   ccgcgagaag gcgatacatt tgggtggcat ccttcaacat cgaatgcacg cgcgttccgg 120
   gtcctatatt tgactccgaa gtcgacaaaa gcgtgcagat gaagaccccc atctgcgtga 180
   ttctctcggc cgatgatgca ttcagctcca agtcccgcaa gatgattgac cactgcaaaa 240
   gggtcgaggt ctccacattg tggataggtg agcaag 276
<210> 219
   <211> 276
   <212> DNA
   <213> Artificial Sequence
<220>
   <221> source
   <222> 1..276
   <223> /mol_type="DNA"
   /note="subject"
   /organism="Artificial Sequence"
<400> 219
   aggcccccac aaatgacatc tccatctttg atcgcgtagt catacccctt ctccggagtc 60
   cctcgagact ttgtaatgtt tgggtggtga ccatcgacat cgaatacatc agcctttcta 120
   ctgcgaaact tccgtccgaa ctctgcaaag cagtgtaggt gaatgcctcc atcctcgtga 180
   tgctctcttc caatgatgca ctcagctccc agaagtgaaa gtttatccat aactcggaaa 240
   gcatcgagct ctccacattg tgcgtatgtg agcaag 276
<210> 220
   <211> 256
   <212> PRT
   <213> Artificial Sequence
<220>
   <221> SOURCE
   <222> 1..256
   <223> /mol_type="protein"
   /note="query"
   /organism="Artificial Sequence"
<400> 220
   Arg Arg Val Leu Asn Ile Thr Thr Arg Lys Lys Val Asp Asn Met Leu
   1               5                   10                  15
   Pro Val Val Val Ala Glu Asp Asn Thr Thr Thr Val Gly Pro Tyr Thr
               20                  25                  30
   Ser Val Ser Pro Leu Leu Ser Leu Phe Val Pro Asn Ala Arg Thr Thr
           35                  40                  45
   Arg Thr Pro Val Thr Asn Pro Ala Val Arg Asn Ser Ser Asp Ile Phe
       50                  55                  60
   Ala Val Gly Tyr Arg Glu Arg Val Arg Ile Asp Leu Met Gly Gly Gly
   65                  70                  75                  80
   Thr Phe Met Trp Arg Arg Ile Val Phe Met Leu Lys Gly Ser Asp Leu
                   85                  90                  95
   Arg Val Ala Met Asn Ser Ser Asp Ser Gly Asn Ile Pro Asn Gln Leu
               100                 105                 110
   Phe Asp Gln Thr Thr Glu Gly Gly Cys Arg Arg Val Ile Gly Pro Leu
           115                 120                 125
   Leu Gly Val Thr Asn Ala Arg Asp Glu Leu Gln Ser Tyr Val Phe Arg
       130                 135                 140
   Gly Gln Glu Asp Ser Asp Trp Ala Asp Gln Phe Thr Ala Pro Leu Asp
   145                 150                 155                 160
   Thr Arg Arg Ile Thr Val Lys Ser Asp Lys Leu Arg Val Ile Arg Pro
                   165                 170                 175
   Gly Asn Glu Thr Gly Ala Ser Arg Leu Phe Lys Phe Trp Tyr Pro Ile
               180                 185                 190
   Arg Arg Thr Ile Ser Tyr Glu Asp Asp Leu Glu Ser Asp Val Val Gly
           195                 200                 205
   Asp Arg Pro Phe Ser Thr Ala Gly Leu Arg Gly Val Gly Asp Met Tyr
       210                 215                 220
   Val Met Asp Ile Met Gly Ile Thr Asn Leu Val Asn Asp Ala Pro Pro
   225                 230                 235                 240
   Thr Ser Tyr Arg Phe Gly Pro Glu Gly Ser Phe Tyr Trp His Glu Arg
                   245                 250                 255
<210> 221
   <211> 265
   <212> PRT
   <213> Artificial Sequence
<220>
   <221> SOURCE
   <222> 1..265
   <223> /mol_type="protein"
   /note="subject"
   /organism="Artificial Sequence"
<220>
   <221> VARIANT
   <222> 234
   <223> /
   unknown or other
   unknown or other
   unknown or other
<220>
   <221> VARIANT
   <222> 93
   <223> unknown or other
<220>
   <221> VARIANT
   <222> 122
   <223> unknown or other
<400> 221
   Arg Arg Ile Leu Asn Val Ala Ser Ile Lys Lys Arg Asp Asn Met Leu
   1               5                   10                  15
   Ser Met Val Gln Thr Ser Asp Gln Ala Pro Pro Thr Ile Gly Pro Ile
               20                  25                  30
   Thr Val Gly Thr Gly Phe Ala Ser Leu Tyr Met Pro Ser Ala Arg Pro
           35                  40                  45
   Leu Gly Pro Ala Asn Asp Val Gly Glu Ser Thr Arg Gln Arg Arg Asp
       50                  55                  60
   Thr Phe Ser Val Gly Tyr Lys Glu Arg Val Gln Val Asp Ile His Gly
   65                  70                  75                  80
   Gly Gly Val Trp Lys Trp Arg Arg Val Val Phe Thr Xaa Lys Gly Asp
                   85                  90                  95
   Gln Leu Tyr Asn Lys Gly Glu Phe Ile Gly Asp Trp Thr Gln Pro Phe
               100                 105                 110
   Glu Ser Lys Ala Val Lys Pro Ser Pro Xaa Asp Pro Asn Pro Leu Gly
           115                 120                 125
   Val Asp Met Val Arg Leu Ile Gly Gln Pro Thr Thr Glu Gln His Asn
       130                 135                 140
   Ala Ile Arg Asp Leu Ile Trp Asp Gly His Glu Gly Ile Asp Trp Thr
   145                 150                 155                 160
   Ser Glu Phe Thr Ala Lys Val Ser Thr Thr Arg Ile Thr Val Leu Ser
                   165                 170                 175
   Asp Lys Thr Tyr His Phe Asn Pro Gly Asn Glu Ser Gly Met Ser Arg
               180                 185                 190
   Thr Phe Arg Leu Trp Tyr Pro Thr Arg Lys Arg Leu Ile Tyr Asp Asp
           195                 200                 205
   Asp Glu Arg Gly Gly Ala Val Phe Ser Arg Gly Ser Pro Val Ser Val
       210                 215                 220
   Thr Ser Arg Gln Gly Met Gly Asp Leu Xaa Ile Tyr Asp Ile Ala Tyr
   225                 230                 235                 240
   Ala Val Val Pro Ser Ala Gly Asn Pro Gly Ser Met Thr Trp Ala Pro
                   245                 250                 255
   Glu Gly Thr Tyr Tyr Trp His Glu Arg
               260                 265
<210> 222
   <211> 300
   <212> PRT
   <213> Artificial Sequence
<220>
   <221> SOURCE
   <222> 1..300
   <223> /mol_type="protein"
   /note="query"
   /organism="Artificial Sequence"
<400> 222
   Met Ser Val Ala Arg Arg Ser Gly Arg Arg Thr Arg Lys Ala Phe Arg
   1               5                   10                  15
   Arg Gly Gly Arg Ala Thr Arg Ser Arg Gly Arg Ser Thr Arg Arg Ser
               20                  25                  30
   Ser Arg Arg Arg Val Arg Arg Arg Thr Met Thr Thr Arg Arg Val Leu
           35                  40                  45
   Asn Ile Thr Thr Arg Lys Lys Val Asp Asn Met Leu Pro Val Val Val
       50                  55                  60
   Ala Glu Asp Asn Thr Thr Thr Val Gly Pro Tyr Thr Ser Val Ser Pro
   65                  70                  75                  80
   Leu Leu Ser Leu Phe Val Pro Asn Ala Arg Thr Thr Arg Thr Pro Val
                   85                  90                  95
   Thr Asn Pro Ala Val Arg Asn Ser Ser Asp Ile Phe Ala Val Gly Tyr
               100                 105                 110
   Arg Glu Arg Val Arg Ile Asp Leu Met Gly Gly Gly Thr Phe Met Trp
           115                 120                 125
   Arg Arg Ile Val Phe Met Leu Lys Gly Ser Asp Leu Arg Val Ala Met
       130                 135                 140
   Asn Ser Ser Asp Ser Gly Asn Ile Pro Asn Gln Leu Phe Asp Gln Thr
   145                 150                 155                 160
   Thr Glu Gly Gly Cys Arg Arg Val Ile Gly Pro Leu Leu Gly Val Thr
                   165                 170                 175
   Asn Ala Arg Asp Glu Leu Gln Ser Tyr Val Phe Arg Gly Gln Glu Asp
               180                 185                 190
   Ser Asp Trp Ala Asp Gln Phe Thr Ala Pro Leu Asp Thr Arg Arg Ile
           195                 200                 205
   Thr Val Lys Ser Asp Lys Leu Arg Val Ile Arg Pro Gly Asn Glu Thr
       210                 215                 220
   Gly Ala Ser Arg Leu Phe Lys Phe Trp Tyr Pro Ile Arg Arg Thr Ile
   225                 230                 235                 240
   Ser Tyr Glu Asp Asp Leu Glu Ser Asp Val Val Gly Asp Arg Pro Phe
                   245                 250                 255
   Ser Thr Ala Gly Leu Arg Gly Val Gly Asp Met Tyr Val Met Asp Ile
               260                 265                 270
   Met Gly Ile Thr Asn Leu Val Asn Asp Ala Pro Pro Thr Ser Tyr Arg
           275                 280                 285
   Phe Gly Pro Glu Gly Ser Phe Tyr Trp His Glu Arg
       290                 295                 300
<210> 223
   <211> 302
   <212> PRT
   <213> Artificial Sequence
<220>
   <221> SOURCE
   <222> 1..302
   <223> /mol_type="protein"
   /note="subject"
   /organism="Artificial Sequence"
<400> 223
   Met Ala Tyr Ala Arg Arg Arg Phe Thr Ser Ser Arg Ala Arg Lys Pro
   1               5                   10                  15
   Ile Arg Arg Ser Arg Arg Pro Ala Gly Lys Arg Arg Ser Tyr Arg Arg
               20                  25                  30
   Ser Thr Arg Lys Arg Gly Met Ser Thr Arg Thr Leu Leu Asn Lys Thr
           35                  40                  45
   Ser Gln Lys Lys Arg Asp Asn Met Leu Ser Phe Ser Asn Thr Thr Ala
       50                  55                  60
   Glu Asp Pro Phe Ser Thr Asp Tyr Thr Ile Gly Pro Ala Val Met Arg
   65                  70                  75                  80
   Arg Pro Val Gly Ala Asp Leu Pro Ala Glu Phe Ile Tyr Val Trp Asn
                   85                  90                  95
   Ala Thr Gly Arg Pro His Glu Leu Ser Thr Gly Phe Arg Gly Ser Lys
               100                 105                 110
   Ile Asp Thr Ser Leu Arg Thr Asn Thr Thr Ile Phe Ala Val Gly Leu
           115                 120                 125
   Lys Glu Arg Ile Lys Leu Glu Thr Asn Asn Ser Ala Pro Trp Arg Trp
       130                 135                 140
   Arg Arg Ile Cys Phe Thr Ser Lys Asn Asp Tyr Gly Glu Ala Asp Pro
   145                 150                 155                 160
   Asp Glu Ser Asp Tyr Phe Arg Arg Thr Ser Asn Gly Met Val Arg Leu
                   165                 170                 175
   Leu Arg Ala Gln Ser Gln Ala Glu Glu Ile Asn Asp Asp Val Phe Asp
               180                 185                 190
   Gly Gln Arg Asn Val Asp Trp Leu Ser Ala Ile Thr Ala Pro Ile Asn
           195                 200                 205
   Arg Arg His Val Ser Val His Tyr Asp Arg Thr Arg Thr Ile Gln Ser
       210                 215                 220
   Gly Asn Gln Ser Gly Val Thr Arg Thr Tyr Arg Gln Trp His Pro Met
   225                 230                 235                 240
   Arg Lys Asn Val Val Tyr Ala Asp Glu Gln Ala Gly Glu Gly Met Val
                   245                 250                 255
   Asp Ser Gly Val Ser Val Thr Gly Lys Val Gly Met Gly Asn Tyr Tyr
               260                 265                 270
   Val Met Asp Ile Phe Thr Lys His Gly Leu Asn Asp Asp Gln Ser Thr
           275                 280                 285
   Leu Ser Tyr Glu Pro Glu Ser Thr Phe Phe Trp His Glu Lys
       290                 295                 300
<210> 224
   <211> 261
   <212> **PRT**
   <213> Artificial Sequence
<220>
   <221> SOURCE
   <222> 1..261
   <223> /mol_type="protein"
   /note="query"
   /organism="Artificial Sequence"
<400> 224
   Arg Thr Met Thr Thr Arg Arg Val Leu Asn Ile Thr Thr Arg Lys Lys
   1               5                   10                  15
   Val Asp Asn Met Leu Pro Val Val Val Ala Glu Asp Asn Thr Thr Thr
               20                  25                  30
   Val Gly Pro Tyr Thr Ser Val Ser Pro Leu Leu Ser Leu Phe Val Pro
           35                  40                  45
   Asn Ala Arg Thr Thr Arg Thr Pro Val Thr Asn Pro Ala Val Arg Asn
       50                  55                  60
   Ser Ser Asp Ile Phe Ala Val Gly Tyr Arg Glu Arg Val Arg Ile Asp
   65                  70                  75                  80
   Leu Met Gly Gly Gly Thr Phe Met Trp Arg Arg Ile Val Phe Met Leu
                   85                  90                  95
   Lys Gly Ser Asp Leu Arg Val Ala Met Asn Ser Ser Asp Ser Gly Asn
               100                 105                 110
   Ile Pro Asn Gln Leu Phe Asp Gln Thr Thr Glu Gly Gly Cys Arg Arg
           115                 120                 125
   Val Ile Gly Pro Leu Leu Gly Val Thr Asn Ala Arg Asp Glu Leu Gln
       130                 135                 140
   Ser Tyr Val Phe Arg Gly Gln Glu Asp Ser Asp Trp Ala Asp Gln Phe
   145                 150                 155                 160
   Thr Ala Pro Leu Asp Thr Arg Arg Ile Thr Val Lys Ser Asp Lys Leu
                   165                 170                 175
   Arg Val Ile Arg Pro Gly Asn Glu Thr Gly Ala Ser Arg Leu Phe Lys
               180                 185                 190
   Phe Trp Tyr Pro Ile Arg Arg Thr Ile Ser Tyr Glu Asp Asp Leu Glu
           195                 200                 205
   Ser Asp Val Val Gly Asp Arg Pro Phe Ser Thr Ala Gly Leu Arg Gly
       210                 215                 220
   Val Gly Asp Met Tyr Val Met Asp Ile Met Gly Ile Thr Asn Leu Val
   225                 230                 235                 240
   Asn Asp Ala Pro Pro Thr Ser Tyr Arg Phe Gly Pro Glu Gly Ser Phe
                   245                 250                 255
   Tyr Trp His Glu Arg
               260
<210> 225
   <211> 278
   <212> PRT
   <213> Artificial Sequence
<220>
   <221> SOURCE
   <222> 1..278
   <223> /mol_type="protein"
   /note="subject"
   /organism="Artificial Sequence"
<400> 225
   Arg Lys Met Ser Lys Arg Arg Ile Leu Asn Ile Thr Ser Thr Lys Lys
   1               5                   10                  15
   Lys Asp Thr Met Thr Gly Tyr Thr Asn Ser Thr Pro Ala Asn Gln Thr
               20                  25                  30
   Gly Gly Thr Val Tyr Ser Thr Asp Pro Ala Ile Val Thr Gly Gly Leu
           35                  40                  45
   Asn Ala Asn Gln Cys Asn Ala Phe Leu Trp Cys Ala Thr Ala Arg Thr
       50                  55                  60
   Asn Gln Val Ile Asn Pro Thr Gln Asn Thr Leu Val Pro Gly Asn Lys
   65                  70                  75                  80
   Phe Tyr Thr Ala Cys Arg Thr Asp Ser Asn Pro Tyr Met Val Gly Leu
                   85                  90                  95
   Ser Glu Lys Ile Glu Ile Gln Cys Asn Ser Gly Met Pro Trp Gln Trp
               100                 105                 110
   Arg Arg Ile Cys Trp Thr Gln Lys Gly Gly Gln Gly Leu Pro Ala Asn
           115                 120                 125
   Ser Thr Ser Phe Asn Leu Ser Gln Gln Asn Ala Ile Thr Gly Trp Asn
       130                 135                 140
   Arg Val Met Asn Gln Val Ala Gly Asn Pro Gly Thr Gly Asp Gln Tyr
   145                 150                 155                 160
   Asn Leu Phe His His Ile Phe Arg Gly Gln Val Gly Ser Asp Trp Val
                   165                 170                 175
   Asp Val Met Thr Ala Pro Leu Asp Pro Gln His Ile Thr Val Lys Tyr
               180                 185                 190
   Asp Lys Val Val Thr Leu Ala Ser Gly Asn Glu Glu Gly Phe Ile Arg
           195                 200                 205
   Ala Tyr Arg Arg Trp His Pro Met Lys Lys Thr Leu Ser Tyr Ala Asp
       210                 215                 220
   Glu Glu Tyr Gly Asn Val Glu Asn Ala Asp Ala Phe Ser Gln Val Gly
   225                 230                 235                 240
   Lys Lys Gly Met Gly Asp Tyr Tyr Val Leu Asp Leu Phe Arg Ala Arg
                   245                 250                 255
   Gln Gly Ala Gln Thr Asn Asp Ser Ile Ser Ile Arg Pro Cys Ala Thr
               260                 265                 270
   Leu Tyr Trp His Glu Lys
           275
<210> 226
   <211> 195
   <212> PRT
   <213> Artificial Sequence
<220>
   <221> SOURCE
   <222> 1..195
   <223> /mol_type="protein"
   /note="query"
   /organism="Artificial Sequence"
<400> 226
   Met Ser Ser Phe Arg Phe Gln Ala Arg Tyr Ala Leu Leu Thr Tyr Pro
   1               5                   10                  15
   Gln Cys Gly Asp Leu Asp Pro Phe Ala Val Val Asn His Leu Ala Gly
               20                  25                  30
   Leu Gly Ala Glu Cys Ile Ile Gly Arg Glu Asn His Ala Asp Gly Gly
           35                  40                  45
   Leu His Leu His Ala Phe Val Asp Phe Gly Val Lys Tyr Arg Thr Arg
       50                  55                  60
   Asn Ala Arg Ala Phe Asp Val Glu Gly Cys His Pro Asn Val Ser Pro
   65                  70                  75                  80
   Ser Arg Gly Thr Pro Glu Glu Gly Phe Asp Tyr Ala Ile Lys Asp Gly
                   85                  90                  95
   Asp Val Val Ala Gly Gly Leu Glu Arg Pro Val Ala Gly Arg Val Asp
               100                 105                 110
   Ala Thr Ser Gly Val Trp Ala Glu Ile Ile Asn Ala Glu Ser Glu Gln
           115                 120                 125
   Glu Phe Trp Asp Leu Cys Lys Arg Leu Ala Pro Arg Ser Leu Val Thr
       130                 135                 140
   Ser Phe Thr Gln Leu Arg Ala Tyr Ala Ala Trp Lys Phe Pro Pro Ile
   145                 150                 155                 160
   Arg Val Pro Tyr Glu Thr Pro Glu Gly Val Asn Ile Asp Thr Ser Trp
                   165                 170                 175
   Val Ala Glu Leu Asn Gln Trp Val Gln Gln Asn Leu Gly Arg Gly Glu
               180                 185                 190
   Thr Gly Gly
           195
<210> 227
   <211> 198
   <212> PRT
   <213> Artificial Sequence
<220>
   <221> SOURCE
   <222> 1..198
   <223> /mol_type="protein"
   /note="subject"
   /organism="Artificial Sequence"
<400> 227
   Met Ser Ser Phe Arg Phe Gln Ala Arg Tyr Ala Leu Leu Thr Tyr Ala
   1               5                   10                  15
   Gln Cys Gly Asp Leu Asp Pro Phe Ala Val Ser Asp His Leu Ser Gly
               20                  25                  30
   Leu Arg Ala Glu Cys Ile Ile Gly Arg Glu Thr His Ala Asp Glu Gly
           35                  40                  45
   Ile His Leu His Ala Phe Val Asp Phe Gly Val Lys Phe Ser Thr Arg
       50                  55                  60
   Asn Val Arg Val Phe Asp Val Gln Gly His His Pro Asn Val Glu Pro
   65                  70                  75                  80
   Ser Arg Gly Arg Pro Ala Gly Gly Tyr Asp Tyr Ala Ile Lys Asp Gly
                   85                  90                  95
   Asp Val Val Ala Gly Gly Leu Asp Arg Pro Ala Asp Asp Gly Gly Val
               100                 105                 110
   Gly Val Ser Lys Thr Thr Gln Lys Trp Ala Thr Ile Val Gly Ala Gln
           115                 120                 125
   Thr Arg Asp Glu Phe Trp Ala Leu Leu Ala Glu Leu Asp Pro Gly Ala
       130                 135                 140
   Met Val Arg Ser Phe Thr Gln Cys Arg Ala Tyr Ala Asp His Arg Phe
   145                 150                 155                 160
   Arg Glu Ser Pro Glu Arg Tyr Val Thr Pro Glu Ser Ile Ile Ile Asp
                   165                 170                 175
   Thr Gly His Val Asp Gly Leu Asn Glu Trp Ala Leu Ser Asn Ile Val
               180                 185                 190
   Gly His Arg Arg Gly Gly
           195
<210> 228
   <211> 220
   <212> PRT
   <213> Artificial Sequence
<220>
   <221> SOURCE
   <222> 1..220
   <223> /mol_type="protein"
   /note="query"
   /organism="Artificial Sequence"
<400> 228
   Ser Phe Arg Phe Gln Ala Arg Tyr Ala Leu Leu Thr Tyr Pro Gln Cys
   1               5                   10                  15
   Gly Asp Leu Asp Pro Phe Ala Val Val Asn His Leu Ala Gly Leu Gly
               20                  25                  30
   Ala Glu Cys Ile Ile Gly Arg Glu Asn His Ala Asp Gly Gly Leu His
           35                  40                  45
   Leu His Ala Phe Val Asp Phe Gly Val Lys Tyr Arg Thr Arg Asn Ala
       50                  55                  60
   Arg Ala Phe Asp Val Glu Gly Cys His Pro Asn Val Ser Pro Ser Arg
   65                  70                  75                  80
   Gly Thr Pro Glu Glu Gly Phe Asp Tyr Ala Ile Lys Asp Gly Asp Val
                   85                  90                  95
   Val Ala Gly Gly Leu Glu Arg Pro Val Ala Gly Arg Val Asp Ala Thr
               100                 105                 110
   Ser Gly Val Trp Ala Glu Ile Ile Asn Ala Glu Ser Glu Gln Glu Phe
           115                 120                 125
   Trp Asp Leu Cys Lys Arg Leu Ala Pro Arg Ser Leu Val Thr Ser Phe
       130                 135                 140
   Thr Gln Leu Arg Ala Tyr Ala Ala Trp Lys Phe Pro Pro Ile Arg Val
   145                 150                 155                 160
   Pro Tyr Glu Thr Pro Glu Gly Val Asn Ile Asp Thr Ser Trp Val Ala
                   165                 170                 175
   Glu Leu Asn Gln Trp Val Gln Gln Asn Leu Gly Arg Gly Glu Thr Gly
               180                 185                 190
   Gly Lys Leu Ser Cys Thr Ser Gln Arg Leu Gln Ser Ala Ala Thr Arg
           195                 200                 205
   Ala Ser Met Leu Gly Gly Gly Leu Ala Gln Arg Leu
       210                 215                 220
<210> 229
   <211> 216
   <212> PRT
   <213> Artificial Sequence
<220>
   <221> SOURCE
   <222> 1..216
   <223> /mol_type="protein"
   /note="subject"
   /organism="Artificial Sequence"
<400> 229
   Thr Phe Arg Leu Gln Ser Arg Tyr Val Leu Leu Thr Tyr Ala Gln Cys
   1               5                   10                  15
   Gly Asp Leu Asp Pro Trp Leu Val His Asn His Val Val Ala Phe Pro
               20                  25                  30
   Ala Glu Cys Ile Ile Gly Arg Glu Asn His Ala Asp Gly Gly Ile His
           35                  40                  45
   Leu His Ala Phe Val Asp Phe Gly Lys Arg Val Asp Ile Arg Asp Asn
       50                  55                  60
   Arg Arg Phe Asp Val Glu Gly Phe His Pro Asn Ile Ala Pro Cys Gly
   65                  70                  75                  80
   Arg Thr Pro Gln Lys Met Leu Asp Tyr Ala Ile Lys Asp Gly Asp Val
                   85                  90                  95
   Val Ala Gly Gly Leu Asn Pro Ile Leu Asp Leu Pro Asp Ala Glu Thr
               100                 105                 110
   Asp Ser Ile Trp His Arg Ile Ala Asn Ala Pro Ser Val Asp Glu Phe
           115                 120                 125
   Trp Ala Ile Thr Arg Glu Leu Ala Pro Arg Ala Leu Leu Cys Asn His
       130                 135                 140
   Gln Ser Leu Arg Ala Tyr Ala Glu Trp His Tyr Arg Pro Val Ala Thr
   145                 150                 155                 160
   Arg Tyr Glu His Pro His Gly Leu Ser Leu Arg Thr Asp His Val Pro
                   165                 170                 175
   Gln Leu Asp Glu Trp Val Arg Asp Asn Leu Ser Gly Ser Ser Lys Ser
               180                 185                 190
   Tyr Thr Tyr Thr Arg Ser Ala Ser Gly Arg Ala Ser Leu Pro Ser Gly
           195                 200                 205
   Gly Pro Ser Leu Val Gln Ser Leu
       210                 215
<210> 230
   <211> 212
   <212> PRT
   <213> Artificial Sequence
<220>
   <221> SOURCE
   <222> 1..212
   <223> /mol_type="protein"
   /note="query"
   /organism="Artificial Sequence"
<400> 230
   Phe Arg Phe Gln Ala Arg Tyr Ala Leu Leu Thr Tyr Pro Gln Cys Gly
   1               5                   10                  15
   Asp Leu Asp Pro Phe Ala Val Val Asn His Leu Ala Gly Leu Gly Ala
               20                  25                  30
   Glu Cys Ile Ile Gly Arg Glu Asn His Ala Asp Gly Gly Leu His Leu
           35                  40                  45
   His Ala Phe Val Asp Phe Gly Val Lys Tyr Arg Thr Arg Asn Ala Arg
       50                  55                  60
   Ala Phe Asp Val Glu Gly Cys His Pro Asn Val Ser Pro Ser Arg Gly
   65                  70                  75                  80
   Thr Pro Glu Glu Gly Phe Asp Tyr Ala Ile Lys Asp Gly Asp Val Val
                   85                  90                  95
   Ala Gly Gly Leu Glu Arg Pro Val Ala Gly Arg Val Asp Ala Thr Ser
               100                 105                 110
   Gly Val Trp Ala Glu Ile Ile Asn Ala Glu Ser Glu Gln Glu Phe Trp
           115                 120                 125
   Asp Leu Cys Lys Arg Leu Ala Pro Arg Ser Leu Val Thr Ser Phe Thr
       130                 135                 140
   Gln Leu Arg Ala Tyr Ala Ala Trp Lys Phe Pro Pro Ile Arg Val Pro
   145                 150                 155                 160
   Tyr Glu Thr Pro Glu Gly Val Asn Ile Asp Thr Ser Trp Val Ala Glu
                   165                 170                 175
   Leu Asn Gln Trp Val Gln Gln Asn Leu Gly Arg Gly Glu Thr Gly Gly
               180                 185                 190
   Lys Leu Ser Cys Thr Ser Gln Arg Leu Gln Ser Ala Ala Thr Arg Ala
           195                 200                 205
   Ser Met Leu Gly
       210
<210> 231
   <211> 212
   <212> PRT
   <213> Artificial Sequence
<220>
   <221> SOURCE
   <222> 1..212
   <223> /mol_type="protein"
   /note="subject"
   /organism="Artificial Sequence"
<400> 231
   Phe Arg Phe Ala Ala Lys Tyr Gly Leu Leu Thr Tyr Pro Gln Cys Gly
   1               5                   10                  15
   Asp Leu Asp Pro Trp Ala Val Asn Asp Met Leu Gly Arg Leu Gly Ala
               20                  25                  30
   Glu Cys Val Ile Gly Arg Glu Asn His Gln Ala Gly Gly Val His Leu
           35                  40                  45
   His Ala Phe Phe Met Phe Glu Arg Lys Phe Glu Ser Arg Asn Val Arg
       50                  55                  60
   Val Phe Asp Val Asp Gly His His Pro Asn Ile Val Arg Gly Tyr Ser
   65                  70                  75                  80
   Thr Pro Glu Asp Gly Trp Ala Tyr Ala Thr Lys Asp Gly Asp Val Val
                   85                  90                  95
   Ala Gly Ala Leu Glu Cys Pro Arg Pro Arg Thr Glu Val Ser Glu Ser
               100                 105                 110
   Ser Ser Lys Trp Ala Arg Ala Ile Leu Ala Glu Thr Arg Glu Glu Phe
           115                 120                 125
   Phe Ala Ile Val Ala Glu Leu Asp Pro Arg Ala Leu Cys Val Ser Phe
       130                 135                 140
   Gly Ser Leu Arg Ala Tyr Ala Asp Trp Lys Tyr Arg Pro Ala Arg Asp
   145                 150                 155                 160
   Pro Tyr Ile Thr Pro Glu Gly Ile Ser Phe Asp Thr Ser Glu Leu Pro
                   165                 170                 175
   Glu Leu Asp Leu Trp Val Gln Gln Thr Leu Ser Gly Leu Ser Thr Gly
               180                 185                 190
   Lys Gln Pro Pro Pro Ser Ala Pro Leu Arg Gly Val Pro Glu Gln Ile
           195                 200                 205
   Ser Ala Arg Gly
       210
<210> 232
   <211> 107
   <212> PRT
   <213> Artificial Sequence
<220>
   <221> SOURCE
   <222> 1..107
   <223> /mol_type="protein"
   /note="query"
   /organism="Artificial Sequence"
<400> 232
   Met Gly Lys Thr Ile Trp Ala Arg Ser Leu Gly Asn His Ala Tyr Phe
   1               5                   10                  15
   Gly Gly Leu Phe Ser Leu Asp Glu Asn Leu Asp Gly Val Glu Tyr Ala
               20                  25                  30
   Ile Phe Asp Asp Phe Gly Gly Ile Lys Phe Leu Pro Ser Tyr Lys Phe
           35                  40                  45
   Trp Leu Gly His Gln Lys Gln Phe Tyr Val Thr Asp Lys Tyr Lys Gly
       50                  55                  60
   Lys Lys Leu Val His Trp Ala Arg Pro Ser Ile Trp Leu Ser Asn Ser
   65                  70                  75                  80
   Asp Pro Arg Asp Glu Leu Gly Val Asp Thr Glu Trp Leu Asn Ala Asn
                   85                  90                  95
   Cys Asp Phe Val Tyr Leu Asp Ser Pro Ile Val
               100                 105
<210> 233
   <211> 110
   <212> PRT
   <213> Artificial Sequence
<220>
   <221> SOURCE
   <222> 1..110
   <223> /mol_type="protein"
   /note="subject"
   /organism="Artificial Sequence"
<400> 233
   Thr Gly Lys Thr Val Trp Ala Arg Ser Leu Gly Asp His Ala Tyr Phe
   1               5                   10                  15
   Gly Gly Leu Phe Ser Met Asp Glu Asp Ile Ser Ser Ala Thr Tyr Ala
               20                  25                  30
   Val Phe Asp Asp Phe Gly Gly Leu Lys Phe Leu Pro Ser Tyr Lys Phe
           35                  40                  45
   Trp Met Gly His Gln Lys Gln Phe Tyr Val Thr Asp Lys Tyr Lys Gly
       50                  55                  60
   Lys Lys Leu Val Glu Trp Gly Arg Pro Ser Ile Trp Ile Asn Asn Glu
   65                  70                  75                  80
   Asp Pro Arg Glu Glu His Gly Val Arg Cys Asp Glu Ile Glu Trp Leu
                   85                  90                  95
   Glu Gln Asn Cys Ile Phe Ile Arg Leu Asp Ser Thr Ile Val
               100                 105                 110
<210> 234
   <211> 107
   <212> PRT
   <213> Artificial Sequence
<220>
   <221> SOURCE
   <222> 1..107
   <223> /mol_type="protein"
   /note="query"
   /organism="Artificial Sequence"
<400> 234
   Met Gly Lys Thr Ile Trp Ala Arg Ser Leu Gly Asn His Ala Tyr Phe
   1               5                   10                  15
   Gly Gly Leu Phe Ser Leu Asp Glu Asn Leu Asp Gly Val Glu Tyr Ala
               20                  25                  30
   Ile Phe Asp Asp Phe Gly Gly Ile Lys Phe Leu Pro Ser Tyr Lys Phe
           35                  40                  45
   Trp Leu Gly His Gln Lys Gln Phe Tyr Val Thr Asp Lys Tyr Lys Gly
       50                  55                  60
   Lys Lys Leu Val His Trp Ala Arg Pro Ser Ile Trp Leu Ser Asn Ser
   65                  70                  75                  80
   Asp Pro Arg Asp Glu Leu Gly Val Asp Thr Glu Trp Leu Asn Ala Asn
                   85                  90                  95
   Cys Asp Phe Val Tyr Leu Asp Ser Pro Ile Val
               100                 105
<210> 235
   <211> 108
   <212> PRT
   <213> Artificial Sequence
<220>
   <221> SOURCE
   <222> 1..108
   <223> /mol_type="protein"
   /note="subject"
   /organism="Artificial Sequence"
<400> 235
   Leu Gly Lys Thr Leu Trp Ala Arg Ser Leu Gly Asn His Ala Tyr Phe
   1               5                   10                  15
   Gly Gly Leu Phe Ser Leu Glu Glu Glu Leu Glu Gly Val Asp Tyr Ala
               20                  25                  30
   Ile Phe Asp Asp Met Gln Gly Gly Leu Lys Phe Phe His Ser Tyr Lys
           35                  40                  45
   Phe Trp Leu Gly Ala Gln Ser Gln Phe Trp Ala Thr Asp Lys Tyr Arg
       50                  55                  60
   Gly Lys Lys Leu Ile His Trp Gly Arg Pro Ser Ile Tyr Ile Cys Asn
   65                  70                  75                  80
   Gln Asn Pro Leu Cys Asp Glu Gly Val Asp His Asp Trp Leu Ile Gly
                   85                  90                  95
   Asn Cys Asp Phe Val Glu Ile Thr Glu Ser Leu Leu
               100                 105
<210> 236
   <211> 106
   <212> PRT
   <213> Artificial Sequence
<220>
   <221> SOURCE
   <222> 1..106
   <223> /mol_type="protein"
   /note="query"
   /organism="Artificial Sequence"
<400> 236
   Met Gly Lys Thr Ile Trp Ala Arg Ser Leu Gly Asn His Ala Tyr Phe
   1               5                   10                  15
   Gly Gly Leu Phe Ser Leu Asp Glu Asn Leu Asp Gly Val Glu Tyr Ala
               20                  25                  30
   Ile Phe Asp Asp Phe Gly Gly Ile Lys Phe Leu Pro Ser Tyr Lys Phe
           35                  40                  45
   Trp Leu Gly His Gln Lys Gln Phe Tyr Val Thr Asp Lys Tyr Lys Gly
       50                  55                  60
   Lys Lys Leu Val His Trp Ala Arg Pro Ser Ile Trp Leu Ser Asn Ser
   65                  70                  75                  80
   Asp Pro Arg Asp Glu Leu Gly Val Asp Thr Glu Trp Leu Asn Ala Asn
                   85                  90                  95
   Cys Asp Phe Val Tyr Leu Asp Ser Pro Ile
               100                 105
<210> 237
   <211> 107
   <212> PRT
   <213> Artificial Sequence
<220>
   <221> SOURCE
   <222> 1..107
   <223> /mol_type="protein"
   /note="subject"
   /organism="Artificial Sequence"
<400> 237
   Leu Gly Lys Thr Leu Trp Ala Arg Ser Leu Gly Arg His Ala Tyr Phe
   1               5                   10                  15
   Gly Gly Leu Phe Cys Leu Asp Glu Ser Leu Glu Asp Val Asp Tyr Ala
               20                  25                  30
   Val Phe Asp Asp Met Gln Gly Gly Leu Glu Phe Phe His Ala Tyr Lys
           35                  40                  45
   Phe Trp Leu Gly Ala Gln Lys Gln Phe Tyr Ala Thr Asp Lys Tyr Arg
       50                  55                  60
   Gly Lys Gln Leu Val Asn Trp Gly Arg Pro Ser Ile Tyr Ile Ser Asn
   65                  70                  75                  80
   Thr Asn Pro Leu Ala Asp Lys Gly Ala Asp Val Asp Trp Leu Met Gly
                   85                  90                  95
   Asn Cys Val Ile Val His Val Asp Arg Pro Ile
               100                 105
<210> 238
   <211> 87
   <212> PRT
   <213> Artificial Sequence
<220>
   <221> SOURCE
   <222> 1..87
   <223> /mol_type="protein"
   /note="query"
   /organism="Artificial Sequence"
<400> 238
   Thr Ser Ser Gly Val Arg Lys Ile Leu Ile Gly Gln Ile Ser Leu Leu
   1               5                   10                  15
   Pro Pro Leu Ile Leu Val Ala Leu Arg Leu Ser Pro Thr Asn Ser Ala
               20                  25                  30
   Ser Ser Ala Pro Val Met Lys Gln Val Leu Arg Ala Cys Leu Ser Phe
           35                  40                  45
   Gly Thr Pro Phe Val Val Pro Ser Leu Thr Lys Met Thr Leu Ser Pro
       50                  55                  60
   Thr Leu Ser Val Ile Gly Pro Phe Leu Pro Gln Val Tyr Glu Val Leu
   65                  70                  75                  80
   Gly Ile Cys Thr Ser Trp Ile
                   85
<210> 239
   <211> 91
   <212> PRT
   <213> Artificial Sequence
<220>
   <221> SOURCE
   <222> 1..91
   <223> /mol_type="protein"
   /note="subject"
   /organism="Artificial Sequence"
<400> 239
   Thr Ser Ser Gly Val Lys Ile Val Leu Thr Ile Asp Ser Ser Leu Leu
   1               5                   10                  15
   Asp His Leu Phe Leu Ala Leu Pro Arg Ser Ser Pro His Gln Glu Ala
               20                  25                  30
   Leu Ala Ile Phe Leu Leu Val Pro Glu Cys Cys Val Ala Leu Gly Ala
           35                  40                  45
   Cys Arg Ile Pro Ser Leu Ala Leu Arg Phe Ala Glu Ala Val Thr Gly
       50                  55                  60
   Leu Ser Lys Gly Ser Ser Asn Ile Leu Gly Pro Phe Leu Glu Gln Ile
   65                  70                  75                  80
   Val Gln Met Leu Lys Thr Cys Val Ser Ala Ile
                   85                  90
<210> 240
   <211> 1626
   <212> **DNA**
   <213> **Artificial** Sequence
<220>
   <221> source
   <222> 1..1626
   <223> /mol_type="DNA"
   /note="MSSI1.162"
   /organism="Artificial Sequence"
<400> 240
   gacttctgat tgattgatgc ctgccgactt ttgataattt tttgatagat gatttttctt 60
   gactgaaata aaaaaacccc ttattgttgt aagtgcttgg ttcacaactt aataagaggt 120
   tttaattatg atgtcacatg atggcacacc tgtcaatttt ttgtccgata ttcaacccag 180
   tgagaaaagc tgggacactc accgagccga agccgaaagc gtccgattgc tttacagtct 240
   ttcgactgag ttcaccaaat acgctagccg tatttatgac tgttcccaaa tcctaaaatt 300
   tgcccctacc cctgataagc tggtacttaa acacgccttt ttctgtcgtg ttaggtattg 360
   ccctgtttgc cagtggcgta ggtcgttatt atggcgggcg gtcatgtttc agcagttacc 420
   agcaatcaaa gagaagtacc ctagttatcg ttgggtgttt cttaccctta ctgtcaaaaa 480
   tcccccagtt actgaattaa gggacacctt aaaggctatg aattcagctt ggcaacgcct 540
   agcacaaact aaacgcttta aaggggtcgt taaaggcttt attcgcacaa ccgaagttac 600
   aaggggtaaa gatggcgaca tgatggctca cccccatttc catgcgctgt tattagttca 660
   aagtaattat ttcactacaa attatattaa acaaaatgat tgggtcgaaa tgtggcaaaa 720
   ggcgttaagg gtcgattatg ccccatcggt caacgtaaaa gccgttaaac cccctaaaaa 780
   gggtgaaaag gataatctcg ataaggcgat atgtgaaacg ctcaaatata gcgttaaacc 840
   gtctgatatt gccaaagatg acgatggggg cgaatggttg catgaaatga ccagacaaac 900
   cctaaatatg cgttttattg ccactggtgg cattttgaag ggtgtactta aacccgatga 960
   gcaagtaaca caacaagaaa tgcttacccc cactggtgaa gatgaagcac caaccgaaca 1020
   aaaacggatt ggatttagat tttaccccca tcatgggcgt tatgtttttt cgccagctat 1080
   acgaattttt gacttgctgt ttttgcaagt cgtaagttcg cccttatagc aaatgtgttg 1140
   tgcctgtaag tgctgtaagg catatccagc gttagcaata aacggcttta tcgtttattg 1200
   cgttagcgtc tttatatccc caacctttcc ccctttgaag tcccagcttt tttttgtggc 1260
   tgttctattg ttgctcttta ttgaccttac gccacataaa aaaagtgggt tacatatcgg 1320
   ttgaatttgg gttgcaatag tctagggaca ttcaaaaata atccttcttg gtagtattac 1380
   gaaatctcat gtaatccgca ttggtggcgg tttacccaaa aacaaaaaca agaaaaattc 1440
   gtcttgcgaa tttttattgc tttttgcttt ttggggtata aaaacctata gaaaaactat 1500
   aaaaaaccta tagaattata ggtgcttact gggggtttgc cttgtattta ttattaaggg 1560
   ataactctga ttttatttta tatgattttt tatactggca aacctgttga ataccgctta 1620
   aatact 1626
<210> 241
   <211> 273
   <212> DNA
   <213> Artificial Sequence
<220>
   <221> source
   <222> 1..273
   <223> /mol_type="DNA"
   /note="query"
   /organism="Artificial Sequence"
<400> 241
   aaacacgcct ttttctgtcg tgttaggtat tgccctgttt gccagtggcg taggtcgtta     60
   ttatggcggg cggtcatgtt tcagcagtta ccagcaatca aagagaagta ccctagttat    120
   cgttgggtgt ttcttaccct tactgtcaaa aatcccccag ttactgaatt aagggacacc    180
   ttaaaggcta tgaattcagc ttggcaacgc ctagcacaaa ctaaacgctt taaaggggtc    240
   gttaaaggct ttattcgcac aaccgaagtt aca                                 273
<210> 242
   <211> 273
   <212> DNA
   <213> Artificial Sequence
<220>
   <221> source
   <222> 1..273
   <223> /mol_type="DNA"
   /note="subject"
   /organism="Artificial Sequence"
<400> 242
   aaacaagcct ttttttgccg tgttcgttac tgcggtatgt gccaatggcg taggtcatta     60
   ctctggaaag ccttgatgta taaaacatac gatgaaatta tgcagacaca cccgacacat    120
   cgctttgtgt ttttaaccct gacgcttaaa aatccgccta tcggtgactt gcgcgaaacc    180
   ttgcagcaca tgaataaatc atggcaacgc ttaaaagatc gtaaagaatt tatagcaggc    240
   gttgaaggtt gggtacgcac aacggaagtc aca                                 273
<210> 243
   <211> 89
   <212> DNA
   <213> Artificial Sequence
<220>
   <221> source
   <222> 1..89
   <223> /mol_type="DNA"
   /note="query"
   /organism="Artificial Sequence"
<400> 243
   aattatatta aacaaaatga ttgggtcgaa atgtggcaaa aggcgttaag ggtcgattat 60
   gccccatcgg tcaacgtaaa agccgttaa 89
<210> 244
   <211> 89
   <212> DNA
   <213> Artificial Sequence
<220>
   <221> source
   <222> 1..89
   <223> /mol_type="DNA"
   /note="subject"
   /organism="Artificial Sequence"
<400> 244
   aattatatta atcaagaaga atggacgaga ctgtggcgaa aggcgttaaa agtcgattat 60
   gagccagtgg tgcatgtgca agtcgttaa 89
<210> 245
   <211> 313
   <212> PRT
   <213> Artificial Sequence
<220>
   <221> SOURCE
   <222> 1..313
   <223> /mol_type="protein"
   /note="query"
   /organism="Artificial Sequence"
<400> 245
   Val Asn Phe Leu Ser Asp Ile Gln Pro Ser Glu Lys Ser Trp Asp Thr
   1               5                   10                  15
   His Arg Ala Glu Ala Glu Ser Val Arg Leu Leu Tyr Ser Leu Ser Thr
               20                  25                  30
   Glu Phe Thr Lys Tyr Ala Ser Arg Ile Tyr Asp Cys Ser Gln Ile Leu
           35                  40                  45
   Lys Phe Ala Pro Thr Pro Asp Lys Leu Val Leu Lys His Ala Phe Phe
       50                  55                  60
   Cys Arg Val Arg Tyr Cys Pro Val Cys Gln Trp Arg Arg Ser Leu Leu
   65                  70                  75                  80
   Trp Arg Ala Val Met Phe Gln Gln Leu Pro Ala Ile Lys Glu Lys Tyr
                   85                  90                  95
   Pro Ser Tyr Arg Trp Val Phe Leu Thr Leu Thr Val Lys Asn Pro Pro
               100                 105                 110
   Val Thr Glu Leu Arg Asp Thr Leu Lys Ala Met Asn Ser Ala Trp Gln
           115                 120                 125
   Arg Leu Ala Gln Thr Lys Arg Phe Lys Gly Val Val Lys Gly Phe Ile
       130                 135                 140
   Arg Thr Thr Glu Val Thr Arg Gly Lys Asp Gly Asp Met Met Ala His
   145                 150                 155                 160
   Pro His Phe His Ala Leu Leu Leu Val Gln Ser Asn Tyr Phe Thr Thr
                   165                 170                 175
   Asn Tyr Ile Lys Gln Asn Asp Trp Val Glu Met Trp Gln Lys Ala Leu
               180                 185                 190
   Arg Val Asp Tyr Ala Pro Ser Val Asn Val Lys Ala Val Lys Pro Pro
           195                 200                 205
   Lys Lys Gly Glu Lys Asp Asn Leu Asp Lys Ala Ile Cys Glu Thr Leu
       210                 215                 220
   Lys Tyr Ser Val Lys Pro Ser Asp Ile Ala Lys Asp Asp Asp Gly Gly
   225                 230                 235                 240
   Glu Trp Leu His Glu Met Thr Arg Gln Thr Leu Asn Met Arg Phe Ile
                   245                 250                 255
   Ala Thr Gly Gly Ile Leu Lys Gly Val Leu Lys Pro Asp Glu Gln Val
               260                 265                 270
   Thr Gln Gln Glu Met Leu Thr Pro Thr Gly Glu Asp Glu Ala Pro Thr
           275                 280                 285
   Glu Gln Lys Arg Ile Gly Phe Arg Phe Tyr Pro His His Gly Arg Tyr
       290                 295                 300
   Val Phe Ser Pro Ala His Thr Asn Phe
   305                 310
<210> 246
   <211> 299
   <212> PRT
   <213> Artificial Sequence
<220>
   <221> SOURCE
   <222> 1..299
   <223> /mol_type="protein"
   /note="subject"
   /organism="Artificial Sequence"
<400> 246
   Ile Asn Phe Leu Thr Glu Ile Ser Pro His Glu Val Ser Trp Asp Glu
   1               5                   10                  15
   His Arg Ser Asp Ala Glu Ser Val Lys Ile Leu Tyr Asn Tyr Ser Val
               20                  25                  30
   Glu Leu Ala Lys Tyr Ala Asp Arg Ile Asn Gly Cys Ser Gly Ile Leu
           35                  40                  45
   Lys Phe Gly Val Ser Pro Asn Gln Gly Lys Leu Val Leu Lys Gln Ala
       50                  55                  60
   Phe Phe Cys Arg Val Arg His Cys Pro Val Cys Gln Trp Arg Arg Ser
   65                  70                  75                  80
   Leu Leu Trp Arg Ala Val Met Phe Gln Gln Leu Pro Lys Ile Gln Lys
                   85                  90                  95
   Leu Tyr Pro Thr His Arg Trp Val Phe Leu Thr Met Thr Val Arg Asn
               100                 105                 110
   Pro Pro Ile Ile Glu Leu Arg Asp Thr Leu Lys His Met Asn Glu Ser
           115                 120                 125
   Trp Gln Arg Leu Ile Gln Thr Lys Ala Phe Lys Ser Gly Val Ala Gly
       130                 135                 140
   Phe Ile Arg Thr Thr Glu Val Thr Arg Gly Lys Asp Ala Glu Met Met
   145                 150                 155                 160
   Ser His Pro His Tyr His Ala Leu Leu Leu Val Lys Pro Gly Tyr Phe
                   165                 170                 175
   Ser Lys Tyr Tyr Ile Asn Gln Ser Glu Trp Val Glu Met Trp Ala Lys
               180                 185                 190
   Ala Leu Arg Ala Asp Tyr Leu Pro Ser Val Asn Val Lys Ala Ile Arg
           195                 200                 205
   Ala Gly Asn Asn Glu Lys Ser Lys Lys Ala Leu Glu Lys Gln Ile Cys
       210                 215                 220
   Glu Thr Leu Lys Tyr Ser Val Lys Pro Ser Asp Leu Ala Val Glu Arg
   225                 230                 235                 240
   Asp Lys Gly Ala Trp Leu His Glu Met Thr Lys Gln Val His Lys Met
                   245                 250                 255
   Arg Phe Val Ala Thr Gly Gly Val Leu Lys Gly Ile Leu Lys Pro Asp
               260                 265                 270
   Asp Glu Ile Thr Asn Glu Glu Met Ile Ser Ser Ser Glu Glu Thr Glu
           275                 280                 285
   Asp Val Gly Glu Gly Arg Val Ala Phe Gln Phe
       290                 295
<210> 247
   <211> 313
   <212> PRT
   <213> Artificial Sequence
<220>
   <221> SOURCE
   <222> 1..313
   <223> /mol_type="protein"
   /note="query"
   /organism="Artificial Sequence"
<400> 247
   Val Asn Phe Leu Ser Asp Ile Gln Pro Ser Glu Lys Ser Trp Asp Thr
   1               5                   10                  15
   His Arg Ala Glu Ala Glu Ser Val Arg Leu Leu Tyr Ser Leu Ser Thr
               20                  25                  30
   Glu Phe Thr Lys Tyr Ala Ser Arg Ile Tyr Asp Cys Ser Gln Ile Leu
           35                  40                  45
   Lys Phe Ala Pro Thr Pro Asp Lys Leu Val Leu Lys His Ala Phe Phe
       50                  55                  60
   Cys Arg Val Arg Tyr Cys Pro Val Cys Gln Trp Arg Arg Ser Leu Leu
   65                  70                  75                  80
   Trp Arg Ala Val Met Phe Gln Gln Leu Pro Ala Ile Lys Glu Lys Tyr
                   85                  90                  95
   Pro Ser Tyr Arg Trp Val Phe Leu Thr Leu Thr Val Lys Asn Pro Pro
               100                 105                 110
   Val Thr Glu Leu Arg Asp Thr Leu Lys Ala Met Asn Ser Ala Trp Gln
           115                 120                 125
   Arg Leu Ala Gln Thr Lys Arg Phe Lys Gly Val Val Lys Gly Phe Ile
       130                 135                 140
   Arg Thr Thr Glu Val Thr Arg Gly Lys Asp Gly Asp Met Met Ala His
   145                 150                 155                 160
   Pro His Phe His Ala Leu Leu Leu Val Gln Ser Asn Tyr Phe Thr Thr
                   165                 170                 175
   Asn Tyr Ile Lys Gln Asn Asp Trp Val Glu Met Trp Gln Lys Ala Leu
               180                 185                 190
   Arg Val Asp Tyr Ala Pro Ser Val Asn Val Lys Ala Val Lys Pro Pro
           195                 200                 205
   Lys Lys Gly Glu Lys Asp Asn Leu Asp Lys Ala Ile Cys Glu Thr Leu
       210                 215                 220
   Lys Tyr Ser Val Lys Pro Ser Asp Ile Ala Lys Asp Asp Asp Gly Gly
   225                 230                 235                 240
   Glu Trp Leu His Glu Met Thr Arg Gln Thr Leu Asn Met Arg Phe Ile
                   245                 250                 255
   Ala Thr Gly Gly Ile Leu Lys Gly Val Leu Lys Pro Asp Glu Gln Val
               260                 265                 270
   Thr Gln Gln Glu Met Leu Thr Pro Thr Gly Glu Asp Glu Ala Pro Thr
           275                 280                 285
   Glu Gln Lys Arg Ile Gly Phe Arg Phe Tyr Pro His His Gly Arg Tyr
       290                 295                 300
   Val Phe Ser Pro Ala His Thr Asn Phe
   305                 310
<210> 248
   <211> 315
   <212> PRT
   <213> Artificial Sequence
<220>
   <221> SOURCE
   <222> 1..315
   <223> /mol_type="protein"
   /note="subject"
   /organism="Artificial Sequence"
<400> 248
   Ile Asn Phe Leu Thr Glu Ile Ser Pro His Glu Val Ser Trp Asp Glu
   1               5                   10                  15
   His Arg Ser Asp Ala Glu Ser Val Lys Ile Leu Tyr Asn Tyr Ser Val
               20                  25                  30
   Glu Leu Ala Lys Tyr Ala Asp Arg Ile Asn Gly Cys Ser Gly Ile Leu
           35                  40                  45
   Lys Phe Gly Val Ser Pro Asn Gln Gly Lys Leu Val Leu Lys Gln Ala
       50                  55                  60
   Phe Phe Cys Arg Val Arg His Cys Pro Val Cys Gln Trp Arg Arg Ser
   65                  70                  75                  80
   Leu Leu Trp Arg Ala Val Met Phe Gln Gln Leu Pro Lys Ile Gln Lys
                   85                  90                  95
   Leu Tyr Pro Thr His Arg Trp Val Phe Leu Thr Met Thr Val Arg Asn
               100                 105                 110
   Pro Pro Ile Ile Glu Leu Arg Asp Thr Leu Lys His Met Asn Glu Ser
           115                 120                 125
   Trp Gln Arg Leu Ile Gln Thr Lys Ala Phe Lys Ser Gly Val Ala Gly
       130                 135                 140
   Phe Ile Arg Thr Thr Glu Val Thr Arg Gly Lys Asp Ala Glu Met Met
   145                 150                 155                 160
   Ser His Pro His Tyr His Ala Leu Leu Leu Val Lys Pro Gly Tyr Phe
                   165                 170                 175
   Ser Lys Tyr Tyr Ile Asn Gln Ser Glu Trp Val Glu Met Trp Ala Lys
               180                 185                 190
   Ala Leu Arg Ala Asp Tyr Leu Pro Ser Val Asn Val Lys Ala Ile Arg
           195                 200                 205
   Ala Gly Asn Asn Glu Lys Ser Lys Lys Ala Leu Glu Lys Gln Ile Cys
       210                 215                 220
   Glu Thr Leu Lys Tyr Ser Val Lys Pro Ser Asp Leu Ala Val Glu Arg
   225                 230                 235                 240
   Asp Lys Gly Ala Trp Leu His Glu Met Thr Lys Gln Val His Lys Met
                   245                 250                 255
   Arg Phe Val Ala Thr Gly Gly Val Leu Lys Gly Ile Leu Lys Pro Asp
               260                 265                 270
   Asp Glu Ile Thr Asn Glu Glu Met Ile Ser Ser Ser Glu Glu Thr Glu
           275                 280                 285
   Asp Val Gly Glu Gly Arg Val Ala Phe Gln Phe Asn Ser Glu Tyr Arg
       290                 295                 300
   Arg Tyr Val Tyr Ala Pro Lys Phe Asn Glu Tyr
   305                 310                 315

## Description

The present invention relates to CMI (Cow Milk Isolate) nucleotide sequences as well as probes and primers comprising part of said nucleotide sequences and antibodies against polypeptides encoded by said nucleotide sequences. Finally, the present invention relates to the use of said compounds as an early marker for the future development of diseases such as cancer and diseases of the CNS and as a target for treatment and prevention of these diseases.

### Background

Several epidemiological analyses conducted in recent decades indicate that the long-term consumption of "red" meat processed by different ways (including smoked or air-dried meat and meat as component of sausages consumed rare, undercooked or grilled) can be regarded as a risk factor for colon cancer (World Cancer Report 2007, zur Hausen 2012). "Red" meat is regarded as comprising beef, pork, mutton, lamb and goat meat, in contrast to "white" meat (poultry meat/fish).

Thus far, chemical carcinogenic substances being produced during roasting, grilling, barbecuing, smoking and air-drying were blamed as risk factors for cancer. However, often the fact was disregarded that the same substances are also produced in comparable concentrations during analogous ways of preparation of poultry meat/fish. Accordingly, this does not support the assumption that these chemical substances play an exclusive role as regards the development of colon cancer. Since, in addition, the current epidemiological analyses suggest that beef is the main risk factor it has been postulated that an additional species-specific - presumably infectious - factor contributes to the triggering of this type of cancer (zur Hausen, 2012). The results of the correlation of analyses of the global spreading of domesticated bovine species with the global incidence of colon cancer seem to suggest that the consumption of meat of bovine species stemming from European/Asian cattle (Bos taurus) but not from breedings of zebu, water buffalo or yak might be of importance as a main risk factor (zur Hausen, 2015)

Thus, the technical problem underlying the present invention is to identify specific nucleotide sequences that might be associated with diseases such as cancer or diseases of the CNS and, thus, to provide means for diagnosis and therapy.

### Brief Description of the Present Invention

The solution to said technical problem is achieved by providing the embodiments characterized in the claims.

During the experiments resulting in the present invention sera of cattle were screened for infectious agents - starting from the assumption that the presence in sera is also indicative for the presence of these agents in "red" meat. Sera from healthy cows were screened and new viral nucleic acid components could be isolated. The DNA sequences and open reading frames of several of these components showed a recognizable relationship to two sequences which were already described for transmissible spongiform encephalopathies (TSE) for TSE-diseases of sheep, cattle and humans.

The TSE isolates have also been suspected to play a role in cancer induction (Manuelidis, 2011), thus, it is reasonable to assume that the viral sequences described might be associated with the development of diseases like cancer, specifically colon and breast cancers but also Hodgkin's disease and others, and diseases of the CNS (Multiple sclerosis MS, amyotrophic lateral sclerosis, transmissible spongiforme encephalopathies/ Prion-linked diseases, Parkinson's disease, Alzheimer disease).

### Brief description of the drawings

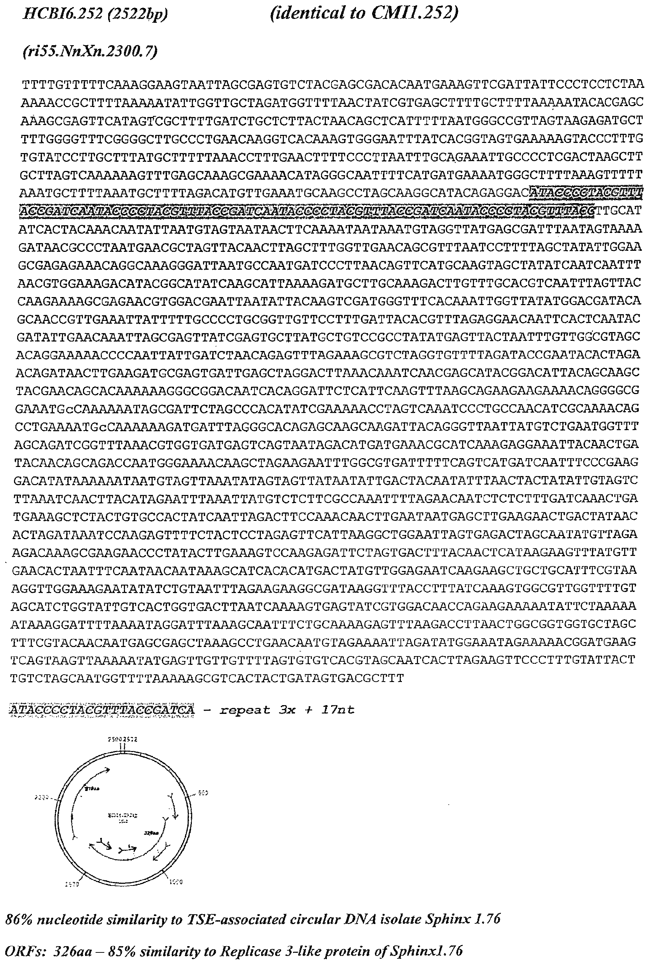

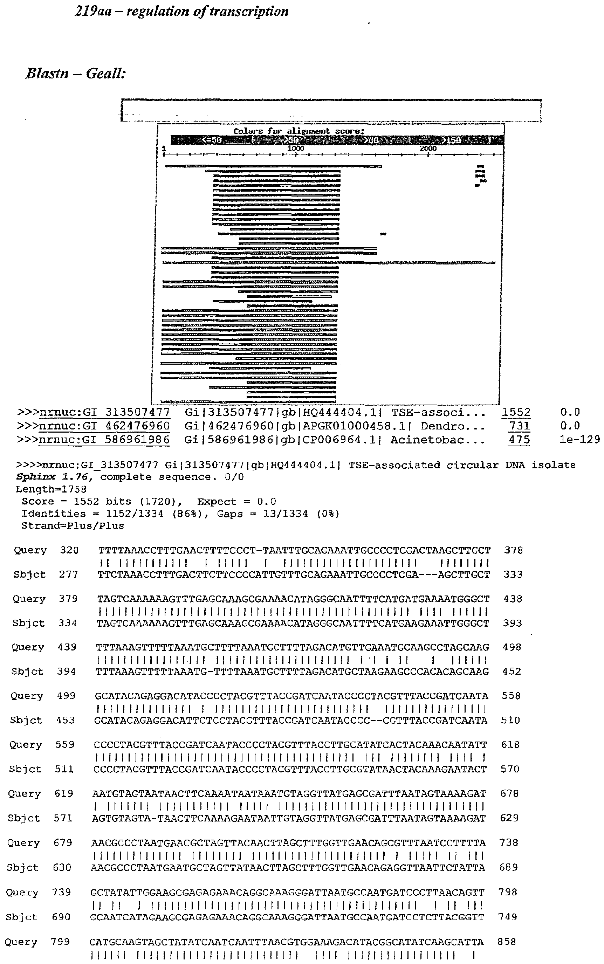

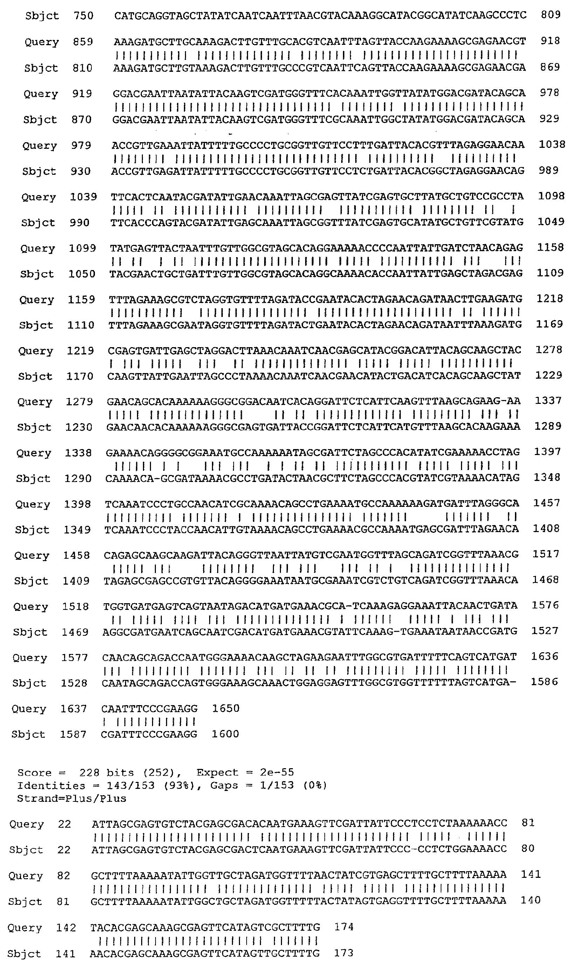

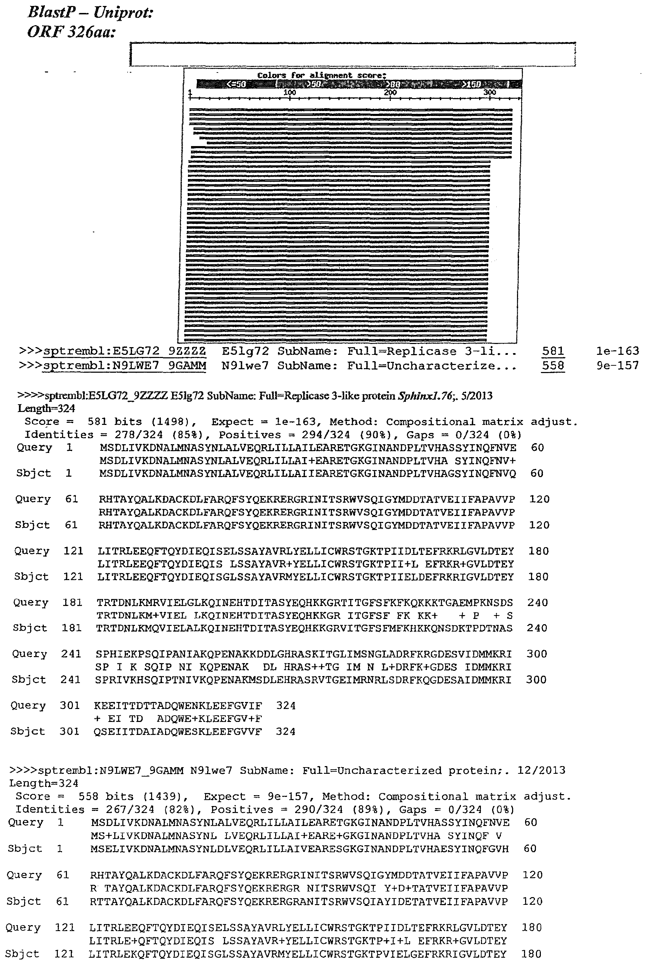

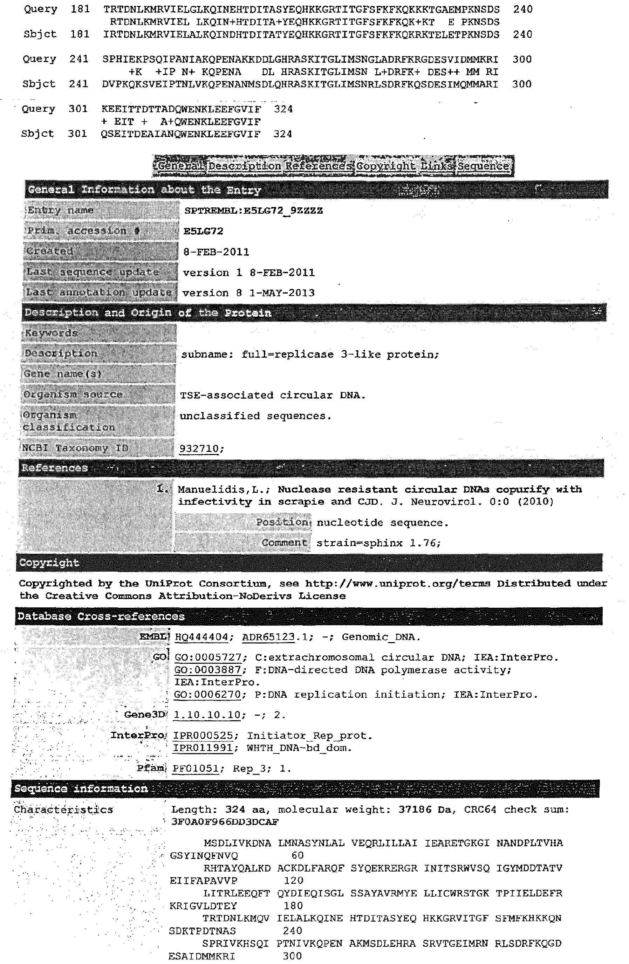

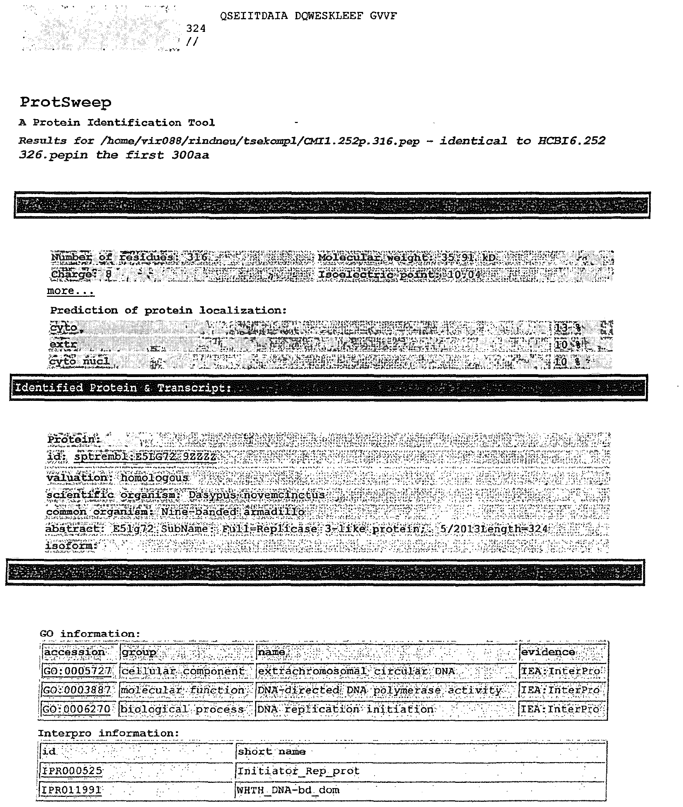

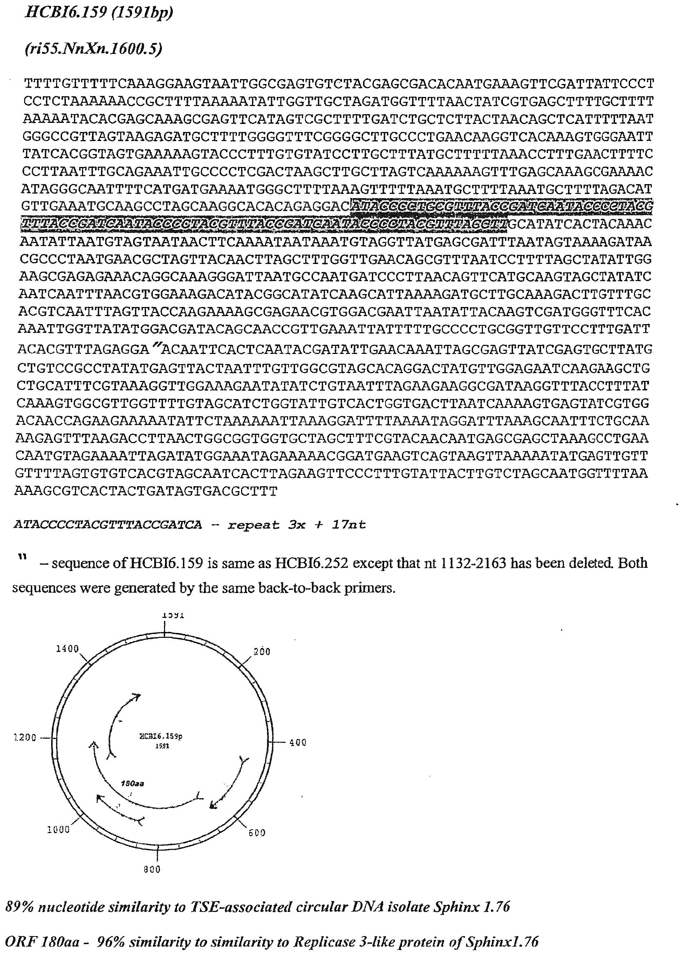

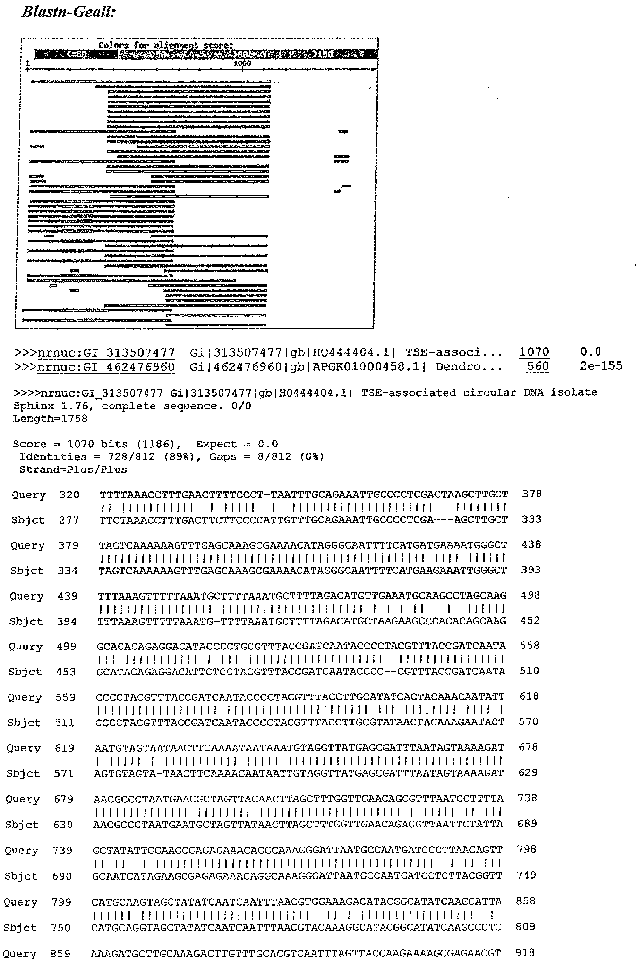

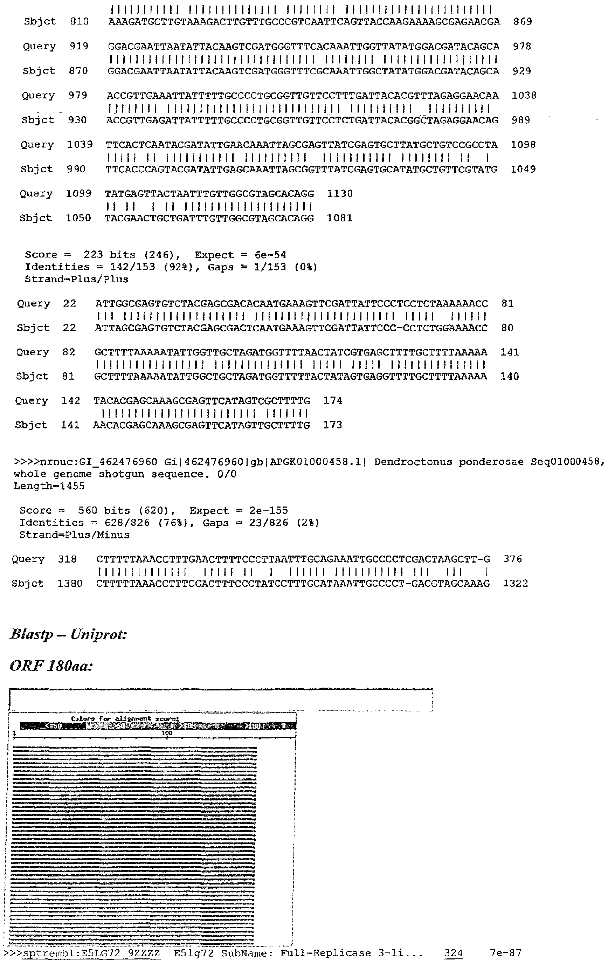

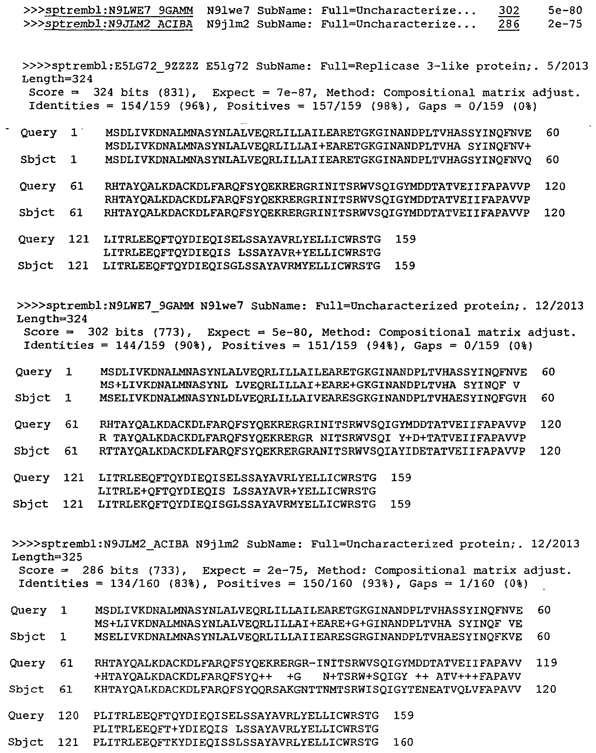

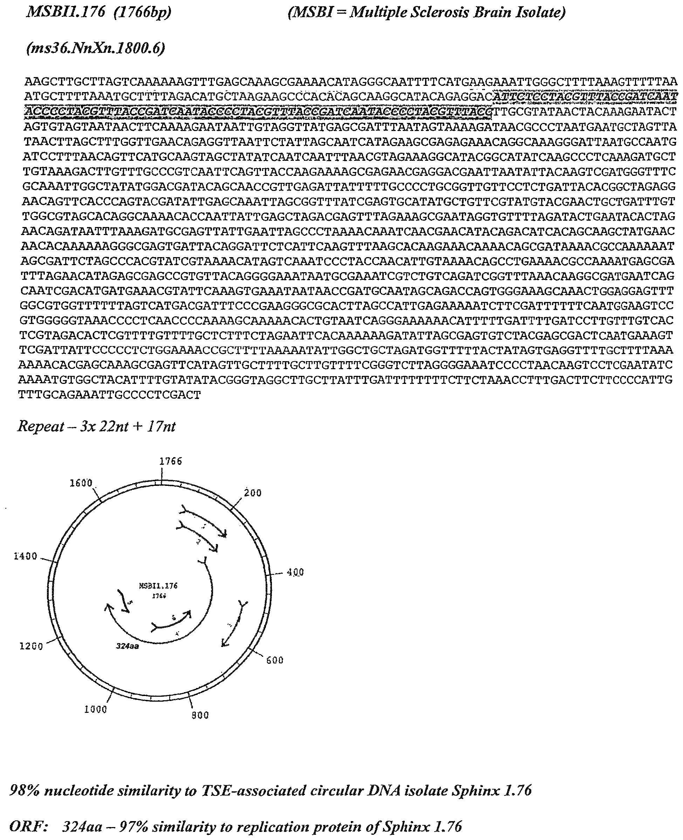

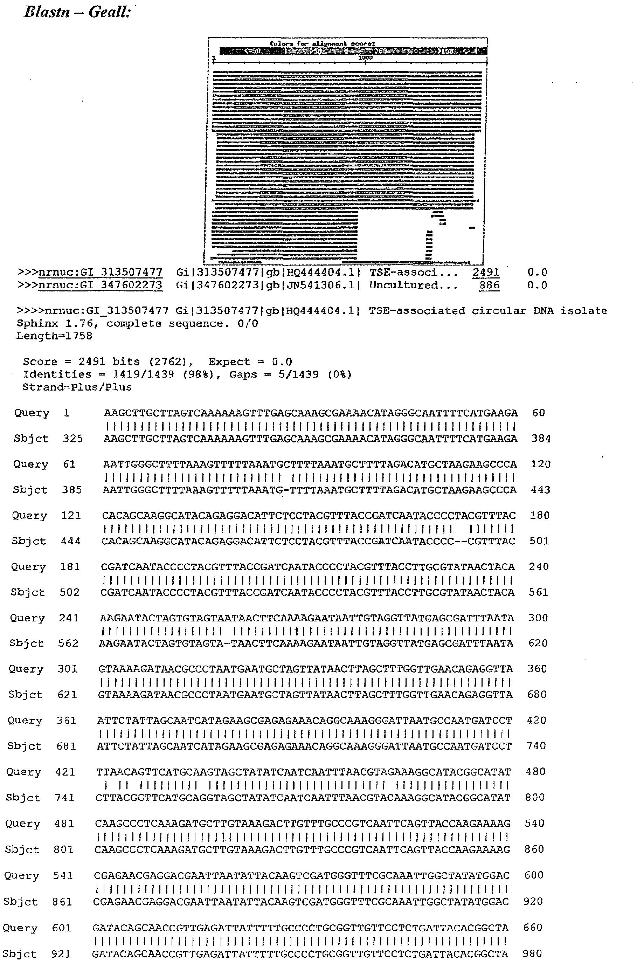

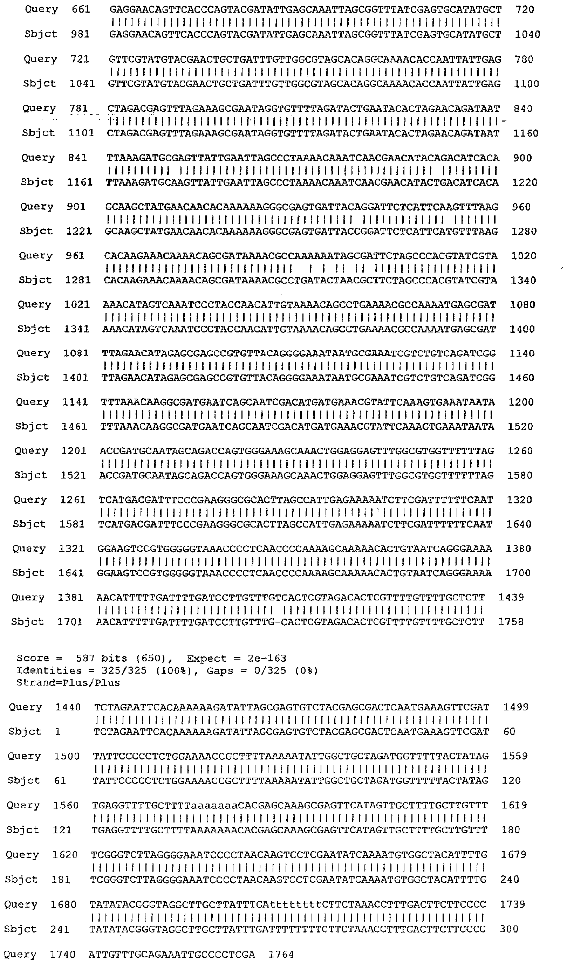

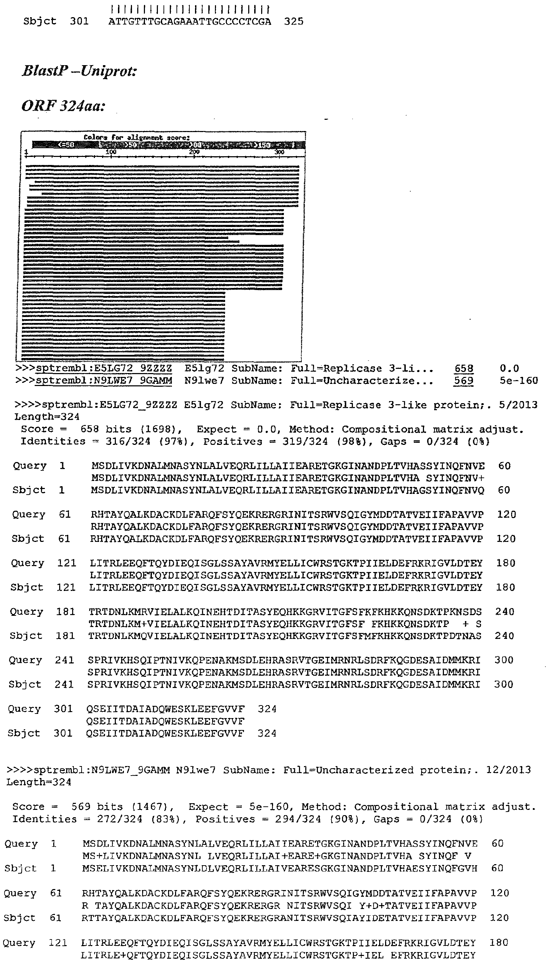

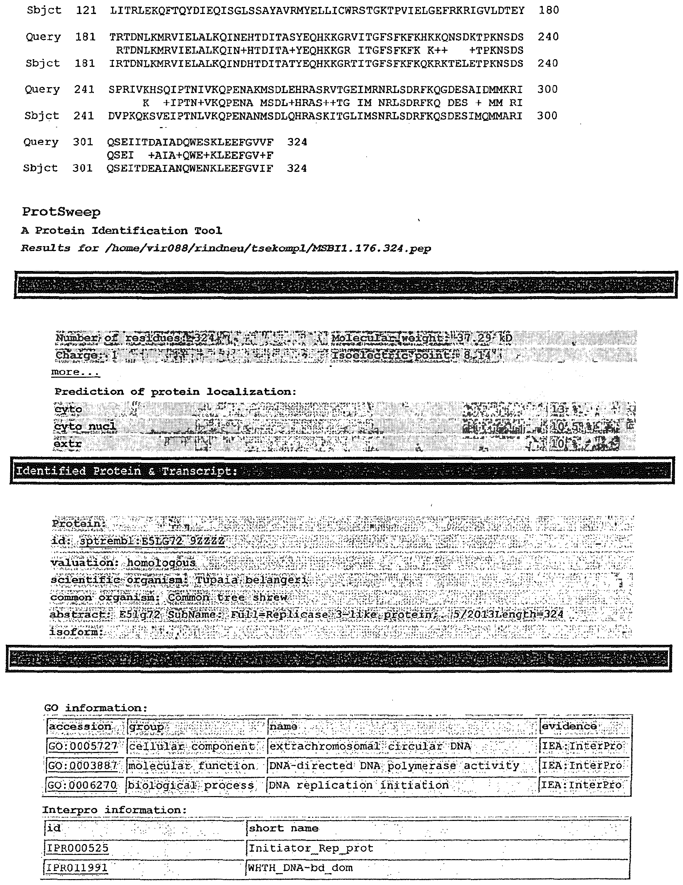

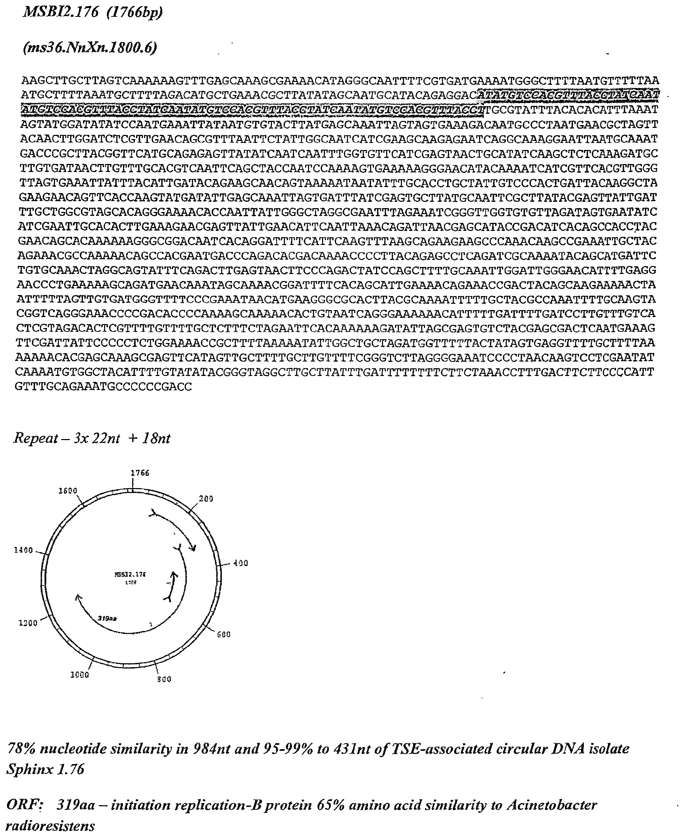

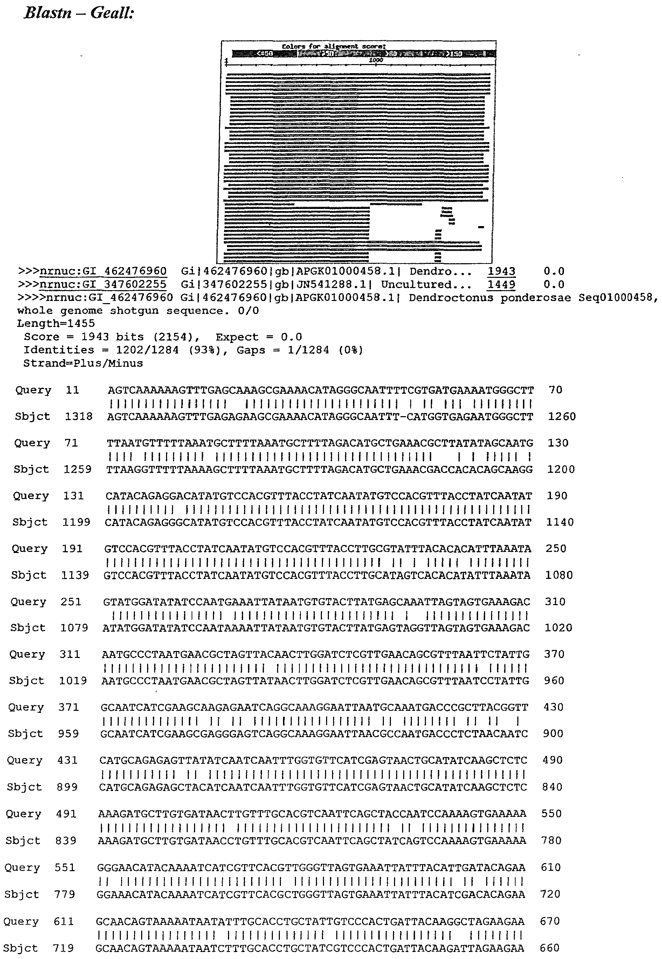

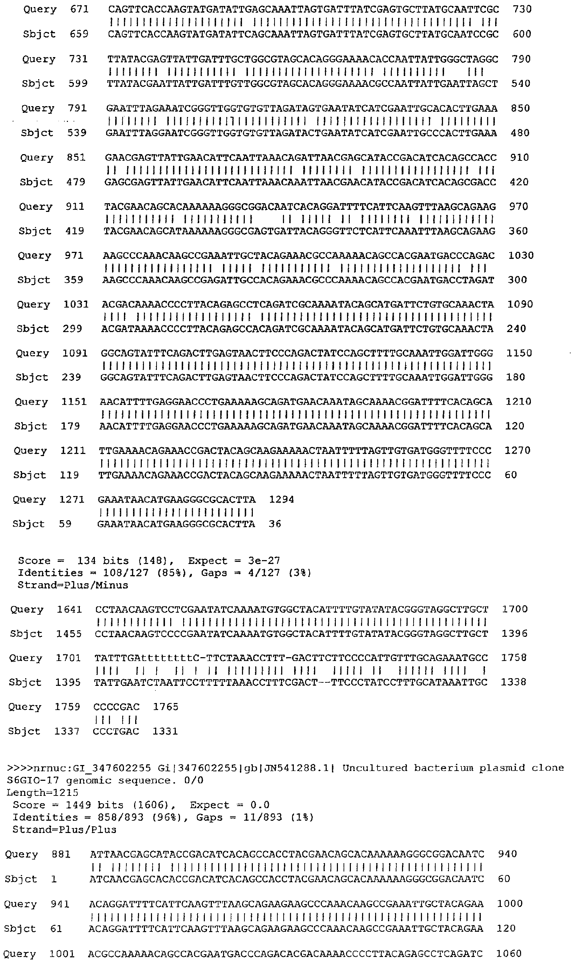

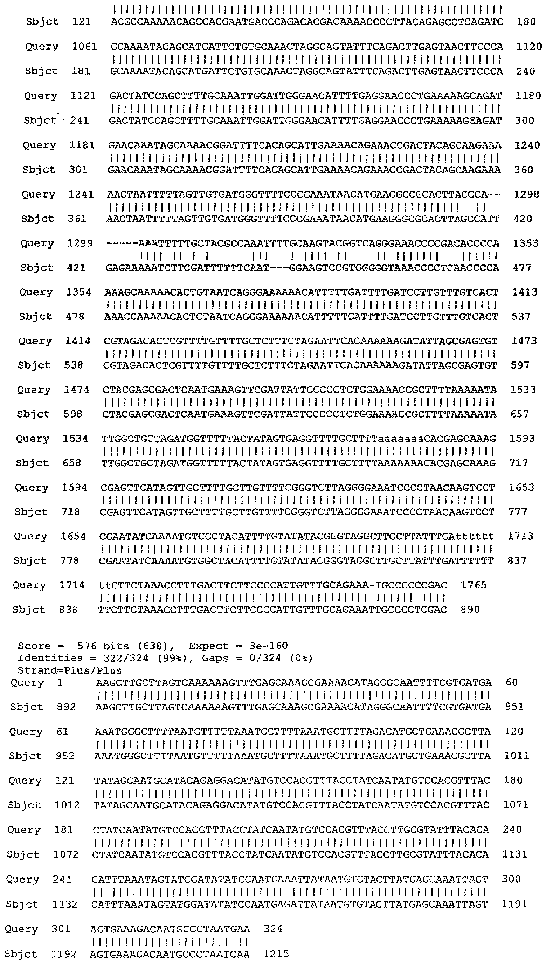

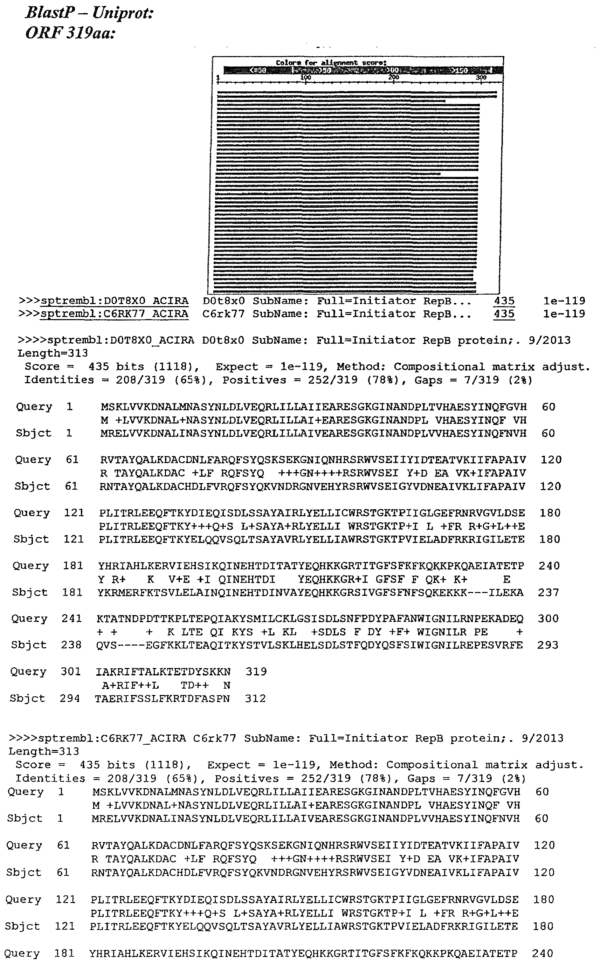

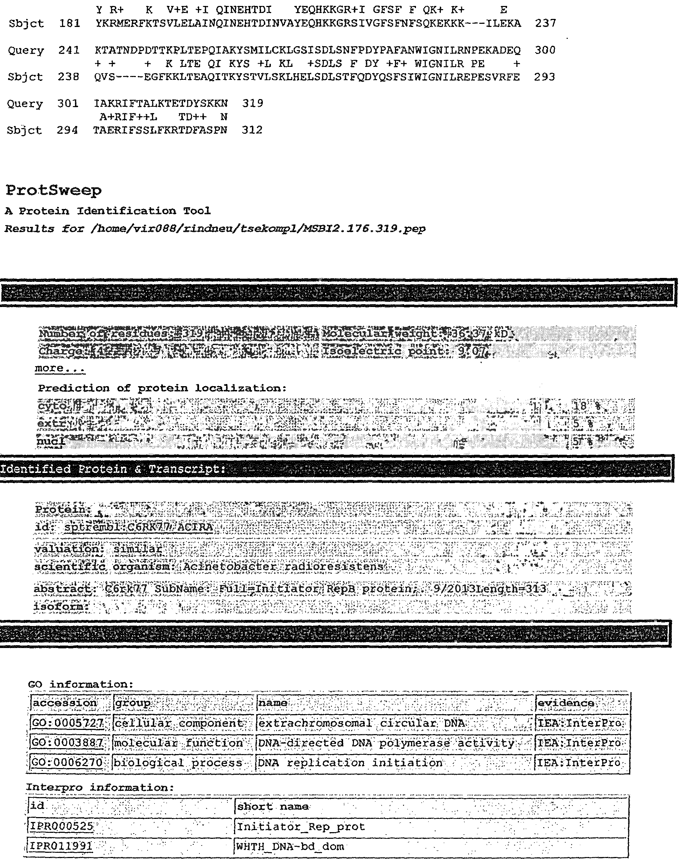

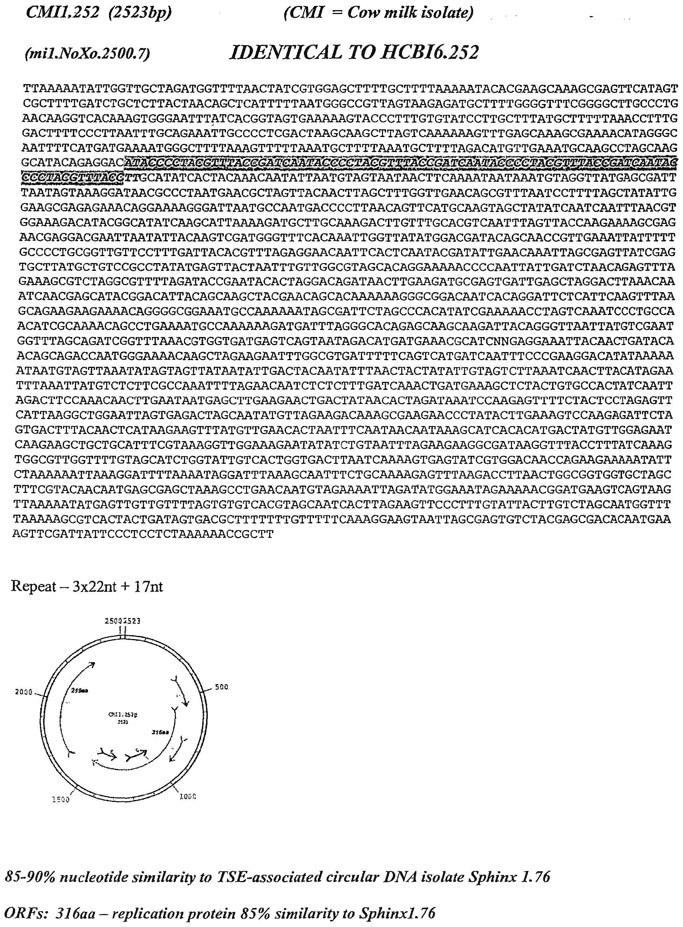

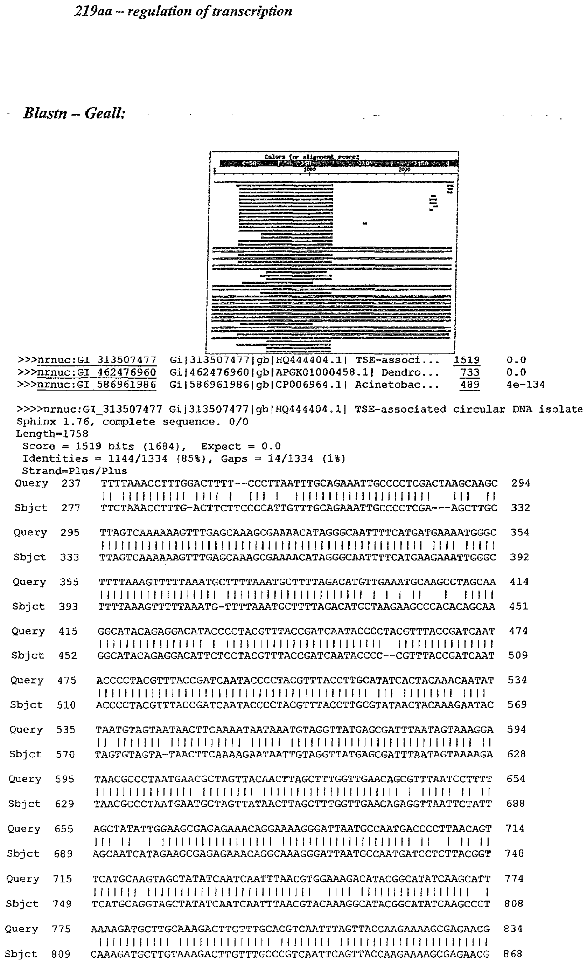

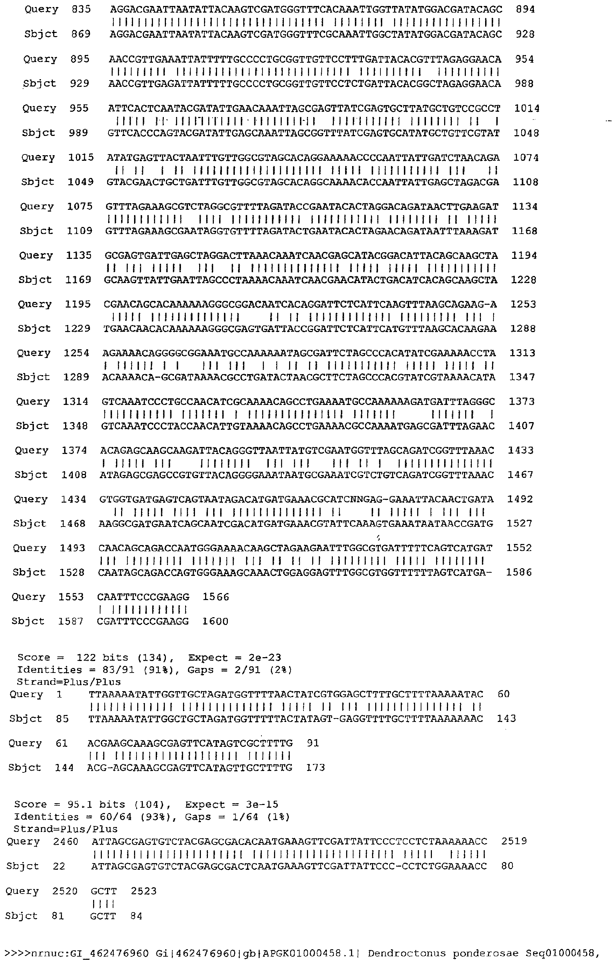

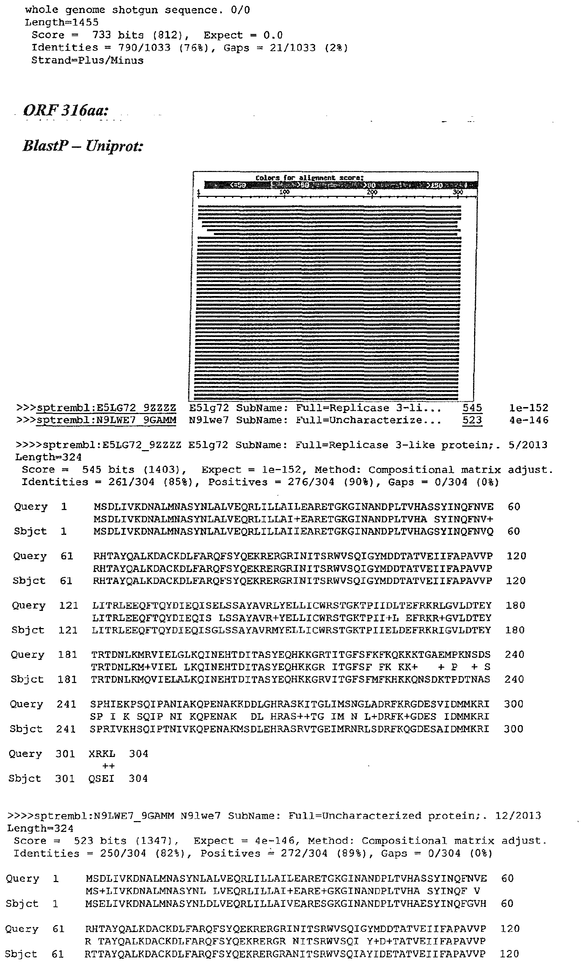

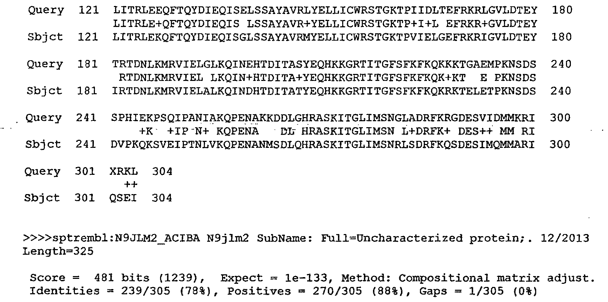

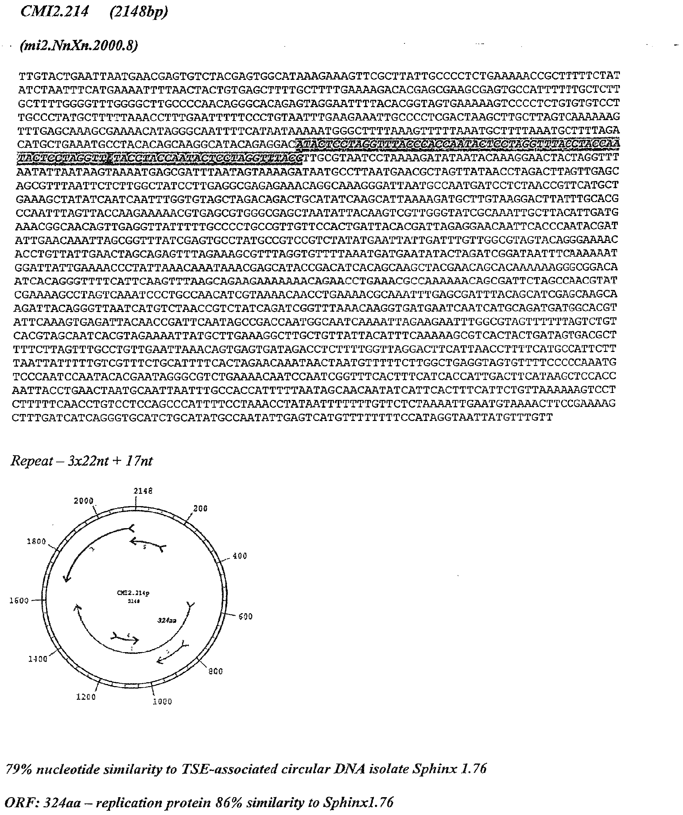

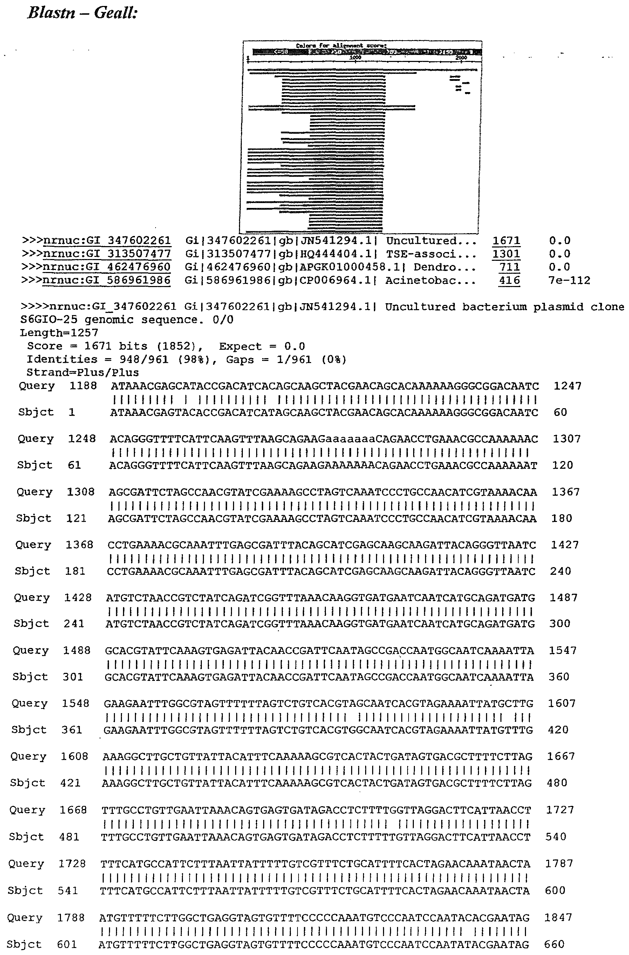

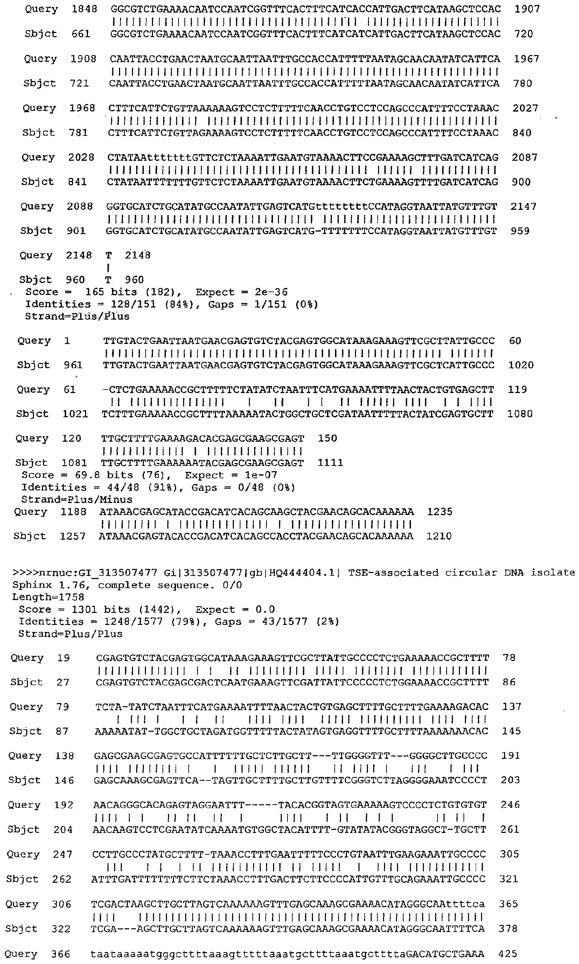

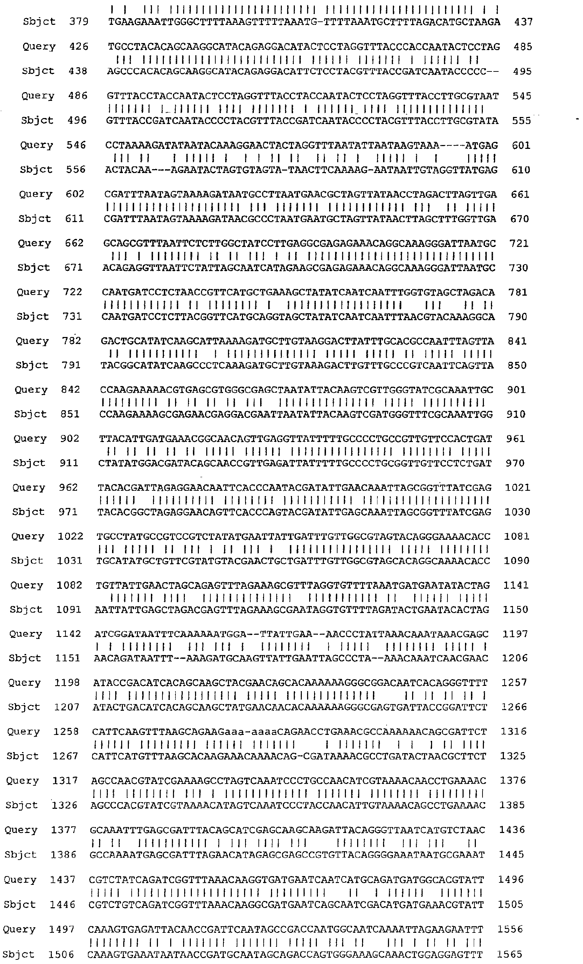

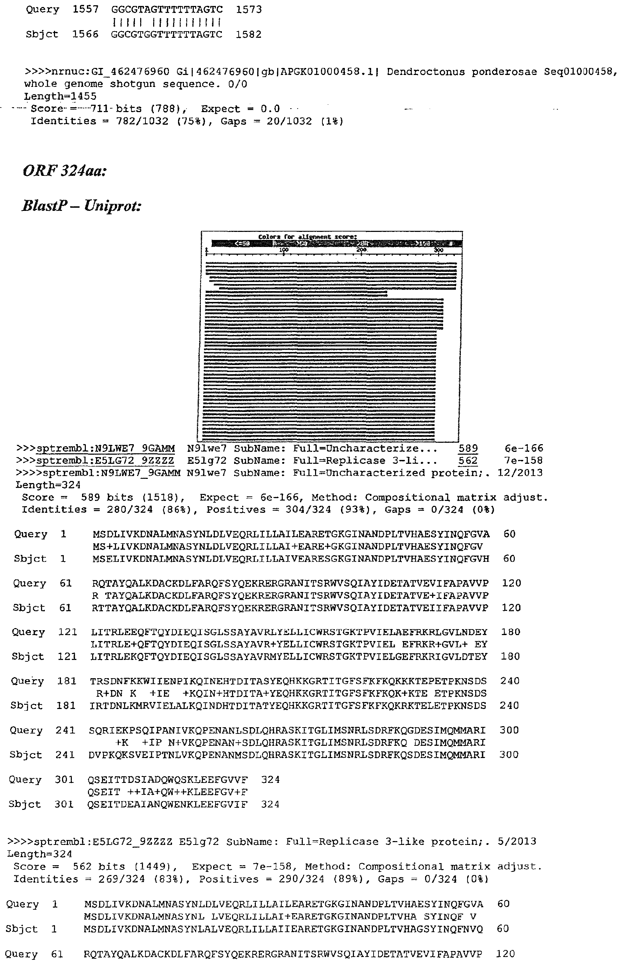

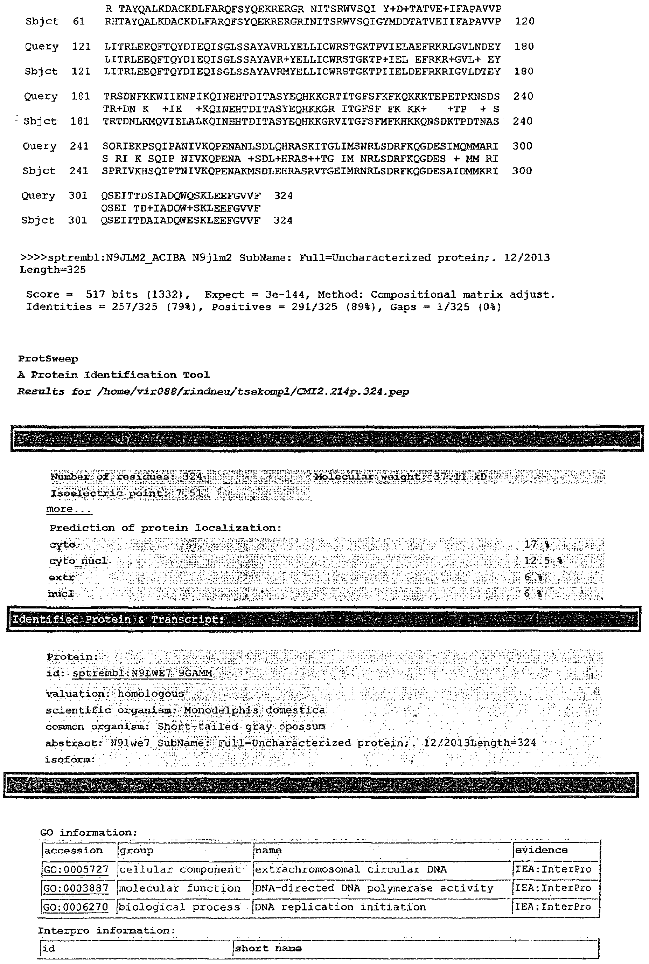

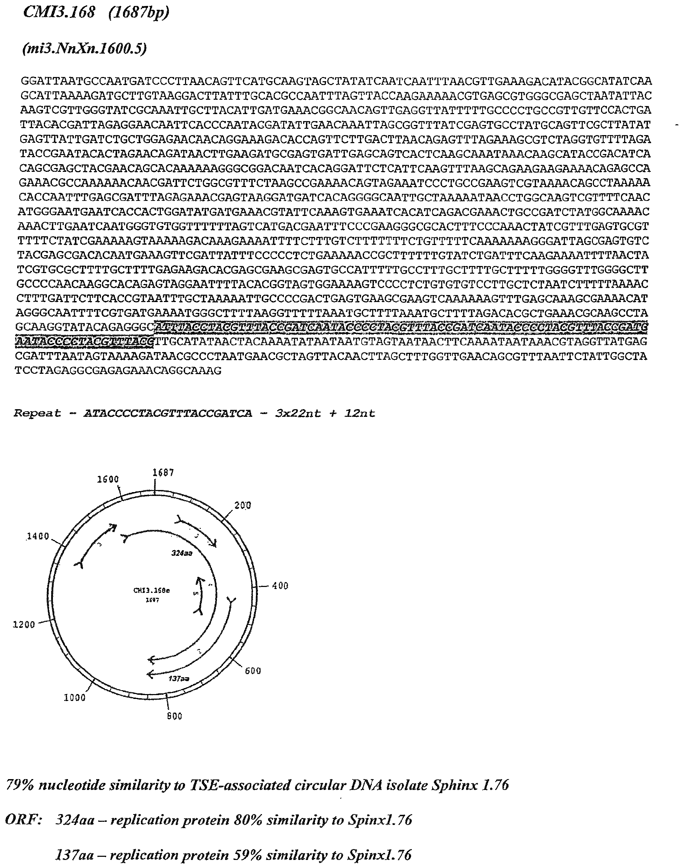

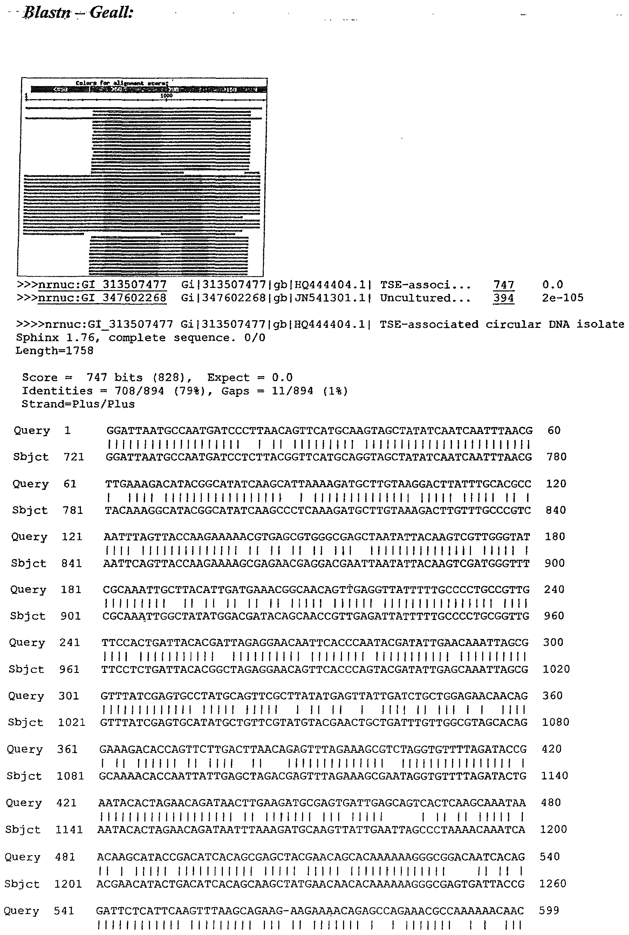

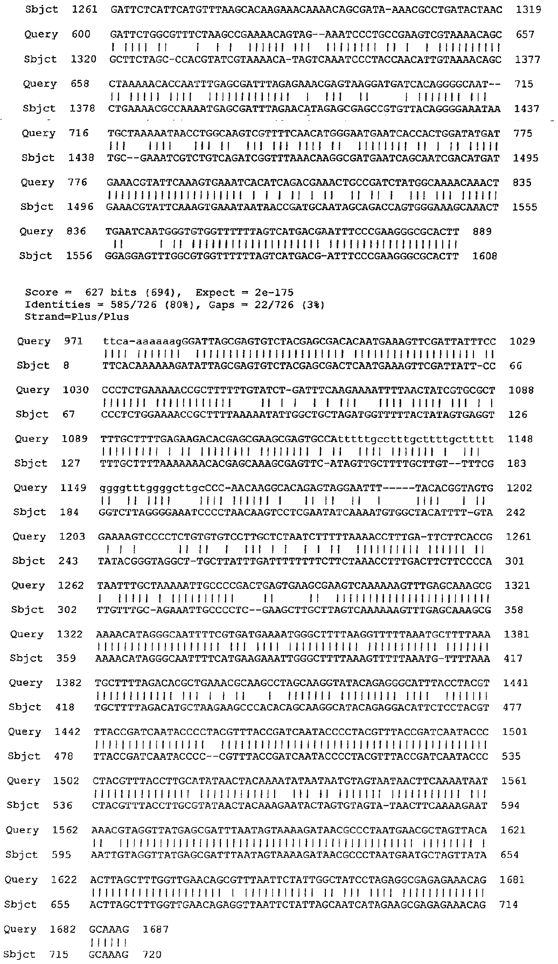

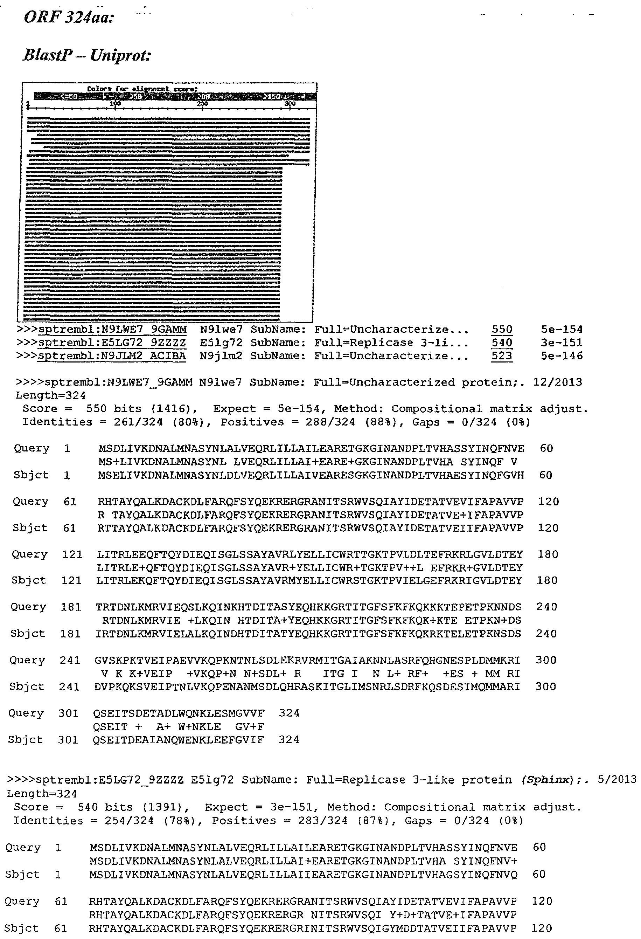

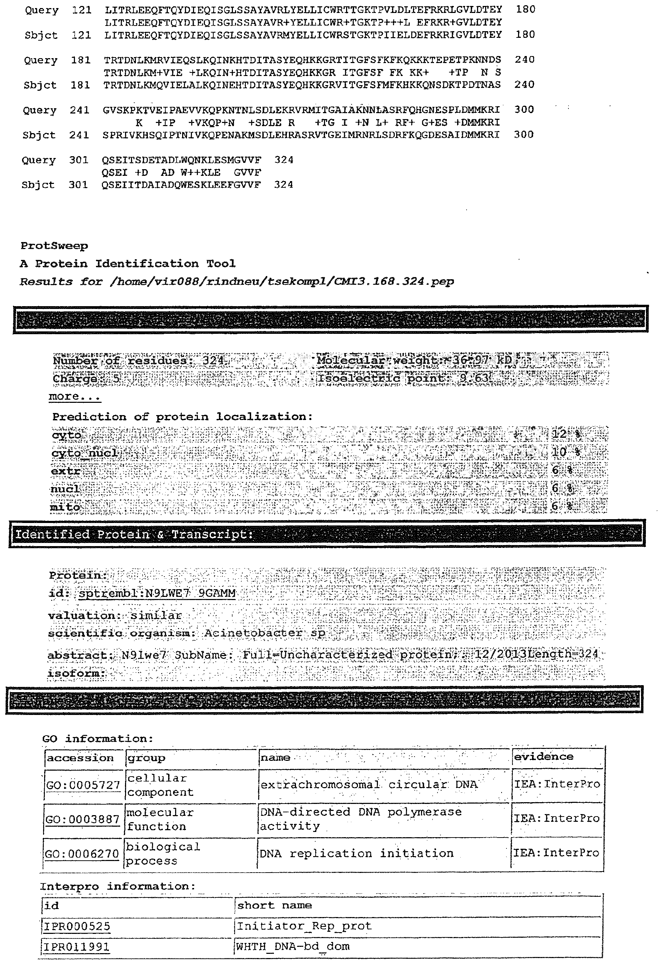

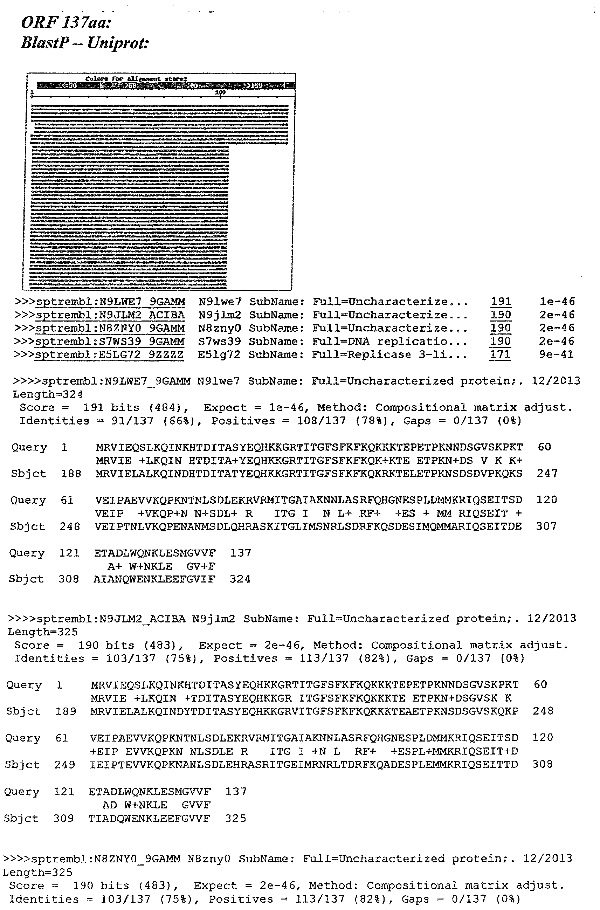

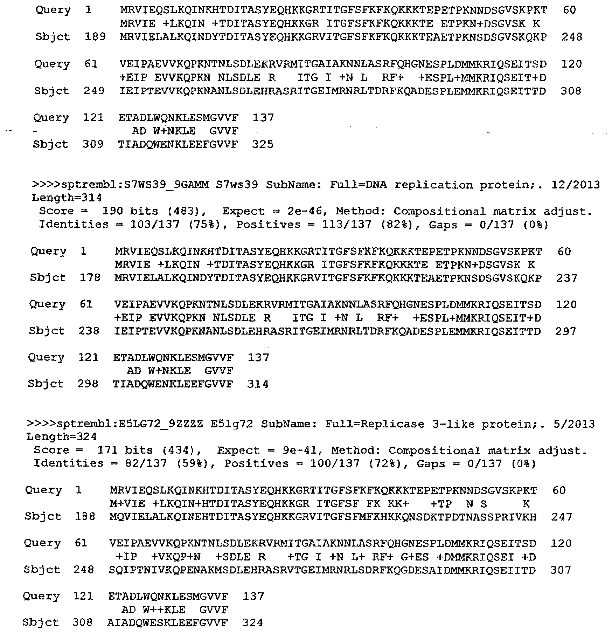

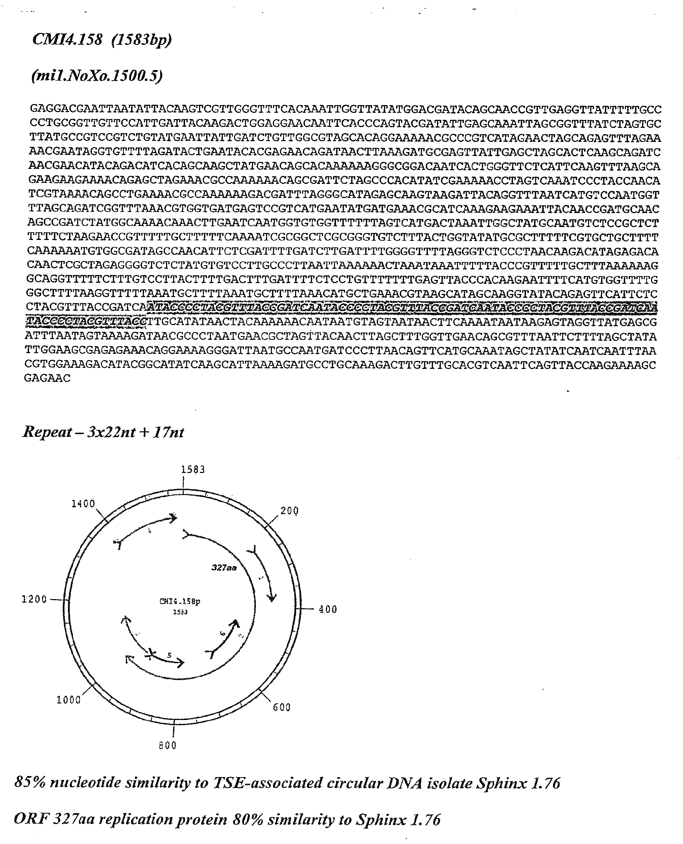

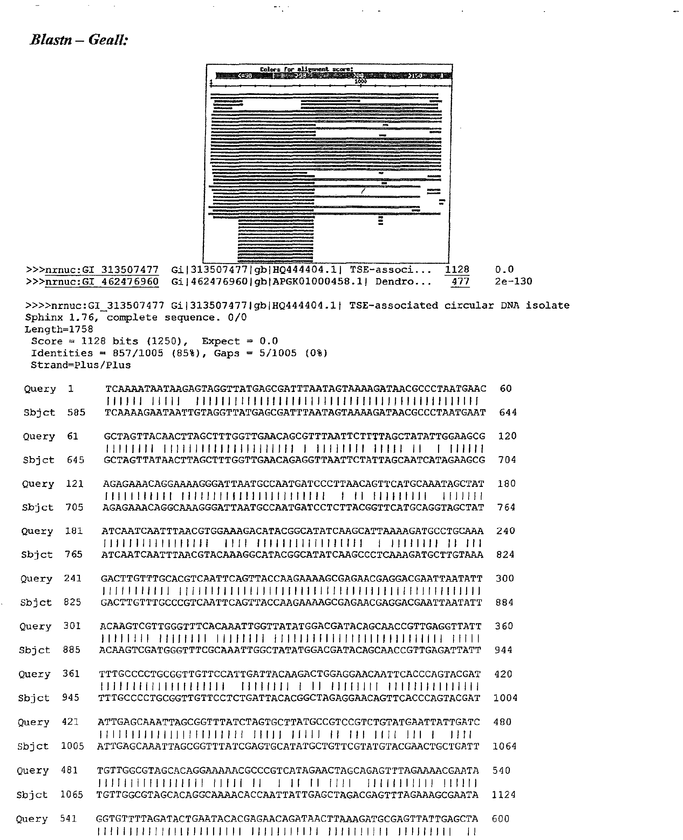

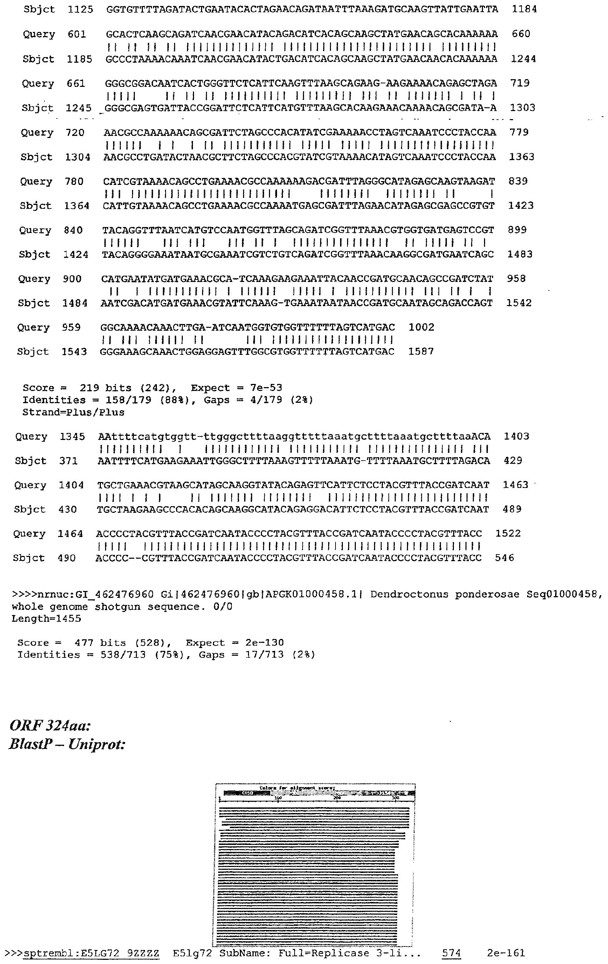

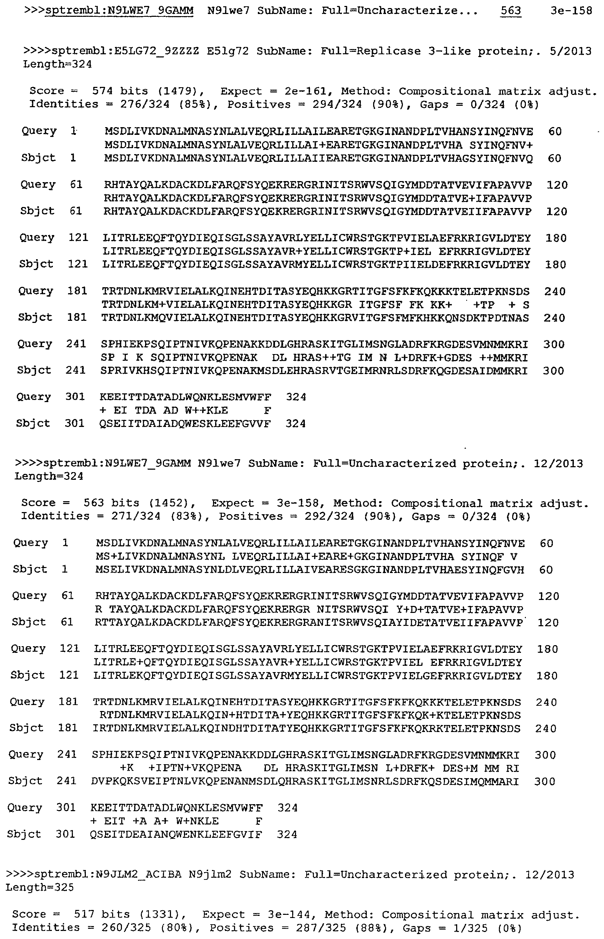

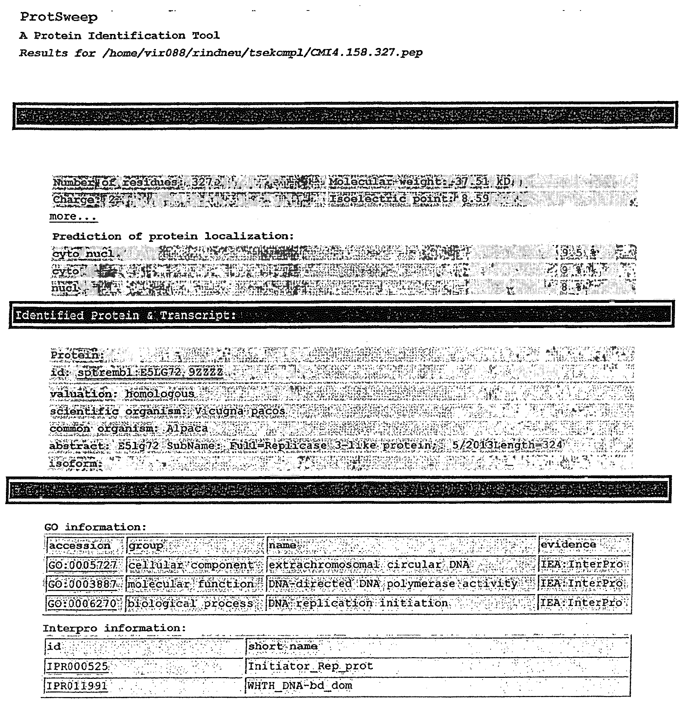

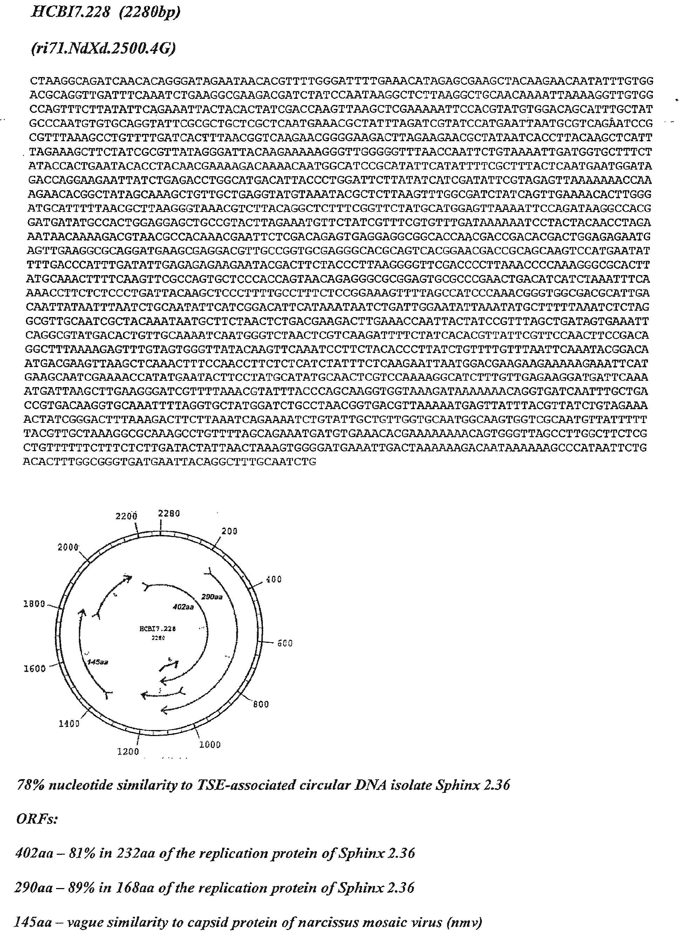

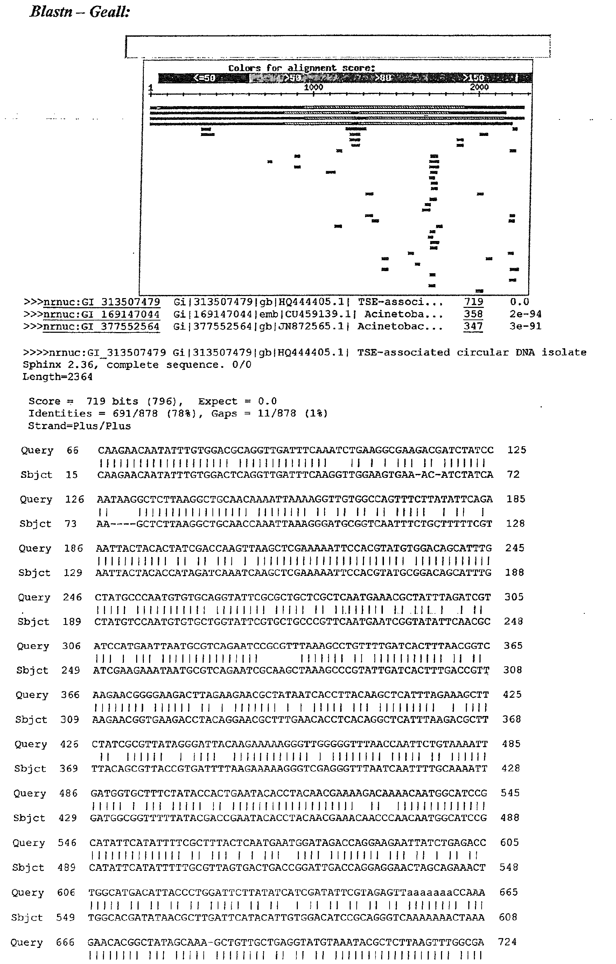

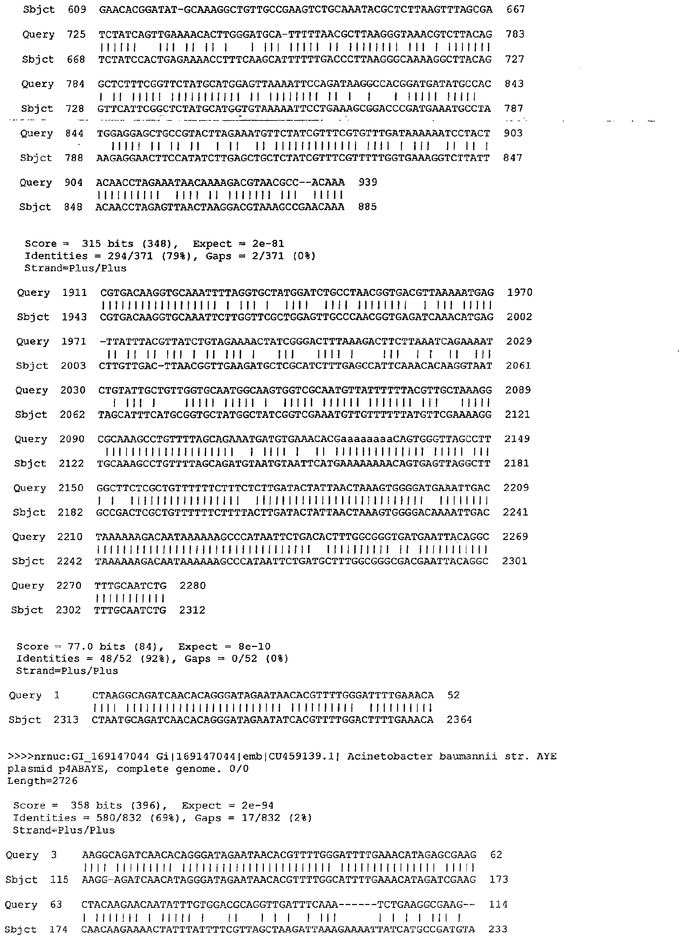

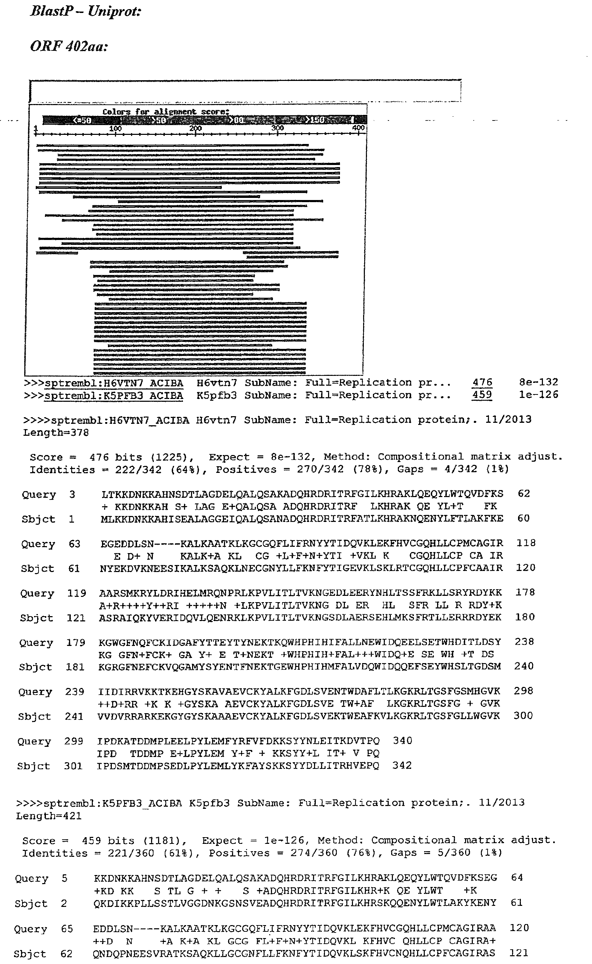

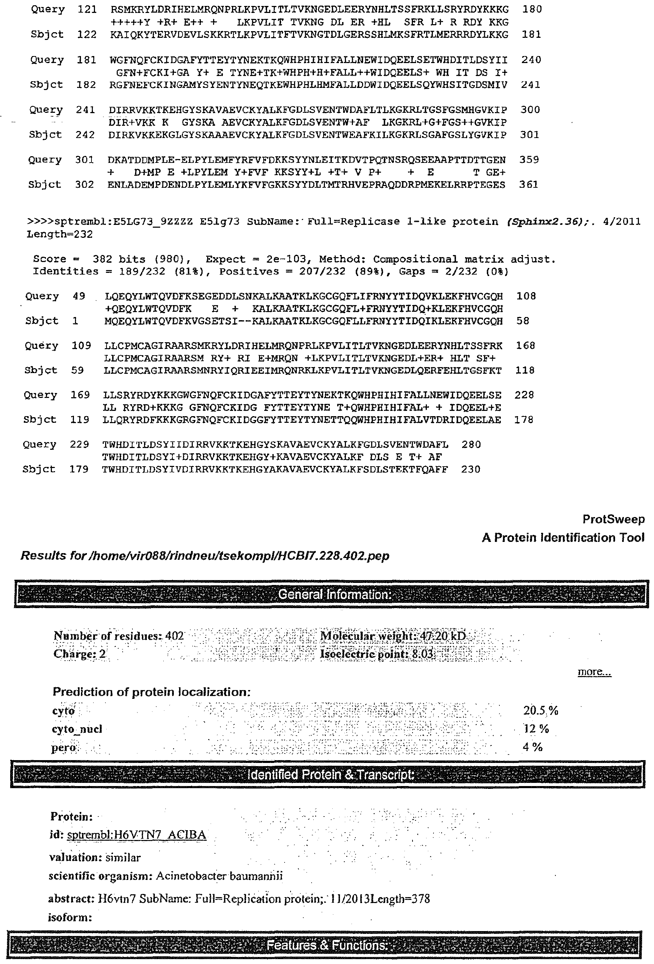

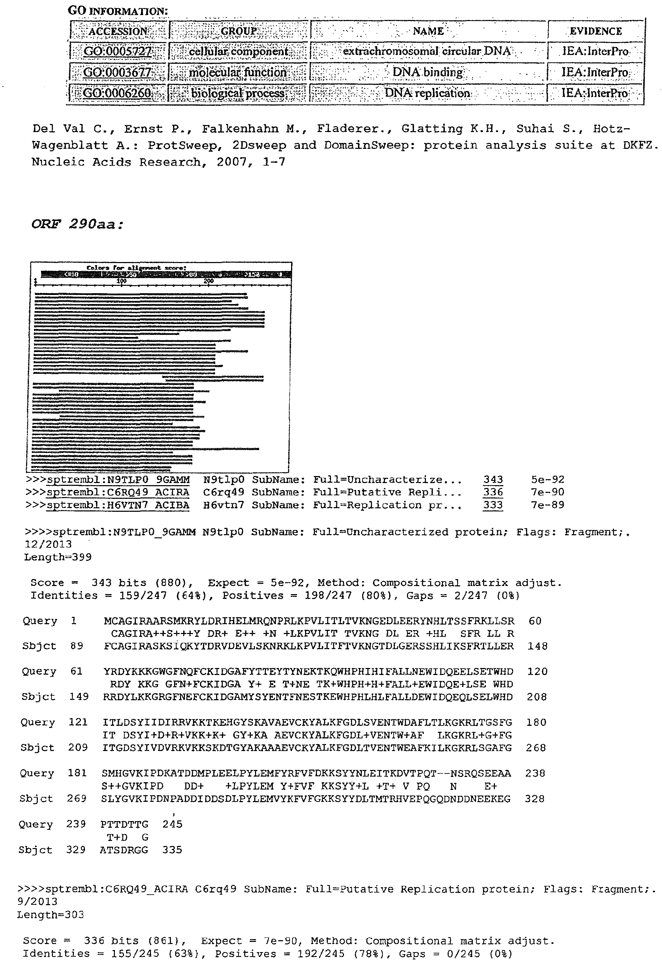

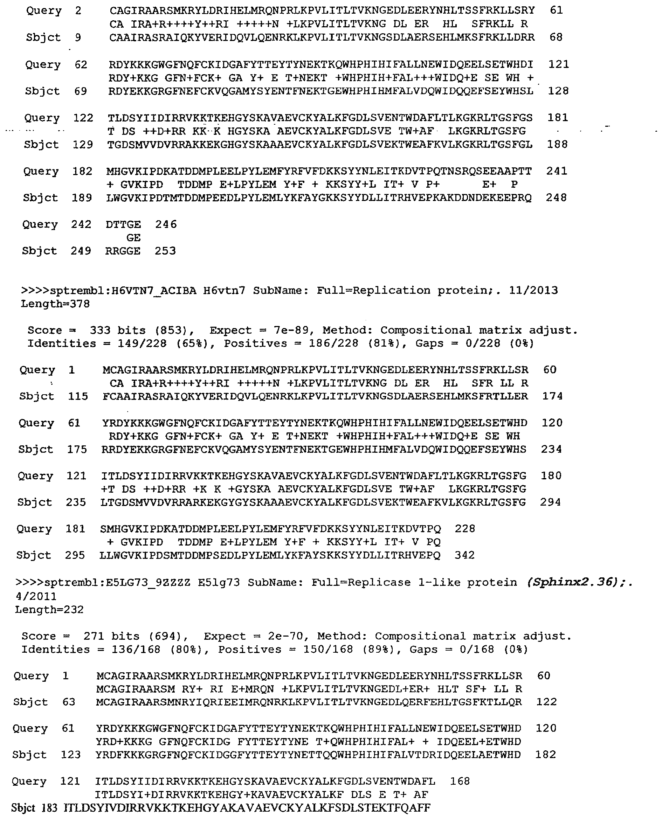

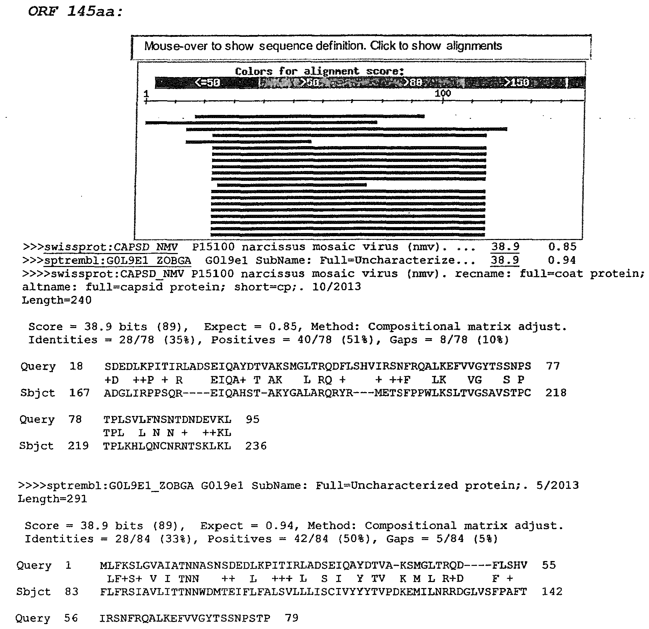

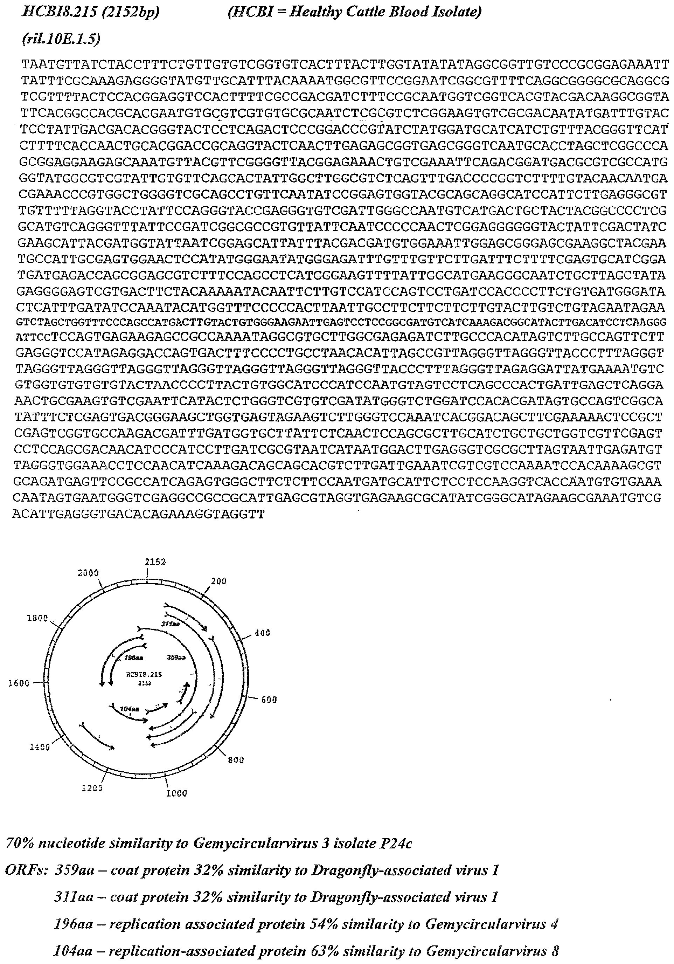

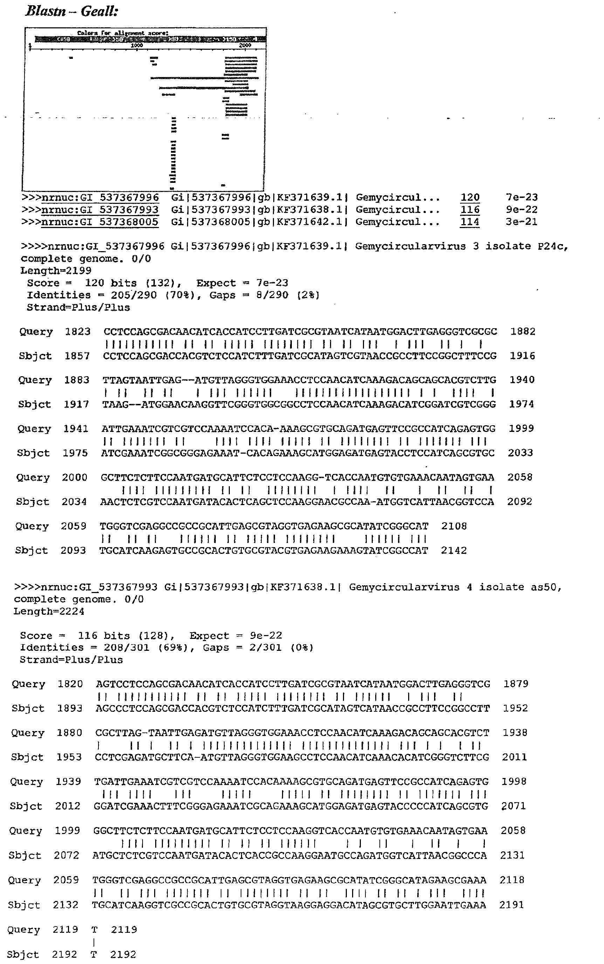

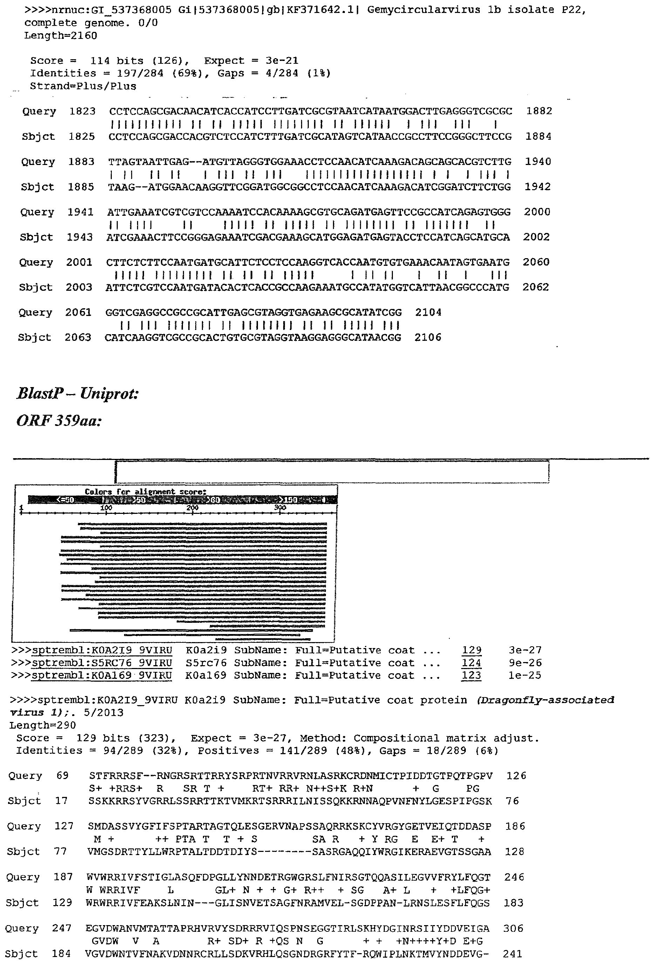

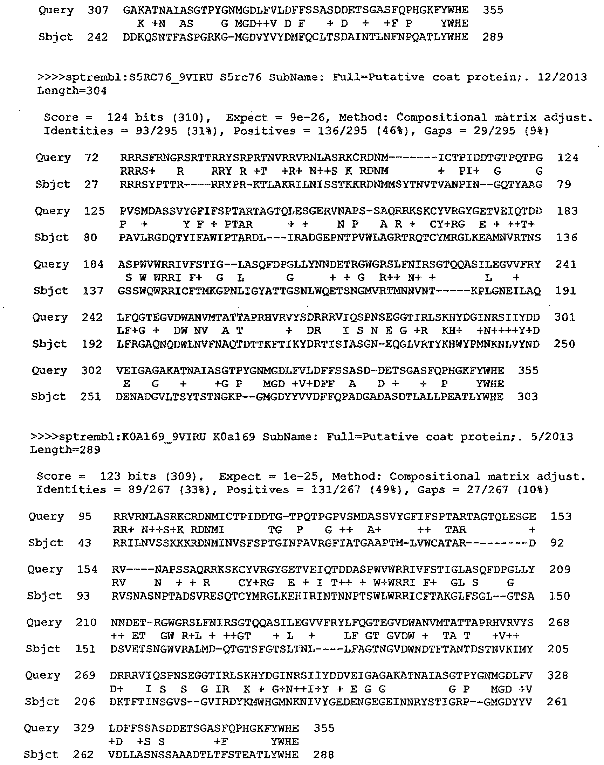

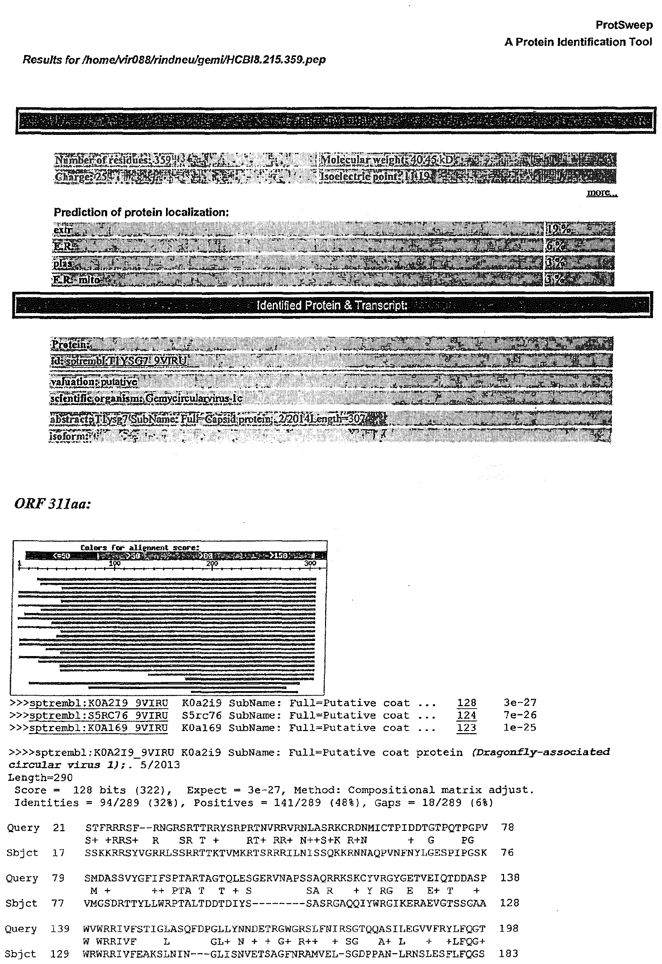

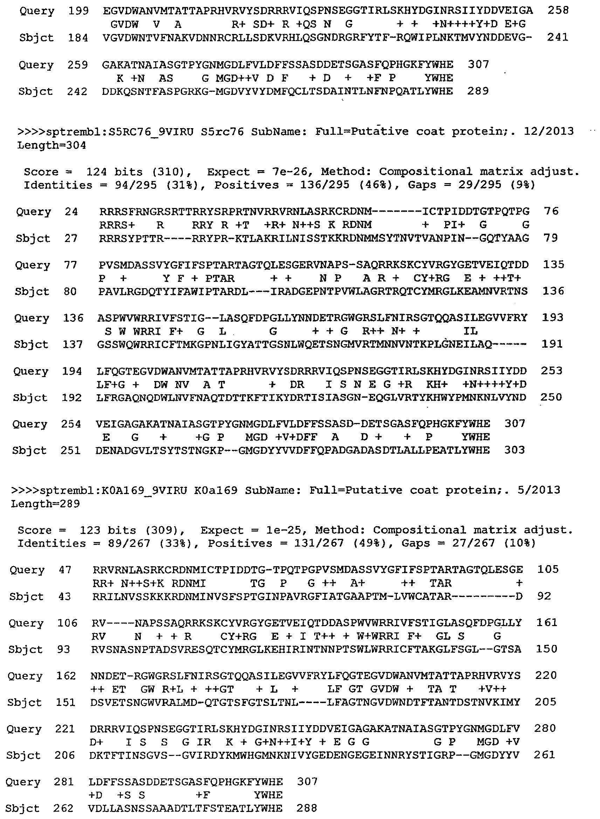

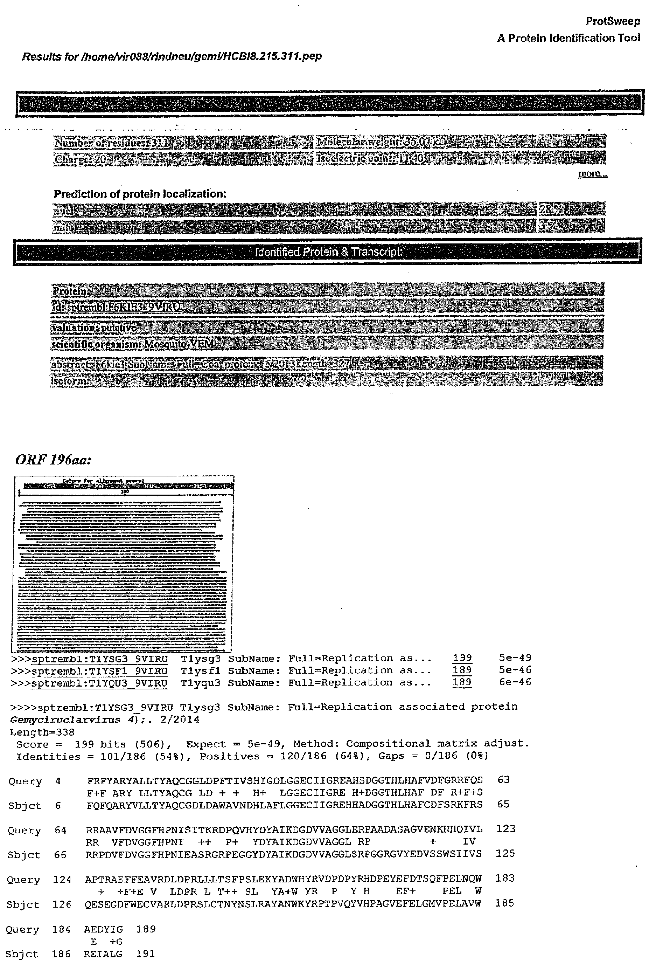

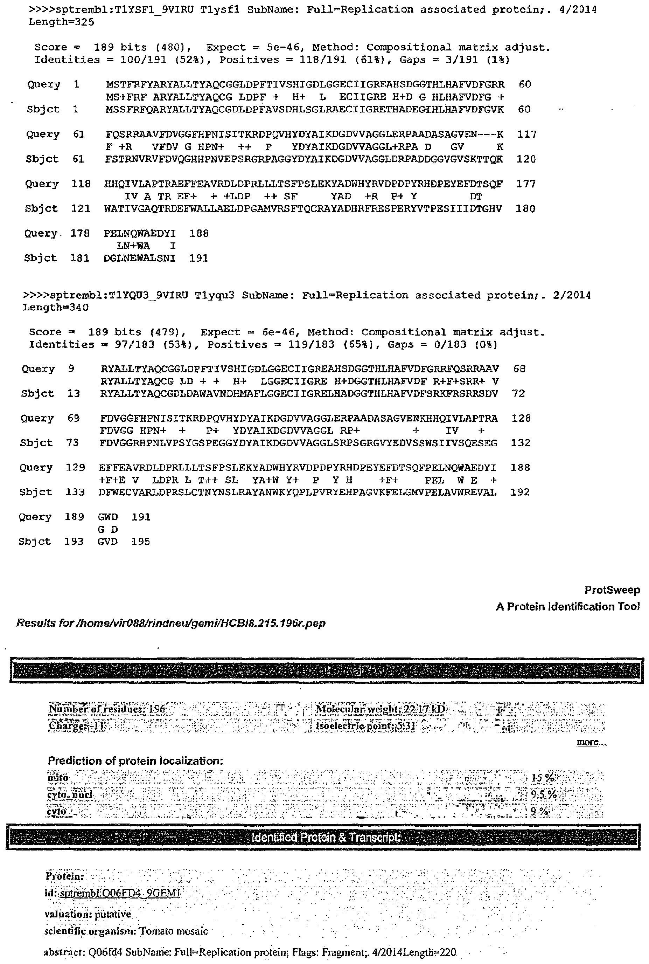

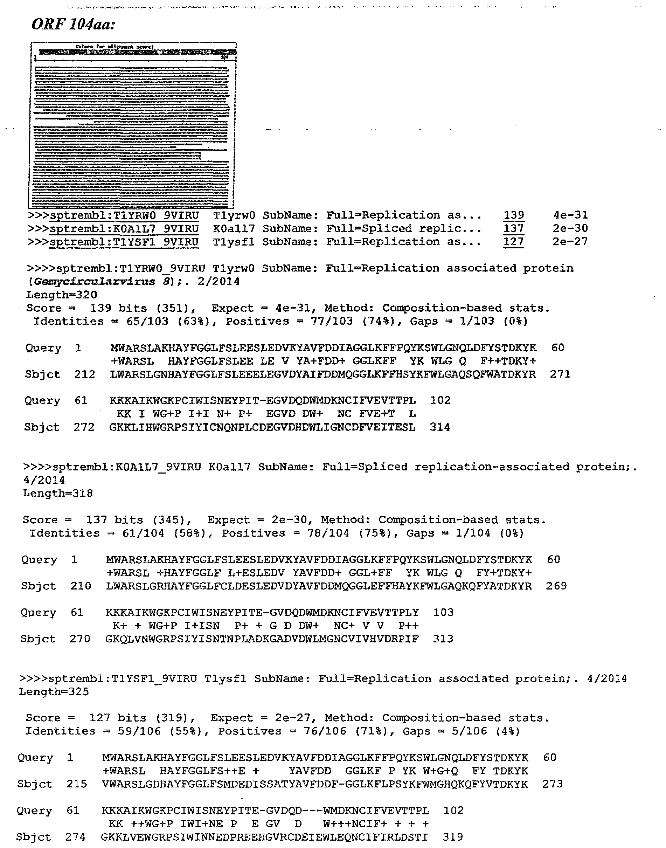

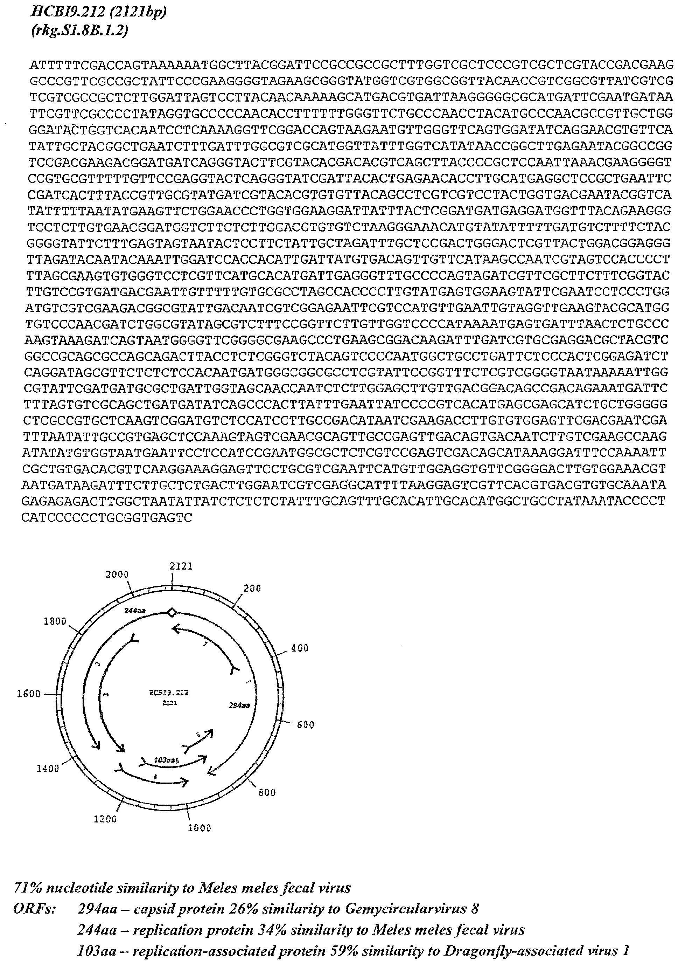

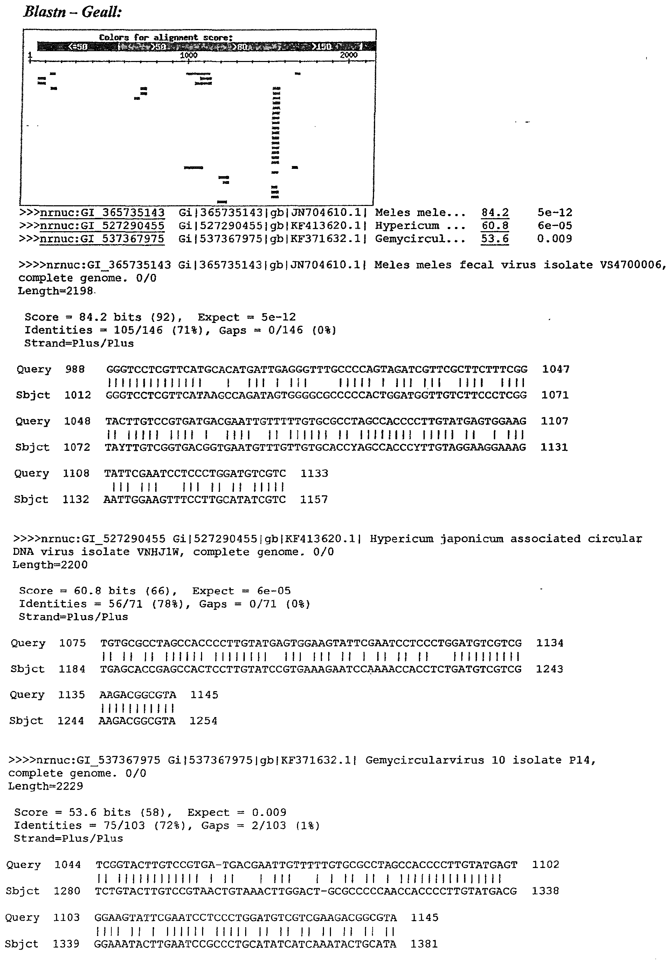

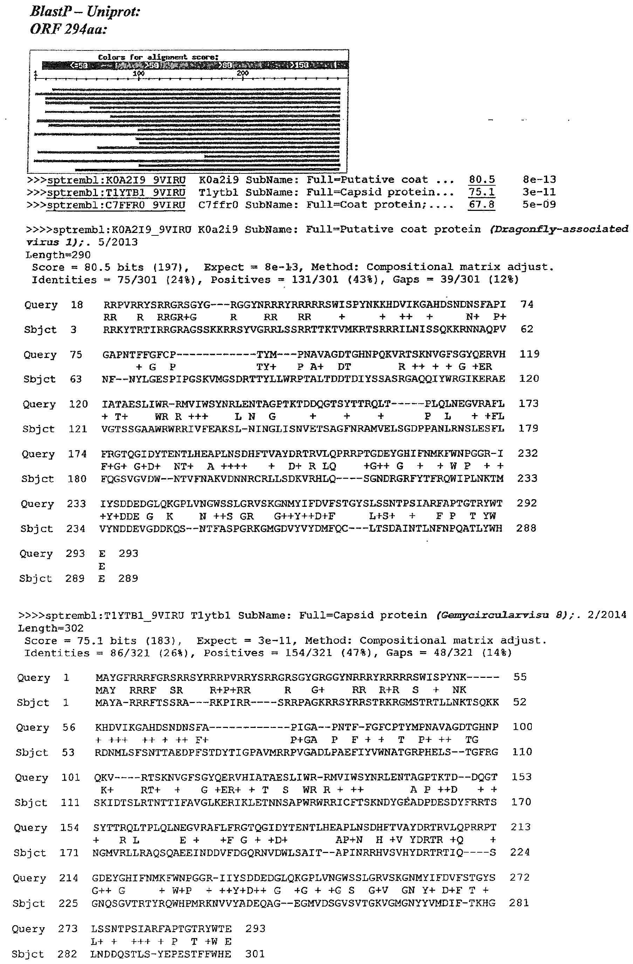

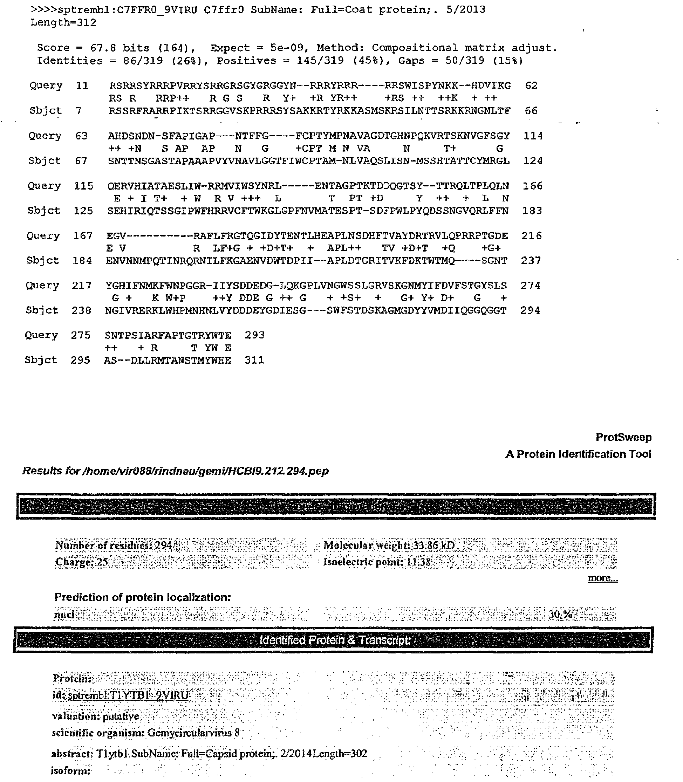

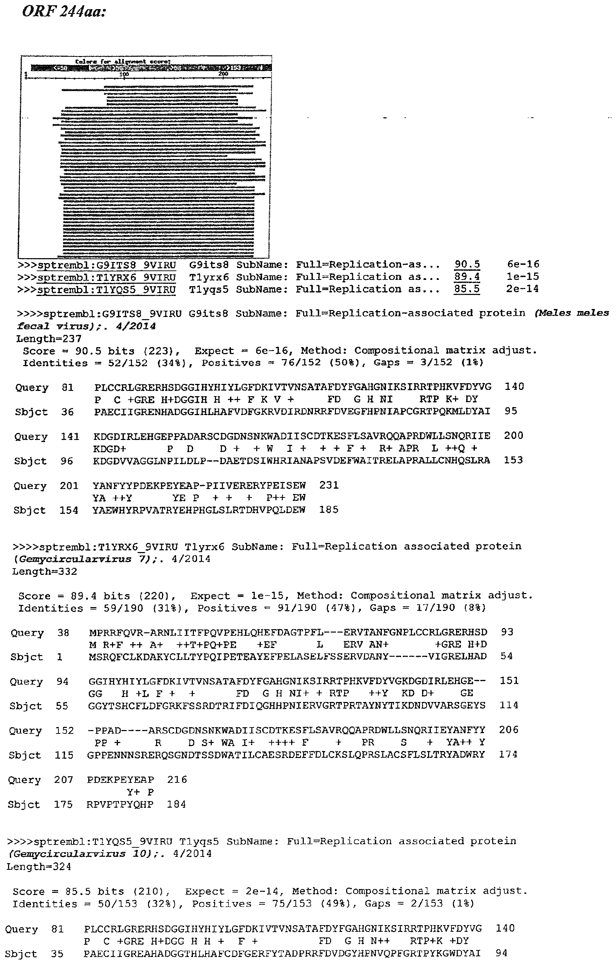

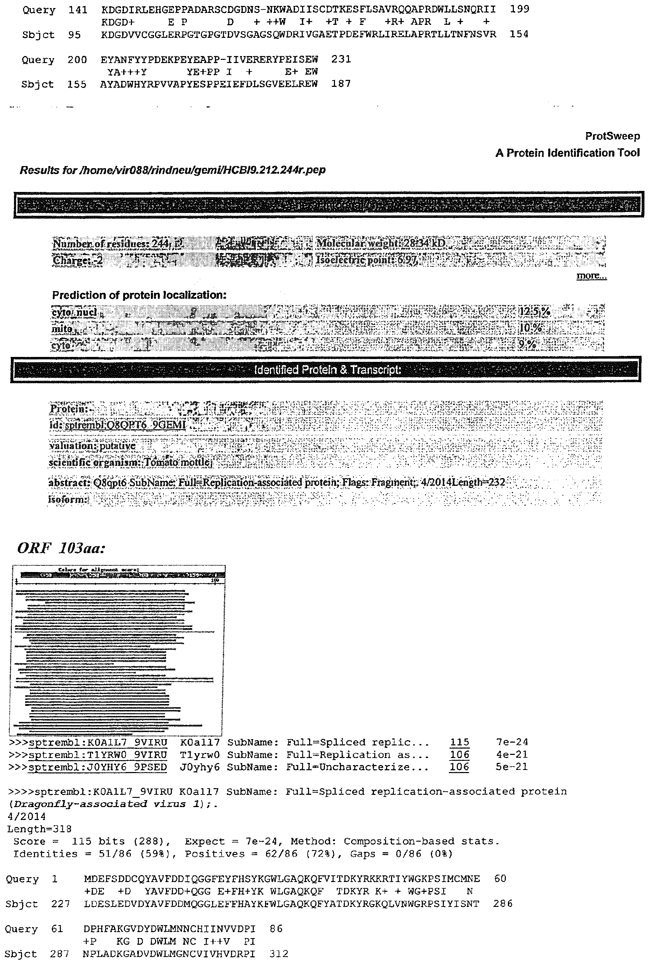

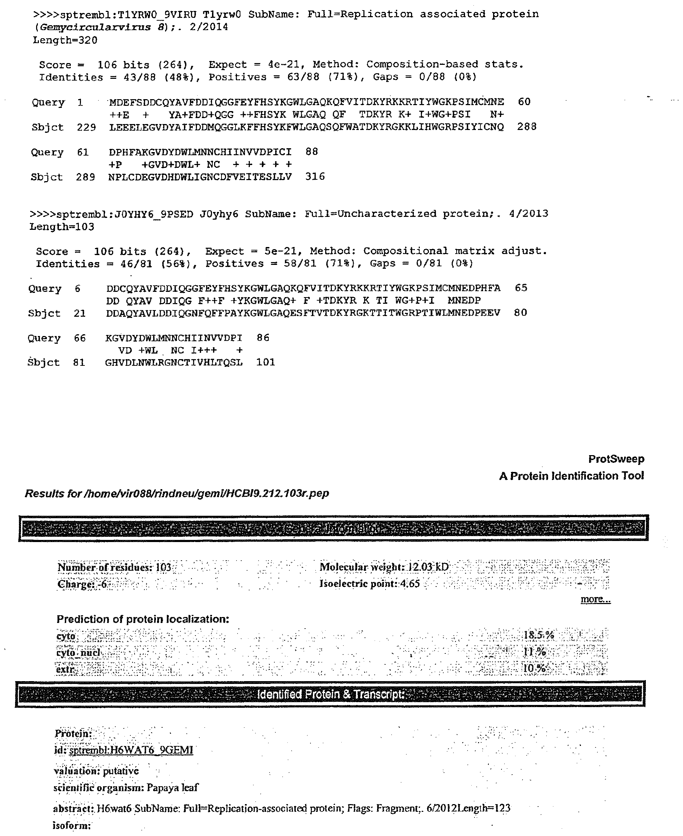

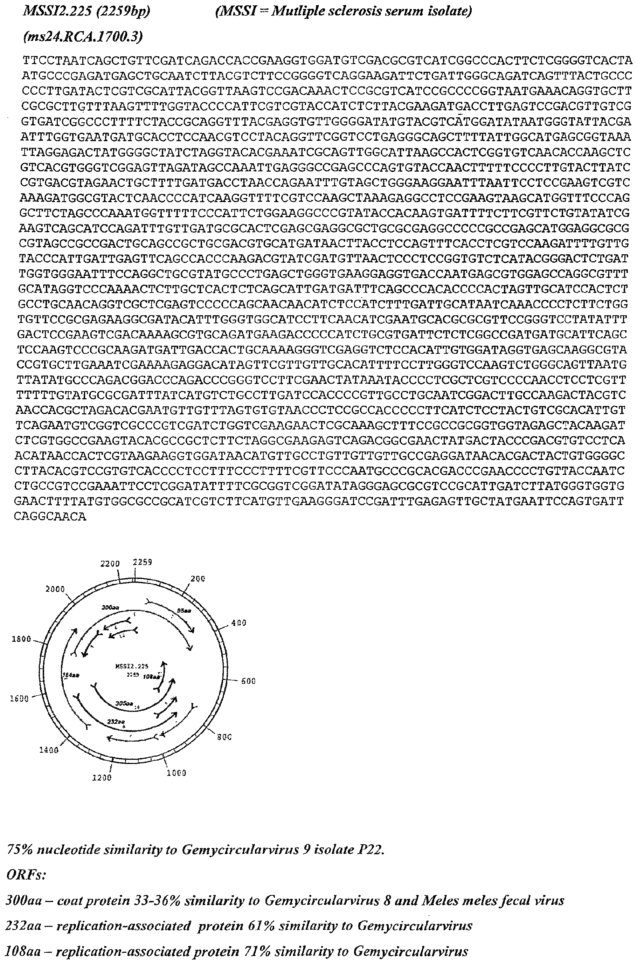

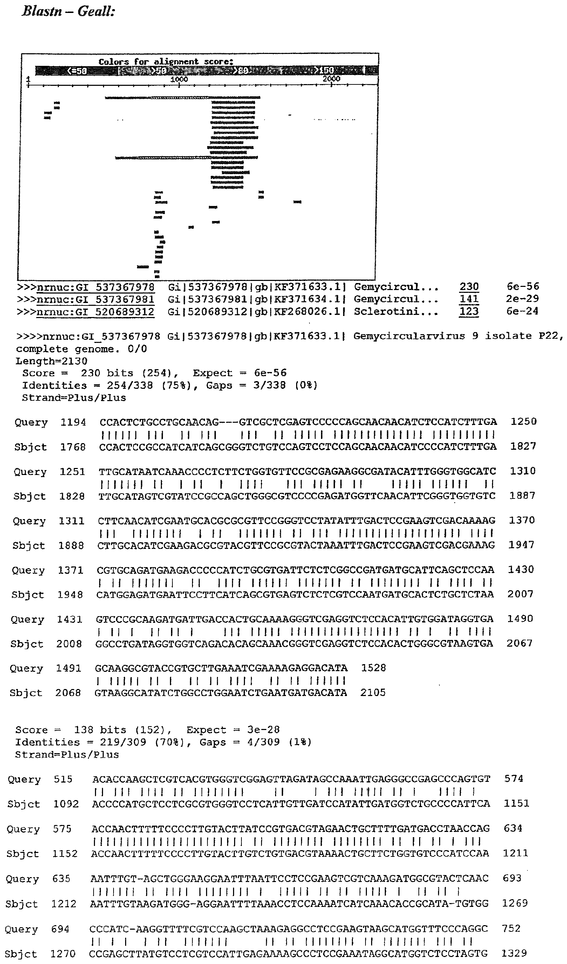

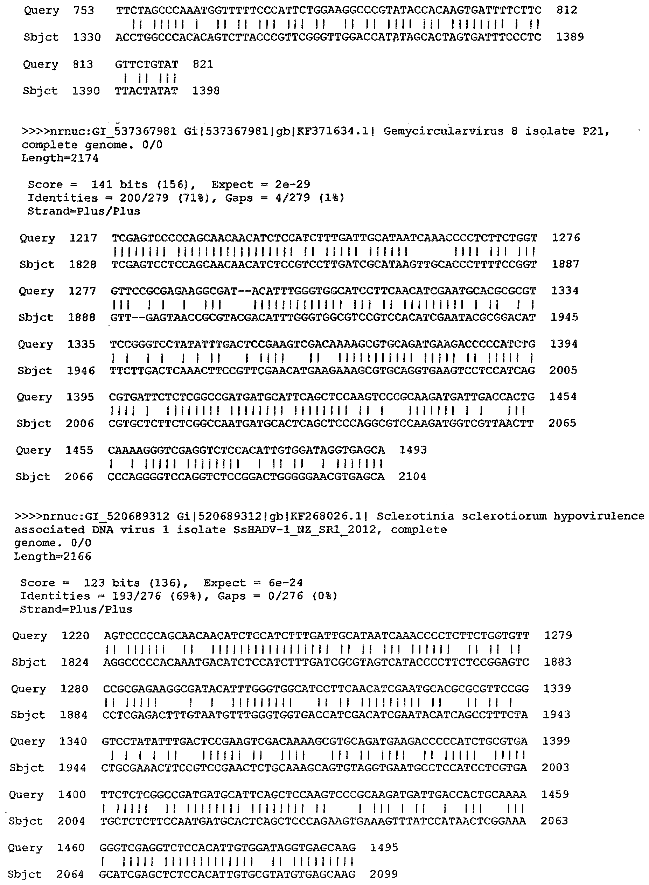

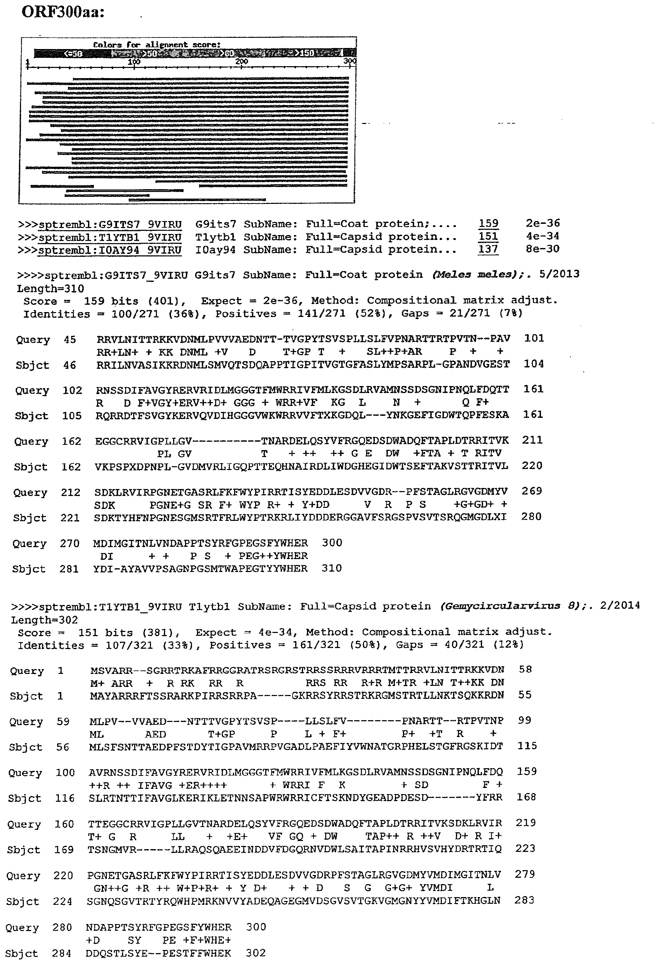

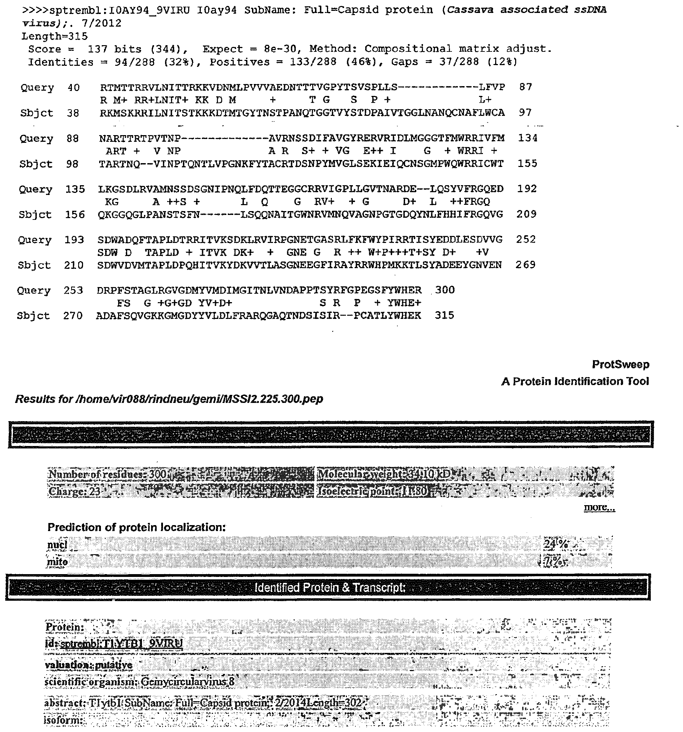

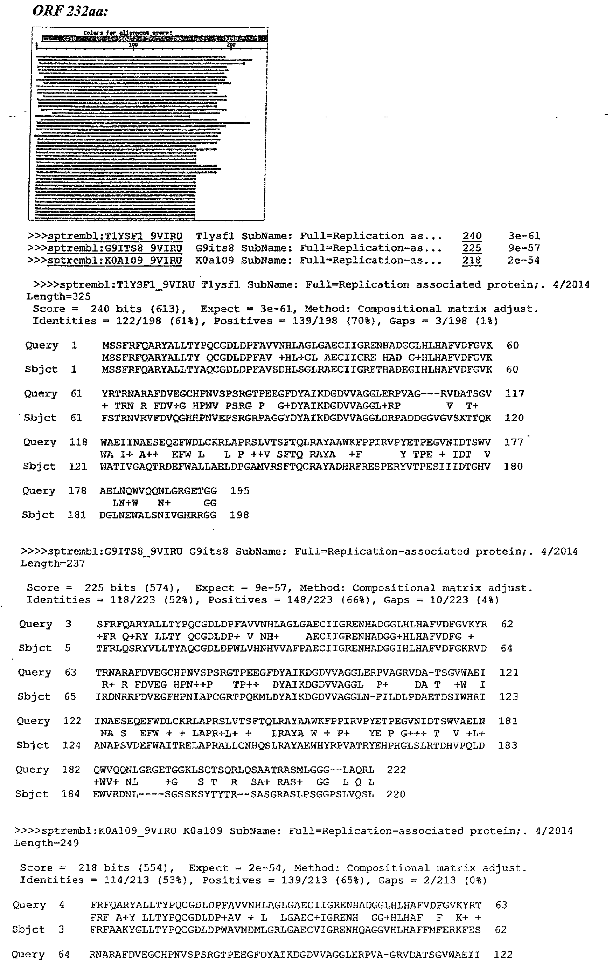

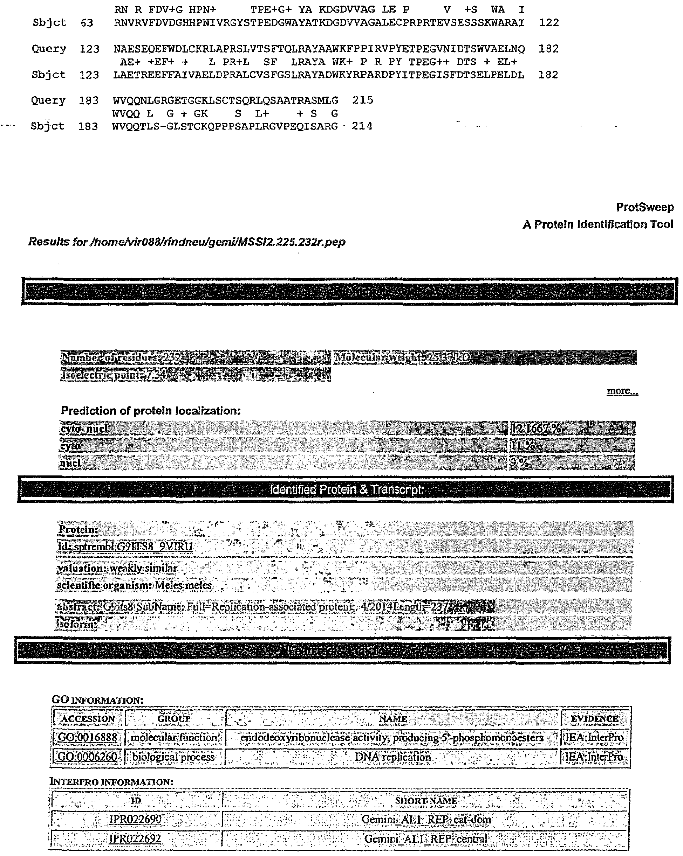

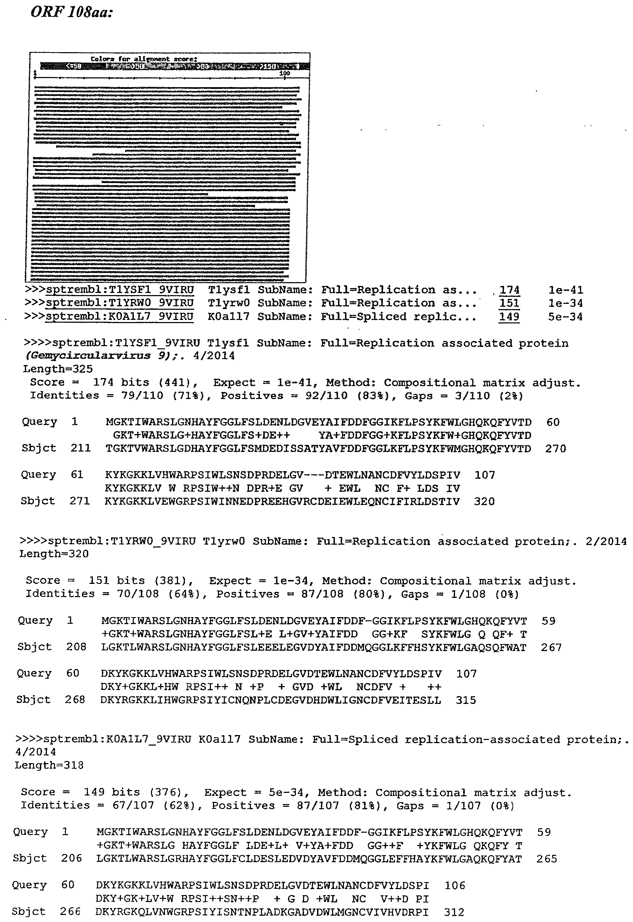

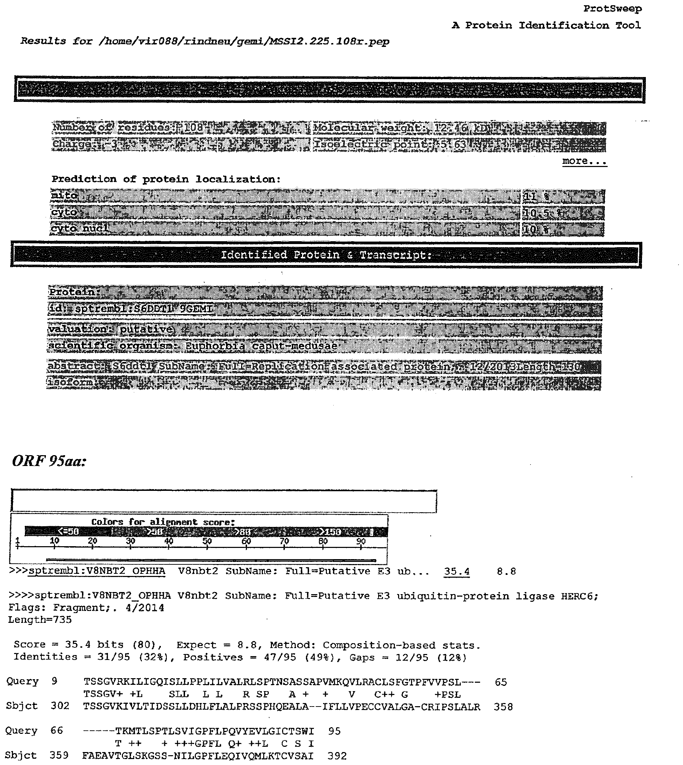

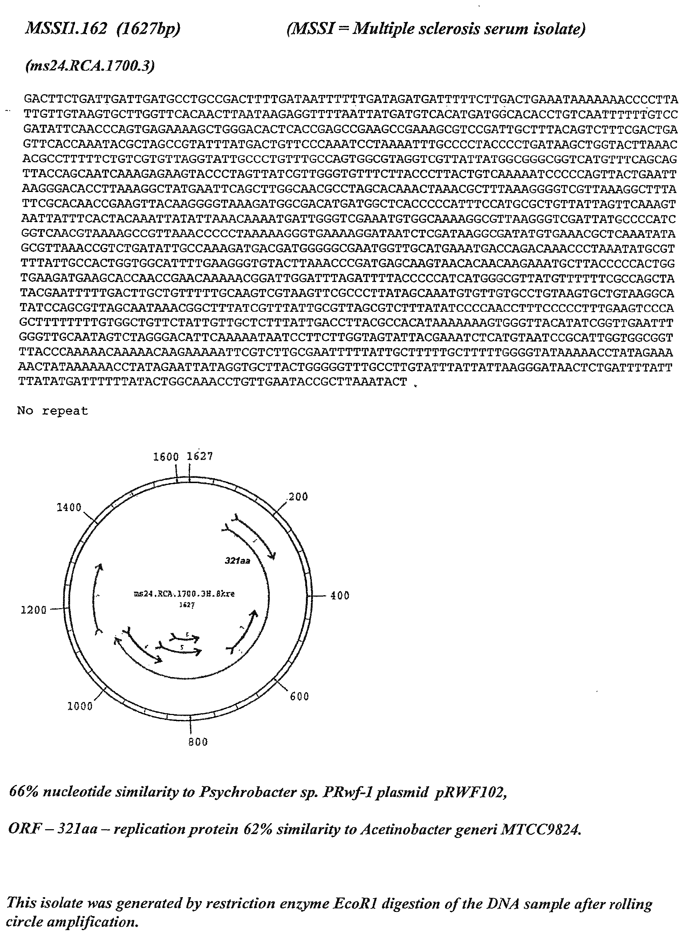

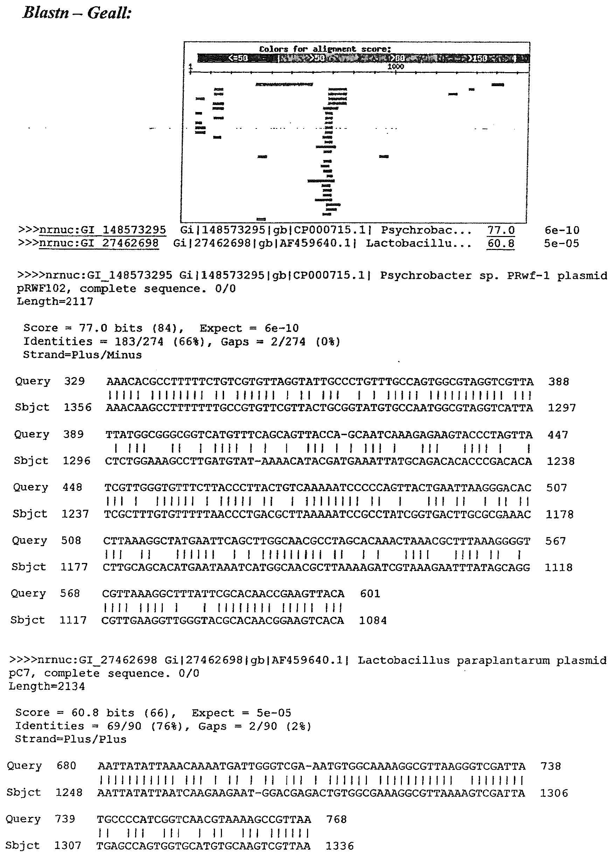

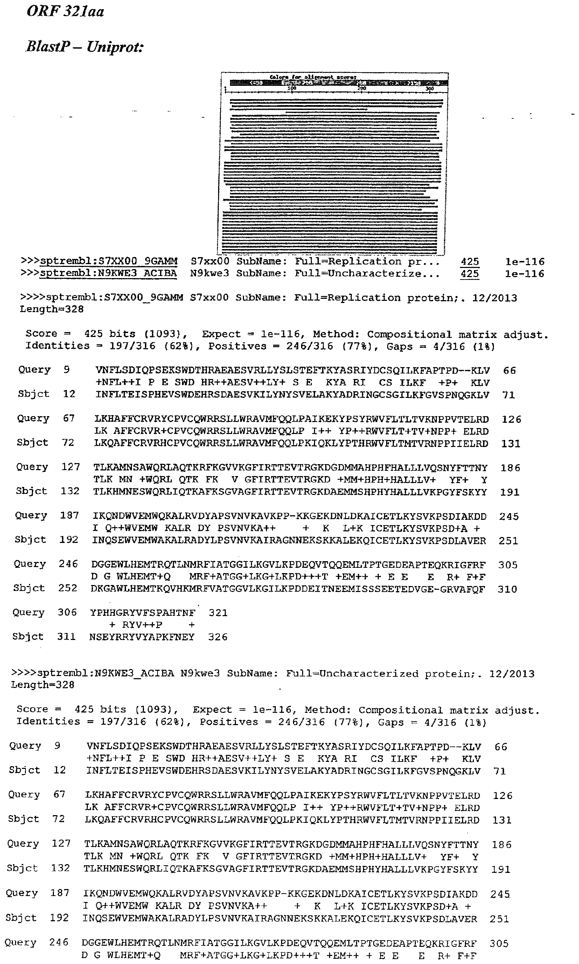

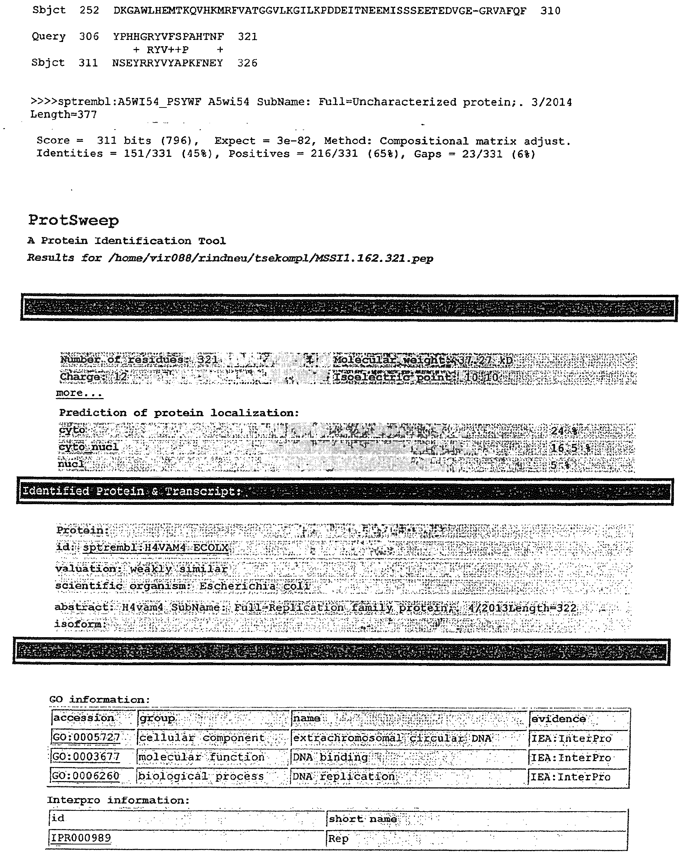

Abbreviations for Figures 1-4: Rep = replication protein; CP = capsid protein
CMI:cattle milk isolate
HCBI: healty cattle blood isolate
MSCI: MS brain isolate
MSSI: MS serum isolate
Sphinx: slow progressive hidden infection of variable (X) latency

### Figure 1:

### Group 1 - Isolates from bovine serum, bovine milk and multiple sclerosis (MS) brain samples (post mortem) - all related to Sphinx1.76

(a) HCBI6.252 (2522 bp) (Healthy Cattle Blood Isolate; identical to CMI1.252 (Cow Milk Isolate)).
(b) HCBI6.159 (1591 bp): A deletion in HCBI6.252 apparently resulted in HCBI6.159 - both were rescued independently from the bovine serum using back-to-back primer, i.e. circular DNA molecules and not an artefact. This could indicate that these infectious organisms have 2 forms - a large and small molecule related to or complementing each other
(c) MSBI1.176 (Multiple Sclerosis Brain Isolate) (1766 bp) - 98% similar to Sphinx1.76
(d) MSBI2.176 (1766 bp) - isolated from the same MS-brain sample as MSB1.176
(e) CMI1.252 (2523 bp) (Cow Milk Isolate; identical to HCBI6.252)
(f) CMI2.214 (2148 bp)
(g) CMI3.168 (1687 bp)
(h) CMI4.158 (1583 bp)

The isolates were all generated by using back-to-back primers designed on the replication gene of Sphinx1.76.

Primers (several isolates were isolated twice by applying both primer pairs independently).

Nn (forward GGATTAATGCCAATGATCC), Xn (reverse CTTTGCCTGTTTCTCT CG), and/or No (forward GAGGACGAATTAATATTACAAGTC), Xo (reverse GTTCTCGTTTTCTTGGTAA)

### Figure 2:

### Group 2: Isolate HCBI7.228 (2280bp)(Healthy Cattle Blood Isolate) related to Sphinx2.36

This sample was generated by PCR from bovine serum using back-to-back primers
Primers: Nd (forward CAGATTGCAAAGCCTGTAATTCAT), Xd (reverse CTAAGGCAGATCAACACAGGGATA)

### Figure 3:

### Group 3: Isolates from bovine serum and multiple sclerosis serum and MS brain samples (post mortem) - distantly related to known ssDNA viruses

Nucleotide sequences of (a) HCBI8.215 (2152 bp) and (b) HCBI9.212 (2121 bp) (Healthy Cattle Blood Isolate) as well as (c) MSSI2.225 (2259 bp) (Multiple Sclerosis Serum Isolate). An identical sequence (MSBI3.224) to MSSI2.225 was also isolated from an MS-brain sample.

These HCBI isolates were generated as follows:
Serum samples were subjected to ultracentrifugation on Optiprep gradient centrifugation. The gradient was fractionated, DNA extracted from fractions and subjected to rolling circle amplification. Products were digested with BamHI or EcoRI enzymes. Products were cloned and sequenced. Complete genomes were obtained by amplification of original DNA samples with back-to-back primers designed on the respective gradient clones. Complete genomes were sequenced in full.

The MSSI isolate (as well as the clone MSBI3.224 from the MS-brain) was obtained after rolling circle amplification of the extracted DNA from MS serum and MS brain, restriction enzyme digestion, cloning of the product and subsequent sequencing. The complete genome was obtained by amplification with back-to-back primers designed on the original clone. The complete genome was sequenced in full.

### Figure 4:

### Group 4 - distantly related to Psychrobacter spp plasmid: Nucleotide sequence of MSSI1.162 and putative open reading frames

MS-serum isolate MSSI1.162 (group 4) was generated by rolling circle amplification of a multiple MS-serum sample. The product was digested with restriction enzyme HindIII, cloned and sequenced.

### Figure 5:

### Genome organisation of 4 groups of isolates

The isolates are grouped according to their sequence similarity to the Sphinx1.76 genome (group 1), Sphinx2.36 (group 2), myco-like Gemycircularviruses (group 3) and Psychrobacter spp. Plasmid (group 4).
(A) group 1
(B) groups 2,3 and 4

### Figure 6:

### Alignment of a replication gene/ iteron-like repeat region between 8 isolates and Sphinx1.76

### Figure 7:

### Schematic outline of latent infection of different types of brain cells with Herpes type genomes and BMF factor

### Figure 8:

### Spontaneous reactivation of Herpes DNA in a cell concomitantly infected by Herpes and BMF DNA

Amplification of BMF and inhibition of Herpes DNA replication.

### Figure 9:

### Schematic outline of the recognition of foreign antigens by the immune system, T cell response

### Figure 10:

### Mononuclear inflammatory cells surrounding a small vein in an early lesion

Lymphocytes, monocytes, plasma cells and occasional macrophages.

### Figure 11:

### Advancing age of the lesion (plaque) on the left with normal white matter on the right

Macrophages are present in the lesion (arrows) and at the interphase.

### Figure 12:

### Schematic outline of the pathogenesis concept for multiple sclerosis

EBV is used as an example for the role of Herpes-type viruses.

### Figure 13:

Tentative Scheme of MS pathogenesis

### Detailed description of the present invention

In the present application a new concept for the pathogenesis of multiple sclerosis and cancer is presented: Interaction of an Amplifying Virus and the amplified DNA of a Bovine Milk (or serum) Factor (BMF)

### Introduction

The incidence of multiple sclerosis (MS) increased in several parts of the world (*reviewed in Kurtzke, 2000, Alcalde-Cabero* et *al., 2013*)*.* This increase has been mainly attributed to environmental factors. Migrants from high to lower risk areas retain the MS risk of their birth place only if they are at least age 15 at migration, frequently interpreted to be due to an infection acquired during early childhood (*reviewed in Kurtzke, 2000).* Clustering of cases and the geographic epidemiology has also been widely discussed: A rising incidence of MS was noted in females linked to urbanization (*Kotzamani* et *al., 2012).*

Demyelinization is a characteristic feature of MS lesions. Four fundamentally different patterns of demyelination were found, defined on the basis of myelin protein loss, the location and extension of plaques, the patterns of oligodendrocyte destruction, and the immunopathological evidence of complement activation (*Lucchinetti et al., 2000, Metz et al., 2014*)*.* At a given time point of the disease the patterns of demyelination were heterogeneous between patients; but they were homogenous within multiple active lesions from the same patient, potentially pointing to different contributing factors.

Two of the risk factors seem to deserve special attention: the relatively consistent results pointing to a possible role of different, predominantly herpes-group viruses, and the consumption of fresh cow milk, potentially including other dairy products (*see below*)*.* In addition, Vitamin D deficiency plays a role as a risk factor.

### - Viral Risk Factors

Apparently all Herpes virus types share two properties which seem to be relevant for the subsequent discussion:
During their persistence in a latent stage, spontaneous reactivation may occur, in part regulated by specific gene functions, partly also by epigenetic mechanisms (*reviewed in Nicoll et al., 2012, Grinde, 2013).* Reactivation may also be triggered by interaction with extracellular cytokines, such as transforming growth factor β.

Spontaneous induction of a lytic cycle has been observed for virtually every human pathogenic Herpes virus type. The high antibody titers against Epstein-Barr virus structural proteins in EBV-positive Burkitt's lymphomas and nasopharyngeal cancers (*reviewed in Henle, W. and Henle, G.,* 1977) may serve here as one example. Reactivations of human Herpes virus type 6, Varicella-Zoster virus, Herpes simplex virus and others are not rare events and may affect a number of different cell types (*Hu Knox et al., 2000*).

A second remarkable property of herpes virus infection represents the amplification of various double- or single-stranded small DNA virus genomes upon infection of cells containing such DNAs in a latent state. This has been noted for human and monkey Polyoma viruses, JC and SV40, for human and bovine Papilloma viruses, as well as for single-stranded Adeno-associated (AAV) and Anello-/TT-viruses (*Schlehofer and zur Hausen, 1990, Heilbronn* et *al., 1993, Borkosky* et *al., 2012).* The Herpes-group viruses used in these studies were Herpes simplex virus, human cytomegalovirus and Epstein-Barr virus. The potential to induce amplification of latent small viral DNA genomes is also shared by Adeno- and Vaccinia viruses *(Schlehofer and zur Hausen, 1990).* The helper effect of Herpes-and adenovirus-induced amplification of parvoviruses has been intensively studied for adeno-associated viruses. The replication of the latter seems to depend on this helper effect but in turn leads to a reduction of Herpes- or adenovirus replication due to the preferential amplification of small viral DNA (*Schlehofer et al., 1983, Matz* et *al., 1984, Bantel-Schaal and zur Hausen, 1988, Schmitt et al., 1989, Schlehofer and zur Hausen, 1990, Heilbronn et al., 1990a, Heilbronn* et *al., 1990b).*

Spontaneous induction of Herpes-group viruses and the amplification of latent small viral DNA form the basis for the subsequent postulation of the mechanism underlying MS development.

### - Bovine milk factor

Several reports noted a correlation between consumption of non-pasteurized cow milk and MS development (*Murray TJ. 1976, Sepcić* et *al., 1993, Malosse and Perron, 1993),* whereas others stressed long-time consumption of cow milk as a risk factor, in particular when consumed in the early phase of life (*Agranoff and Goldberg, 1974, Christensen, 1975, Warren, 1984, Butcher, 1976, 1986, Winer et al., 2001, Munger et al., 2011a).*

If a specific factor in cow milk exists which increases the risk for MS development, one can anticipate a protective role of long-term breast-feeding. Long-term breast-feeding (for six months and more) has indeed repeatedly been reported as having a protective effect for MS development (*Christensen, 1975, Warren, 1984, Tarrats et al., 2002, Conradi* et *al., 2013*)*.* The existence of a cow milk factor would also not exclude a specific genetic predisposition for the development of MS. A monogenic predisposition for MS has been reported in a chromosomal localization close to BRCA1 (*Holzmann et al., 2013)* .

### - Vitamin-D deficiency

A role of vitamin-D deficiency has repeatedly been implicated for the initiation of MS (*reviewed in Ascherio* et *al. 2012, 2013)* .

A convincing relationship between vitamin D deficiency and Epstein Barr Virus reactivation originates from early studies on EBV reactivation by transforming growth factor beta (TGF-β). A serum factor, purified and labeled as Epstein-Barr virus-inducing factor (EIF) (*Bauer* et *al.,* 1982) proved to be identical to the subsequently described TGF-β molecule (*Frolik* et *al., 1983, Bauer* et *al., 1991).* TGF-β in turn is negatively regulated by activated vitamin D receptors *(Isik* et *al., 2012, Ito et al., 2013, Zerr et al., 2014).* This could very well explain the season-related preferential onset of MS and of exacerbations.

The relationship between low vitamin D and EBV reactivation is further supported by studies describing a correlation between low vitamin D and elevated immunoreactivity against Epstein-Barr virus prior to the clinical manifestation of multiple sclerosis (*Munger et al., 2011b, Décard et al., 2012*) and a higher rate of EBV excretion of EBV-positive MS patients in comparison to EBV-positive healthy controls (*Yea et al., 2013*)*.*

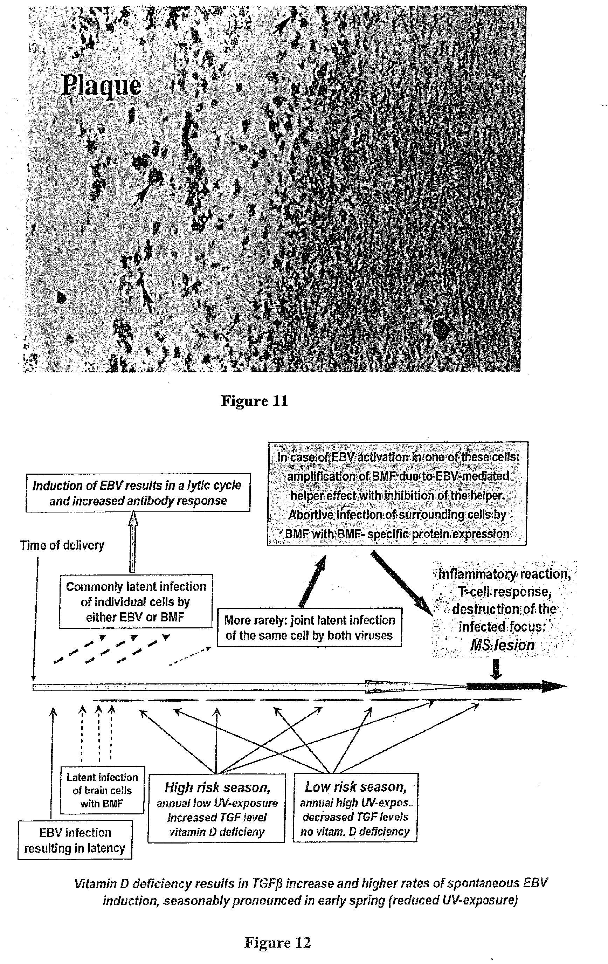

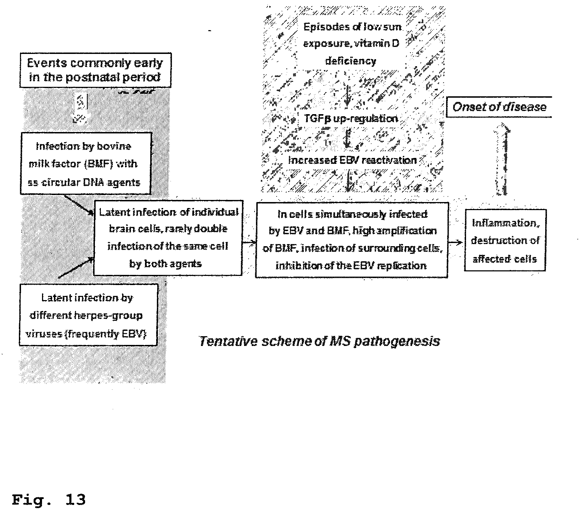

Thus, at least two factors have been implicated as potential etiological contributors for both diseases of the CNS (e.g. MS) and cancer (e.g. colon and breast cancer): vitamin D deficiency and the reactivation of various herpes group viruses, mainly Epstein-Barr virus (EBV), human herpes virus type 6, and varicella-zoster virus. According to the present invention the identification of several novel types of small circular single-stranded DNAs, presumably of viral origin, from cattle sera of the present invention and commercially available dairy products, show a unifying concept. The inventors have demonstrated in the present invention that co-infection of cells with herpes-group viruses and small single-stranded or double-stranded DNA viruses results in an substantial amplification of small viral DNA with partial inhibition of the herpes virus. Some of the molecules identified in dairy cattle sera and milk are distantly related to DNA reported in prion-linked brain lesions and have been found in two autopsy lesions of patients with multiple sclerosis. The amplification of these single-stranded DNA molecules by reactivation of a co-latently persisting herpes virus genome could result in their amplification and evoking a local immune response resulting in destruction of the affected brain cells. This model could in part explain the North-South incidence gradient of multiple sclerosis, which is thought to be linked to vitamin-D deficiency and herpes virus reactivation (c.f. Fig. 12 and 13).

In addition, the full-length genomes of the isolates from MS brains and sera were isolated and re-circularized before transfection into the human cell line 293TT. Transfected cells were harvested on day 3 and total RNA extracted using the miRNA Easy Kit (Qiagen). Samples were further purified (DNase digestion, ribosome removal) and subjected to high throughput RNA sequencing. RNA transcripts have been obtained for MSBI1,176, MSBI2,176 (Group 1), MSSI2.225 (Group 3) and MSSI1.162 (Group 4). The RNA transcripts clearly show that the isolates replicate in human cells.

The inventors consider Vitamin D deficiency and herpes virus reactivations as risk factors also for breast and colon cancers. Reactivation of dual latent infections within the same cell, outlined above for multiple sclerosis pathogenesis, could therefore also play a particular important role in the aetiology of these cancers.

Thus, it is considered by the inventors that multiple sclerosis (MS) and also the other below mentioned diseases result from
- Latent infections of the same cell with two different infectious agents, one of them most likely a herpes-type virus(in particular EBV, HHV-6, VZV, but also HSV, HHV-7), the other one acquired by bovine milk consumption (bovine milk factor - BMF) as the first event. Each of them latently infects individual cells, but occasionally genomes of both agents occur within the same cell.
- Reactivation of the herpes-type virus most frequently, but not only, Epstein Barr virus (EBV) to a lytic cycle as a second precondition. For EBV this is probably linked to increased levels of transforming growth factor β (TGF β) which is negatively regulated by activated vitamin D receptors;
- As third event, amplification of BMF, resulting in partial suppression of Herpes-type DNA synthesis and formation of BMF particles or spreading of its nucleic acid to neighbouring cells via neuronal interconnections;
- This is followed by an infection of neighbouring cells with expression of BMF protein;
- Finally, T-cell response against BMF leads to the destruction of affected cells and in case of MS to plaque formation. This supports the clinical observation of the focal appearance of lesions, commonly starting from a central vein and the intensive localized immune response in early lesions.

Transmissions of agents present in milk or dairy products may lead to latent infections in human brain cells followed by amplification of these agents in case of co-latency and spontaneous induction of a Herpes virus DNA or Herpes virus - like DNA. Potential BMF candidate agents are described in Examples 2-5 and the accompanying figures.

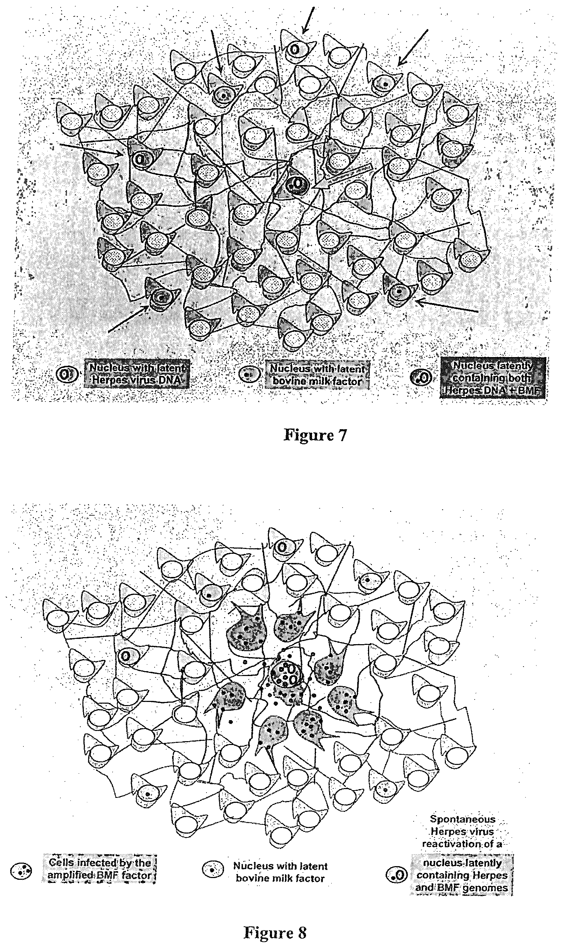

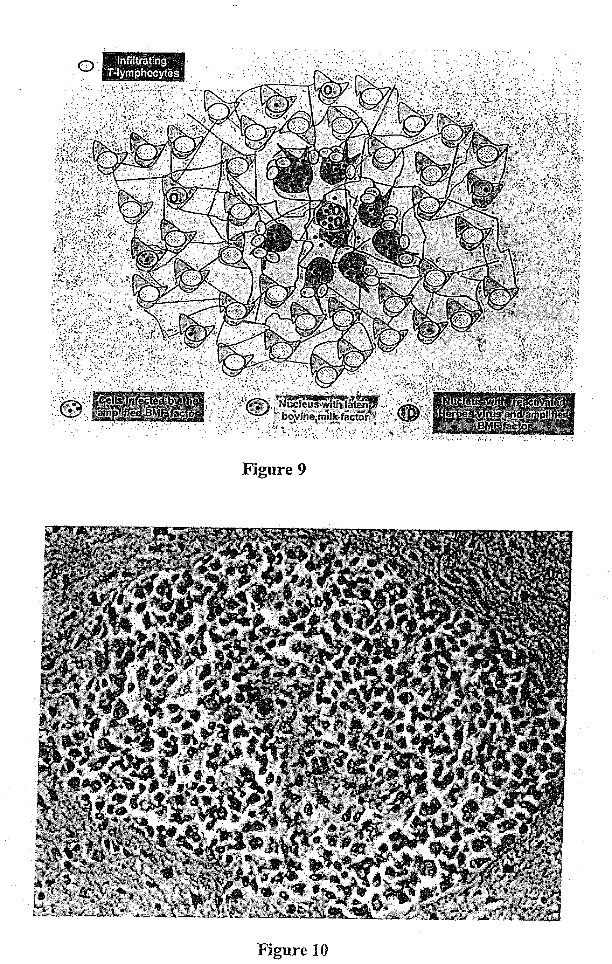

The presence of presumably circular single stranded DNA related to Sphinx-sequences, Anello-, Circo-, and Gemycircularvirus families, as well as *Psychrobacter* species in cattle sera (see Examples 2-5), in commercially available milk samples, as well as in florid MS lesions and MS serum permits the development of a concept for MS pathogenesis. It integrates observations of involvement of Herpes-type viruses, most prominently of EBV, of their property to amplify small double- and single-stranded DNA viruses, of viral cow milk factors, of vitamin-D deficiency, the EBV inducing property of TGFβ, and the partial season dependence of MS onset and of exacerbation in the course of disease. This concept is schematically outlined in Figures 7-13.

An initial dual latent infection of the same or closely flanking cells by a herpes virus genome and the postulated BMF, followed by a trigger for Herpes virus reactivation and the subsequent preferential amplification of single-stranded DNA are defined as the primary event. In the case of latent EBV infection, vitamin D deficiency with the subsequent up-regulation of TNF-β as an EBV-inducing factor could be the important trigger for up-regulation. Probably abortive infection of neighbouring cells with viral antigen expression results in an active T-cell response and the destruction of the affected cells. The frequently described seasonality of MS onset and of new rounds of MS exacerbations, the repeatedly reported North-South gradient of MS incidence should reflect the degree of sun-light exposure, negatively correlating vitamin D levels with TGFβ concentration and EBV reactivation.

Thus, the inventors anticipate the presence of different BMF sequences also in susceptible normal human brain cells in a latent form. The remarkable heterogeneity of the BMF isolates may also find its reflection in variations for pathologic characteristics of MS in humans (*Lucchinetti et al., 2000, Metz* et *al., 2014*). It would not be too surprising if eventually "high" and "low" risk types will be identified, in a certain analogy to human papillomavirus pathogenicity (*zur Hausen,* 1985). The majority of those carriers will not develop MS, since the latter should require latent co-infection of a BMF-positive cell with a Herpes-type virus and spontaneous induction of the latter. This should be a rare event, increasing, however, under conditions resulting in frequent Herpes virus reactivations.

As a final point, it should be of interest to also apply this concept to other presumably autoimmune diseases and certain cancers occurring at increased frequency under conditions of vitamin D deficiency. As far as cancers are concerned, this specifically accounts for colon- and breast cancer, and possibly for ovarian, prostate, pancreatic cancer and lung cancers.

The risk for insulin-dependent diabetes mellitus has been repeatedly linked to cow milk consumption and vitamin D deficiency (*reviewed in Scott, 1990, in Grant, 2006, in Hyppönen et al, 2010*). The latter system seems to come particularly close to the MS situation.

Accordingly, the present invention relates to a CMI polynucleic acid comprising:
(a) a nucleotide sequence depicted in Figure 1 (e) [SEQ ID. No: 46], Figure 1(f) [SEQ ID. No: 57], Figure 1(g) [SEQ ID. No.: 70] or Figure 1(h) [SEQ ID. No.: 90];(b) a nucleotide sequence having at least 99% identity to a nucleotide sequence of (a);(c) a nucleotide sequence being complementary to a nucleotide sequence of (a)or (b); or(d) a nucleotide sequence which is redundant as a result of the degeneracy of the genetic code compared to any of the above-given nucleotide sequences.

The term "polynucleic acid" refers to a single-stranded or double-stranded nucleic acid sequence. A polynucleic acid may consist of deoxyribonucleotides or ribonucleotides, nucleotide analogues or modified nucleotides or may have been adapted for diagnostic or therapeutic purposes. A polynucleic acid may also comprise a double stranded cDNA clone which can be used, for example, for cloning purposes.

The CMI polynucleic acids of the invention can be prepared according to well-known routine methods, for example, by (a) isolating the entire DNA or RNA from a sample, (b) detecting the CMI sequence by hybridization or PCR and (c) cloning of the CMI sequence into a vector.

Polynucleic acid sequences according to the present invention can be characterized and isolated according to any of the techniques known in the art, such as amplification by means of sequence-specific primers, hybridization with sequence-specific probes under more or less stringent conditions, sequence determination of the genetic information of HCBI, MSBI, MSSI or CMI etc.

The present invention also provides polynucleic acid sequences which are redundant as a result of the degeneracy of the genetic code compared to any of the above-given nucleotide sequences. These variant polynucleic acid sequences will thus encode the same amino acid sequence as the polynucleic acids they are derived from.

The CMI polynucleic acids of the invention might be present as an extrachromosomal episome, might be integrated into the host's genome and/or might be linked to a host cell DNA.

The present invention also relates to an oligonucleotide primer comprising or consisting of part of a polynucleic acid as defined above, with said primer being able to act as primer for specifically sequencing or specifically amplifying CMI polynucleic acid of the invention.

The term "primer" refers to a single stranded DNA oligonucleotide sequence capable of acting as a point of initiation for synthesis of a primer extension product which is complementary to the nucleic acid strand to be copied. The length and the sequence of the primer must be such that they allow priming the synthesis of the extension products. Preferably the primer is about 5-50 nucleotides. Specific length and sequence will depend on the complexity of the required DNA or RNA targets, as well as on the conditions of primer use such as temperature and ionic strength.

The fact that amplification primers do not have to match exactly with a corresponding template sequence to warrant proper amplification is amply documented in the Literature. The amplification method used can be, for example, polymerase chain reaction (PCR), ligase chain reaction (LCR), nucleic acid sequence-based amplification (NASBA), transcription-based amplification system (TAS), strand displacement amplification (SDA) or amplification by means of Qb replicase or any other suitable method to amplify nucleic acid molecules using primer extension. During amplification, the amplified products can be labelled either using labelled primers or by incorporating labelled nucleotides.

Labels may be isotopic (32P, 35S, etc.) or non-isotopic (biotin, digoxigenin, etc.). The amplification reaction is repeated between 20 and 70 times, advantageously between 25 and 45 times. Any of a variety of sequencing reactions known in the art can be used to directly sequence the viral genetic information and determine the ORF by translating the sequence of the sample into the corresponding amino acid sequence. Exemplary sequencing reactions include those based on techniques developed by Sanger or Maxam and Gilbert. It is also contemplated that a variety of automated sequencing procedures may be utilized when performing the subject assays including sequencing by mass spectrometry (see, for example: PCT publication WO 94/16101). It will be evident to one skilled in the art that, for example the occurrence of only two or three nucleic bases needs to be determined in the sequencing reaction.

Preferably, these primers are about 5 to 50 nucleotides long, more preferably from about 10 to 25 nucleotides. Most preferred are primers having a length of at least 13 bases.

The present invention also relates to an oligonucleotide probe comprising or consisting of part of a CMI polynucleic acid as defined above, with said probe being able to act as a hybridization probe for specific detection of a HCBI, MSBI or CMI polynucleic acid according to the invention.

The probe can be labelled or attached to a solid support. The term "probe" refers to single stranded sequence-specific oligonucleotides which have a sequence which is complementary to the target sequence of an HCBI, MSBI, MSSI or CMI polynucleic acid to be detected.

Preferably, these probes are about 5 to 50 nucleotides long, more preferably from about 10 to 25 nucleotides. Most preferred are probes having a length of at least 13 bases.

The term "solid support" can refer to any substrate to which an oligonucleotide probe can be coupled, provided that it retains its hybridization characteristics and provided that the background level of hybridization remains low. Usually the solid substrate will be a microtiter plate, a membrane (e.g. nylon or nitrocellulose) or a microsphere (bead). Prior to application to the membrane or fixation it may be convenient to modify the nucleic acid probe in order to facilitate fixation or improve the hybridization efficiency. Such modifications may encompass homopolymer tailing, coupling with different reactive groups such as aliphatic groups, NH₂ groups, SH groups, carboxylic groups, or coupling with biotin or haptens. The oligonucleotides according to the present invention, used as primers or probes may also contain or consist of nucleotide analogues such as phosphorothioates, alkylphosphoriates or peptide nucleic acids or may contain intercalating agents. These modifications will necessitate adaptions with respect to the conditions under which the oligonucleotide should be used to obtain the required specificity and sensitivity. However, the eventual results will be essentially the same as those obtained with the unmodified oligonucleotides.

The introduction of these modifications may be advantageous in order to positively influence characteristics such as hybridization kinetics, reversibility of the hybrid-formation, biological stability of the oligonucleotide molecules, etc.

The present invention also relates to a recombinant expression vector comprising a CMI polynucleic acid of the invention as defined above operably linked to prokaryotic, eukaryotic or viral transcription and translation control elements as well as host cells containing such vector.

The term "vector" may comprise a plasmid, a cosmid, an artificial chromosome, a phage, or a virus or a transgenic non-human animal. Particularly useful for vaccine development may be

CMI recombinant molecules, BCG or adenoviral vectors, as well as avipox recombinant viruses.

The term "recombinant expression" used within the context of the present invention refers to the fact that the polypeptides of the present invention are produced by recombinant expression methods be it in prokaryotes, or lower or higher eukaryotes as discussed in detail below.

The term "host cell" refers to cells which can be or have been, used as recipients for a recombinant vector or other transfer polynucleotide, and include the progeny of the original cell which has been transfected.

It is understood that the progeny of a single parental cell may not necessarily be completely identical in morphology or in genomic or total DNA complement as the original parent, due to natural, accidental, or deliberate mutation or recombination. The term "lower eukaryote" refers to host cells such as yeast, fungi and the like. Lower eukaryotes are generally (but not necessarily) unicellular. Preferred lower eukaryotes are yeasts, particularly species within Saccharomyces, Schizosaccharomyces, Kluiveromyces, Pichia (e. g. Pichia pastoris), Hansenula (e. g. Hansenula polymorph), Schwaniomyces, Schizosaccharomyces, Yarowia, Zygosaccharomyces and the like. Saccharomyces cerevisiae, S. carlsbergensis and K. lactis are the most commonly used yeast hosts, and are convenient fungal hosts.

The term "higher eukaryote" refers to host cells derived from higher animals, such as mammals, reptiles, insects, and the like. Presently preferred higher eukaryote host cells are derived from Chinese hamster (e. g. CHO), monkey (e. g. COS and Vero cells), baby hamster kidney (BHK), pig kidney (PK15), rabbit kidney 13 cells (RK13), the human osteosarcoma cell line 143 B, the human cell line HeLa and human hepatoma cell lines like Hep G2, the 293TT cell line (Buck et al., 2004) and insectcell lines (e.g. Spodoptera frugiperda). The host cells may be provided in suspension or flask cultures, tissue cultures, organ cultures and the like. Alternatively the host cells may also be transgenic non-human animals.

The term "prokaryotes" refers to hosts such as E. coli, Lactobacillus, Lactococcus, Salmonella, Streptococcus, Bacillus subtilis or Streptomyces. Also these hosts are contemplated within the present invention.

The segment of the CMI DNA encoding the desired sequence inserted into the vector sequence may be attached to a signal sequence. Said signal sequence may be that from a CMI source, but particularly preferred constructs according to the present invention contain signal sequences appearing in the CMI genome before the respective start points of the proteins.

Higher eukaryotes may be transformed with vectors, or may be infected with a recombinant virus, for example a recombinant vaccinia virus. Techniques and vectors for the insertion of foreign DNA into vaccinia virus are well known in the art, and utilize, for example homologous recombination. A wide variety of viral promoter sequences, possibly terminator sequences and poly(A)-addition sequences, possibly enhancer sequences and possibly amplification sequences, all required for the mammalian expression, are available in the art. Vaccinia is particularly preferred since vaccinia halts the expression of host cell proteins. For vaccination of humans the avipox and Ankara Modified Virus (AMV) are particularly useful vectors.

Also known are insect expression transfer vectors derived from baculovirus Autographa californica nuclear polyhedrosis virus (AcNPV), which is a helper-independent viral expression vector. Expression vectors derived from this system usually use the strong viral polyhedrin gene promoter to drive the expression of heterologous genes. Different vectors as well as methods for the introduction of heterologous DNA into the desired site of baculovirus are available to the person skilled in the art for baculovirus expression. Also different signals for posttranslational modification recognized by insect cells are known in the art.

The present invention also relates to a polypeptide having an amino acid sequence encoded by a CMI polynucleic acid as defined above, or a part or an analogue thereof being substantially similar and biologically equivalent. The term "polypeptide" refers to a polymer of amino acids and does not refer to a specific length of the product. Thus, peptides, oligopeptides, and proteins are included within the definition of polypeptide. This term also does not refer to or exclude post-expression modifications of the polypeptide, for example, glycosylations, acetylations, phosphorylations and the like. Included within the definition are, for example, polypeptides containing one or more analogues of an amino acid (including, for example, unnatural amino acids, peptide nucleic acid (PNA), etc.), polypeptides with substituted linkages, as well as other modifications known in the art, both naturally occurring and non-naturally occurring.

The polypeptides of the invention can be prepared by classical chemical synthesis. The synthesis can be carried out in homogeneous solution or in solid phase. The polypeptides according to this invention can also be prepared by means of recombinant DNA techniques. The present invention also relates to a method for production of a recombinant polypeptide as defined above, comprising: (a) transformation of an appropriate cellular host with a recombinant vector, in which a polynucleic acid or a part thereof as defined above has been inserted under the control of the appropriate regulatory elements, (b) culturing said transformed cellular host under conditions enabling the expression of said insert, and (c) harvesting said polypeptide.

The present invention also relates to an antibody raised upon immunization with at least one polypeptide as defined above, with said antibody being specifically reactive with any of said polypeptides, and with said antibody being preferably a monoclonal antibody. The term "antibody", preferably, relates to antibodies which consist essentially of pooled monoclonal antibodies with different epitopic specificities, as well as distinct monoclonal antibody preparations. Monoclonal antibodies are made from an antigen containing, e.g., a polypeptide encoded by a CMI polynucleic acid of the invention or a fragment thereof by methods well known to those skilled in the art. As used herein, the term "antibody" (Ab) or "monoclonal antibody" (Mab) is meant to include intact molecules as well as antibody fragments (such as, for example, Fab and F(ab')2 fragments) which are capable of specifically binding to protein. Fab and F(ab')2 fragments lack the Fc fragment of intact antibody, clear more rapidly from the circulation, and may have less non-specific tissue binding than an intact antibody. Thus, these fragments are preferred, as well as the products of a FAB or other immunoglobulin expression library. Moreover, antibodies useful for the purposes of the present invention include chimerical, single chain, and humanized antibodies.

Preferably, the antibody or antigen binding fragment thereof carries a detectable label. The antibody/fragment can be directly or indirectly detectably labeled, for example, with a radioisotope, a fluorescent compound, a bioluminescent compound, a chemiluminescent compound, a metal chelator or an enzyme. Those of ordinary skill in the art will know of other suitable labels for binding to the antibody, or will be able to ascertain such, using routine experimentation.

The present invention also relates to a method for the detection of CMI polynucleic acids according to the invention present in a biological sample, comprising: (a) optionally extracting sample polynucleic acid, (b) amplifying the polynucleic acid as described above with at least one primer as defined above, optionally a labelled primer, and (c) detecting the amplified polynucleic acids.

The term "polynucleic acid" can also be referred to as analyte strand and corresponds to a single- or double-stranded polynucleic acid molecule.

The term "labelled" refers to the use of labelled nucleic acids. This may include the use of labelled nucleotides incorporated during the polymerase step of the amplification or labelled primers, or by any other method known to the person skilled in the art.

The present invention also relates to a method for the detection of CMI polynucleic acids according to the invention present in a biological sample, comprising: (a) optionally extracting sample polynucleic acid, (b) hybridizing the polynucleic acid as described above with at least one probe as defined above, and (c) detecting the hybridized polynucleic acids.

The hybridization and washing conditions are to be understood as stringent and are generally known in the art. However, according to the hybridization solution (SSC, SSPE, etc.), these probes should be hybridized at their appropriate temperature in order to attain sufficient specificity.

According to the hybridization solution (SSC, SSPE, etc.), these probes should be stringently hybridized at their appropriate temperature in order to attain sufficient specificity. However, by slightly modifying the DNA probes, either by adding or deleting one or a few nucleotides at their extremities (either 3' or 5'), or substituting some non-essential nucleotides (i. e. nucleotides not essential to discriminate between types) by others (including modified nucleotides or inosine) these probes or variants thereof can be caused to hybridize specifically at the same hybridization conditions (i.e. the same temperature and the same hybridization solution). Also changing the amount (concentration) of probe used may be beneficial to obtain more specific hybridization results. It should be noted in this context, that probes of the same length, regardless of their GC content, will hybridize specifically at approximately the same temperature in TMACI solutions.

Suitable assay methods for purposes of the present invention to detect hybrids formed between the oligonucleotide probes and the HCBI, MSBI, MSSI or CMI polynucleic acid sequences in a sample may comprise any of the assay formats known in the art, such as the conventional dot-blot format, sandwich hybridization or reverse hybridization. For example, the detection can be accomplished using a dot blot format, the unlabelled amplified sample being bound to a membrane, the membrane being incorporated with at least one labelled probe under suitable hybridization and wash conditions, and the presence of bound probe being monitored.

An alternative and preferred method is a "reverse" dot-blot format, in which the amplified sequence contains a label. In this format, the unlabelled oligonucleotide probes are bound to a solid support and exposed to the labelled sample under appropriate stringent hybridization and subsequent washing conditions. It is to be understood that also any other assay method which relies on the formation of a hybrid between the polynucleic acids of the sample and the oligonucleotide probes according to the present invention may be used.

The present invention also relates to a method for detecting a polypeptide encoded by a CMI polynucleic acid of the present invention or an antibody against said polypeptide present in a biological sample, comprising: (a) contacting the biological sample for the presence of such polypeptide or antibody as defined above, and (b) detecting the immunological complex formed between said antibody and said polypeptide.

The immunoassay methods according to the present invention may utilize antigens from different domains of the new and unique polypeptide sequences of the present invention. It is within the scope of the invention to use for instance single or specific oligomeric antigens, dimeric antigens, as well as combinations of single or specific oligomeric antigens. The HCBI, MSBI, MSSI or CMI antigens of the present invention may be employed in virtually any assay format that employs a known antigen to detect antibodies or cell-mediated immune responses. Thus, the present invention also encompasses the detection of cell-mediated immune responses against HCBI, MSBI, MSSI or CMI antigens and the application of therapeutic interferences based on cell-mediated immune responses against HCBI, MSBI, MSSI or CMI antigens.

Of course, an assay format that denatures the CMI conformational epitope should be avoided or adapted. A common feature of all of these assays is that the antigen is contacted with the body component suspected of containing CMI antibodies under conditions that permit the antigen to bind to any such antibody present in the component. Such conditions will typically be physiologic temperature, pH and ionic strength using an excess of antigen. The incubation of the antigen with the specimen is followed by detection of immune complexes comprised of the antigen.

Design of the immunoassays is subject to a great deal of variation, and many formats are known in the art. Protocols may, for example, use solid supports, or immunoprecipitation. Most assays involve the use of labeled antibody or polypeptide; the labels may be, for example, enzymatic, fluorescent, chemiluminescent, radioactive, or dye molecules. Assays which amplify the signals from the immune complex are also known; examples of which are assays which utilize biotin and avidin or streptavidin, and enzyme-labeled and mediated immunoassays, such as ELISA assays.

The immunoassay may be in a heterogeneous or in a homogeneous format, and of a standard or competitive type. In a heterogeneous format, the polypeptide is typically bound to a solid matrix or support to facilitate separation of the sample from the polypeptide after incubation. Examples of solid supports that can be used are nitrocellulose (e. g., in membrane or microtiter well form), polyvinyl chloride (e. g., in sheets or microtiter wells), polystyrene latex (e. g., in beads or microtiter plates, polyvinylidine fluoride (known as Immunolon), diazotized paper, nylon membranes, activated beads, and Protein A beads. The solid support containing the antigenic polypeptides is typically washed after separating it from the test sample, and prior to detection of bound antibodies. Both standard and competitive formats are known in the art.

In a homogeneous format, the test sample is incubated with the combination of antigens in solution. For example, it may be under conditions that will precipitate any antigen-antibody complexes which are formed. Both standard and competitive formats for these assays are known in the art.

In a standard format, the amount of HCBI, MSBI, MSSI or CMI antibodies in the antibody-antigen complexes is directly monitored. This may be accomplished by determining whether (labelled) anti-xenogeneic (e. g. anti-human) antibodies which recognize an epitope on anti-CMI antibodies will bind due to complex formation. In a competitive format, the amount CMI antibodies in the sample is deduced by monitoring the competitive effect on the binding of a known amount of labelled antibody (or other competing ligand) in the complex.

Complexes formed comprising anti-CMI antibody (or in the case of competitive assays, the amount of competing antibody) are detected by any of a number of known techniques, depending on the format. For example, unlabeled HCBI, MSBI, MSSI or CMI antibodies in the complex may be detected using a conjugate of anti-xenogeneic Ig complexed with a label (e. g. an enzyme label).

In an immunoprecipitation or agglutination assay format the reaction between the CMI antigens and the antibody forms a network that precipitates from the solution or suspension and forms a visible layer or film of precipitate. If no anti-CMI antibody is present in the test specimen, no visible precipitate is formed.

There currently exist three specific types of particle agglutination (PA) assays. These assays are used for the detection of antibodies to various antigens when coated to a support. One type of this assay is the hemagglutination assay using red blood cells (RBCs) that are sensitized by passively adsorbing antigen (or antibody) to the RBC. The addition of specific antigen/antibodies present in the body component, if any, causes the RBCs coated with the purified antigen to agglutinate.

To eliminate potential non-specific reactions in the hemagglutination assay, two artificial carriers may be used instead of RBC in the PA. The most common of these are latex particles.

The solid phase selected can include polymeric or glass beads, nitrocellulose, microparticles, microwells of a reaction tray, test tubes and magnetic beads. The signal generating compound can include an enzyme, a luminescent compound, a chromogen, a radioactive element and a chemiluminescent compound. Examples of enzymes include alkaline phosphatase, horseradish peroxidase and beta-galactosidase. Examples of enhancer compounds include biotin, anti-biotin and avidin. Examples of enhancer compounds binding members include biotin, anti-biotin and avidin.

The above methods are useful for evaluating the risk of developing diseases like cancer or an autoimmune disease due to the deleterious effects of the presence of a subgenomic HCBI, MSBI, MSSI or CMI polynucleotide sequence by itself or linked to a particular host gene or gene fragment within the patient's cells and allow taking appropriate counter measures.

Thus, the present invention also relates to an antisense oligonucleotide or iRNA specific for the CMI virus polynucleic acid of the invention.

The generation of suitable antisense oligonucleotides or iRNAs includes determination of a site or sites within the CMI polynucleic acid for the antisense interaction to occur such that the desired effect, e.g., inhibition of expression of the polypeptide, will result. A preferred intragenic site is (a) the region encompassing the translation initiation or termination codon of the open reading frame (ORF) of the gene or (b) a region of the mRNA which is a "loop" or "bulge", i.e., not part of a secondary structure. Once one or more target sites have been identified, oligonucleotides are chosen which are sufficiently complementary to the target, i.e., hybridize sufficiently well and with sufficient specificity, to give the desired effect. In the context of this invention, "hybridization" means hydrogen bonding, which may be Watson-Crick, Hoogsteen or reversed Hoogsteen hydrogen bonding, between complementary nucleoside or nucleotide bases. "Complementary" as used herein, refers to the capacity for precise pairing between two nucleotides. For example, if a nucleotide at a certain position of an oligonucleotide is capable of hydrogen bonding with a nucleotide at the same position of a DNA or RNA molecule, then the oligonucleotide and the DNA or RNA are considered to be complementary to each other at that position. The oligonucleotide and the DNA or RNA are complementary to each other when a sufficient number of corresponding positions in each molecule are occupied by nucleotides which can hydrogen bond with each other. Thus, "specifically hybridizable" and "complementary" are terms which are used to indicate a sufficient degree of complementarity or precise pairing such that stable and specific binding occurs between the oligonucleotide and the DNA or RNA target. It is understood in the art that the sequence of an antisense compound does not need to be 100% complementary to that of its target nucleic acid to be specifically hybridizable. An antisense compound is specifically hybridizable when binding of the compound to the target DNA or RNA molecule interferes with the normal function of the target DNA or RNA to cause a loss of utility, and there is a sufficient degree of complementarity to avoid non-specific binding of the antisense compound to non-target sequences under conditions in which specific binding is desired, i.e., in the case of therapeutic treatment.

"Oligonucleotide" (in particular in the context of antisense compounds) refers to an oligomer or polymer of ribonucleic acid (RNA) or deoxyribonucleic acid (DNA) or mimetics thereof. This term includes oligonucleotides composed of naturally-occurring nucleobases, sugars and covalent internucleoside (backbone) linkages as well as oligonucleotides having non-naturally-occurring portions which function similarly. Such modified or substituted oligonucleotides are often preferred over native forms because of desirable properties such as, for example, enhanced cellular uptake, enhanced affinity for nucleic acid target and increased stability in the presence of nucleases. While antisense oligonucleotides are a preferred form of the antisense compound, the present invention comprehends other oligomeric antisense compounds, including but not limited to oligonucleotide mimetics such as are described below. The antisense compounds in accordance with this invention comprise from about 8 to about 50 nucleobases (i.e. from about 8 to about 50 linked nucleosides). Particularly preferred antisense compounds are antisense oligonucleotides, even more preferably those comprising from about 15 to about 25 nucleobases. Antisense compounds include ribozymes, external guide sequences (EGS), oligonucleotides (oligozymes), and other short catalytic RNAs or catalytic oligonucleotides which hybridize to the target nucleic acid and inhibit its expression. The antisense compounds also include an iRNA comprising a sense sequence and an antisense sequence, wherein the sense and antisense sequences form an RNA duplex and wherein the antisense sequence comprises a nucleotide sequence sufficiently complementary to the nucleotide sequence of an HCBI, MSBI, MSSI or CMI polynucleic acid of the present invention.

Alternatively, the invention provides a vector allowing to transcribe an antisense oligonucleotide of the invention, e.g., in a mammalian host. Preferably, such a vector is a vector useful for gene therapy. Preferred vectors useful for gene therapy are viral vectors, e.g. adenovirus, adeno-associated virus, herpes simplex virus, vaccinia, or, an RNA virus such as a retrovirus. Preferably, the retroviral vector is a derivative of a murine or avian retrovirus. Examples of such retroviral vectors which can be used in the present invention are: Moloney murine leukemia virus (MoMuLV), Harvey murine sarcoma virus (HaMuSV), murine mammary tumor virus (MuMTV) and Rous sarcoma virus (RSV). Most preferably, a non-human primate retroviral vector is employed, such as the gibbon ape leukemia virus (GaLV), providing a broader host range compared to murine vectors. Since recombinant retroviruses are defective, assistance is required in order to produce infectious particles. Such assistance can be provided, e.g., by using helper cell lines that contain plasmids encoding all of the structural genes of the retrovirus under the control of regulatory sequences within the LTR. Suitable helper cell lines are well known to those skilled in the art. Said vectors can additionally contain a gene encoding a selectable marker so that the transduced cells can be identified. Moreover, the retroviral vectors can be modified in such a way that they become target specific. This can be achieved, e.g., by inserting a polynucleotide encoding a sugar, a glycolipid, or a protein, preferably an antibody. Those skilled in the art know additional methods for generating target specific vectors. Further suitable vectors and methods for in vitro- or in vivo-gene therapy are described in the literature and are known to the persons skilled in the art; see, e.g., WO 94/29469 or WO 97/00957. The CMI polynucleotide sequences of the invention may also serve as a suitable vector itself, either composed solely of rearranged CMI sequences or of chimeric

CMI host cell DNA sequences. In addition, the nucleotide sequences of the invention may be used for the construction of artificial chromosomes.

In order to achieve expression only in the target organ, the DNA sequences for transcription of the antisense oligonucleotides can be linked to a tissue specific promoter and used for gene therapy. Such promoters are well known to those skilled in the art.

Within an oligonucleotide structure, the phosphate groups are commonly referred to as forming the internucleoside backbone of the oligonucleotide. The normal linkage or backbone of RNA and DNA is a 3' to 5' phosphodiester linkage. Specific examples of preferred antisense compounds useful in the present invention include oligonucleotides containing modified backbones or non-natural internucleoside linkages. Oligonucleotides having modified backbones include those that retain a phosphorus atom in the backbone and those that do not have a phosphorus atom in the backbone. Modified oligonucleotide backbones which can result in increased stability are known to the person skilled in the art, preferably such modification is a phosphorothioate linkage.

A preferred oligonucleotide mimetic is an oligonucleotide mimetic that has been shown to have excellent hybridization properties, and is referred to as a peptide nucleic acid (PNA). In PNA compounds, the sugar-backbone of an oligonucleotide is replaced with an amide containing backbone, in particular an aminoethylglycine backbone. The nucleobases are retained and are bound directly or indirectly to aza nitrogen atoms of the amide portion of the backbone.

Modified oligonucleotides may also contain one or more substituted or modified sugar moieties. Preferred oligonucleotides comprise one of the following at the 2' position: OH; F; 0-, S-, or N-alkyl; 0-, S-, or N-alkenyl; 0-, S- or N-alkynyl; or 0-alkyl-O-alkyl, wherein the alkyl, alkenyl and alkynyl may be substituted or unsubstituted C₁ to C₁₀ alkyl or C₂ to C₁₀ alkenyl and alkynyl. A particularly preferred modified sugar moiety is a 2'-O-methoxyethyl sugar moiety.

Antisense-oligonucleotides of the invention may also include nucleobase modifications or substitutions. Modified nucleobases include other synthetic and natural nucleobases such as 5-methylcytosine (5-me-C), 5-hydroxymethyl cytosine, xanthine, hypoxanthine, 2-aminoadenine, 6-methyl and other alkyl derivatives of adenine and guanine, 2-propyl and other alkyl derivatives of adenine and guanine, 2-thiouracil, 2-thiothymine and 2-thiocytosine etc., with 5-methylcytosine substitutions being preferred since these modifications have been shown to increase nucleic acid duplex stability.

Another modification of the oligonucleotides of the invention involves chemically linking to the oligonucleotide one or more moieties or conjugates which enhance the activity, cellular distribution or cellular uptake of the oligonucleotide. Such moieties include lipid moieties such as a cholesterol moiety, cholic acid, a thioether, a thiocholesterol, an aliphatic chain, e.g., dodecandiol or undecyl residues, a phospholipid, a polyamine or a polyethylene glycol chain, or adamantane acetic acid, a palmityl moiety, or an octadecylamine or hexylamino-carbonyl-oxycholesterol moiety.

The present invention also includes antisense compounds which are chimeric compounds. "Chimeric" antisense compounds or "chimeras," in the context of this invention, are antisense compounds, particularly oligonucleotides, which contain two or more chemically distinct regions, each made up of at least one monomer unit, i.e., a nucleotide in the case of an oligonucleotide compound. These oligonucleotides typically contain at least one region wherein the oligonucleotide is modified so as to confer upon the oligonucleotide increased resistance to nuclease degradation, increased cellular uptake, and/or increased binding affinity for the target nucleic acid. An additional region of the oligonucleotide may serve as a substrate for enzymes capable of cleaving RNA:DNA or RNA:RNA hybrids. By way of example, RNase H is a cellular endonuclease which cleaves the RNA strand of an RNA:DNA duplex. Activation of RNase H, therefore, results in cleavage of the RNA target, thereby greatly enhancing the efficiency of oligonucleotide inhibition of gene expression. Consequently, comparable results can often be obtained with shorter oligonucleotides when chimeric oligonucleotides are used, compared to phosphorothioate deoxyoligonucleotides hybridizing to the same target region. Chimeric antisense compounds of the invention may be formed as composite structures of two or more oligonucleotides, modified oligonucleotides, oligonucleosides and/or oligonucleotide mimetics as described above. Such compounds have also been referred to in the art as hybrids or gapmers.

The present invention also relates to a pharmaceutical composition comprising an antibody or antisense oligonucleotide of the invention and a suitable excipient, diluent or carrier. Preferably, in a pharmaceutical composition, such compound as described above is combined with a pharmaceutically acceptable carrier. "Pharmaceutically acceptable" is meant to encompass any carrier, which does not interfere with the effectiveness of the biological activity of the active ingredient and that is not toxic to the host to which it is administered. Examples of suitable pharmaceutical carriers are well known in the art and include phosphate buffered saline solutions, water, emulsions, such as oil/water emulsions, various types of wetting agents, sterile solutions etc. Such carriers can be formulated by conventional methods and the active compound can be administered to the subject at an effective dose.

An "effective dose" refers to an amount of the active ingredient that is sufficient to prevent the disease or to affect the course and the severity of the disease, leading to the reduction or remission of such pathology. An "effective dose" useful for treating and/or preventing these diseases or disorders may be determined using methods known to one skilled in the art.

Administration of the suitable compositions may be effected by different ways, e.g. by intravenous, intraperitoneal, oral, subcutaneous, intramuscular, topical or intradermal administration. The route of administration, of course, depends on the kind of therapy and the kind of compound contained in the pharmaceutical composition. The dosage regimen will be determined by the attending physician and other clinical factors. As is well known in the medical arts, dosages for any one patient depends on many factors, including the patient's size, body surface area, age, sex, the particular compound to be administered, time and route of administration, the kind of therapy, general health and other drugs being administered concurrently.

The present invention also relates to a vaccine for immunizing a mammal against CMI infection, comprising at least one polypeptide or CMI polynucleic acid as defined above or corresponding VLP (virus-like particle) or peptide/protein/DNA complexes, in a pharmaceutically acceptable carrier. It also involves molecular and immunological tests in animals (in particular cattle) and within their products (e.g. milk and dairy products).

It may also include small chemicals for targeted therapy derived from the analysis of structural components of these agents.

A "vaccine" is an immunogenic composition capable of eliciting protection against CMI, whether partial or complete. A vaccine may also be useful for treatment of an already infected individual, in which case it is called a therapeutic vaccine.

The term "therapeutic" refers to a composition capable of treating CMI infection or diseases linked to this infection. The term "effective amount" refers to an amount of epitope-bearing polypeptide sufficient to induce an immunogenic response in the individual to which it is administered, or to otherwise detectably immunoreact in its intended system (e. g., immunoassay). Preferably, the effective amount is sufficient to effect treatment, as defined above. The exact amount necessary will vary according to the application. For vaccine applications or for the generation of polyclonal antiserum/antibodies, for example, the effective amount may vary depending on the species, age, and general condition of the individual, the severity of the condition being treated, the particular polypeptide selected and its mode of administration, etc. Effective amounts will be found within a relatively large, non-critical range. An appropriate effective amount can be readily determined using routine experimentation. Preferred ranges of proteins for prophylaxis of HCBI, MSBI, MSSI or CMI caused diseases are 0.01 to 100 µg/dose, preferably 0.1 to 50 µg/dose. Several doses may be needed per individual in order to achieve a sufficient immune response and subsequent protection against an HCBI, MSBI, MSSI or CMI infection and an HCBI, MSBI or CMI linked disease, respectively.

Pharmaceutically acceptable carriers include any carrier that does not itself induce the production of antibodies harmful to the individual receiving the vaccine. Suitable carriers are typically large, slowly metabolized macromolecules such as proteins, polysaccharides, polylactic acids, polyglycolic acids, polymeric amino acids, and amino acid copolymers. Such carriers are well known to those of ordinary skill in the art.

Preferred adjuvants to enhance effectiveness of the composition include, but are not limited to: aluminium hydroxide (alum), N-acetyl-muramyl-L-threonyl-D-isoglutamine (thr-MDP) as found in U.S. Patent No. 4,606,918, N-acetyl-normuramyl-L-alanyl-D-isoglutamine (nor-MDP), N-acetylmuramyl-L-alanyl-D-isoglutaminyl-L-alanine-2-(1'-2'-dipalmitoyl sn-glycero-3-hydroxy-phosphoryloxy)-ethylamine (MTP-PE) and RIBI, which contains three components extracted from bacteria, monophosphoryl lipid A, trehalose dimycolate, and cell wall Skeleton (MPL +TDM + CWS) in a 2% squalene/Tween 80 emulsion. Any of the 3 components MPL, TDM or CWS may also be used alone or combined 2 by 2. Additionally, adjuvants such as Stimulon (Cambridge Bioscience, Worcester, MA) or SAF-1 (Syntex) may be used. Further, Complete Freund's Adjuvant (CFA) and Incomplete Freund's Adjuvant (IFA) may be used for non-human applications and research purposes.

The immunogenic compositions typically will contain pharmaceutically acceptable vehicles, such as water, saline, glycerol, ethanol, etc. Additionally, auxiliary substances, such as wetting or emulsifying agents, pH buffering substances, preservatives, and the like, may be included in such vehicles.

Typically, the immunogenic compositions are prepared as injectables, either as liquid solutions or suspensions. Solid forms suitable for solution in, or suspension in, liquid vehicles prior to injection may also be prepared. The preparation also may be emulsified or encapsulated in liposomes for enhanced adjuvant effect. The proteins may also be incorporated into Immune Stimulating Complexes together with saponins, for example Quil A (ISCOMS).

Immunogenic compositions used as vaccines comprise a "sufficient amount" or "an immunologically effective amount" of the proteins of the present invention, as well as any other of the above mentioned components, as needed. "Immunologically effective amount" means that the administration of that amount to an individual, either in a single dose or as part of a series, is effective for treatment, as defined above. This amount varies depending upon the health and physical condition of the individual to be treated, the capacity of the individual's immune system to synthesize antibodies, the degree of protection desired, the formulation of the vaccine, the treating doctor's assessment of the medical situation, and other relevant factors. It is expected that the amount will fall in a relatively broad range that can be determined through routine trials. Usually the amount will vary from 0.01 to 1000 µg/dose, more particularly from 0.1 -100 µg/dose.

### Example 1

### Material and Methods

### (A) Fractionation of bovine sera on density-sedimentation gradients with subsequent cloning

Initially, pools of 5 sera from a total of 120 bovine sera were subjected to Optiprep-(iodixanol)-density gradient ultracentrifugation after prior benzonase treatment to remove all free DNA and RNA (Buck et al., 2005). Protein-associated DNA was extracted from fractions (Qiagen PCR Purification Kit) and 1µ1 DNA/fraction subjected to RCA (rolling circle amplification) in a solution of 50µM Exo-resistant random primers (Thermo Scientific), 3.2µmol each dNTPs (Takara) and 10U phi29 polymerase (Biolabs). Restriction digested products (EcoR1 or BamH1) were separated by agarose gel electrophoresis, eluted and cloned into vector pUC19 prior to sequencing.

(B) Rolling circle amplification of DNA extracted from sera, cow milk or brain tissue: DNA was extracted by phenolchloroform from milk and post mortem brain tissue and sera from MS patients. DNA from all serum samples was extracted using the High Pure Viral Nucleic Acid Kit (Roche). RCA (rolling circle amplification) with random primers on DNA from protein-associated fractions, restriction digestion, cloning and sequencing of resulting fragments (refer above). Abutting primers used were designed either on the individual isolated DNA sequences, as well as on the replication genes of Sphinx1.76 or Sphinx2.36 and used in inverted PCR on RCA amplified DNA from single bovine sera and cow milk, as well as sera from multiple sclerosis patients and post mortem multiple sclerosis brain samples.

### Example 2

### Concept for the pathogenesis of multiple sclerosis: Isolation of circular DNA molecules (bovine agents) from bovine serum, cow milk and multiple sclerosis brain (Group 1)

The epidemiology of colon cancer suggested the involvement of an infectious factor present in red meat derived from cattle of European/Asian descent (zur Hausen, 2012; zur Hausen, 2015) and cow milk consumption has been suspected to play a role in multiple sclerosis. In attempts to isolate these putative factors, sera from 120 healthy 5-year old cows were obtained from the Veterinary Faculty of the University of Leipzig and analyzed for the presence of circular episomal DNA. Since the first isolates HCBI6.252 (Healthy Cattle Blood Isolate)(2522bp) and HCBI6.159 (1591bp) revealed a distant relationship to DNA related to sequences found in brain lesions of animals linked to prion-associated conditions (Manuelidis, 2011). The inventors concomitantly analysed 8 sera (from patients in relapse), 2 CSF and 1 PBMC from MS patients, as well as 12 biopsies from post mortem brain tissue for Sphinx-related sequences. Two circular DNA molecules related to Sphinx1.76(1758bp acc no. HQ444404) were isolated from one MS brain sample - MSBI1.176 (Multiple Sclerosis Brain Isolate) (1766bp) and MS2.176 (1766bp). Since there is an elevated MS risk after cow milk consumption, the inventors investigated commercially available pasteurized milk for the presence of related DNA. Indeed, they isolated episomal single-stranded DNA molecules from all 4 milk samples (CMI1.252 (Cow Milk Isolate), CMI2.214, CMI3.168 and CMI4.158) (HCBI6.252 and CMI1.252 are near identical). This was taken as an indication that milk excretion of these agents is indeed occurring.

The inventors used 2 primer pairs designed on Sphinx1.76 for inverted PCR on all human and bovine samples. These primers pairs were: forward 5'-GGATTAATGCCAATGATCC-3' (nt 721-739), reverse 5'-CGAGAGAAACAGGCAAAG-3'(nt703-720) and forward 5'-GAGGACGAATTAATATTACAAGTC-3' (nt868-891), reverse 5'-TTACCAAGAAAAGCGAGAAC-3' (nt848-867). The resulting sequences are all distantly similar (ranging from 79% - 98%) to the Sphinx1.76isolate. HCBI6.159 apparently evolved from a deletion in HCBI6.252 (deletion of nt 1129-2060 in HCBI6.252) as their overlapping sequences are identical. HCBI6.159 was isolated independently from HCBI6.252 and it is therefore highly unlikely that it is an artifact. MSBI1.176 is 98% identical to Sphinx1.76, but the nature (patterns) of the single sequence differences are such that these can be regarded as two separate agents. As the Sphinx1.76 construct was not available in the inventor's laboratory, it could not have resulted from laboratory contamination. The inventors isolated a second very distantly Sphinx1.76-related (but identical in size) circular DNA molecule MSBI2.176 from the same brain biopsy.

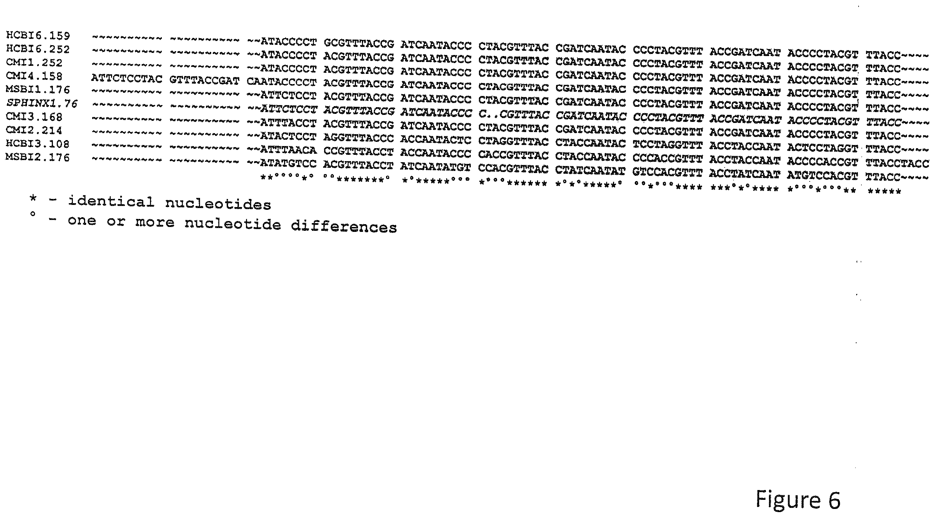

The large ORFs of the isolate of group 1 encode for replication protein (ProtSweep, del Val et al., 2007) sharing high similarity between them. Another common feature is the presence of iteron-like tandem repeats (3x22nt plus 17/18nt of the repeat in each isolate). Alignment of this repeat region indicates only single nucleotide variation in the core (Figure 6). These iteron-like repeats may constitute binding sites for Rep proteins (Chattoraj, 2000, Dziewit et al., 2013).

Nucleotide sequence accession number: The complete sequences of 8 isolates have been deposited in the EMBL Databank under the ace. no.:

| | |
|---|---|
| CMI1.2522 | Acc no. LK931487 |
| CMI2.214 | Acc no. LK931488 |
| CMI3.168 | Acc no. LK931489 |
| CMI4.158 | Acc no. LK931490 |
| MSBI1.17 | Acc no. LK931491 |
| MSBI2.176 | Acc no. LK931492 |
| HCBI6.252 | Acc no. LK931493 |
| HCBI6.159 | Acc no. LK931494 |

In this context, diseases of the CNS (e.g. Multiple sclerosis MS, amyotrophic lateral sclerosis, transmissible spongiforme encephalopathies/ Prion-linked diseases, Parkinson's disease, Alzheimer disease) are also highly interesting since the similar sequences described by Manuelidis are primarily found in the CNS.

### Example 3

### Isolation of a bovine factor from bovine serum (group 2)

DNA from bovine serum was extracted followed by rolling circle amplification of the DNA. PCR amplification using abutting primers (forward (nt2313-2336) 5'CTAATGCAGATCAACACAGGGATA-3' and reverse (nt2312-2291) 5'-GAATTACAGGCTTTGCAATCTG-3') designed on the replication gene of Sphinx2.36 (2364bp, acc no. HQ444405) (Manuelidis 2011) resulted in one circular DNA isolate HCBI7.228 (2280bp, acc no. LK931498) (group 2).

### Example 4

### Isolation of sequences from bovine sera and multiple sclerosis serum distantly related to a novel group of single-stranded DNA viruses, the myco-like Gemycircularviruses (Group 3)

A large number of circular ssDNA viruses have recently been identified by metagenomic analyses (Rosario et al., 2012a). The group of Gemycircularviruses has been isolated from fungi, faeces of various animals and plants leaves (Rosario et al., 2012b; Sikorski et al. 2013). The inventors report the isolation of the genomes of 3 novel viruses distantly related to this virus group.

Two sequences HCBI8.215 (2152bp) and HCBI9.2112 (2121bp) were isolated after density gradient fractionation of bovine serum pools. Fragments obtained after restriction digest (BamH1 and EcoR1) were cloned into pUC19. Their full-length genomes were verified by inverted PCR using abutting primers. One multiple sclerosis serum isolate MSSI2.225 rescued using abutting primers and inverted PCR was related to the myco-like Gemycircularviruses isolated from cattle sera (HCBI8.215 and HCBI9.212). The inventors subsequently isolated a sequence MSBI3.224 from a post-mortem-MS affected brain tissue which proved to be identical to MSSI2.225.

The genome organization of all 3 isolates revealed a putative spliced replication protein coded for on the negative strand and the coat protein (CP) on the positive strand. The CP was arginine rich and a similarity to the TTV ORF1 protein was indicated in a DomainSweep analysis (del Val et al., 2007). Putative rolling circle motifs I, II and III and a Walker B motif of each were identified as follows: HCBI8.215 (LLTYA, HLHAFVD, YAIKD; VFDDI), for HCBI9.212 (LLKMP, HYHIYLG, YVGKD; VFDDI) and for MSSI2.225 (LLTYP, HLHAFVD, YAIKD; IFDDF). The GRS motifs were AVFDVGGFHPNISITK, TAFDYFGAHGNIKSIR and RAFDVEGCHPNVSPSR respectively. The nona-nucleotide motif for both HCBI8.215 and MSSI2.225 is (TAATGTTAT) and for HCBI9.212 (TAATATTAT).

These 3 isolates probably constitute a novel group of viruses as their capsid proteins share similarities with the ORF1 of TT viruses from the family *Anelloviridae,* and not to that of the plant virus family *Geminiviridae* as in the Gemycircularviruses. The isolation of these 3 Gemycircularvirus-related genomes directly from animal and diseased human tissue links them to have some etiological relevance for diseases such as multiple sclerosis.

| | |
|---|---|
| HCBI8.215 | Acc no. LK931483 |
| HCBI9.212 | Acc no. LK931484 |
| MSSI2.225 | Acc no. LK931485 |

### Example 5

### Bacterial plasmid-related circular DNA from human serum (group 4)

*Psychrobacter* species are frequently present as food contaminants and have been isolated from human tissues including brain, cerebrospinal fluid and blood. It is considered as an opportunistic human pathogen (Caspar et al., 2013; Lloyd-Puryear et al., 1991). The inventors report the isolation of a circular DNA molecule, MSSI1.162, from serum taken from a multiple sclerosis patient during relapse.

Rolling circle amplification and restriction digest were performed on DNA extracted from sera from patients with multiple sclerosis. The resulting fragment was cloned into vector pUC19. The full-length genome was verified by inverted PCR amplification using primers designed on the sequence identified initially: forward primer 5'-GACTTCTGATTGATTGATGCCTG-3'and reverse 5'-CCTGTTGAATACCGCTTAAATACT-3'. All products were sequenced by primer walking. MSSI1.162 (multiple sclerosis serum isolate) (1627bp) (Acc no. LK931486) shares 66% nucleotide similarity by BlastN analysis to the pRWF102 plasmid of the *Psychrobacter* species PRwf-1. The putative protein (321 amino acids) encoded by the large ORF shows weak similarity to replication protein family of E. coli as analysed by ProtSweep (del Val et a., 2007).

This new isolate is only distantly related to known *Psychrobacter* species and their plasmids. It may therefore represent a yet unknown human pathogen.

### Summary:

The presence of presumably infectious agents and their nucleic acids in the serum of healthy cows should imply that the same particles are also present in red meat.

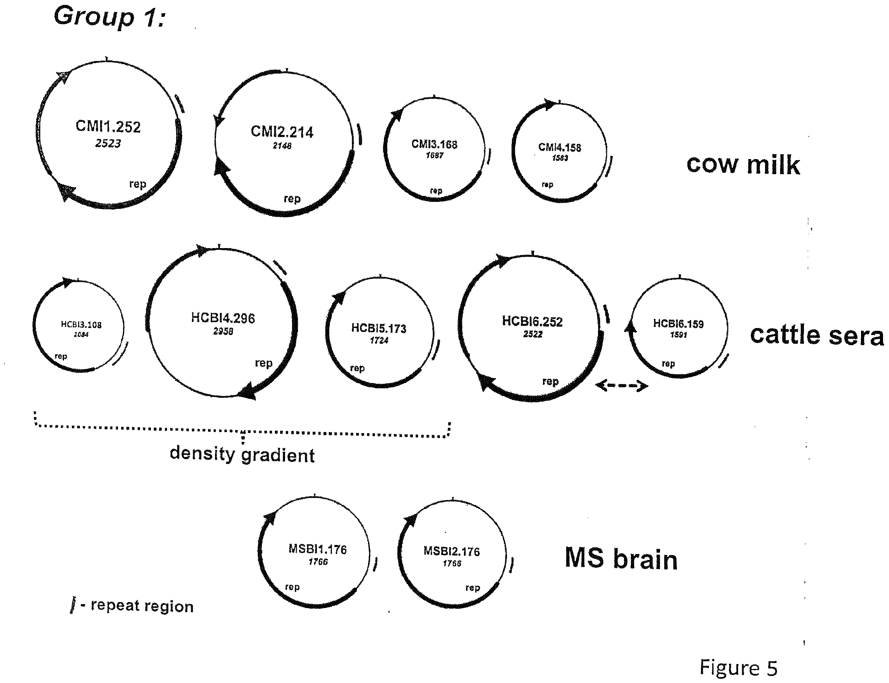

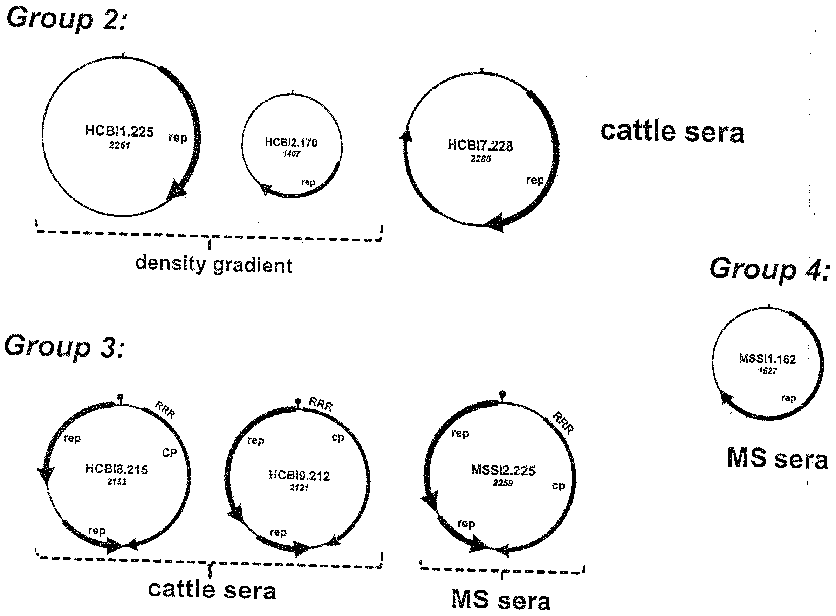

The inventors isolated 13 novel single-stranded DNA molecules from cattle serum and milk and MS brain tissue and sera. These isolates are grouped in 4 groups according to their sequence similarity to the Sphinxl.76 genome (group 1), Sphinx2.36 genome (group 2), myco-like Gemycircularviruses (group 3) and *Psychrobacter* spp. plasmid (group 4). Their genome organizations are presented in Figure 5 A, B. The main feature of all the sequences is the presence of a replication-associated protein encoding ORF.

All the isolates are presumably single-stranded DNA because of the bias of RCA towards amplification of single-stranded DNA *(del Solar et al., 1998*). A taxonomic classification of the isolates is, at this stage, not possible. Only group 3 can be defined as clearly related to the myco-like Gemycircularviruses. This relatively novel group of viruses have been identified by metagenomics and isolated mainly from faeces of a very large variety of animals and insects, as well as plants and fungi. They share some characteristics with the Geminiviruses of plants *(Sikorski et al., 2013*). Isolation from tissues of animals or humans has not yet been described. The putative replication-associated protein of all 3 isolates described in this application has an arginine-rich region with a similarity to human single-stranded TT viruses.

Infection of human cells by such agents should evoke a strong immune reaction, quite distinct from human TT viruses, where reasonable evidence for vertical transmission has been obtained *(reviewed in zur Hausen and de Villiers, 2014*). This could explain the high susceptibility to environmental factors for MS development during the first 15 years of life: primary infection may initially lead to rounds of replication and spreading of BMF probably via blood cells, eventually resulting in latent brain cell infection. This initial infection should induce an immune response, probably neutralizing the agent in subsequent rounds of infection prior to entry of the brain. The isolates reported here seem to represent excellent candidates for the postulated bovine milk factor (BMF).

A high variability in size was noted in group 1. The circular isolate HCBI6.159 seem to have evolved from HCBI6.252 through deletion of 931 nucleotides from the latter. The isolates all possess a replication gene and have an iteron-like repeat region in common (*Dziewit et al., 2013*). Alignment of this region between 8 isolates and Sphinx1.76 reveals a central identical core (Figure 6). Group 2 and 4 isolates do not have repeat regions.

The "Sphinx" sequences (*Manuelidis, 2011*) show high homologies to plasmid sequences of the bacterium Acinetobacter (*Vallenet* et *al., 2008; Longkumer et al., 2013*)*.* The sequences obtained in the present invention also exhibit striking homologies to the corresponding plasmid sequences. Although a large number of plasmids have been isolated and sequenced from Acinetobacter, thus far none of them corresponded exactly to the bovine and human sequences reported in this invention. Interestingly, a group of scientists in the UK published serological data over a period of years pointing to an increased selective formation of antibodies against Acinetobacter proteins but not against other bacterial antigenes obtained from patients suffering from multiple sclerosis (see review article: *Ebringer* et *al., 2012).* These results could not be confirmed by the group of Chapman (*Chapman et al., 2005).* However, it has to be stressed that the group of Chapman used a different strain of Acinetobacter (*Acinetobacter calcoaceticus).* Unequivocal results were obtained by the group of Ebringer for three strains of Acinetobacter *(Acinetobacter 1woffii*, *A. radioesistens* and a specific isolate, A. 11171). However, the results obtained for *A*. *junii* 17908 were less impressive and significant reactivity was hardly detectable (*Hughes et al., 2001*)*.* These results suggest that we are dealing with strain-specific reactivities wherein this sero-reactivity is due to strain-specific plasmids exhibiting homologies to the DNA sequences obtained in the present invention.

The isolate MSSI1.162 (group 4) has similarity to a plasmid of the *Psychrobacter* spp. *Pyschrobacter* species have been considered as an opportunistic human pathogen (*Caspar et al.,* 2013) and has been isolated from a case of meningitis (*Lloyd-Puryear et al., 1991*). These bacteria have repeatedly been reported as contaminants during and after cold-storage of meat *(de Filippis et al., 2013)* and were frequently isolated from milk and a variety of cheeses (*Coton* et *al., 2012).*

It is of interest to note that Manuelidis reported two "Sphinx-structures", labeled as "large" (2.36) and "small" (1.76) Sphinx. Although most of the present sequences substantially differed from her isolates, the inventors also obtained large and small Sphinx-like sequences from the same probes. Circular HCBI6.159 seems to have evolved from HCBI6.252 through a deletion of 931 nucleotides from the latter. It cannot be excluded that the other larger isolates may have smaller counterparts which were not isolated. It remains, however, to be determined whether the two structures found here persist within the same protein coat or complement each other.

The isolation of DNA of similar, in part even identical single-stranded circular nucleic acids from cattle sera, commercially available cow milk and florid MS tissues argues in favour of the concept outlined above.

### References

Buck CB, Pastrana DV, Lowy DR, Schiller JT. Efficient intracellular assembly of papillomaviral vectors. J. Virol. 2004; 78:751-757.

Chapman MD, Hughes LE, Wilson CD, Namnyak S, Thompson EJ, Giovannoni G. No evidence for production of intrathecal immunoglobulin G against Acinetobacter or Pseudomonas in multiple sclerosis. Eur Neurol. 2005; 53(1):27-31. de Villiers EM, Borkosky SS, Kimmel R, Gunst K, and Fei JW. (2011) The diversity of Torque teno viruses: In vitro replication leads to the formation of additional replication-competent subviral Molecules. J Virol 2011; 85(14):7284-7295 Ebringer A, Hughes L, Rashid T, Wilson C. Acinetobacter Immune Responses in Multiple Sclerosis: Etiopathogenetic Role and Its Possible Use as a Diagnostic Marker. Arch Neurol. 2005; 62:33-36.

Ebringer A, Rashid T, Wilson C. The role of Acinetobacter in the pathogenesis of multiple sclerosis examined by using Popper sequences. Med Hypotheses. 2012; 78(6):763-769.

Hughes, L.E., Bonell, S., Natt, R.S., Wilson, C., Tiwana, H., Ebringer, A., Cunningham, P., Chamoun, V., Thompson, E.J., Croker, J., and Vowles, J. Antibody responses to Acinetobacter spp. and Pseudomonas aeroginosa in multiple sclerosis: prospects for diagnosis using the myelin-Acinetobacter-neurofilament antibody index. Clin. Diagn. Laboratory Immunol. 2001; 8: 1181-1188.

Longkumer T, Kamireddy S, Muthyala VR, Akbarpasha S, Pitchika GK, Kodetham G, Ayaluru M, Siddavattam D. Scientific Reports 2013; 3:2240.

Manuelidis L. Nuclease resistant circular DNAs copurify with infectivity in scrapie and CJD. J. Neurovirol. 2011; 17:131-145.

Wold, MS. Replication protein A: heterotrimeric, single-stranded DNA-binding protein required for eukaryotic DNA metabolism. Ann. Review Biochem. 1997; 66:61-92

Vallenet D, Nordmann P, Barbe V, Poirel L, Mangenot S, Bataille E, Dossat C, Gas S, Kreimeyer A, Lenoble P, Oztas S, Poulain J, Segurens B, Robert C, Abergel C, Claverie J-M, Raoult D, Medigue C, Weissenbach J, Cruveiller S. Comparative analysis of Acinetobacters: three genomes for three lifestyles. PLoS One 2008; 3(3):e1805-e1805.

Xu B, Zhi N, Hu G, Wan Z, Zheng X, Liu X, Wong S, Kajigaya S, Zhao K, Mao Q, Young NS. Hybrid DNA virus in Chinese patients with seronegative hepatitis discovered by deep sequencing. Proc Natl Acad Sci U S A. 2013; 110: 10264-9.

zur Hausen H. Red meat consumption and cancer: Reasons to suspect involvement of bovine infectious factors in colorectal cancer. Int J Cancer 2012; 130:2475-2483.

zur Hausen H., de Villiers, E.-M., Prenatal Infections with Subsequent Immune Tolerance could explain the Epidemiology of Common Childhood Cancers, World Cancer Report, IARC, Lyon, 2014, pp. 261-265.

Abdel-Hag NM, Asmar BI. Human herpes virus 6 (HHV6) infection. Indian J Pediatr. 2004; 71: 89-96.

Agranoff BW, Goldberg D. Diet and the geographical distribution of multiple sclerosis. Lancet II 1974; 1061.

Alcalde-Cabero E, Almazán-Isla J, Garcia-Merino A, de Sá J, de Pedro-Cuesta J. Incidence of multiple sclerosis among European Economic Area populations, 1985-2009: the framework for monitoring. BMC Neurol. 2013; 13: 58-80.

Alenda R, Alvarez-Lafuente R, Costa-Frossard L, Arroyo R, Mirete S, Alvarez-Cermeño JC, Villar LM. Identification of the major HHV-6 antigen recognized by cerebrospinal fluid IgG in multiple sclerosis. Eur J Neurol. 2014 Apr 12. doi: 10.1111/ene.12435.

Almohmeed YH, Avenell A, Aucott L, Vickers MA. Systematic review and meta-analysis of the sero-epidemiological association between Epstein Barr virus and multiple sclerosis. PLoS One. 2013; 8: e61110.

Angelini DF, Serafini B, Piras E, Severa M, Coccia EM, Rosicarelli B, Ruggieri S, Gasperini C, Buttari F, Centonze D, Mechelli R, Salvetti M, Borsellino G, Aloisi F, Battistini L. Increased CD8+ T cell response to Epstein-Barr virus lytic antigens in the active phase of multiple sclerosis. PLoS Pathog. 2013; 9: e1003220.

Ascherio A, Munger KL, Lünemann JD. The initiation and prevention of multiple sclerosis. Nat Rev Neurol. 2012; 8: 602-12.

Ascherio A. Environmental factors in multiple sclerosis. Expert Rev. Neurother. 2013; 13 (12s) 3-9.

Ashtari F, Jamshidi F, Shoormasti RS, Pourpak Z, Akbari M. Cow's milk allergy in multiple sclerosis patients. J Res Med Sci. 2013; 18 (Suppl 1): S62-5.

Bager P, Nielsen NM, Bihrmann K, Frisch M, Wohlfart J, Koch-Henriksen N, Melbye M, Westergaard T Sibship characteristics and risk of multiple sclerosis: a nationwide cohort study in Denmark.. Am J Epidemiol. 2006; 163: 1112-7.

Bantel-Schaal U, zur Hausen H. Adeno-associated viruses inhibit SV40 DNA amplification and replication of herpes simplex virus in SV40-transformed hamster cells. Virology 1988; 164: 64-74.

Banwell B, Bar-Or A, Cheung R, Kennedy J, Krupp LB, Becker DJ, Dosch HM; Wadsworth Pediatric Multiple Sclerosis Study Group. Abnormal T-cell reactivities in childhood inflammatory demyelinating disease and type 1 diabetes. Ann Neurol. 2008; 63: 98-111.

Baranzini SE, Mudge J, van Velkinburgh JC, Khankhanian P, Khrebtukova I, Miller NA, Zhang L, Farmer AD, Bell CJ, Kim RW, May GD, Woodward JE, Caillier SJ, McElroy JP, Gomez R, Pando MJ, Clendenen LE, Ganusova EE, Schilkey FD, Ramaraj T, Khan OA, Huntley JJ, Luo S, Kwok PY, Wu TD, Schroth GP, Oksenberg JR, Hauser SL, Kingsmore SF. Genome, epigenome and RNA sequences of monozygotic twins discordant for multiple sclerosis. Nature. 2010; 464: 1351-6.

Bauer G, Höfler P, zur Hausen H. Epstein-Barr virus induction by a serum factor. I. Induction and cooperation with additional inducers. Virology. 1982; 121: 184-94.

Bauer G, Götschl M, Höfler P.Tumor-promoting activity of Epstein-Barr-virus-inducing factor transforming growth factor type beta (EIF/TGF-beta) is due to the induction of irreversible transformation. Int J Cancer. 1991; 47: 881-8.

Ben Fredj N, Rotola A, Nefzi F, Chebel S, Rizzo R, Caselli E, Frih-Ayed M, Di Luca D, Aouni M. Identification of human herpes viruses 1 to 8 in Tunisian multiple sclerosis patients and healthy blood donors. J Neurovirol. 2012; 18 :12-19.

Beretich BD, Beretich TM. Explaining multiple sclerosis by ultraviolet exposure: a geospatial analysis. Mult Scler 2009; 15: 891-8.

Birkeland SA, Storm HH, Lamm LU, Barlow L, Blohmé I, Forsberg B, Eklund B, Fjeldborg O, Friedberg M, Frödin L, et al. Cancer risk after renal transplantation in the Nordic countries, 1964-1986. Int J Cancer. 1995 Jan 17;60(2):183-9.

Borkosky SS, Whitley C, Kopp-Schneider A, zur Hausen H, de Villiers EM. Epstein-Barr virus stimulates torque teno virus replication: a possible relationship to multiple sclerosis. PLoS One. 2012; 7: e32160.

Brecht I, Weissbrich B, Braun J, Toyka KV, Weishaupt A, Buttmann M. Intrathecal, polyspecific antiviral immune response in oligoclonal band negative multiple sclerosis. PLoS One. 2012; 7: e40431.

Buchanan R, Bonthius DJ. Measles virus and associated central nervous system sequelae. Semin Pediatr Neurol. 2012; 19: 107-14.

Butcher PJ, The distribution of multiple sclerosis in relation to the dairy industry and milk consumption. N Z Med J. 1976; 83: 427-30.

Butcher PJ. Milk consumption and multiple sclerosis-an etiological hypothesis. Med Hypotheses. 1986; 19: 169-78.

Caserta MT, Mock DJ, Dewhurst S. Human herpes virus 6. Clin Infect Dis. 2001; 33: 829-33. Review.

Christensen JC. Multiple sclerosis: some epidemiological clues to etiology.

Acta Neurol Latinoam. 1975; 21: 66-85. Review.

Christensen T. Human herpes viruses in MS. Int MS J. 2007; 14: 41-7. Review.

Conradi S, Malzahn U, Paul F, Quill S, Harms L, Then Bergh F, Ditzenbach A, Georgi T, Heuschmann P, Rosche B. Breastfeeding is associated with lower risk for multiple sclerosis. Mult Scler. 2013; 19: 553-8.

Cusick MF, Libbey JE, Fujinami RS. Multiple sclerosis: autoimmunity and viruses: Curr. Opin. Rheumatol. 2013; 25: 496-501.

Décard BF, von Ahsen N, Grunwald T, Streit F, Stroet A, Niggemeier P, Schottstedt V, Riggert J, Gold R, Chan A. Low vitamin D and elevated immunoreactivity against Epstein-Barr virus before first clinical manifestation of multiple sclerosis. J Neurool Neurosurg Psychiatry. 2012; 83: 1170-3.

Dewhurst S. Human herpes virus type 6 and human herpes virus type 7 infections of the central nervous system. Herpes. 2004; 11 Suppl 2: 105A-111A. Review.

Disanto G, Morahan JM, Barnett MH, Giovannoni G, Ramagopalan SV. The evidence for a role of B cells in multiple sclerosis. Neurology 2012; 78: 823-32.

Engels EA, Biggar RJ, Hall HI, Cross H, Crutchfield A, Finch JL, Grigg R, Hylton T, Pawlish KS, McNeel TS, Goedert JJ. Cancer risk in people infected with human immunodeficiency virus in the United States. Int J Cancer. 2008; 123: 187-94.

Ferrò MT, Franciotta D, Prelle A, Bestetti A, Cinque P. Active intrathecal herpes simplex virus type 1 (HSV-1) and human herpes virus-6 (HHV-6) infection at onset of multiple sclerosis. J Neurovirol. 2012; 18: 437-4

Fotheringham J, Jacobson S. Human herpes virus 6 and multiple sclerosis: potential mechanisms for virus-induced disease. Herpes 2005; 12: 4-9.

Fraussen J, Vrolix K, Martinez-Martinez P, Losen M, De Baets MH, Stinissen P, Somers V. B cell characterization and reactivity analysis in multiple sclerosis. Autoimmun Rev. 2009; 8: 654-8.

Frolik CA, Dart LL, Meyers CA, Smith DM, Sporn MB. Purification and initial characterization of a type beta transforming growth factor from human placenta. Proc Natl Acad Sci U S A. 1983; 80: 3676-80.

Gaitán MI, Sati P, Inati SJ, Reich DS. Initial investigation of the blood-barrier in MS lesions at 7 tesla. Mult Scler. 2013; 19: 1068-73.

Geeraedts F, Wilczak N, van Binnendijk R, De Keyser J. Search for morbillivirus proteins in multiple sclerosis brain tissue. Neuroreport. 2004; 19: 15: 27-32.

Grant WB. Epidemiology of disease risks in relation to vitamin D insufficiency. Prog Biophys Mol Biol. 2006; 92: 65-79.

Grinde B. Herpes viruses: latency and reactivation - viral strategies and host response. J Oral Microbiol. 2013; doi: 10.3402/jom.v510.22766.

Guggenmos J, Schubart AS, Ogg S, Andersson M, Olsson T, Mather IH, Linington C. Antibody cross-reactivity between myelin oligodendrocyte glycoprotein and the milk protein butyrophilin in multiple sclerosis. J. Immunol. 2004; 172: 661-8.

Hawkes CH, Giovannoni G, Keir G, Cunnington M, Thompson EJ. Seroprevalence of herpes simplex virus type 2 in multiple sclerosis. Acta Neurol Scand. 2006; 114: 363-7.

Heilbronn R, Bürkle A, Stephan S, zur Hausen H. The adeno-associated virus rep gene suppresses herpes simplex virus-induced DNA amplification. J Virol. 1990a; 64: 3012-8.

Heilbronn R, Weller SK, zur Hausen H. Herpes simplex virus type 1 mutants for the origin-binding protein induce DNA amplification in the absence of viral replication. Virology. 1990b; 179: 478-81.

Heilbronn R, Albrecht I, Stephan S, Bürkle A, zur Hausen H. Human cytomegalovirus induces JC virus DNA replication in human fibroblasts. Proc Natl Acad Sci U S A. 1993; 90: 11406-10.

Henle W, Henle G. Evidence for an etiologic relation of the Epstein-Barr virus to human malignancies. Laryngoscope. 1977; 87: 467-73.

Hollis BW, Roos BA, Draper HH, Lambert PW. Vitamin D and its metabolites in human and bovine milk. J Nutr. 1981; 111: 1240-8. Holzmann C, Bauer I, Meyer P. Co-occurrence of multiple sclerosis and cancer in a BRCA1 positive family. Eur J Med Genet. 2013; 56: 577-9.

Hsiao FC, Tai AK, Deglon A, Sutkowski N, Longnecker R, Huber BT. EBV LMP-2A employs a novel mechanism to transactivate the HERV-K18 superantigen through its ITAM. Virology. 2009; 385: 261-6.

Hu Knox KK, Brewer JH, Henry JM, Harrington DJ, Carrigan DR. Human herpes virus 6 and multiple sclerosis: systemic active infections in patients with early disease. Clin Infect Dis. 2000; 31: 894-903.

Hyppönen E. Vitamin D and increasing incidence of type 1 diabetes-evidence for an association? Diabetes Obes Metab. 2010; 12: 737-43.

Isik S, Ozuguz U, Tutuncu YA, Erden G, Berker D, Acar K, Aydin Y, Akbaba G, Helvaci N, Guler S. Serum transforming growth factor-beta levels in patients with vitamin D deficiency. Eur J Intern Med. 2012; 23: 93-7.

Ito I, Waku T, Aoki M, Abe R, Nagai Y, Watanabe T, Nakajima Y, Ohkido I, Yokoyama K, Miyachi H, Shimizu T, Murayama A, Kishimoto H, Nagasawa K, Yanagisawa J. A nonclassical vitamin D receptor pathway suppresses renal fibrosis. Clin Invest. 2013; 123: 4579-94.

James E, Dobson R, Kuhle J, Baker D, Giovannoni G, Ramagopalan SV. The effect of vitamin D-related interventions on multiple sclerosis relapses: a meta-analysis. Mult Scler. 2013; 19: 1571-9.

Kakalacheva K, Münz C, Lünemann JD. Viral triggers of multiple sclerosis. Biochim Biophys Acta. 2011; 1812: 132-40.

Karner W, Bauer G. Activation of a varicella-zoster virus-specific IgA response during acute Epstein-Barr virus infection. J Med Virol. 1994; 44: 258-62.

Koch-Henriksen N, Sorensen PS. Why does the north-south gradient of incidence of multiple sclerosis seem to have disappeared on the northern hemisphere? J Neurol Sci. 2011; 311: 58-63.

Koch-Henriksen N, Stenager E, Laursen B. The use of epidemiological multiple sclerosis registers in research: the Danish MS Registry. Acta Neurol Scand Suppl. 2012; 195: 7-12.

Kotzamani D, Panou T, Mastorodemos V, Tzagournissakis M, Nikolakaki H, Spanaki C, Plaitakis A. Rising incidence of multiple sclerosis in females associated with urbanization. Neurology. 2012; 78: 1728-35.

Kurtzke JF. Multiple sclerosis in time and space-geographic clues to cause. J Neurovirol. 2000; 6 Suppl 2: S134-40.

Kurtzke JF. Epidemiology in multiple sclerosis: a pilgrim's progress. Brain. 2013; 136: 2904-17.

Kuusisto H, Hyöty H, Kares S, Kinnunen E, Elovaara I. Human herpes virus 6 and multiple sclerosis: a Finnish twin study. Mult Scler. 2008; 14: 54-8.

Latif N, Rana F, Guthrie T. Breast cancer and HIV in the era of highly active antiretroviral therapy: two case reports and review of the literature. Breast J. 2011; 17: 87-92.

Lebrun C, Debouverie M, Vermersch P, Clavelou P, Rumbach L, de Seze J, Wiertlevski S, Defer G, Gout O, Berthier F, Danzon A. Cancer risk and impact of disease-modifying treatments in patients with multiple sclerosis. Mult Scler. 2008; 14: 399-405.

Lemire JM, Adams JS, Sakai R, Jordan SC. 1 alpha,25-dihydroxyvitamin D3 suppresses proliferation and immunoglobulin production by normal human peripheral blood mononuclear cells. J Clin Invest 1984; 74: 657-61.

Libbey JE, Cusick MF, Fujinami RS. Role of pathogens in multiple sclerosis. Intern Rev Immunol. Early Online 1-18, 2013; DOI: 10.3/08830185.2013.823422

Liu H, Fu Y, Li B, Yu X, Xie J, Cheng J, Ghabrial SA, Li G, Yi X, Jiang D. Widespread horizontal gene transfer from circular single-stranded DNA viruses to eukaryotic genomes. BMC Evol Biol. 2011 Sep 26;11:276. doi: 10.1186/1471-2148-11-276.

Longkumer T, Kamireddy S, MuthyalaVR, Akbarpasha S, Pitchika GK, Kodetham G, Ayaluru M, Siddavattam D. Acinetobacter phage genome is similar to Sphinx 2.36, the circular DNA copurified with TSE infected particles. Sci Rep 2013; 3: 2240.doi: 10.1038/srep02240.

Lucchinetti C, Brück W, Parisi J, Scheithauer B, Rodriguez M, Lassmann H.Heterogeneity of multiple sclerosis lesions: implications for the pathogenesis of demyelination. Ann Neurol. 2000; 47: 707-17.

Magliozzi R, Serafini B, Rosicarelli B, Chiapetta G, Veroni C., Reynolds R, Aloisi F. B-cell enrichment and Epstein-Barr virus infection in inflammatory cortical lesions in secondary progressive multiple sclerosis. J Neuropathol Exp Neurol 2013; 72: 29-41.

Malosse D, Perron H. Correlation analysis between bovine populations, other farm animals, house pets, and multiple sclerosis prevalence. Neuroepidemiology. 1993; 12: 15-27.

Marrie RA, Wolfson C. Multiple sclerosis and varicella zoster virus infection: a review. Epidemiol Infect. 2001; 127: 315-25. Review.

Matz B, Schlehofer JR, zur Hausen H. Identification of a gene function of herpes simplex virus type 1 essential for amplification of simian virus 40 DNA sequences in transformed hamster cells. Virology. 1984; 134: 328-37.

Mesliniene S, Ramrattan L, Goldings S, Sheikh-Ali M. Role of vitamin D in the onset, progression and severity of multiple sclerosis. Endocr Pract. 2013; 19: 129-36.

Metz I, Weigand SD, Popescu BF, Frischer JM, Parisi JE, Guo Y, Lassmann H, Brück W, Lucchinetti CF. Pathologic heterogeneity persists in early active multiple sclerosis lesions. Ann Neurol. 2014 Apr 26. doi: 10.1002/ana.24163.

Midgard R, Glattre E, Grønning M, Riise T, Edland A, Nyland H. Multiple sclerosis and cancer in Norway. A retrospective cohort study. Acta Neurol Scand. 1996; 93: 411-5.

Mirandola P, Stefan A, Brambilla E, Campadelli-Fiume G, Grimaldi LM. Absence of human herpes virus 6 and 7 from spinal fluid and serum of multiple sclerosis patients. Neurology. 1999; 53: 1367-8.

Müller K, Heilmann C, Poulsen LK, Barington T, Bendtzen K. The role of monocytes and T cells in 1,25-dihydroxyvitamin D3 mediated inhibition of B cell function in vitro. Immunopharmacology 1991; 21: 121-8.

Munger KL, Chitnis, T, Frazier AL, Giovannucci E, Spiegelman D, Ascherio A. Dietary intake of vitamin D during adolescence and risk of multiple sclerosis. J Neurol. 2011a; 258: 479-85.

Munger KL Levin LI, O'Reilly EJ, Falk KI, Aschewrio A. Anti-Epstein-Barr virus antibodies as serological markers of multiple sclerosis: a prospective study among United States military personnel. Mult Scler 2011b; 17: 1185-93.

Murray TJ. An unusual occurrence of multiple sclerosis in a small rural community. Can J Neurol Sci. 1976; 3: 163-6.

Nicoll MP, ProençaJT, Efstathiou S. The molecular basis of herpes simplex virus latency. FEMS Microbiol Rev. 2012; 36: 684-705.

Nielsen NM, Rostgaard K, Rasmussen S, Koch-Henriksen N, Storm HH, Melbye M, Hjalgrim H. Cancer risk among patients with multiple sclerosis: a population-based register study. Int J Cancer. 2006; 118: 979-84.

Nora-Krukle Z, Chapenko S, Logina I, Millers A, Platkajis A, Murovska M. Human herpes virus 6 and 7 reactivation and disease activity in multiple sclerosis. Medicina (Kaunas). 2011; 47: 527-31.

Nordal HJ, Vandvik B, Norrby E. Multiple sclerosis: local synthesis of electrophoretically restricted measles, rubella, mumps and herpes simplex virus antibodies in the central nervous system. Scand J Immunol. 1978; 7: 473-9.

O'Gorman C, Lin R, Stankovich J, Broadley SA, Modelling genetic susceptibility to multiple sclerosis with family data. Neuroepidemiology. 2013; 40: 1-12.

Ohara Y. Multiple sclerosis and measles virus. Jpn J Infect Dis. 1999; 52: 198-200. Review.

Olival GS, Lima BM, Sumita LM, Serafim V, Fink MC, Nali LH, Romano CM, Thomaz RB, Cavenaghi VB, Tilbery CP, Penalva-de-Oliveira AC. Multiple sclerosis and herpes virus interaction. Arq Neuropsiquiatr. 2013; 71: 727-30.

Ongrádi J, Rajda C, Maródi CL, Csiszár A, Vecsei L. A pilot study on the antibodies to HHV-6 variants and HHV-7 in CSF of MS patients. J Neurovirol. 1999; 5: 529-32.

Opsahl ML, Kennedy PG Investigating the presence of human herpes virus 7 and 8 in multiple sclerosis and normal control brain tissue. J Neurol Sci. 2006; 240: 37-44.

Ordoñez G, Pineda B, Garcia-Navarrete R, Sotelo J. Brief presence of varicella-zoster vral DNA in mononuclear cells during relapses of multiple sclerosis. Arch Neurol. 2004; 61: 529-32.

Owens GP, Gilden D, Burgoon MP, Yu X, Bennett JL. Viruses and multiple sclerosis. Neuroscientist. 2011; 17: 659-76. Review.

Pakpoor J, Pakpoor J, Disanto G, Giovannoni G, Ramagopalan SV. Cytomegalovirus and multiple sclerosis risk. J Neurol. 2013; 260: 1658-60.

Petersen T, Møller-Larsen A, Ellermann-Eriksen S, Thiel S, Christensen T. Effects of interferon-beta therapy on elements in the antiviral immune response towards the human herpes viruses EBV, HSV, and VZV, and to the human endogenous retroviruses HERV-H and HERV-W in multiple sclerosis. J Neuroimmunol. 2012; 249: 105-8.

Pierrot-Deseilligny C, Souberbielle JC. Contribution of vitamin D insufficiency to the pathogenesis of multiple sclerosis. Ther Adv Neurol Disord. 2013; 6: 81-116.

Pisacane A, Impagliazzo N, Russo M, Valiani R, Mandarini A, Florio C, Vivo P. Breast feeding and multiple sclerosis. BMJ. 1994; 308: 1411-2.

Pohl D, Rostasy K, Jacobi C, Lange P, Nau R, Krone B, Hanefeld F. Intrathecal antibody production against Epstein-Barr and other neurotropic viruses in pediatric and adult onset multiple sclerosis. J Neurol. 2010; 257: 212-6.

Provvedini DM, Tsoukas CD, Deftos LJ, Manolagas SC. 1 alpha,25-dihydroxyvitamin D3-binding macromolecules in human B lymphocytes: effects on immunoglobulin production. J Immunol 1986; 136: 2734-40.

Rall GF. Measles virus 1998-2002: progress and controversy. Annu Rev Microbiol. 2003; 57: 343-67. Review.

Rima BK, Duprex WP. Molecular mechanisms of measles virus persistence. Virus Res. 2005; 111: 132-47. Review.

Rosecrans R, Dohnal JC. Seasonal vitamin D changes and the impact on health risk assessment. Clin Biochem, 2014; pii: S0009-9120. doi: 10.1016/j.clinbiochem.2014.02.004.

Ross RT. The varicella-zoster virus and multiple sclerosis. J Clin Epidemiol. 1998; 51: 533-5. Review.

Ruprecht K, Obojes K, Wengel V, Gronen F, Kim KS, Perron H, Schneider-Schaulies J, Rieckmann P. Regulation of human endogenous retrovirus W protein expression by herpes simplex virus type 1: implications for multiple sclerosis. J Neurovirol. 2006; 12: 65-71.

Salzer J, Nyström M, Hallmans G, Steenlund H, Wadell G, Sundström P. Epstein-Barr virus antibodies biobank samples. Mult Scler. 2013; 19: 1587-91.

Sanders V, Felisan S, Waddell A, Tourtellotte W. Detection of herpesviridae in postmortem multiple sclerosis brain tissue and controls by polymerase chain reaction. J Neurovirol. 1996; 2: 249-58.

Schlehofer JR, Gissmann L, Matz B, zur Hausen H. Herpes simplex virus-induced amplification of SV40 sequences in transformed Chinese hamster embryo cells. Int J Cancer. 1983; 32: 99-103.

Schlehofer JR, zur Hausen H. Adenovirus infection induces amplification of persistent viral DNA sequences (simian virus 40, hepatitis B virus, bovine papillomavirus) in human and rodent cells. Virus Res. 1990;17: 53-60.

Schmitt J, Schlehofer JR, Mergener K, Gissmann L, zur Hausen H. Amplification of bovine papillomavirus DNA by N-methyl-N'-nitro-N-nitrosoguanidine, ultraviolet irradiation, or infection with herpes simplex virus. Virology 1989; 172:73-81.

Scott FW. Cow milk and insulin-dependent diabetes mellitus: is there a relationship? Am J Clin Nutr. 1990; 51:489-491.

Sepcic J, Mesaros E, Materljan E, Sepic-Grahovac D. Nutritional factors and multiple sclerosis in Gorski Kotar, Croatia. Neuroepidemiology. 1993; 12: 234-40.

Simpson S Jr, Taylor B, Dwyer DE, Taylor J, Blizzard L, Ponsonby AL, Pittas F, Dwyer T, van der Mei I. Anti-HHV-6 IgG titer significantly predicts subsequent relapse risk in multiple sclerosis. Mult Scler. 2012; 18: 799-806.

Sotelo J, Corona T. Varicella zoster virus and relapsing remitting multiple sclerosis. Mult Scler Int. 2011; 2011: 214763.

Sun LM, Lin CL, Chung CJ, Liang JA, Sung FC, Kao CH. Increased breast cancer risk for patients with multiple sclerosis: a nationwide population-based cohort study. Eur J Neurol. 2013; Sep 19. doi: 10.1111/ene.12267.

Sundqvist E, Bergström T, Daialhosein H, Nyström M, Sundström P, Hillert J, Alfredsson L, Kockum I, Olsson T. Cytomegalovirus seropositivity is negatively associated with multiple sclerosis. Mult Scler. 2013 Sep 2. [Epub ahead of print].

Sundström P, Juto P, Wadell G, Hallmans G, Svenningsson A, Nyström L, Dillner J, Forsgren L. An altered immune response to Epstein-Barr virus in multiple sclerosis: a prospective study. Neurology. 2004; 62: 2277-82.

Sutkowski N, Chen G, Calderon G, Huber BT. Epstein-Barr virus latent membrane protein LMP-2A is sufficient for transactivation of the human endogenous retrovirus HERV-K18 superantigen. J Virol. 2004; 78: 7852-60.

Svejgaard A.The immunogenetics of multiple sclerosis. Immunogenetics. 2008; 60: 275-86.

Tai AK, Luka J, Ablashi D, Huber BT. HHV-6A infection induces expression of HERV-K18-encoded superantigen. J Clin Virol. 2009; 46: 47-8.

Tan IL, van Schijndel RA, Pouwell PJ, van Walderveen MA, Reichenbach JR, Manoliu RA, Barkhof F. MR venography of multiple sclerosis. AJNR Am Neuroradiol. 2000; 21: 1029-42.

Tarrats R, Ordoñez G, Rios C, Sotelo J. Varicella, ephemeral breastfeeding and eczema as risk factors for multiple sclerosis in Mexicans. Acta Neurol Scand. 2002; 105: 88-94..

Taus C, Pucci E, Cartechini E, Fié A, Giuliani G, Clementi M, Menzo S. Absence of HHV-6 and HHV-7 in cerebrospinal fluid in relapsing-remitting multiple sclerosis. Acta Neurol Scand. 2000; 101: 224-8.

Turcanova VL, Bundgaard B, Höllsberg P. Human herpes virus-6B induces expression of the human endogenous retrovirus K18-encoded superantigen. J Clin Virol. 2009; 46: 15-9.

Virtanen JO, Jacobson S. Viruses and multiple sclerosis. CNS Neurol Disord Drug Targets. 2012; 11: 528-44. Review.

Warren TR. The increased prevalence of multiple sclerosis among people who were born and bred in areas where goitre is endemic. Med Hypotheses. 1984; 14: 111-4.

Waubant E, Mowry EM, Krupp L, Chitnis T, Yeh EA, Kuntz N, Ness J, Belman A, Milazzo M, Gorman M, Weinstock-Guttman B, Rodriguez M, James JA. Antibody response to common viruses and human leukocyte antigen-DRB1 in pediatric multiple sclerosis. Mult Scler. 2013; 19: 891-5.

Wikström J. Studies on the clustering of multiple sclerosis in Finland.

Riv Patol Nerv Ment. 1976; 97: 199-204.

Winer S, Astsaturov I, Cheung RK, Schrade K, Gunaratnam L, Wood DD, Moscarello MA, O'Connor P, McKerlie C, Becker DJ, Dosch HM. T cells of multiple sclerosis patients target a common environmental peptide that causes encephalitis in mice. J Immunol. 2001; 166: 4751-6.

Wu H, Li T, Zeng M, Peng T. Herpes simplex virus type 1 infection activates the Epstein-Barr virus replicative cycle via a CREB-dependent mechanism. Cell Microbiol. 2012 ; 14: 546-59.

Yea C, Tellier R, Chong P, Westmacott G, Marrie RA, Bar-Or A, Banwell B; Canadian Pediatric Demyelinating Disease Network. Epstein-Barr virus in oral shedding of children with multiple sclerosis. Neurology. 2013; 81: 1392-9.

Zerr P, Vollath S, Palumbo-Zerr K, Tomcik M, Huang J, Distler A, Beyer C,Dees C, Gela K, Distler O, Schett G, Distler JH. Vitamin D receptor regulates TGF-β signalling in systemic sclerosis. Ann Rheum Dis. 2014 Jan 21. doi: 10.1136/annrheumdis-2013-204378.

zur Hausen H. Genital papillomavirus infections. Prog Med Virol. 1985; 32: 15-21.

zur Hausen, H. and de Villiers, E.M., Diary cattle serum and milk factors contributing to the risk of colon and breast cancers, Int. J. Cancer, 2015, Feb. 3 doi: 10.1002/ijc.29466

Zwart SR, Mehta SK, Ploutz-Snyder R, Bourbeau Y, Locke JP, Pierson DL, Smith SM. Response to vitamin D supplementation during Antarctic winter is related to BMI, and supplementation can mitigate Epstein-Barr virus reactivation. J Nutr. 2011; 141: 692-7.

### References (Examples 2-5)

Buck CB, Pastrana DV, Lowy DR, Schillier JT. 2005. Generation of HPV pseudovirions using transfection and their use in neutralization assays. Methods Mol Med 119: 445-462.

Caspar Y, Recule C, Pouzol P, Lafeuillade B, Mallaret M, Mairin M, Croize J. Psychrobacter arenosus bacteremia after blood transfusion, France. Emerging Infectious Diseases 2013; 19: 1118-1120.

Coton M, Delbes-Paus C, Irlinger F, Desmasures N, Le Fleche A, Stahl V, Montel MC, Coton E. Diversity and assessment of potential risk factors of Gram-negative isolates associated with French cheeses. Food Microbiol. 2012; 29:88-98.

De Filippis F, La Storia A, Villiani F, Ercolini D. Exploring the sources of bacterial spoilers in beefsteaks by culture-independent high-throughput sequencing. PLoS One 2013; 8:e70222.

del Solar G, Giraldo R, Ruiz-Echevarria MJ, Espinosa M, Diaz-Orejas R. Replication and control of circular bacterial plasmids. Microbiol Mol. Biol. Rev. 1998; 62:434-464.

de Villiers EM, Borkosky SS, Kimmel R, Gunst K, Fei JW. The diversity of torque teno viruses: in vitro replication leads to the formation of additional replication-competent subviral molecules. J Virol. 2011; 85: 7284-95.

de Villiers, EM, zur Hausen, H. Concept for the pathogenesis of multiple sclerosis (I): interaction of an amplifying virus and a helper-dependent bovine milk factor. (submitted)

Funk M, Gunst K, Lucansky V, Müller H, zur Hausen H, de Villiers E-M., Isolation of protein-associated circular DNA from healthy cattle serum, Genome Announcements, 2(4): e00846-14, 2014

Dziewit L, Cegielski A, Romaniuk K, Uhrynowski W, Szych A, Niesiobedzki P, Zmuda-Baranowska MJ, Zdanowski MK, Bartosik D. Plasmid diversity in arctic strains of Psychrobacter spp. Extremophiles. 2013; 17: 433-44

Ebringer A, Rashid T, Wilson C. Bovine spongiform encephalopathy, multiple sclerosis, and creutzfeldt-jakob disease are probably autoimmune diseases evoked by Acinetobacter bacteria. Ann N Y Acad Sci. 2005; 1050: 417-28.

Lloyd-Puryear M, Wallace D, Baldwin T, Hollis DG. Meningitis caused by Psychrobacter immobilis in an infant. J Clin. Microbiol. 1991; 29: 2041-2042.

Liu H, Fu Y, Li B, Yu X, Xie J, Cheng J, Ghabrial SA, Li G, Yi X, Jiang D. Widespread horizontal gene transfer from circular single-stranded DNA viruses to eukaryotic genomes. BMC Evol Biol. 2011; 11: 276. doi: 10.1186/1471-2148-11-276.

Longkumer T, Kamireddy S, MuthyalaVR, Akbarpasha S, Pitchika GK, Kodetham G, Ayaluru M, Siddavattam D. Acinetobacter phage genome is similar to Sphinx 2.36, the circular DNA copurified with TSE infected particles. Sci Rep 2013; 3: 2240.doi: 10.1038/srep02240.

Martin DP, Biagini P, Lefeuvre P, Golden M, Roumagnac P, Varsani A. Recombination in eukaryotic single stranded DNA viruses. Viruses. 2011; 3: 1699-738.

Rosario K, Duffy S, Breitbart M. 2012a. A field guide to eukaryotic circular single-stranded DNA viruses: insights gained from metagenomics. Arch. Virol. 157: 1851-1871.

Rosario K, Dayaram A, Marinov M, Ware J, Kraberger S, Stainton D, Breitbart M, Varsani A. 2012b. Diverse circular ssDNA viruses discovered in dragonflies (Odonata: Epiprocta). J Gen. Virol. 93:2668-2681.

Sikorski A, Massaro M, Kraberger S, Young LM, Smalley D, Martin DP, Varsani A. 2013. Novel myco-like DNA viruses discovered in the faecal matter of various animals. Virus Res. 177:209-216.

zur Hausen H, de Villiers EM. Prenatal Infections with Subsequent Immune Tolerance Could Explain the Epidemiology of Common Childhood Cancers. World Cancer Report 2014, IARC Lyon, pp 261-265.

de Villiers E-M, zur Hausen H. Concept for the pathogenesis of multiple sclerosis (I): Interaction of an amplifying virus and a helper-dependent bovine milk factor. (submitted).

del Val C, Ernst P, Falkenhahn M, Fladerer C, Glatting KH, Suhai S, Hotz-Wagenblatt A. 2007. ProtSweep, 2Dsweep and DomainSweep: protein analysis suite at DKFZ. Nucleic Acids Res. 35 (Web Server issue):W444-450.

Chattoraj DK. 2000. Control of plasmid DNA replication by iterons: no longer paradoxical. Mol. Microbiol. 37: 467-476.

zur Hausen H. 2001. Proliferation-inducing viruses in non-permissive systems as possible causes of human cancers. Lancet 357: 381-384.

Whitley C, Gunst K, Müller H, Funk M, zur Hausen H, de Villiers E-M., Novel replication-competent circular DNA molecules from healthy cattle serum, milk and multiple sclerosis-affected human brain tissue, Genome Announcements 2(4): e00849-14, 2014.

Lamberto I, Gunst K, Müller H, zur Hausen H, de Villiers E-M. Mycovirus-like DNA virus sequences from cattle serum, human brain and serum from multiple sclerosis patients, Genome Announcements 2(4): e00848-14, 2014

Gunst K, zur Hausen H, de Villiers E-M., Isolation of bacterial plasmid-related replication-associated circular DNA from serum sample of a multiple sclerosis patient, Genome announcement 2(4), e00847-14, 2014

## Claims

1. A Cow Milk Isolate (CMI) polynucleic acid comprising:
(a) a nucleotide sequence depicted in Figure 1 (e) [SEQ ID. No: 46], Figure 1(f) [SEQ ID. No: 57], Figure 1(g) [SEQ ID. No.: 70] or Figure 1(h) [SEQ ID. No.: 90];
(b) a nucleotide sequence having at least 99% identity to a nucleotide sequence of (a);
(c) a nucleotide sequence being complementary to a nucleotide sequence of (a)or (b); or
(d) a nucleotide sequence which is redundant as a result of the degeneracy of the genetic code compared to any of the above-given nucleotide sequences.

2. An expression vector comprising a CMI polynucleic acid of any one of claims 1 to 3 operably linked to prokaryotic, eukaryotic or viral transcription and translation control elements.

3. A host cell transformed or modified with an expression vector according to claim 2.

4. A polypeptide being encoded by a CMI polynucleic acid of claim 1.

5. An antibody or antigen binding fragment thereof specifically binding to a polypeptide of claim 4.

6. A method for the detection of a CMI polynucleic acid according to claim 1 in a biological sample, comprising: (a) optionally extracting sample polynucleic acid, (b) amplifying the polynucleic acid as described above with at least one oligonucleotide primer comprising part of a CMI polynucleic acid of claim 1, said primer being capable of acting as primer for specifically sequencing or specifically amplifying the nucleic acid of a certain CMI isolate containing a nucleotide sequence according to claim 1, optionally a labelled primer, and (c) detecting the amplified polynucleic acid.

7. A method for the detection of a CMI polynucleic acid according to claim 1 in a biological sample, comprising: (a) optionally extracting sample polynucleic acid, (b) hybridizing the polynucleic acid as described above with at least one oligonucleotide probe comprising part of a CMI polynucleic acid of claim 1, said probe being capable of acting as a hybridization probe for specific detection of the nucleic acid of a certain CMI isolate containing a nucleotide sequence of claim 1, optionally a labelled probe, and (c) detecting the hybridized polynucleic acid.

8. A method for detecting a polypeptide of claim 4 or an antibody of claim 5 present in a biological sample, comprising: (a) contacting the biological sample for the presence and/or concentration of such polypeptide or antibody as defined above, and (b) detecting the immunological complex formed between said antibody and/or said polypeptide.

9. An antisense oligonucleotide reducing or inhibiting the expression of a polynucleic acid of claim 1 or a vector containing said antisense oligonucleotide.

10. A pharmaceutical composition comprising the antibody or antigen binding fragment thereof of claim 5 or the antisense oligonucleotide of claim 9 and a suitable pharmaceutical carrier.

11. A vaccine comprising a CMI polynucleic acid of claim 1 or a polypeptide according to claim 4, preferably wherein the vaccine comprises a VLP or protein/DNA or polypeptide/DNA complex or specific proteins or attenuated infectious agents.

12. Use of a CMI polynucleic acid of claim 1 as a lead component for the development of a medicament for prevention or treatment of cancer, a disease of the CNS or diabetes, preferably wherein the cancer is breast cancer, ovarian cancer, lung cancer, prostate cancer, colorectal cancer or colon cancer and the disease of the CNS is Multiple sclerosis MS, amyotrophic lateral sclerosis, transmissible spongiforme encephalopathies/Prion-linked diseases, Parkinson's disease or Alzheimer disease.

## Patentansprüche

1. Kuhmilchisolat *(cow milk isolate;* CMI)-Polynukleinsäure umfassend:
(a) eine Nukleotidsequenz, die in Abbildung 1(e) [SEQ ID. No: 46], Abbildung 1(f) [SEQ ID. No: 57], Abbildung 1(g) [SEQ ID. No: 70] oder Abbildung 1(h) [SEQ ID. No: 90] dargestellt ist;
(b) eine Nukleotidsequenz mit einer mindestens 99%-igen Ähnlichkeit zu einer Nukleotidsequenz von (a);
(c) eine Nukleotidsequenz, die zu einer Nukleotidsequenz aus (a) oder (b) komplementär ist; oder
(c) eine Nukleotidsequenz, die bedingt durch die Degeneration des genetischen Codes im Vergleich zu einer der oben angegebenen Nukleotidsequenzen redundant ist.

2. Expressionsvektor umfassend eine CMI-Polynukleinsäure gemäß Anspruch 1, die mit prokaryotischen, eukaryotischen oder viralen Transkriptions- und Translationskontrollelementen funktionsfähig verbunden ist.

3. Wirtszelle, die mit einem Expressionsvektor gemäß Anspruch 2 transformiert oder modifiziert ist.

4. Polypeptid, das von einer CMI-Polynukleinsäure gemäß Anspruch 1 kodiert ist.

5. Antikörper oder Antigenbindefragment davon, das an ein Polypeptid gemäß Anspruch 4 spezifisch bindet.

6. Verfahren zur Detektion einer CMI-Polynukleinsäure gemäß Anspruch 1 in einer biologischen Probe, umfassend: (a) wahlweise Extrahieren der Polynukleinsäure der Probe, (b) Amplifizieren der Polynukleinsäure wie oben beschrieben mit mindestens einem Oligonukleotid-Primer, der einen Teil einer CMI-Polynukleinsäure gemäß Anspruch 1 umfasst, wobei der Primer, wahlweise ein markierter Primer, als Primer zum spezifischen Sequenzieren oder spezifischen Amplifizieren der Nukleinsäure eines gewissen CMI-Isolats, das eine Nukleotidsequenz gemäß Anspruch 1 enthält, wirken kann und (c) Detektieren der amplifizierten Polynukleinsäure.

7. Verfahren zur Detektion einer CMI-Polynukleinsäure gemäß Anspruch 1 in einer biologischen Probe, umfassend: (a) wahlweise Extrahieren der Polynukleinsäure der Probe, (b) Hybridisieren der Polynukleinsäure wie oben beschrieben mit mindestens einer Oligonukleotid-Sonde, die einen Teil einer CMI-Polynukleinsäure gemäß Anspruch 1 umfasst, wobei die Sonde, wahlweise eine markierte Sonde, als eine Hybridisierungssonde zur spezifischen Detektion der Nukleinsäure eines gewissen CMI-Isolats, das eine Nukleotidsequenz gemäß Anspruch 1 enthält, wirken kann und (c) Detektieren der hybridisierten Polynukleinsäure.

8. Verfahren zum Detektieren eines Polypeptids gemäß Anspruch 4 oder eines Antikörpers gemäß Anspruch 5, die in einer biologischen Probe vorhanden sind, umfassend: (a) Kontaktieren der biologischen Probe auf das Vorhandensein und/oder die Konzentration eines wie oben definierten Polypeptids oder Antikörpers und (b) Detektieren des immunologischen Komplexes, der sich zwischen dem Antikörper und/oder dem Polypeptid gebildet hat.

9. Antisense-Oligonukleotid, das die Expression einer Polynukleinsäure gemäß Anspruch 1 vermindert oder hemmt, oder ein Vektor, der das Antisense-Oligonukleotid enthält.

10. Pharmazeutische Zusammensetzung, umfassend den Antikörper oder das Antigenbindefragment davon gemäß Anspruch 5 oder das Antisense-Oligonukleotid gemäß Anspruch 9 und ein geeignetes pharmazeutisches Trägermolekül.

11. Impfstoff umfassend eine CMI-Polynukleinsäure gemäß Anspruch 1 oder ein Polypeptid gemäß Anspruch 4, wobei der Impfstoff vorzugsweise ein VLP oder ein Protein/DNA- oder Polypeptid/DNA-Komplex oder spezifische Proteine oder attenuierte Infektionserreger umfasst.

12. Verwendung einer CMI-Polynukleinsäure gemäß Anspruch 1 als eine Ausgangsverbindung zur Entwicklung eines Medikaments zur Vorbeugung oder Behandlung von Krebs, einer Erkrankung des ZNS oder von Diabetes, wobei der Krebs vorzugsweise Brustkrebs, Eierstockkrebs, Lungenkrebs, Prostatakrebs, ein kolorektales Karzinom oder Dickdarmkrebs ist, und die Erkrankung des ZNS Multiple Sklerose MS, amyotrophe Lateralsklerose, transmissible spongiforme Enzephalopathien/Erkrankungen, die mit Prionen im Zusammenhang stehen, Morbus Parkinson oder Morbus Alzheimer ist/sind.

## Revendications

1. Acide polynucléique d'isolat de lait de vache (CMI), comprenant :
(a) une séquence nucléotidique représentée sur la figure 1(e) [SEQ ID. n° : 46], la figure 1(f) [SEQ ID. n° : 57], la figure 1(g) [SEQ ID. n° : 70] ou la figure 1(h) [SEQ ID. n° : 90] ;
(b) une séquence nucléotidique ayant au moins 99 % d'identité par rapport à une séquence nucléotidique de (a) ;
(c) une séquence nucléotidique étant complémentaire d'une séquence nucléotidique de (a) ou (b) ; ou
(d) une séquence nucléotidique qui est redondante en raison de la dégénérescence du code génétique par rapport à l'une quelconque des séquences nucléotidiques ci-dessus.

2. Vecteur d'expression comprenant un acide polynucléique CMI selon l'une quelconque des revendications 1 à 3, lié de manière fonctionnelle à des éléments de commande de translation et de transcription procaryotes, eucaryotes ou viraux.

3. Cellule hôte transformée ou modifiée avec un vecteur d'expression selon la revendication 2.

4. Polypeptide étant codé par un acide polynucléique CMI selon la revendication 1.

5. Fragment de liaison d'anticorps ou d'antigène de celui-ci se liant spécifiquement à un polypeptide selon la revendication 4.

6. Procédé de détection d'un acide polynucléique CMI selon la revendication 1 dans un échantillon biologique, comprenant : (a) éventuellement l'extraction d'acide polynucléique de l'échantillon, (b) l'amplification de l'acide polynucléique tel que décrit ci-dessus avec au moins une amorce oligonucléotidique comprenant une partie d'un acide polynucléique CMI selon la revendication 1, ladite amorce étant capable d'agir comme amorce pour séquencer spécifiquement ou amplifier spécifiquement l'acide nucléique d'un certain isolat CMI contenant une séquence nucléotidique selon la revendication 1, éventuellement une amorce marquée, et (c) la détection de l'acide polynucléique amplifié.

7. Procédé de détection d'un acide polynucléique CMI selon la revendication 1 dans un échantillon biologique, comprenant : (a) éventuellement l'extraction d'un échantillon d'acide polynucléique, (b) l'hybridation de l'acide polynucléique tel que décrit ci-dessus avec au moins une sonde oligonucléotidique comprenant une partie d'un acide polynucléique CMI de la revendication 1, ladite sonde étant capable d'agir comme une sonde d'hybridation pour la détection spécifique de l'acide nucléique d'un certain isolat CMI contenant une séquence nucléotidique de la revendication 1, éventuellement une sonde marquée, et (c) la détection de l'acide polynucléique hybridé.

8. Procédé destiné à détecter un polypeptide de la revendication 4 ou un anticorps de la revendication 5 présent dans un échantillon biologique, comprenant : (a) la mise en contact de l'échantillon biologique pour la présence et/ou la concentration d'un tel polypeptide ou anticorps tel que défini ci-dessus, et (b) la détection du complexe immunologique formé entre ledit anticorps et/ou ledit polypeptide.

9. Oligonucléotide antisens réduisant ou inhibant l'expression d'un acide polynucléique selon la revendication 1 ou un vecteur contenant ledit oligonucléotide antisens.

10. Composition pharmaceutique comprenant l'anticorps ou le fragment de liaison d'antigène de celui-ci selon la revendication 5 ou l'oligonucléotide antisens selon la revendication 9 et un support pharmaceutique approprié.

11. Vaccin comprenant un acide polynucléique CMI selon la revendication 1 ou un polypeptide selon la revendication 4, de préférence dans lequel le vaccin comprend une VLP ou un complexe de protéine/ADN ou des protéines spécifiques ou des agents infectieux atténués.

12. Utilisation d'un acide polynucléique CMI selon la revendication 1 en tant que composant principal pour le développement d'un médicament pour la prévention ou le traitement du cancer, d'une maladie du SNC ou du diabète, de préférence dans laquelle le cancer est le cancer du sein, le cancer de l'ovaire, le cancer du poumon, le cancer de la prostate, le cancer colorectal ou le cancer du côlon et la maladie du SNC est la sclérose en plaques SP, la sclérose latérale amyotrophique, les encéphalopathies spongiformes transmissibles/maladies à prions, la maladie de Parkinson ou la maladie d'Alzheimer.
